# EUROPEAN PATENT SPECIFICATION

(11) **EP 3 162 797 B1**
(45) Date of publication and mention of the grant of the patent: **01.09.2021**
(21) Application number: 15800703.9
(22) Date of filing: 29.06.2015
(51) Int. Cl.: C07D 217/02, C07D 401/10, C07D 401/14, C09K 11/06, H01L 51/50

(54) **POLYCYCLIC COMPOUND AND ORGANIC LIGHT EMITTING DEVICE USING SAME**
POLYCYCLISCHE VERBINDUNG UND ORGANISCHE LICHTEMITTIERENDE VORRICHTUNG DAMIT
COMPOSÉ POLYCYCLIQUE ET DISPOSITIF ÉLECTROLUMINESCENT ORGANIQUE UTILISANT CE COMPOSÉ

(30) Priority: 27.06.2014 KR 20140080248; 23.09.2014 KR 20140127226; 25.03.2015 KR 20150041326
(43) Date of publication of application: 03.05.2017
(73) Proprietor: LT Materials Co., Ltd., Yongin-si, Gyeonggi-do (KR)
(72) Inventor: JANG, So-Hyun, Yongin-City Gyeonggi-do 17118 (KR); KIM, Kee-Yong, Suwon-city Gyeonggi-do 441-470 (KR); CHOI, Jin-Seok, Namsa-Myeon Cheoin-Gu Yongin-City Gyeonggi-do 17118 (KR); CHOI, Dae-Hyuk, Yongin-city Gyeonggi-do 446-987 (KR); EUM, Sung-Jin, Yongin-city Gyeonggi-do 448-150 (KR); LEE, Joo-Dong, Seongnam-city Gyeonggi-do 463-763 (KR)
(74) Representative: Goddar, Heinz J.
(86) International application number: PCT/KR2015/006626
(87) International publication number: WO 2015/199512

(56) References cited:
- EP-A1- 2 213 639
- CN-A- 102 010 411
- JP-A- 2010 270 245
- KR-A- 20100 101 315
- KR-A- 20130 135 178
- KR-A- 20130 135 178
- XIAOYANG SUN ET AL: "Visible-Light-Mediated Fluoroalkylation of Isocyanides with Ethyl Bromofluoroacetates: Unified Synthesis of Mono- and Difluoromethylated Phenanthridine Derivatives", ORGANIC LETTERS , 14(23), 6012-6015 CODEN: ORLEF7; ISSN: 1523-7052, vol. 16, no. 11, 21 May 2014 (2014-05-21), pages 2938-2941, XP055436064, ISSN: 1523-7060, DOI: 10.1021/ol501081h
- JIANG H ET AL: "Synthesis of 6-alkylated phenanthridine derivatives using photoredox neutral somophilic isocyanide insertion", ANGEWANDTE CHEMIE INTERNATIONAL EDITION, VERLAG CHEMIE, vol. 52, no. 50, 9 December 2013 (2013-12-09), pages 13289-13292, XP002760356, ISSN: 1433-7851, DOI: 10.1002/ANIE.201308376 [retrieved on 2013-11-12]
- BO ZHANG ET AL: "6-Trifluoromethyl-phenanthridines through radical trifluoromethylation of isonitriles", ANGEWANDTE CHEMIE INTERNATIONAL EDITION, VERLAG CHEMIE, vol. 52, no. 41, 4 October 2013 (2013-10-04), pages 10792-10795, XP002731186, ISSN: 1433-7851, DOI: 10.1002/ANIE.201306082 [retrieved on 2013-09-02]
- MANDADAPU ANIL K ET AL: "Synthesis of 8-aryl substituted benzo[a]phenanthridine derivatives by consecutive three component tandem reaction and 6-endocarbocyclization", TETRAHEDRON, vol. 68, no. 39, 2012, - 2012, pages 8207-8215, XP028934722, ISSN: 0040-4020, DOI: 10.1016/J.TET.2012.07.067
- MAMORU TOBISU ET AL: "Modular Synthesis of Phenanthridine Derivatives by Oxidative Cyclization of 2-Isocyanobiphenyls with Organoboron Reagents", ANGEWANDTE CHEMIE INTERNATIONAL EDITION, vol. 51, no. 45, 10 October 2012 (2012-10-10), pages 11363-11366, XP055435669, ISSN: 1433-7851, DOI: 10.1002/anie.201206115
- BO ZHANG ET AL: "6-Phosphorylated Phenanthridines from 2-Isocyanobiphenyls via Radical C-P and C-C Bond Formation", ORGANIC LETTERS , 14(23), 6012-6015 CODEN: ORLEF7; ISSN: 1523-7052, vol. 16, no. 1, 9 December 2013 (2013-12-09), pages 250-253, XP055435708, ISSN: 1523-7060, DOI: 10.1021/ol403256e
- GLINKA: "OXIDATIONAL PHOTOCYCLIZATION OF 2-SUBSTITUTED 3,4-DIPHENYLQUINOLINES TO 9-SUBSTITUTED DIBENZO[i,k]PHENANTHRIDINES", POLISH JOURNAL OF CHEMISTRY, POLSKIE TOWARZYSTWO CHEMICZNE, POLISH CHEMICAL SOCIETY, PL, vol. 52, 1 January 1978 (1978-01-01), pages 1039-1043, XP009502416, ISSN: 0137-5083
- GLINKA ET AL: "Oxidative photocyclization of 2-benzyl-3,4-diphenylquinoline to 9-benzyldibenzo[i,k]phenanthridine", ZESZYTY NAUKOWE POLITECHNIKI SLASKIEJ, ZESZYTY NAUKOWE POLITECHNIKI SLASKIEJ, CHEMIA ( 1979 ), VOLUME DATE 1978, (87), 75-9, ZESZYTY NAUKOWE POLITECHNIKI SLASKIEJ, CHEMIA ( 1979 ), VOLUMEDATE 1978, (87), 75-9, vol. 1978, no. 87, 1 January 1978 (1978-01-01), pages 75-79, XP009502449, ISSN: 0434-0787
- FÁTIMA CHURRUCA ET AL: "Direct, Two-Step Synthetic Pathway to Novel Dibenzo[ a,c ]phenanthridines", THE JOURNAL OF ORGANIC CHEMISTRY, vol. 70, no. 8, 1 April 2005 (2005-04-01), pages 3178-3187, XP055248559, US ISSN: 0022-3263, DOI: 10.1021/jo0501036
- CHURRUCA, FATIMA ET AL.: 'Direct, two-step synthetic pathway to novel dibenzo[a,c]phenanthridines' J. ORG. CHEM. vol. 70, 2005, pages 3178 - 3187, XP055248559
- None

## Description

### TECHNICAL FIELD

The present invention relates to a novel polycyclic compound and an organic light emitting device using the same.

### BACKGROUND ART

An electroluminescent device is a self-luminous display device, and has advantages in that the device has a wide viewing angle, an excellent contrast, and quick response time.

An organic light emitting device has a structure in which an organic thin film is arranged between two electrodes. When voltage is applied to an organic light emitting device having such a structure, light is emitted by electrons and holes injected from the two electrodes being dissipated after the electrons and holes are bonded and make a pair in the organic thin film. The organic thin film may be formed as monolayer or a multilayer as necessary.

Materials of the organic thin film may have a light emitting function as necessary. For example, as a material of the organic thin film, compounds capable of forming a light emitting layer alone may be used, or compounds capable of performing as a host or a dopant of a host-dopant-based light emitting layer may also be used. In addition to these, compounds capable of performing hole injection, hole transport, electron blocking, hole blocking, electron transport, or electron injectionmay also be used as a material of the organic thin film.

There have been continuous demands for the development of organic thin film materials in order to improve the performance, life span, or efficiency of an organic light emitting device.

Organic Letters, 14(23), 6012-6015; Angewandte Chemie International Edition, Vol. 52, No. 50, 13289-13292; Angewandte Chemie International Edition, Vol. 52, No. 41, 10792-10795; Tetrahedron, Vol. 68, No. 39, 8207-8215; Angewandte Chemie International Edition, Vol. 51, No. 45, 11363-11366; Polish Journal of Chemistry, Vol. 52, 1039-1043; ZESZYTY NAUKOWE POLITECHNIKI SLASKIEJ, CHEMIA (1979), Vol. date 1978 (87), 75-9, Vol. 1978, No. 87, 75-79; EP 2 213 639 A1; Journal of Organic Chemistry, Vol. 70, No. 8, 3178-3187; CN 102010411 A; and KR 20130135178 A disclose polycyclic compounds.

### SUMMARY OF THE INVENTION

The present invention has been made in an effort to provide a novel polycyclic compound and an organic light emitting device using the same.

The present invention provides a compound according to claim 1 and an organic light emitting device according to claim 13. Further embodiments are disclosed in the dependent claims.

According to the present invention, a compound described in the present specification may be used as a material of an organic material layer of an organic light emitting device. The compound may be used as a hole injection material, a hole transport material, a light emitting material, a hole blocking material, an electron transport material, or an electron injection material, in an organic light emitting device. In particular, the compound of the invention may be used as a material of one or more of a light emitting layer, an electron transport layer, and a hole blocking layer in an organic light emitting device.

### BRIEF DESCRIPTION OF THE DRAWINGS

FIGS. 1 to 3 illustrate examples of laminating order of electrodes and organic material layers of an organic light emitting device according to exemplary embodiments of the present invention.
FIG. 4 is a graph illustrating a PL spectrum of a compound 16 at a wavelength of 302 nm.
FIG. 5 is a graph illustrating an LTPL spectrum of the compound 16 at a wavelength of 382 nm.
FIG. 6 is a graph illustrating a PL spectrum of a compound 18 at a wavelength of 286 nm.
FIG. 7 is a graph illustrating an LTPL spectrum of the compound 18 at a wavelength of 383 nm.
FIG. 8 is a graph illustrating a PL spectrum of a compound 22 at a wavelength of 258 nm.
FIG. 9 is a graph illustrating an LTPL spectrum of the compound 22 at a wavelength of 283 nm.
FIG. 10 is a graph illustrating a PL spectrum of a compound 37 at a wavelength of 261 nm.
FIG. 11 is a graph illustrating an LTPL spectrum of the compound 37 at a wavelength of 308 nm.
FIG. 12 is a graph illustrating a PL spectrum of a compound 41 at a wavelength of 261 nm.
FIG. 13 is a graph illustrating an LTPL spectrum of the compound 41 at a wavelength of 308 nm.
FIG. 14 is a graph illustrating a PL spectrum of a compound 43 at a wavelength of 262 nm.
FIG. 15 is a graph illustrating an LTPL spectrum of the compound 43 at a wavelength of 311 nm.
FIG. 16 is a graph illustrating a PL spectrum of a compound 44 at a wavelength of 270 nm.
FIG. 17 is a graph illustrating an LTPL spectrum of the compound 44 at a wavelength of 311 nm.
FIG. 18 is a graph illustrating a PL spectrum of a compound 73 at a wavelength of 284 nm.
FIG. 19 is a graph illustrating an LTPL spectrum of the compound 73 at a wavelength of 383 nm.
FIG. 20 is a graph illustrating a PL spectrum of a compound 92 at a wavelength of 268 nm.
FIG. 21 is a graph illustrating an LTPL spectrum of the compound 92 at a wavelength of 383 nm.
FIG. 22 is a graph illustrating a PL spectrum of a compound 294 at a wavelength of 265 nm.
FIG. 23 is a graph illustrating an LTPL spectrum of the compound 294 at a wavelength of 299 nm.
FIG. 24 is a graph illustrating a PL spectrum of a compound 324 at a wavelength of 260 nm.
FIG. 25 is a graph illustrating an LTPL spectrum of the compound 324 at a wavelength of 320 nm.
FIG. 26 is a graph illustrating a PL spectrum of a compound 560 at a wavelength of 273 nm.
FIG. 27 is a graph illustrating an LTPL spectrum of the compound 560 at a wavelength of 353 nm.
FIG. 28 is a graph illustrating a PL spectrum of a compound 563 at a wavelength of 273 nm.
FIG. 29 is a graph illustrating an LTPL spectrum of the compound 563 at a wavelength of 353 nm.
FIG. 30 is a graph illustrating a PL spectrum of a compound 568 at a wavelength of 253 nm.
FIG. 31 is a graph illustrating an LTPL spectrum of the compound 568 at a wavelength of 348 nm.
FIG. 32 is a graph illustrating a PL spectrum of a compound 603.
FIG. 33 is a graph illustrating an LTPL spectrum of the compound 603.
FIG. 34 is a graph illustrating a PL spectrum of a compound 604.
FIG. 35 is a graph illustrating an LTPL spectrum of the compound 604.

### <Explanation of Reference Numerals and Symbols>

100: Substrate
200: Positive electrode
300: Organic material layer
301: Hole injection layer
302: Hole transport layer
303: Light emitting layer
304: Hole blocking layer
305: Electron transport layer
306: Electron injection layer
400: Negative electrode

### DETAILED DESCRIPTION

Hereinafter, the present invention will be described in detail.

The compound of the invention may be used as a material of an organic material layer of an organic light emitting device due to the above-described structural properties of a core structure and a substituent.

In the present specification, the term "substituted or unsubstituted" refers to a group that may be substituted or may not be further substituted with one or more substituents selected from the group consisting of halogen; -CN; linear or branched C₁ to C₆₀ alkyl; C₃ to C₆₀ monocyclic or polycyclic cycloalkyl; C₆ to C₆₀ monocyclic or polycyclic aryl; C₂ to C₆₀ monocyclic or polycyclic heteroaryl; -SiR₁₁R₁₂R₁₃; -P(=O)R₁₄R₁₅. The R₁₁ to R₁₅ are the same as or different from each other, and are each independently one selected from the group consisting of hydrogen; deuterium; linear or branched C₁ to C₆₀ alkyl substituted or unsubstituted with linear or branched C₁ to C₆₀ alkyl, C₆ to C₆₀ monocyclic or polycyclic aryl, or C₂ to C₆₀ monocyclic or polycyclic heteroaryl; C₆ to C₆₀ monocyclic or polycyclic aryl unsubstituted or substituted with linear or branched C₁ to C₆₀ alkyl, C₆ to C₆₀ monocyclic or polycyclic aryl, or C₂ to C₆₀ monocyclic or polycyclic heteroaryl; and C₂ to C₆₀ monocyclic or polycyclic heteroaryl unsubstituted or substituted with linear or branched C₁ to C₆₀ alkyl, C₆ to C₆₀ monocyclic or polycyclic aryl, or C₂ to C₆₀ monocyclic or polycyclic heteroaryl.

In the present specification, the "adjacent" group may refer to a substituent substituted at an atom direclty bonded to an atom substituted with substituent, a substituent sterically closest to the substituent, or another substituent substituted at an atom substituted with the substituent. For example, two substituents substituted at ortho positions in a benzene ring and two substituents substituted at the same carbon atom in an aliphatic ring may be construed as the "adjacent" groups.

According to an exemplary embodiment of the present invention, the term "substituted or unsubstituted" refers to a group that may be substituted or may not be further substituted with one or more substituents selected from the group consisting of halogen; -CN; linear or branched C₁ to C₆₀ alkyl; C₆ to C₆₀ monocyclic or polycyclic aryl; C₂ to C₆₀ monocyclic or polycyclic heteroaryl; -SiR₁₁R₁₂R₁₃; and -P(=O)R₁₄R₁₅. The R₁₁ to R₁₅ are the same as or different from each other, and are each independently one selected from the group consisting of hydrogen; deuterium; linear or branched C₁ to C₆₀ alkyl unsubstituted or substituted with linear or branched C₁ to C₆₀ alkyl, C₆ to C₆₀ monocyclic or polycyclic aryl, or C₂ to C₆₀ monocyclic or polycyclic heteroaryl; C₆ to C₆₀ monocyclic or polycyclic aryl unsubstituted or substituted with linear or branched C₁ to C₆₀ alkyl, C₆ to C₆₀ monocyclic or polycyclic aryl, or C₂ to C₆₀ monocyclic or polycyclic heteroaryl; and C₂ to C₆₀ monocyclic or polycyclic heteroaryl unsubstituted or substituted with linear or branched C₁ to C₆₀ alkyl, C₆ to C₆₀ monocyclic or polycyclic aryl, or C₂ to C₆₀ monocyclic or polycyclic heteroaryl.

In the present specification, halogen may be fluorine, chlorine, bromine, or iodine.

In the present specification, alkyl includes linear or branched alkyl having 1 to 60 carbon atoms, and may be further substituted with other substituents. The number of carbon atoms of the alkyl may be 1 to 60, specifically 1 to 40, and more specifically 1 to 20.

In the present specification, alkenyl includes linear or branched alkenyl having 2 to 60 carbon atoms, and may be further substituted with other substituents. The number of carbon atoms of the alkenyl may be 2 to 60, specifically 2 to 40, and more specifically 2 to 20.

In the present specification, alkynyl includes linear or branched alkynyl having 2 to 60 carbon atoms, and may be further substituted with other substituents. The number of carbon atoms of the alkynyl may be 2 to 60, specifically 2 to 40, and more specifically 2 to 20.

In the present specification, cycloalkyl includes monocyclic or polycyclic cycloalkyl having 3 to 60 carbon atoms, and may be further substituted with other substituents. Herein, the term "polycyclic" means a group in which cycloalkyl is directly bonded to or fused with other ring groups. Herein, the term "other ring groups" may be cycloalkyl, but may also be other types of ring groups, for example, heterocycloalkyl, aryl, or heteroaryl. The number of carbon atoms of the cycloalkyl may be 3 to 60, specifically 3 to 40, and more specifically 5 to 20.

In the present specification, heterocycloalkyl includes one or more of O, S, Se, N, and Si as a heteroatom, includes monocyclic or polycyclic heterocycloalkyl having 2 to 60 carbon atoms, and may be further substituted with other substituents. Herein, the term "polycyclic" means a group in which heterocycloalkyl is directly bonded to or fused with other ring groups. Herein, the term "other ring groups" may be heterocycloalkyl, but may also be other types of ring groups, for example, cycloalkyl, aryl, or heteroaryl. The number of carbon atoms of the heterocycloalkyl may be 2 to 60, specifically 2 to 40, and more specifically 3 to 20.

In the present specification, aryl includes monocyclic or polycyclic aryl having 6 to 60 carbon atoms, and may be further substituted with other substituents. Herein, the term "polycyclic" means a group in which aryl is directly bonded to or fused with other ring groups. Herein, the term "other ring groups" may be aryl, but may also be other types of ring groups, for example, cycloalkyl, heterocycloalkyl, or heteroaryl. The aryl includes a spiro group. The number of carbon atoms of the aryl may be 6 to 60, specifically 6 to 40, and more specifically 6 to 25. Specific examples of the aryl include phenyl, biphenyl, triphenyl, naphthyl, anthryl, chrysenyl, phenanthrenyl, perylenyl, fluoranthenyl, triphenylenyl, phenalenyl, pyrenyl, tetracenyl, pentacenyl, indenyl, acenaphthylenyl, fluorenyl, benzofluorenyl, benzochrysenyl, spirobifluorenyl, dibenzophenanthridinyl, or fused rings thereof, but are not limited thereto.

In the present specification, the spiro group is a group including a spiro structure, and may have 15 to 60 carbon atoms. For example, the spiro group may include a structure in which a 2,3-dihydro-1H-indene group or a cyclohexane group is spiro-bonded to a fluorene group. To be specific, the spiro group includes a group of the following structural formulas.

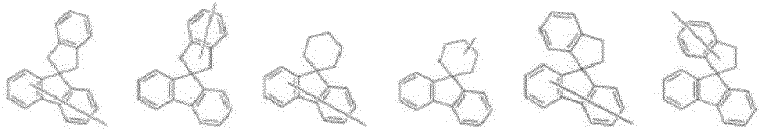

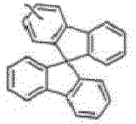

In the present specification, heteroaryl includes one or more of S, O, Se, N, and Si as a heteroatom, includes monocyclic or polycyclic heteroaryl having 2 to 60 carbon atoms, and may be further substituted with other substituents. Herein, the term "polycyclic" means a group in which heteroaryl is directly bonded to or fused with other ring groups. Herein, the term "other ring groups" may be heteroaryl, but may also be other types of ring groups, for example, cycloalkyl, heterocycloalkyl, or aryl. The number of carbon atoms of the heteroaryl may be 2 to 60, specifically 2 to 40, and more specifically 3 to 25. Specific examples of the heteroaryl include pyridyl, pyrolyl, pyrimidyl, pyridazinyl, furanyl, a thiophene group, imidazolyl, pyrazolyl, oxazolyl, isoxazolyl, thiazolyl, isothiazolyl, triazolyl, furazanyl, oxadiazolyl, thiadiazolyl, dithiazolyl, tetrazolyl, pyranyl, thiopyranyl, diazinyl, oxazinyl, thiazinyl, dioxynyl, triazinyl, tetrazinyl, quinolyl, isoquinolyl, quinazolinyl, isoquinazolinyl, naphtyridyl, acridinyl, phenanthridinyl, imidazopyridinyl, diazanaphthalenyl, triazaindene, indolyl, indolizinyl, benzothiazolyl, benzoxazolyl, benzimidazolyl, a benzothiophene group, a benzofuran group, a dibenzothiophene group, a dibenzofuran group, carbazolyl, benzocarbazolyl, dibenzocarbazolyl, phenazinyl, dibenzoxylol, spirobi(dibenzoxylol), dihydrophenazinyl, phenoxazinyl, phenanthridyl, or fused rings thereof, but are not limited thereto.

In the present specification, if at least two adjacent Rs are bonded to each other to form a ring, the ring may be a monocyclic or polycyclic substituted or unsubstituted aliphatic or aromatic hydrocarbon ring.

In the present specification, an aliphatic hydrocarbon ring includes a monocyclic or polycyclic saturated hydrocarbon ring having 3 to 30 carbon atoms, and the above explanation of cycloalkyl can be applied thereto except that adjacent substituents are bonded to each other to form a ring.

In the chemical formulas 6-1 to 8-1,

According to an exemplary embodiment of the present invention, L is C₆ to C₂₀ arylene; or C₂ to C₂₀ heteroarylene.

According to an exemplary embodiment of the present invention, L is substituted or unsubstituted phenylene; substituted or unsubstituted biphenylene; substituted or unsubstituted naphthylene; substituted or unsubstituted anthracenylene; substituted or unsubstituted pyridylene; substituted or unsubstituted pyrimidylene; or substituted or unsubstituted triazinylene.

According to an exemplary embodiment of the present invention, Z is substituted or unsubstituted C₆ to C₆₀ monocyclic to pentacyclic aryl.

According to an exemplary embodiment of the present invention, Z is substituted or unsubstituted phenyl, substituted or unsubstituted biphenyl, substituted or unsubstituted naphthyl, substituted or unsubstituted chrysenyl, substituted or unsubstituted pyrenyl, substituted or unsubstituted triphenylenyl, substituted or unsubstituted anthracenyl, substituted or unsubstituted phenanthrenyl, substituted or unsubstituted fluorenyl, substituted or unsubstituted benzo chrysenyl, or substituted or unsubstituted spirobifluorenyl.

According to an exemplary embodiment of the present invention, Z is substituted or unsubstituted phenyl, substituted or unsubstituted biphenyl, substituted or unsubstituted naphthyl, substituted or unsubstituted chrysenyl, substituted or unsubstituted pyrenyl, substituted or unsubstituted triphenylenyl, substituted or unsubstituted anthracenyl, substituted or unsubstituted phenanthrenyl, substituted or unsubstituted fluorenyl, substituted or unsubstituted benzo chrysenyl, or substituted or unsubstituted spirobifluorenyl, and the term "substituted or unsubstituted" refers to a group that may be substituted or may not be further substituted with at least one selected from halogen, linear or branched C₁ to C₆₀ alkyl, C₃ to C₆₀ monocyclic or polycyclic cycloalkyl, C₆ to C₆₀ monocyclic or polycyclic aryl, C₂ to C₆₀ monocyclic or polycyclic heteroaryl, and may be further substituted.

According to an exemplary embodiment of the present invention, Z is substituted or unsubstituted phenyl, substituted or unsubstituted biphenyl, substituted or unsubstituted naphthyl, substituted or unsubstituted chrysenyl, substituted or unsubstituted pyrenyl, substituted or unsubstituted triphenylenyl, substituted or unsubstituted anthracenyl, substituted or unsubstituted phenanthrenyl, substituted or unsubstituted fluorenyl, substituted or unsubstituted benzo chrysenyl, or substituted or unsubstituted spirobifluorenyl, and the term "substituted or unsubstituted" refers to a group that may be substituted with at least one selected from halogen atoms, methyl, phenyl, naphthyl, pyridyl, and carbazol, and may be further substituted.

According to an exemplary embodiment of the present invention, Z is substituted or unsubstituted C₂ to C₆₀ monocyclic or polycyclic heteroaryl, and the heteroaryl includes at least one selected from N, O, S, Si, and Se as a heteroatom.

According to an exemplary embodiment of the present invention, Z is substituted or unsubstituted benzimidazolyl, substituted or unsubstituted quinolyl, substituted or unsubstituted isoquinolyl, substituted or unsubstituted naphtyridyl, substituted or unsubstituted quinazolyl, substituted or unsubstituted quinoxalyl, substituted or unsubstituted cinolyl, substituted or unsubstituted benzothiazolyl, substituted or unsubstituted benzoxazolyl, substituted or unsubstituted oxadiazolyl, substituted or unsubstituted dibenzofuranyl, substituted or unsubstituted dibenzothiophenyl, substituted or unsubstituted pyridyl, substituted or unsubstituted pyrimidyl, substituted or unsubstituted triazinyl, substituted or unsubstituted pyrazolophthalazinyl, or substituted or unsubstituted carbazolyl.

According to an exemplary embodiment of the present invention, Z is substituted or unsubstituted benzimidazolyl, substituted or unsubstituted quinolyl, substituted or unsubstituted isoquinolyl, substituted or unsubstituted naphtyridyl, substituted or unsubstituted quinazolyl, substituted or unsubstituted quinoxalyl, substituted or unsubstituted cinolyl, substituted or unsubstituted benzothiazolyl, substituted or unsubstituted benzoxazolyl, substituted or unsubstituted oxadiazolyl, substituted or unsubstituted dibenzofuranyl, substituted or unsubstituted dibenzothiophenyl, substituted or unsubstituted pyridyl, substituted or unsubstituted pyrimidyl, substituted or unsubstituted triazinyl, substituted or unsubstituted pyrazolophthalazinyl, or substituted or unsubstituted carbazolyl, and the term "substituted or unsubstituted" refers to a group that may be substituted or may not be further substituted with at least one selected from halogen atoms, monocyclic or polycyclic C₃ to C₆₀ cycloalkyl, monocyclic or polycyclic C₆ to C₆₀ aryl, monocyclic or polycyclic C₂ to C₆₀ heteroaryl, and SiR₁₁R₁₂R₁₃, and may be further substituted, and

R₁₁ to R₁₃ are the same as or different from each other, and are each independently linear or branched C₁ to C₆₀ alkyl; C₆ to C₆₀ monocyclic or polycyclic aryl; or C₂ to C₆₀ monocyclic or polycyclic heteroaryl.

According to an exemplary embodiment of the present invention, Z is substituted or unsubstituted benzimidazolyl, substituted or unsubstituted quinolyl, substituted or unsubstituted isoquinolyl, substituted or unsubstituted naphtyridyl, substituted or unsubstituted quinazolyl, substituted or unsubstituted quinoxalyl, substituted or unsubstituted cinolyl, substituted or unsubstituted benzothiazolyl, substituted or unsubstituted benzoxazolyl, substituted or unsubstituted oxadiazolyl, substituted or unsubstituted dibenzofuranyl, substituted or unsubstituted dibenzothiophenyl, substituted or unsubstituted pyridyl, substituted or unsubstituted pyrimidyl, substituted or unsubstituted triazinyl, substituted or unsubstituted pyrazolophthalazinyl, or substituted or unsubstituted carbazolyl, and the term "substituted or unsubstituted" refers to a group that may be substituted or may not be further substituted with at least one selected from halogen atoms, cyclohexyl, phenyl, naphthyl, triphenylenyl, pyridyl, and Si(CH₃)₃, and may be further substituted.

According to an exemplary embodiment of the present invention, Z is 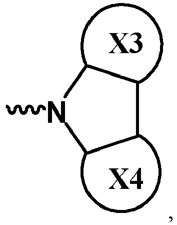 and the X3 and X4 are substituted or unsubstituted C₆ to C₆₀ monocyclic or polycyclic aromatic hydrocarbon rings; or substituted or unsubstituted C₂ to C₆₀ monocyclic or polycyclic aromatic hetero rings.

According to an exemplary embodiment of the present invention, 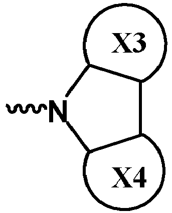 is represented by any one of the following structural formulas.

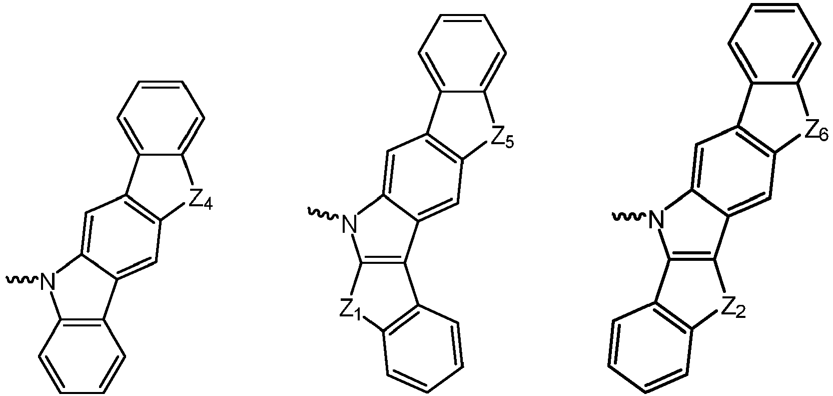

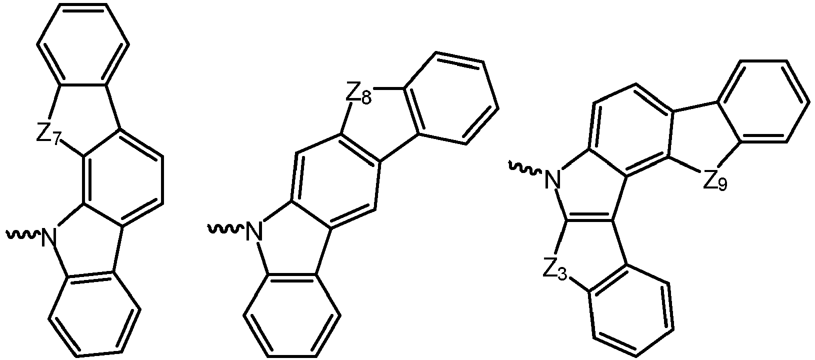

In the structural formulas, Z₁ to Z₃ are the same as or different from each other, and are each independently S or O,
Z₄ to Z₉ are the same as or different from each other, and are each independently CR' R", NR', S, or O,
R' and R" are the same as or different from each other, and are each independently hydrogen; linear or branched substituted or unsubstituted C₁ to C₆₀ alkyl; or substituted or unsubstituted C₆ to C₆₀ monocyclic or polycyclic aryl.

According to an exemplary embodiment of the present invention, the R' and R" are the same as or different from each other, and are independently hydrogen, methyl, phenyl, or naphthyl.

According to an exemplary embodiment of the present invention, Z is -P(=O)R₁₄R₁₅, and R₁₄ and R₁₅ are the same as or different from each other, and are independently one selected from the group consisting of C₆ to C₆₀ monocyclic or polycyclic aryl; and C₂ to C₆₀ monocyclic or polycyclic heteroaryl.

According to an exemplary embodiment of the present invention, Z is -P(=O)R₁₄R₁₅, and R₁₄ and R₁₅ are the same as or different from each other, and are independently C₆ to C₆₀ monocyclic or polycyclic aryl.

According to an exemplary embodiment of the present invention, Z is -P(=O)R₁₄R₁₅, and R₁₄ and R₁₅ are the same as or different from each other, and are independently phenyl, biphenyl, or naphthyl.

According to an exemplary embodiment of the present invention, L is selected from the group consisting of substituted or unsubstituted C₆ to C₆₀ monocyclic or polycyclic arylene and substituted or unsubstituted C₂ to C₆₀ monocyclic or polycyclic heteroarylene, and Z is selected from the group consisting of substituted or unsubstituted C₆ to C₆₀ monocyclic or polycyclic aryl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, spirobifluorenylene, pyridylene, imidazopyridylene, pyrimidylene, triazinylene, carbazolylene, benzimidazolylene, benzocarbazolylene, dibenzocarbazolylene, quinolinylene, isoquinolinylene, quinazolinylene, pyrazoloquinazolinylene, imidazoquinazolinylene, thiazolinylene, benzothiazolinylene, phenanthrolinylene, phenanthridinylene, dibenzo acridinylene, xylolylene, benzoxylolylene, dibenzoxylolylene, benzoxazolylene, naphthyridinylene, oxadiazolylene, dibenzofuranylene, dibenzothiophenylene, dibenzophenanthridinylene, and spirobidibenzoxylolylene, and may be further substituted with halogen; C₁ to C₁₀ alkyl; C₃ to C₃₀ cycloalkyl; C₆ to C₃₀ aryl; and C₂ to C₃₀ heteroaryl, and
Z may be selected from the group consisting of phenyl, biphenyl, naphthyl, anthracenyl, phenanthrenyl, triphenylenyl, chrysenyl, benzo chrysenyl, fluorenyl, pyrenyl, and spirobifluorenyl, and may be further substituted with halogen atoms; C₁ to C₁₀ alkyl; C₃ to C₃₀ cycloalkyl; C₆ to C₃₀ aryl; and C₂ to C₃₀ heteroaryl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, spirobifluorenylene, pyridylene, imidazopyridylene, pyrimidylene, triazinylene, carbazolylene, benzimidazolylene, benzocarbazolylene, dibenzocarbazolylene, quinolinylene, isoquinolinylene, quinazolinylene, pyrazoloquinazolinylene, imidazoquinazolinylene, thiazolinylene, benzothiazolinylene, phenanthrolinylene, phenanthridinylene, dibenzo acridinylene, xylolylene, benzoxylolylene, dibenzoxylolylene, benzoxazolylene, naphthyridinylene, oxadiazolylene, dibenzofuranylene, dibenzothiophenylene, dibenzophenanthridinylene, and spirobidibenzoxylolylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be selected from the group consisting of phenyl, biphenyl, naphthyl, anthracenyl, phenanthrenyl, triphenylenyl, chrysenyl, benzo chrysenyl, fluorenyl, pyrenyl, and spirobifluorenyl, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L is selected from the group consisting of substituted or unsubstituted C₆ to C₆₀ monocyclic or polycyclic arylene and substituted or unsubstituted C₂ to C₆₀ monocyclic or polycyclic heteroarylene, and Z is selected from the group consisting of substituted or unsubstituted C₂ to C₆₀ monocyclic or polycyclic heteroaryl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, spirobifluorenylene, pyridylene, imidazopyridylene, pyrimidylene, triazinylene, carbazolylene, benzimidazolylene, benzocarbazolylene, dibenzocarbazolylene, quinolinylene, isoquinolinylene, quinazolinylene, pyrazoloquinazolinylene, imidazoquinazolinylene, thiazolinylene, benzothiazolinylene, phenanthrolinylene, phenanthridinylene, dibenzo acridinylene, xylolylene, benzoxylolylene, dibenzoxylolylene, benzoxazolylene, naphthyridinylene, oxadiazolylene, dibenzofuranylene, dibenzothiophenylene, dibenzophenanthridinylene, and spirobidibenzoxylolylene, and may be further substituted with halogen; C₁ to C₁₀ alkyl; C₃ to C₃₀ cycloalkyl; C₆ to C₃₀ aryl; and C₂ to C₃₀ heteroaryl, and
Z may be selected from the group consisting of pyridyl, imidazol pyridyl, pyrimidyl, triazinyl, carbazolyl, benzimidazolyl, benzocarbazolyl, dibenzocarbazolyl, quinolinyl, isoquinolinyl, quinazolinyl, pyrazoloquinazolinyl, imidazoquinazolinyl, thiazolinyl, benzothiazolinyl, phenanthrolinyl, phenanthridinyl, dibenzo acridinyl, xylolyl, benzoxylolyl, dibenzoxylolyl, benzoxazolyl, naphthyridinyl, oxadiazolyl, dibenzofuranyl, dibenzothiophenyl, dibenzophenanthridinyl, and spirobidibenzoxylolyl, and may be further substituted with halogen atoms; C₁ to C₁₀ alkyl; C₃ to C₃₀ cycloalkyl; C₆ to C₃₀ aryl; and C₂ to C₃₀ heteroaryl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, spirobifluorenylene, pyridylene, imidazopyridylene, pyrimidylene, triazinylene, carbazolylene, benzimidazolylene, benzocarbazolylene, dibenzocarbazolylene, quinolinylene, isoquinolinylene, quinazolinylene, pyrazoloquinazolinylene, imidazoquinazolinylene, thiazolinylene, benzothiazolinylene, phenanthrolinylene, phenanthridinylene, dibenzo acridinylene, xylolylene, benzoxylolylene, dibenzoxylolylene, benzoxazolylene, naphthyridinylene, oxadiazolylene, dibenzofuranylene, dibenzothiophenylene, dibenzophenanthridinylene, and spirobidibenzoxylolylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be selected from the group consisting of pyridyl, imidazol pyridyl, pyrimidyl, triazinyl, carbazolyl, benzimidazolyl, benzocarbazolyl, dibenzocarbazolyl, quinolinyl, isoquinolinyl, quinazolinyl, pyrazoloquinazolinyl, imidazoquinazolinyl, thiazolinyl, benzothiazolinyl, phenanthrolinyl, phenanthridinyl, dibenzo acridinyl, xylolyl, benzoxylolyl, dibenzoxylolyl, benzoxazolyl, naphthyridinyl, oxadiazolyl, dibenzofuranyle, dibenzothiophenyl, dibenzophenanthridinyl, and spirobidibenzoxylolyl, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L is substituted or unsubstituted C₆ to C₆₀ monocyclic or polycyclic arylene, Z is -SiR₁₁R₁₂R₁₃, and the R₁₁ to R₁₃ are the same as or different from each other, and are each independently one selected from the group consisting of hydrogen atoms; deuterium; linear or branched substituted or unsubstituted C₁ to C₆₀ alkyl; substituted or unsubstituted C₆ to C₆₀ monocyclic or polycyclic aryl; and substituted or unsubstituted C₂ to C₆₀ monocyclic or polycyclic heteroaryl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with halogen; C₁ to C₁₀ alkyl; C₃ to C₃₀ cycloalkyl; C₆ to C₃₀ aryl; and C₂ to C₃₀ heteroaryl,
Z is -SiR₁₁R₁₂R₁₃, and the R₁₁ to R₁₃ are the same as or different from each other, and are each independently one selected from the group consisting of phenyl, biphenyl, naphthyl, and anthracenyl, and may be further substituted with halogen atoms; C₁ to C₁₀ alkyl; C₃ to C₃₀ cycloalkyl; C₆ to C₃₀ aryl; and C₂ to C₃₀ heteroaryl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl,
the Z is -SiR₁₁R₁₂R₁₃, and the R₁₁ to R₁₃ are the same as or different from each other, and are each independently one selected from the group consisting of phenyl, biphenyl, naphthyl, and anthracenyl, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L is substituted or unsubstituted C₆ to C₆₀ monocyclic or polycyclic arylene, Z is -P(=O)R₁₄R₁₅, and the R₁₄ and R₁₅ are the same as or different from each other, and are each independently one selected from the group consisting of hydrogen; deuterium; linear or branched substituted or unsubstituted C₁ to C₆₀ alkyl; substituted or unsubstituted C₆ to C₆₀ monocyclic or polycyclic aryl; and substituted or unsubstituted C₂ to C₆₀ monocyclic or polycyclic heteroaryl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with halogen; C₁ to C₁₀ alkyl; C₃ to C₃₀ cycloalkyl; C₆ to C₃₀ aryl; and C₂ to C₃₀ heteroaryl,
Z is -P(=O)R₁₄R₁₅, and the R₁₄ and R₁₅ are the same as or different from each other, and are are each independently one selected from the group consisting of phenyl, biphenyl, naphthyl, and anthracenyl, and may be further substituted with halogen; C₁ to C₁₀ alkyl; C₃ to C₃₀ cycloalkyl; C₆ to C₃₀ aryl; and C₂ to C₃₀ heteroaryl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl,
Z is -P(=O)R₁₄R₁₅, and the R₁₄ and R₁₅ which are identical to or different from each other, are each independently one selected from the group consisting of phenyl, biphenyl, naphthyl, and anthracenyl, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L is monocyclic or polycyclic substituted or unsubstituted C₆ to C₆₀ monocyclic or polycyclic arylene, Z is monocyclic or polycyclic substituted or unsubstituted C₂ to C₆₀ N-containing monocyclic or polycyclic heteroaryl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be pyridyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with halogen; C₁ to C₁₀ alkyl; C₃ to C₃₀ cycloalkyl; C₆ to C₃₀ aryl; and C₂ to C₃₀ heteroaryl, and
Z may be pyrimidyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be triazinyl substituted or unsubstituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be carbazolyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
the Z may be quinolinyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be quinazolinyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be pyrazoloquinazolinyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, the L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be phenanthrolinyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be benzimidazolyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be benzothiazolyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be benzoxazolyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be oxadiazolyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be naphthyridinyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be phenanthrolinyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, L may be selected from the group consisting of phenylene, biphenylene, naphthylene, anthracenylene, phenanthrenylene, triphenylenylene, chrysenylene, benzo chrysenylene, fluorenylene, pyrenylene, and spirobifluorenylene, and may be further substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl, and
Z may be dibenzophenanthridinyl unsubstituted or substituted with at least one substituent selected from the group consisting of fluorine, methyl, ethyl, propyl, cyclopentyl, cyclohexyl, phenyl, biphenyl, naphthyl, anthracenyl, phenanthrenyl, pyridyl, and pyrimidyl.

According to an exemplary embodiment of the present invention, the compound is represented by the following chemical formula 4 or 5. 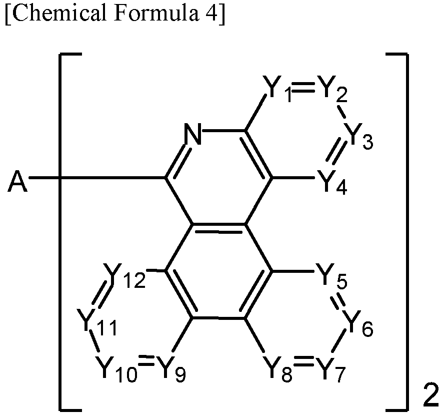 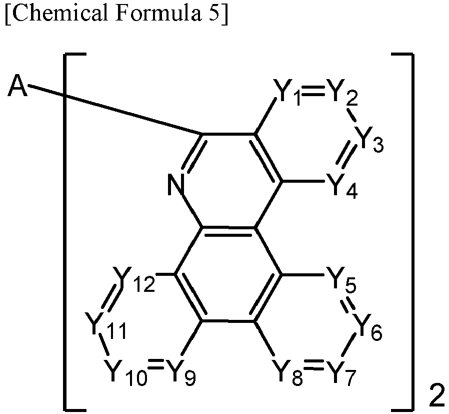 wherein in the chemical formulas 4 and 5,
A is selected from the group consisting of substituted or unsubstituted C₆ to C₆₀ monocyclic or polycyclic arylene, and substituted or unsubstituted C₂ to C₆₀ monocyclic or polycyclic heteroarylene, and
Y₁ to Y₁₂ are the same as defined above

The chemical formulas 4 and 5 mean dimer structures, and the A means a linkage of a dimer.

According to an exemplary embodiment of the present invention, A is C₆ to C₆₀ monocyclic or polycyclic arylene.

According to an exemplary embodiment of the present invention, A is or phenylene, biphenylene, naphthylene, or anthracenylene.

According to an exemplary embodiment of the present application, Formula 9 is represented by the following Formula 10.

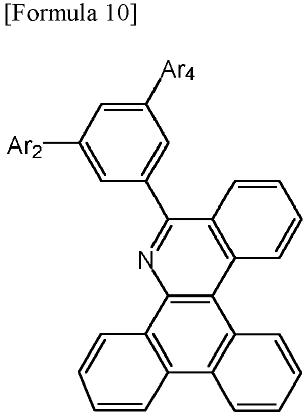

In Formula 10, Ar₂ and Ar₄ are each independently monocyclic or polycyclic substituted or unsubstituted C₆ to C₆₀ aryl; or monocyclic or polycyclic substituted or unsubstituted C₂ to C₆₀ heteroaryl.

According to an exemplary embodiment of the present application, Ar₂ and Ar₄ are each independently selected from the group consisting of a phenyl group which is unsubstituted or substituted with benzoimidazole, a naphthyl group, a phenanthrene group, a dibenzofuran group, a dibenzothiophene group, a carbazole group, a phenanthroline group, and a spirobifluorene group.

According to an exemplary embodiment of the present application, Ar₂ and Ar₄ are the same as each other.

According to an exemplary embodiment of the present invention, the compound of the invention may be selected from the following compounds:

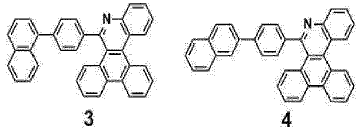

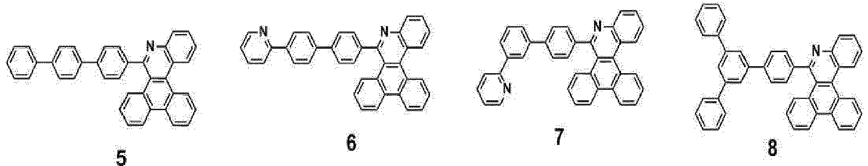

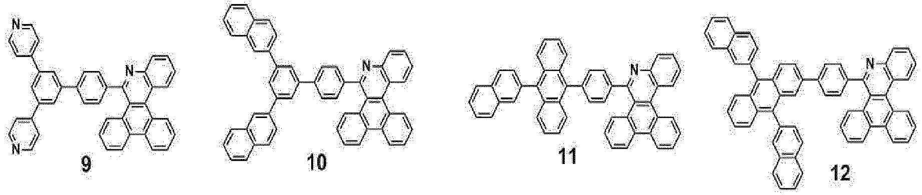

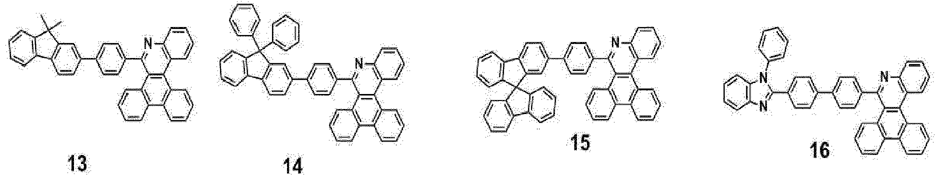

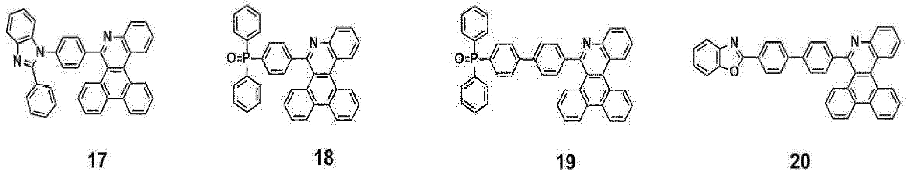

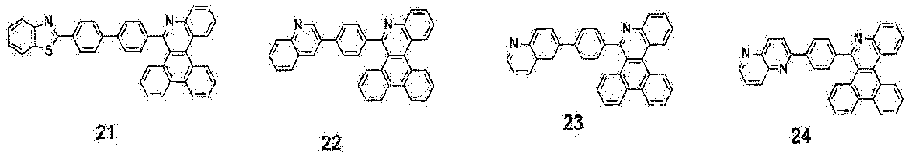

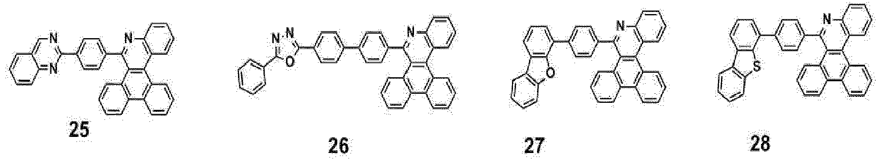

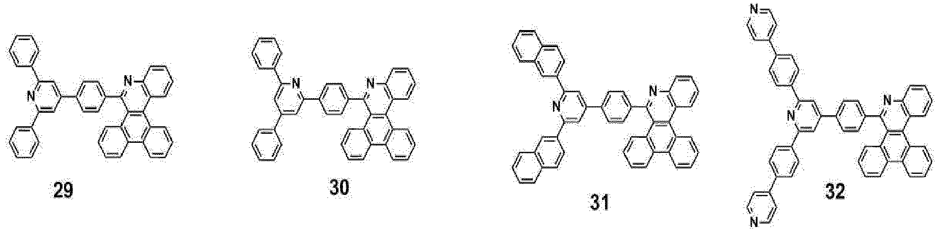

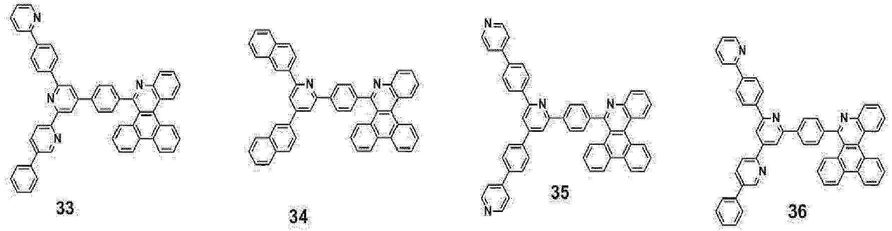

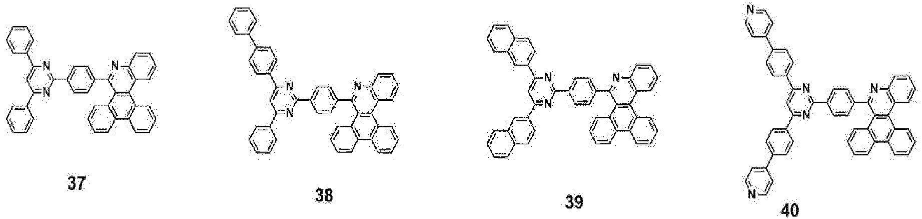

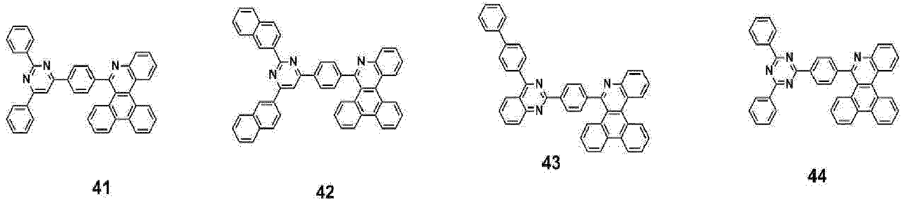

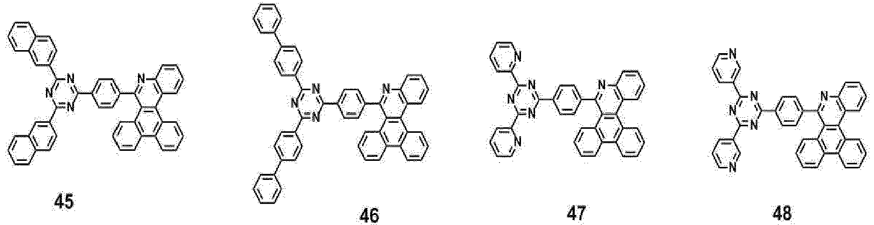

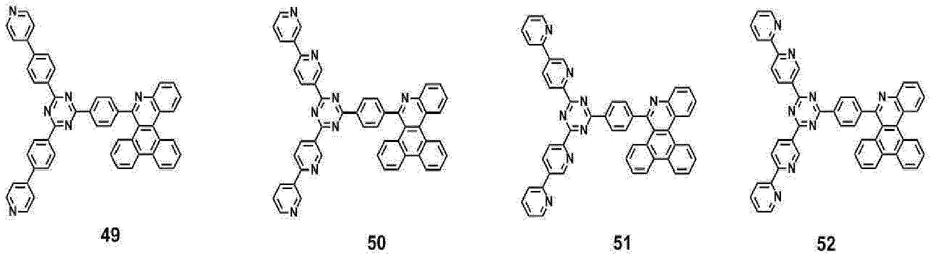

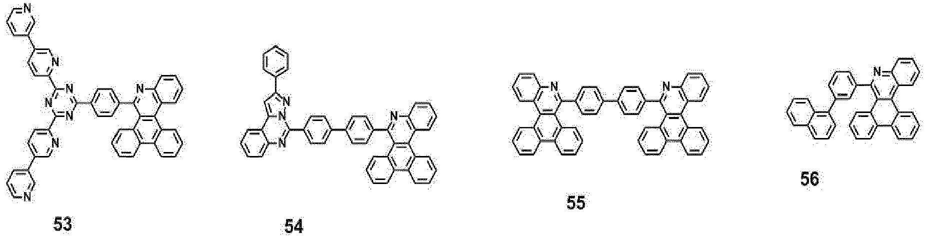

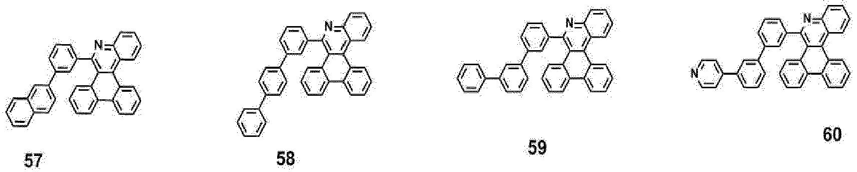

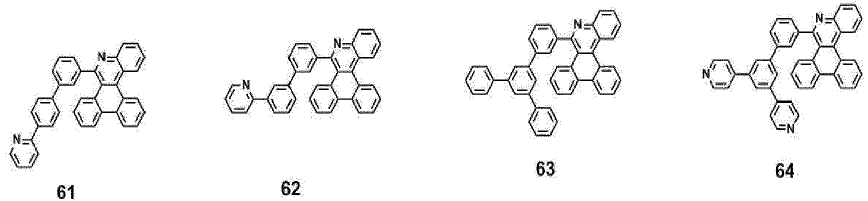

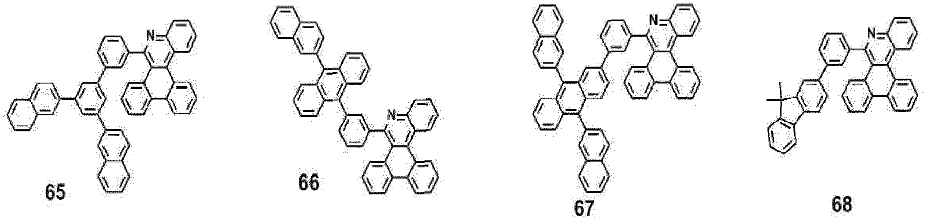

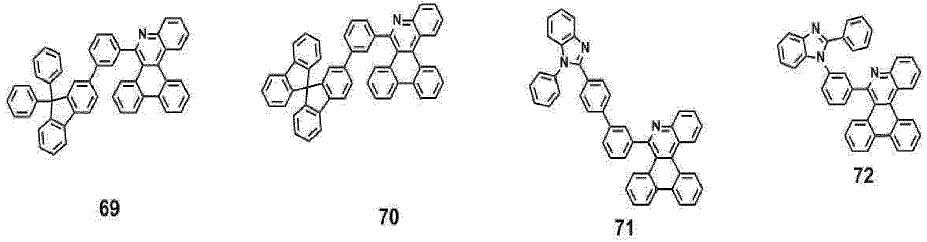

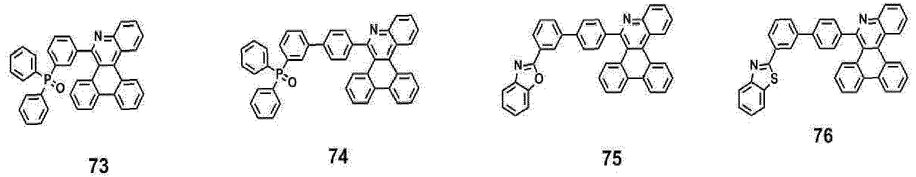

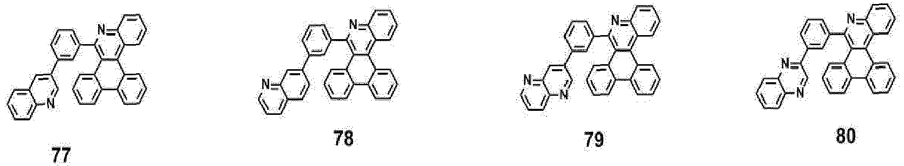

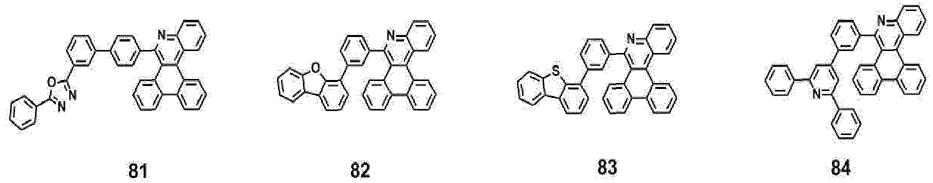

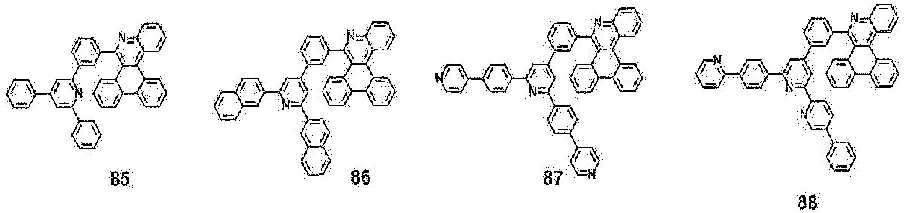

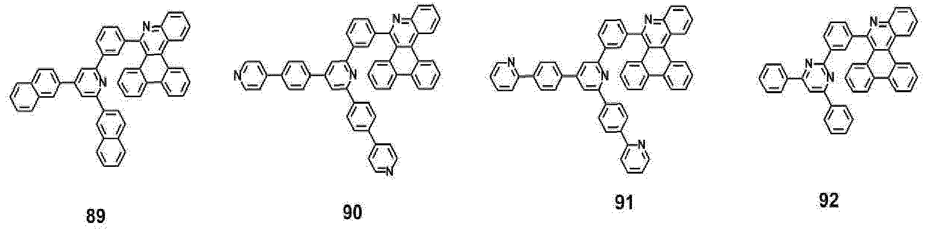

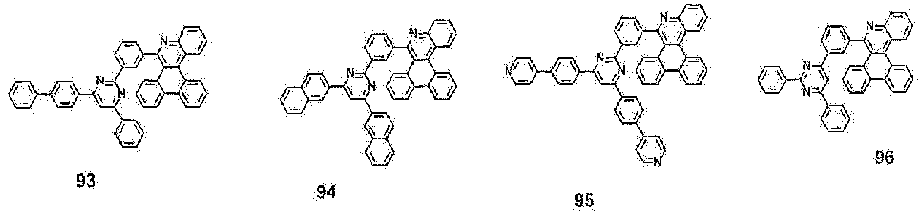

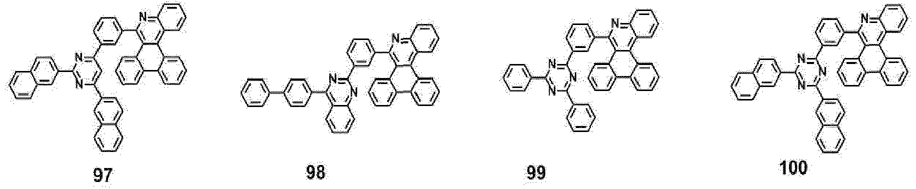

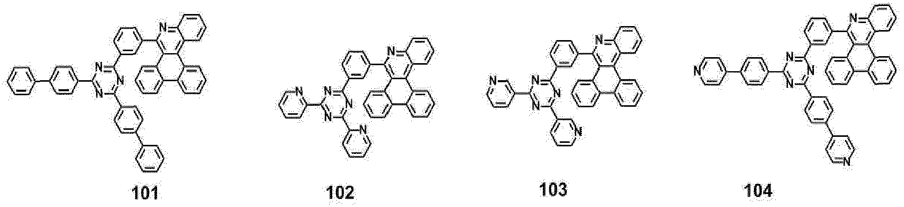

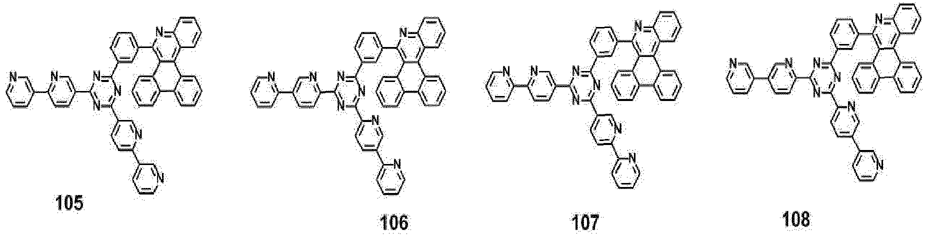

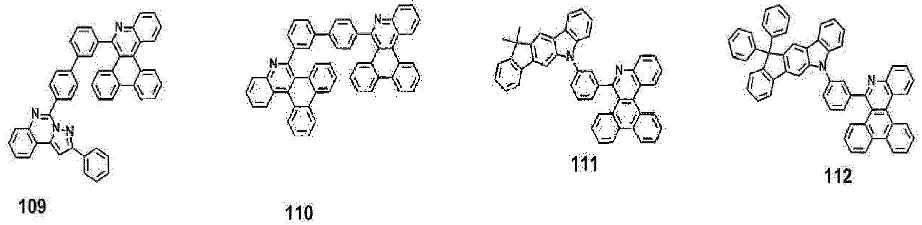

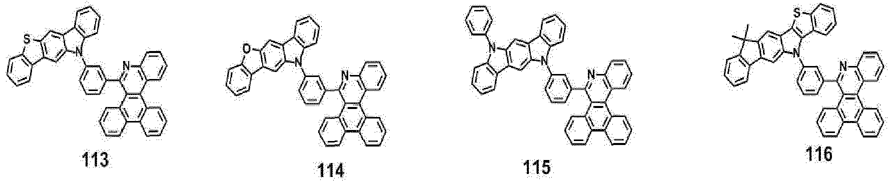

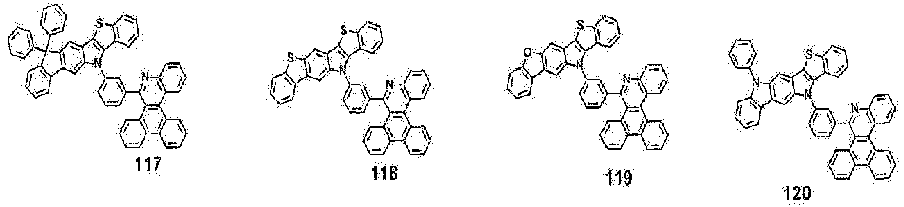

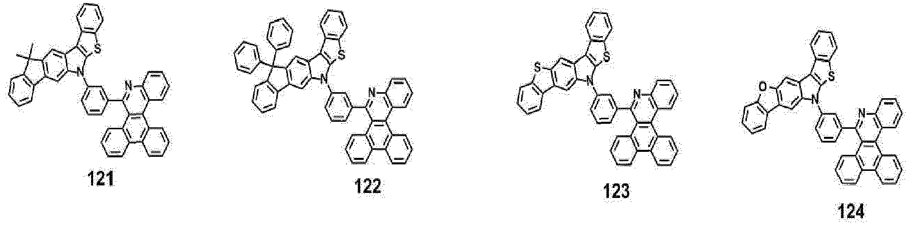

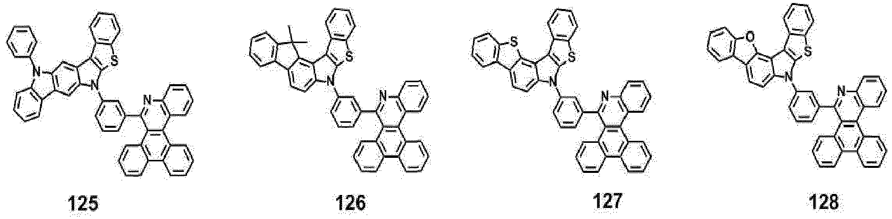

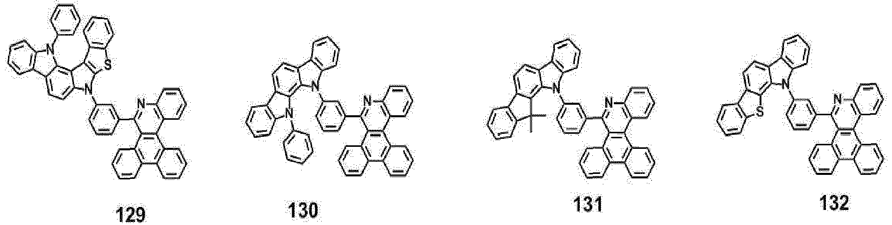

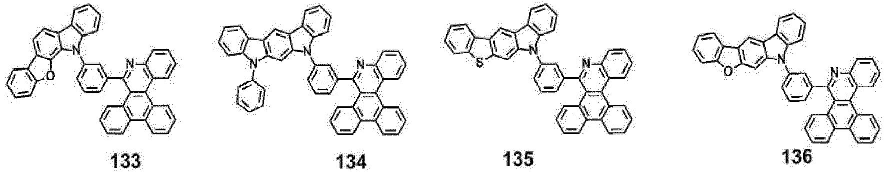

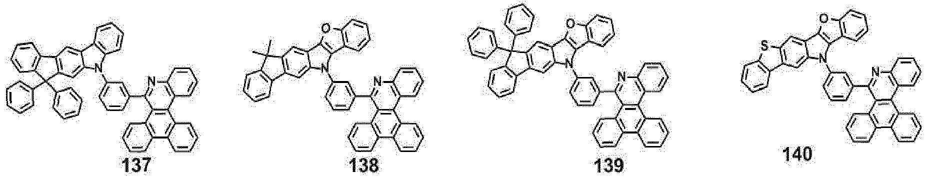

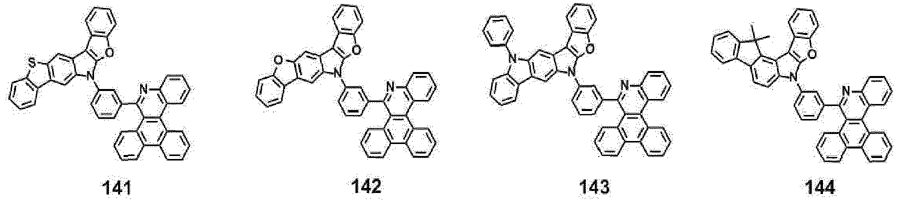

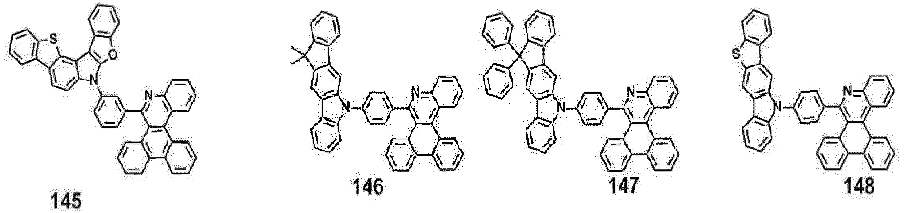

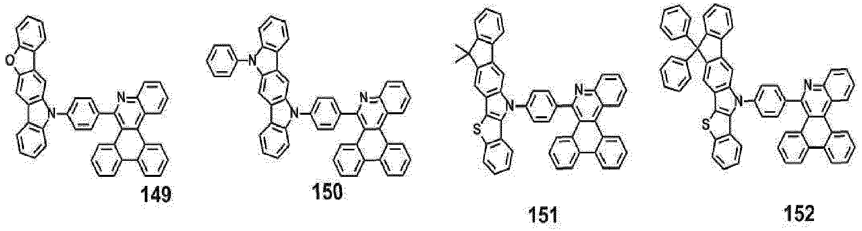

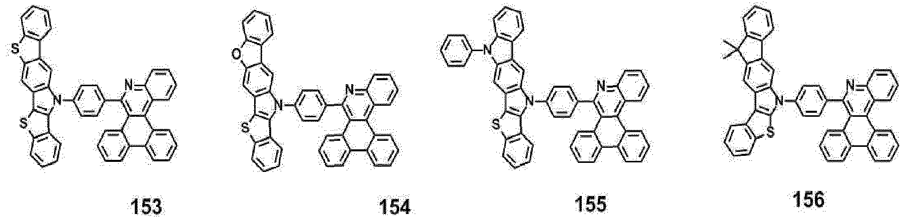

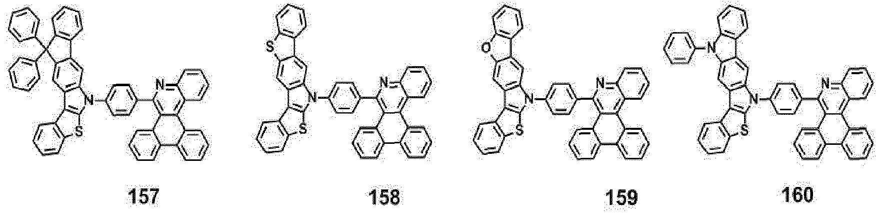

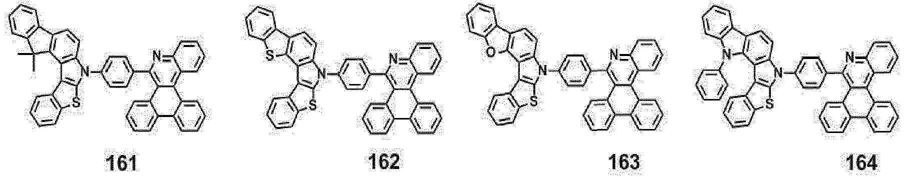

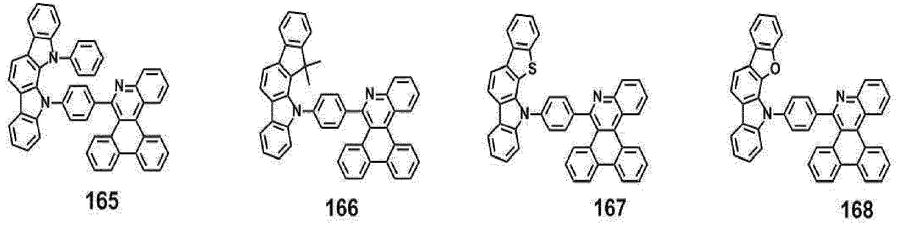

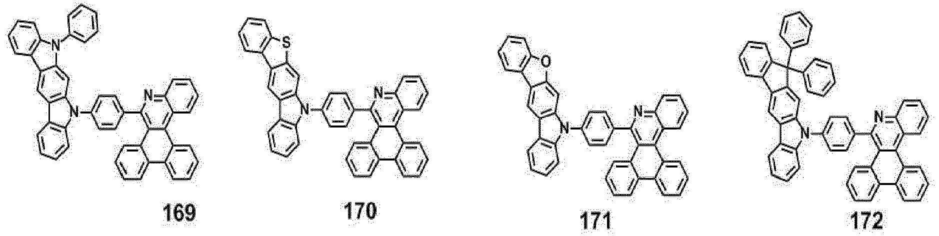

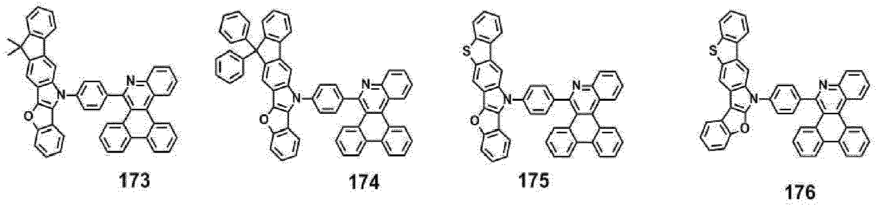

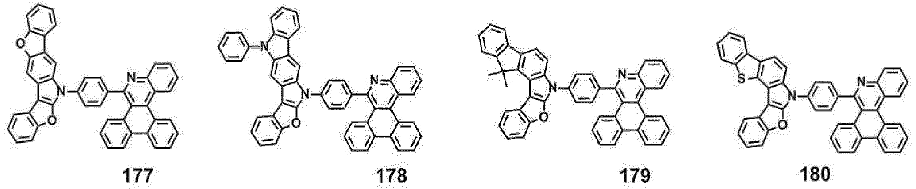

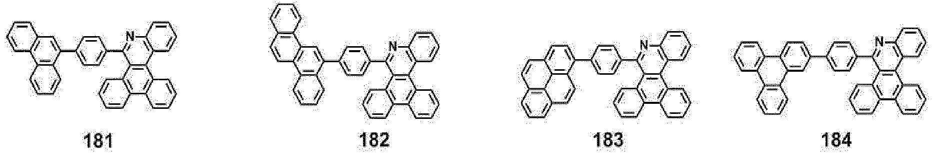

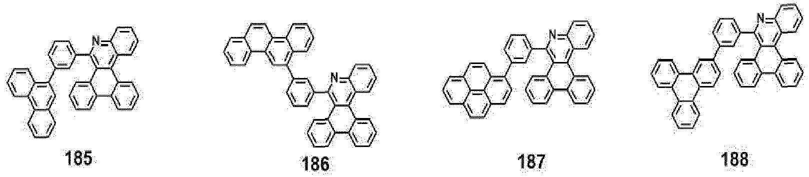

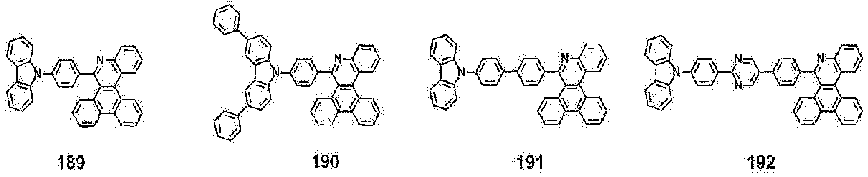

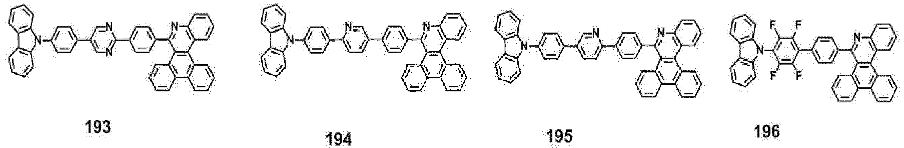

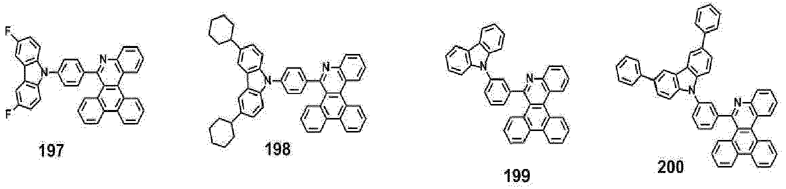

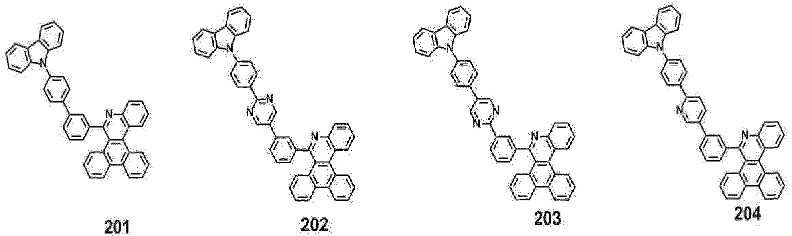

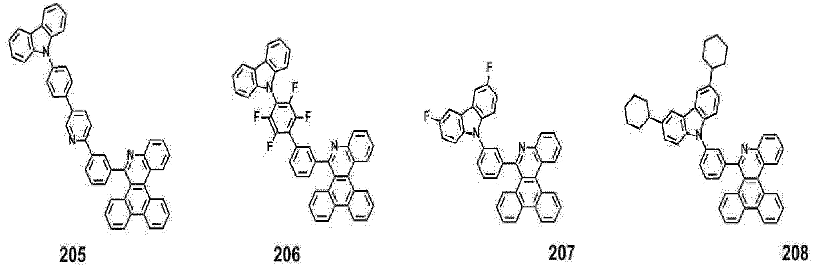

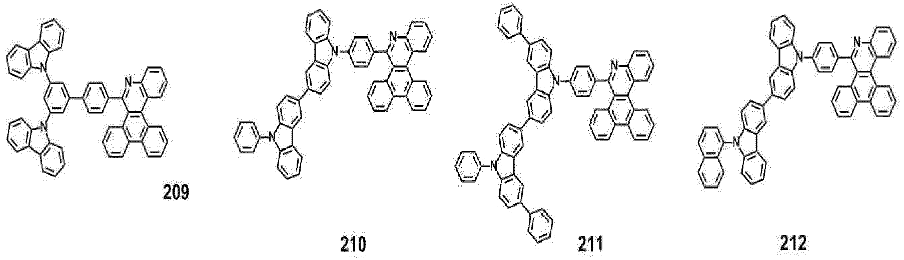

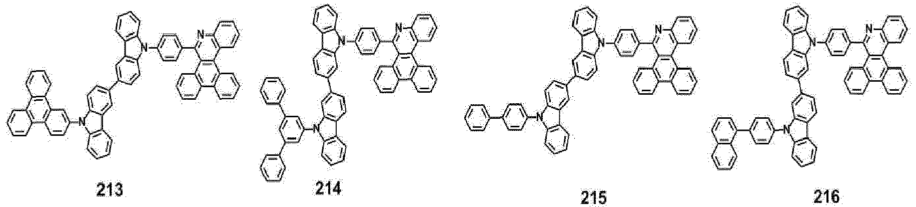

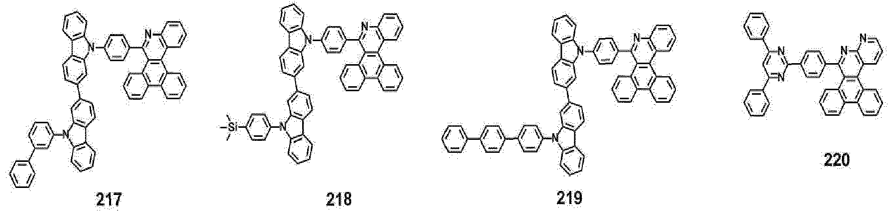

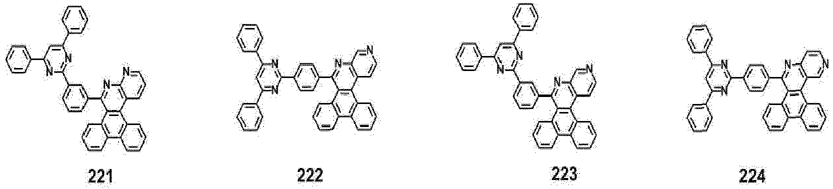

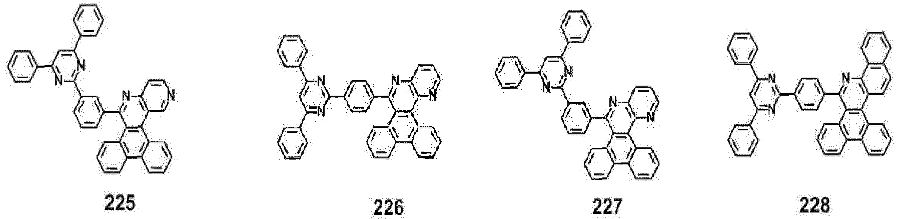

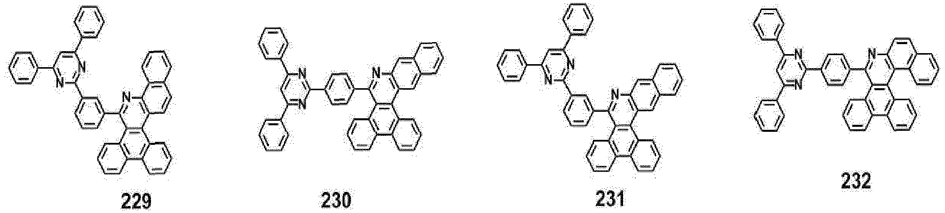

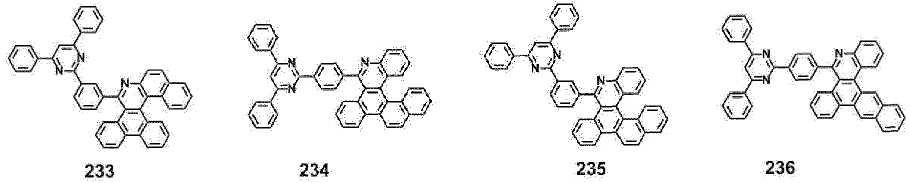

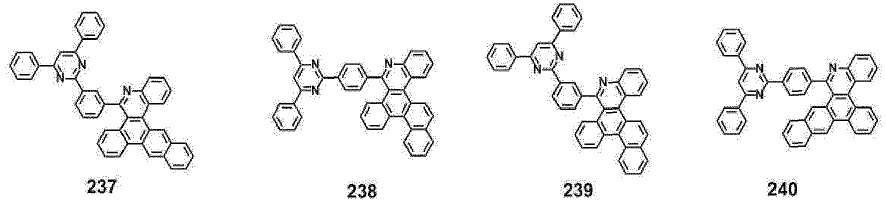

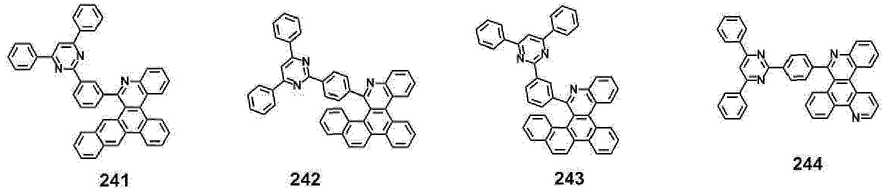

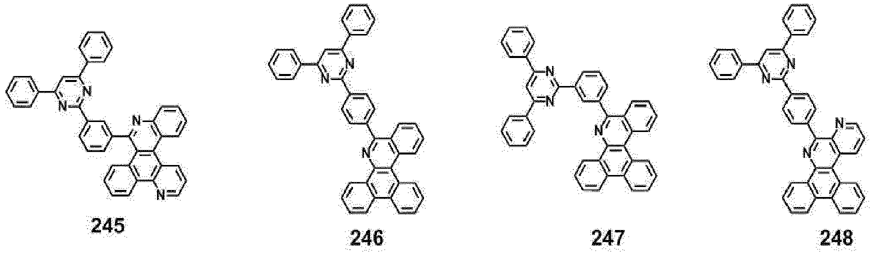

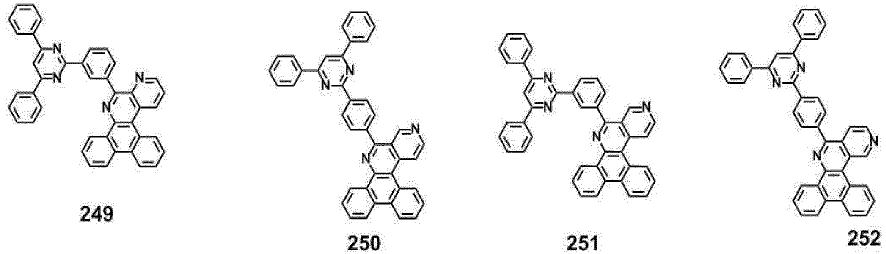

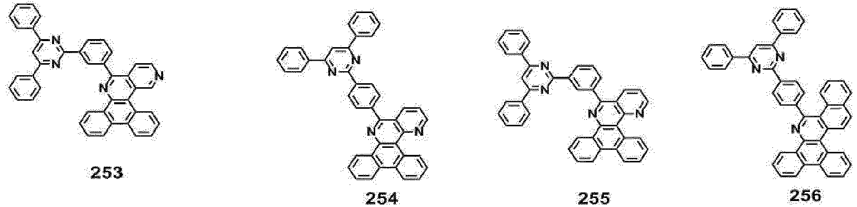

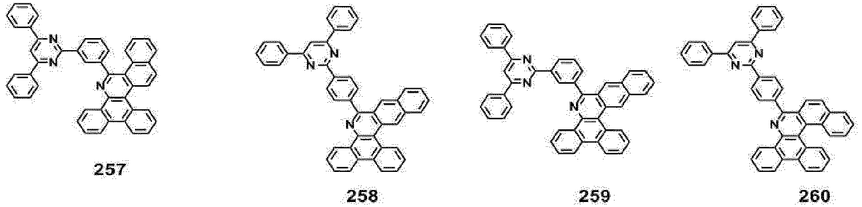

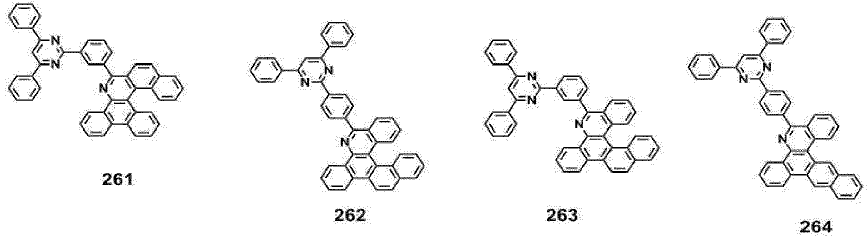

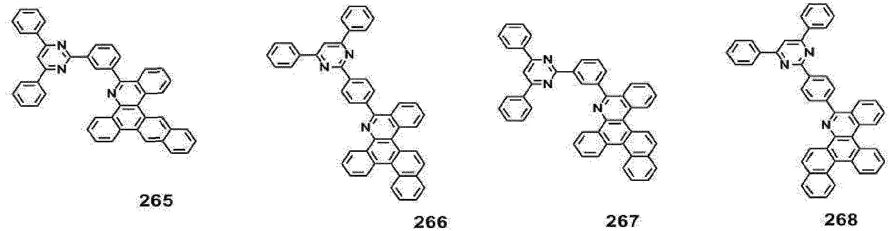

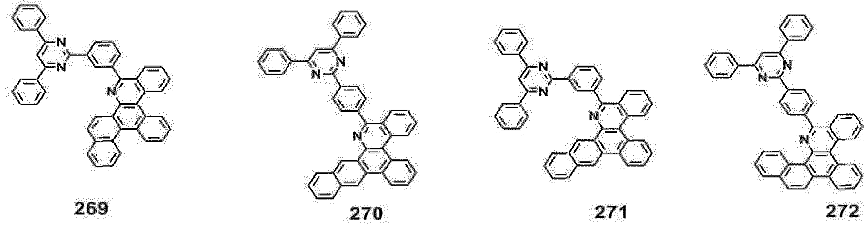

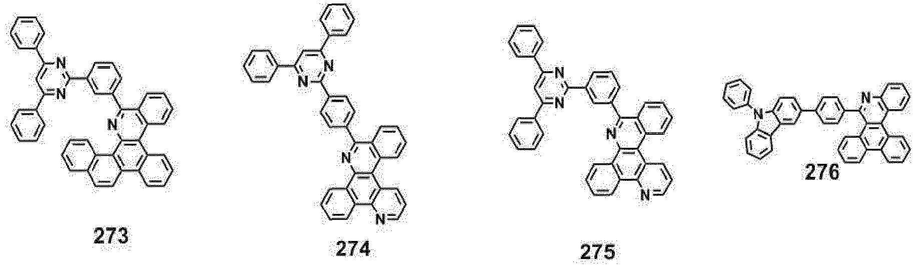

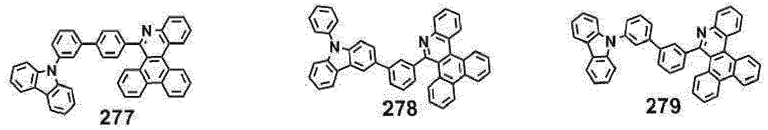

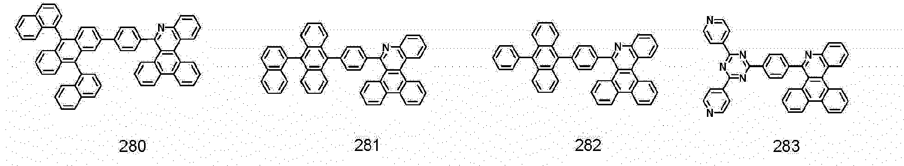

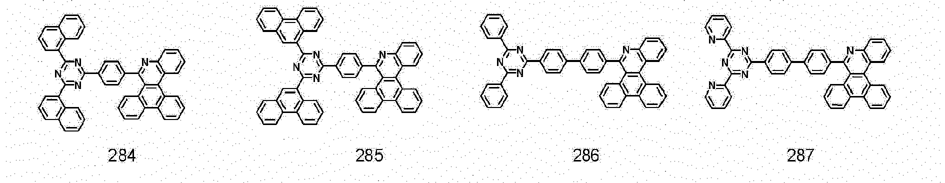

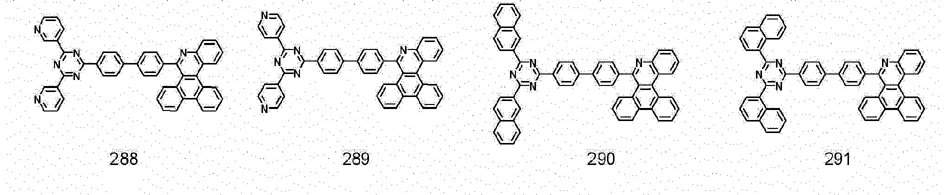

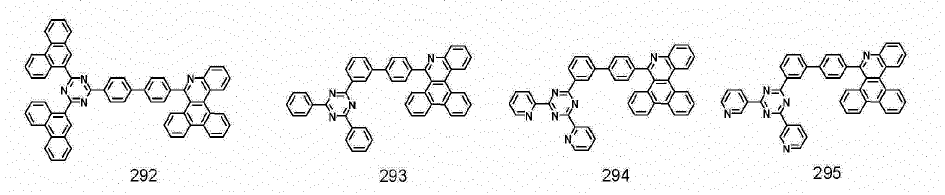

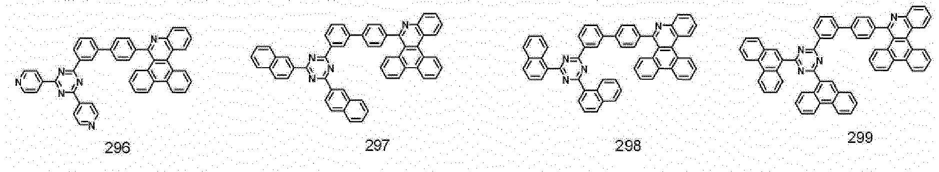

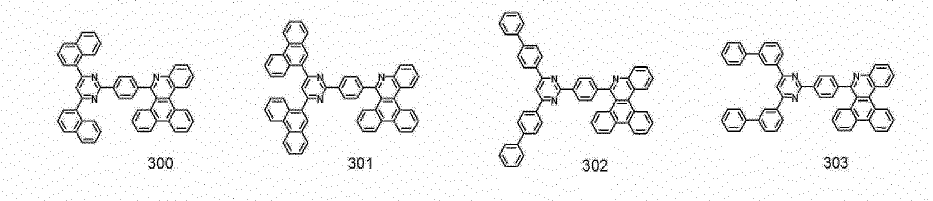

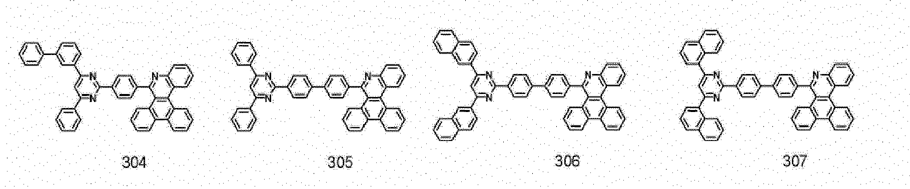

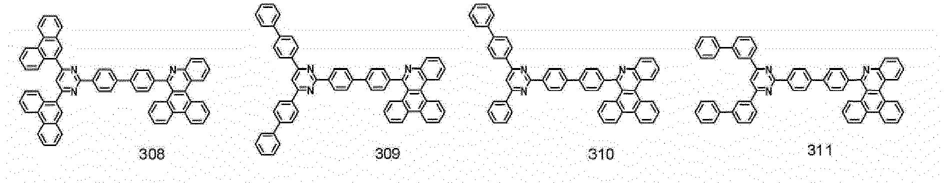

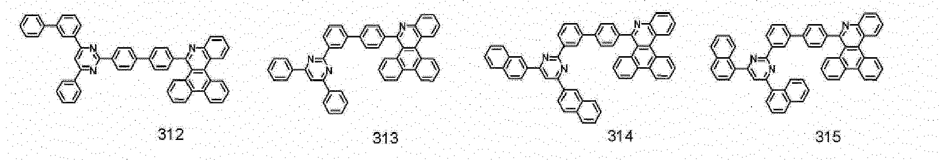

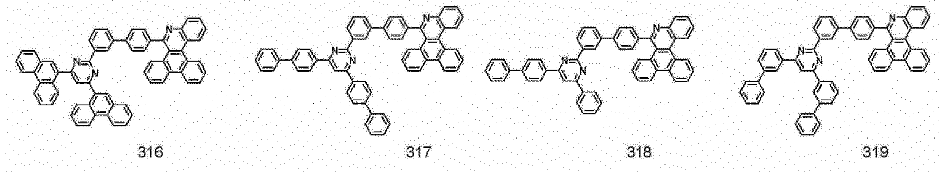

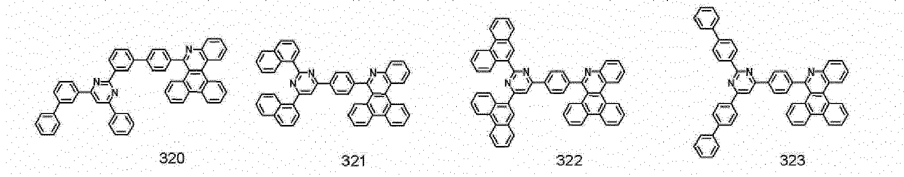

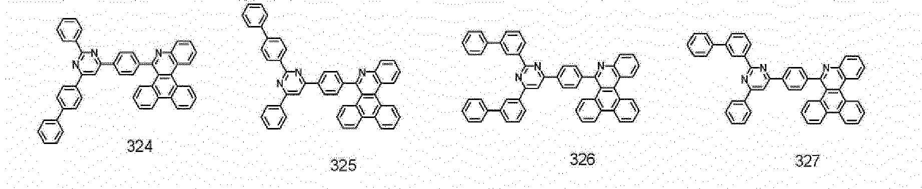

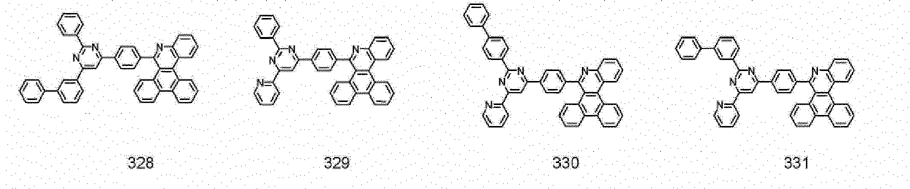

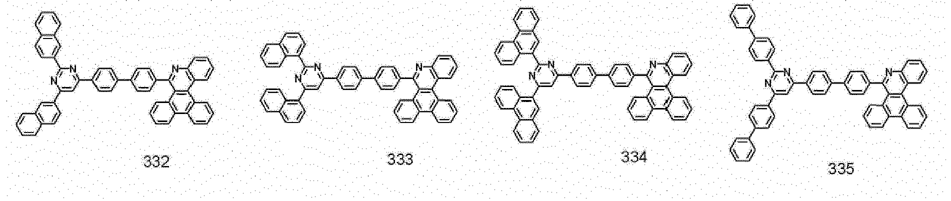

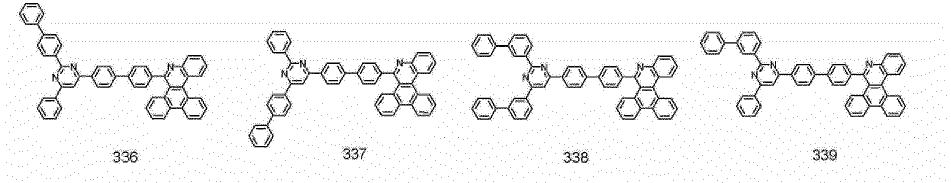

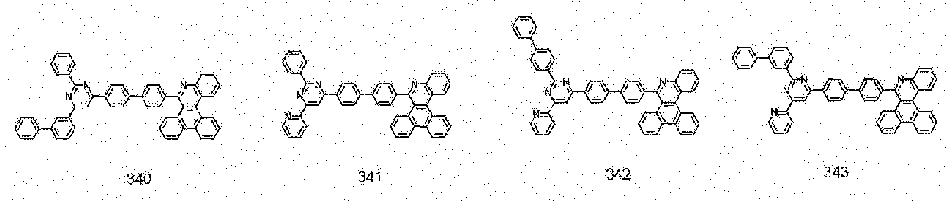

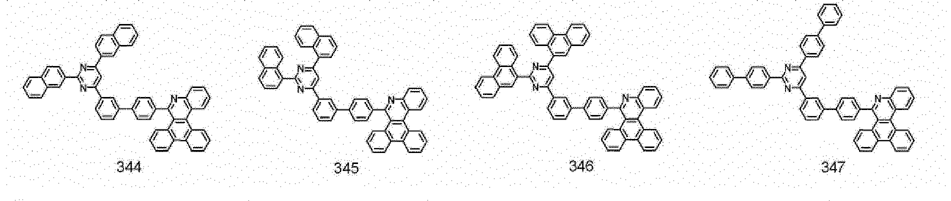

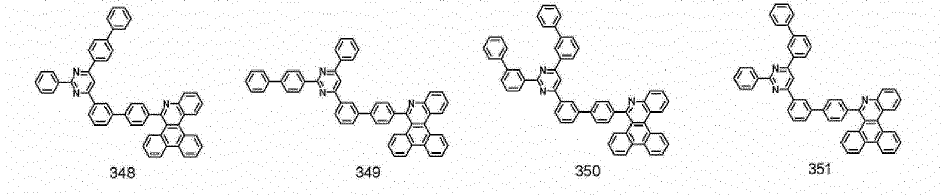

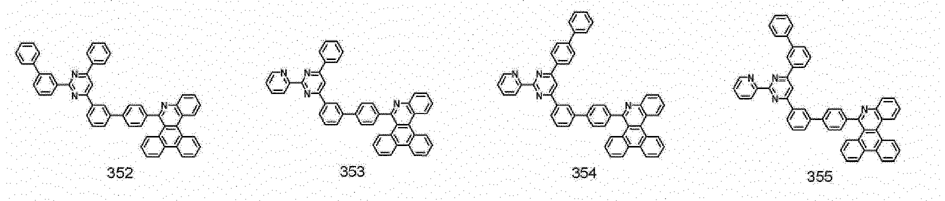

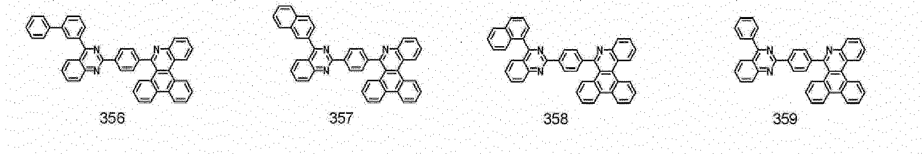

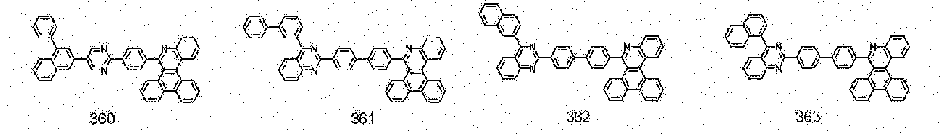

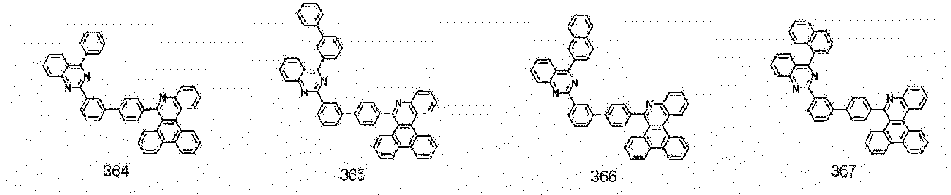

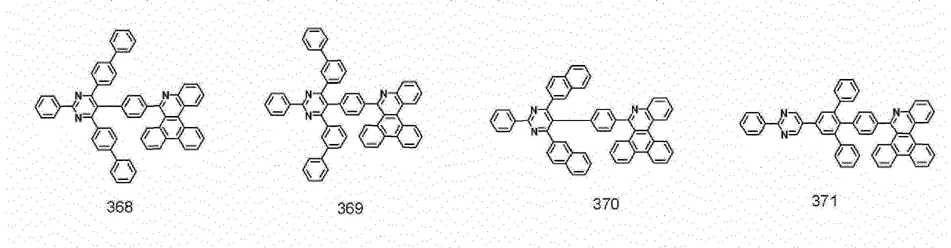

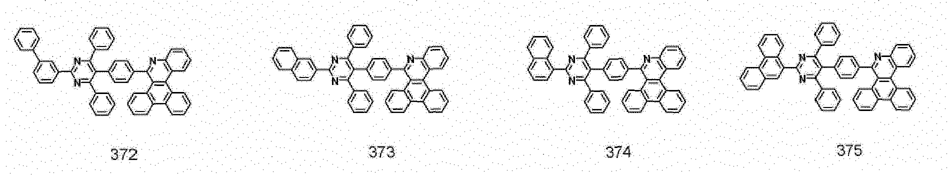

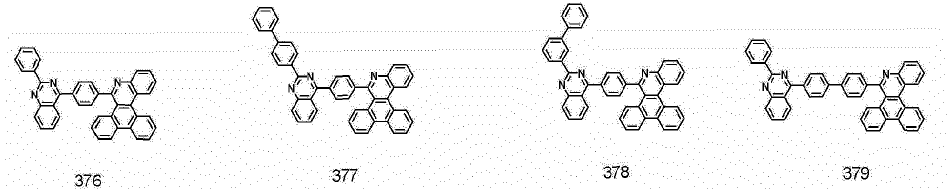

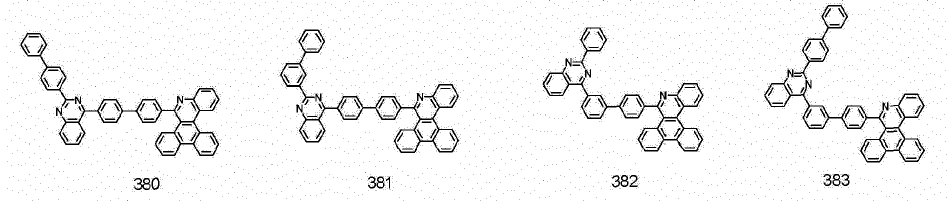

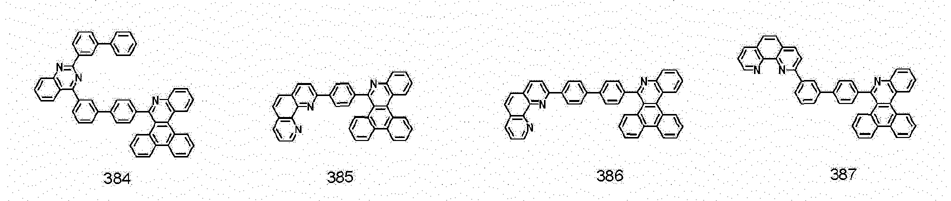

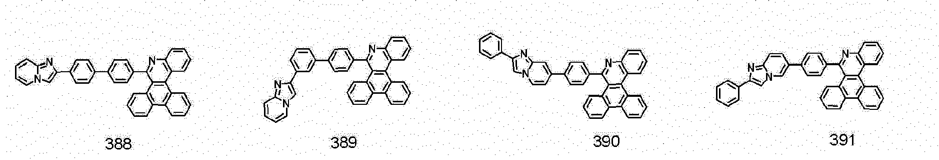

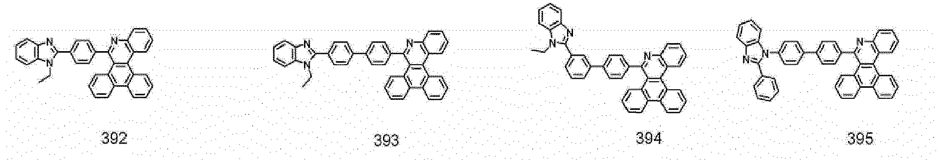

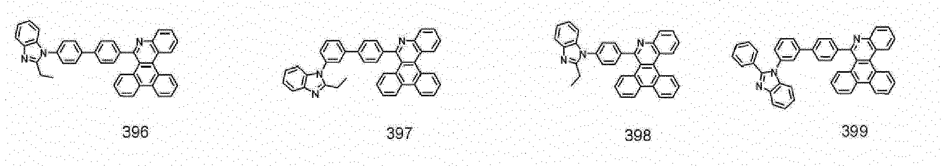

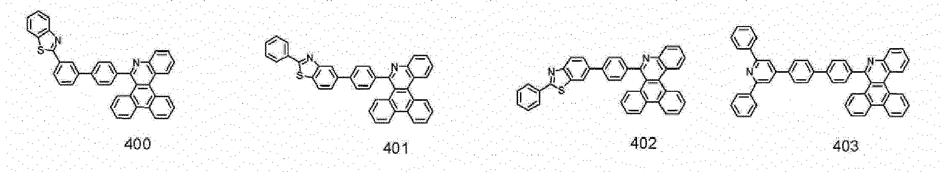

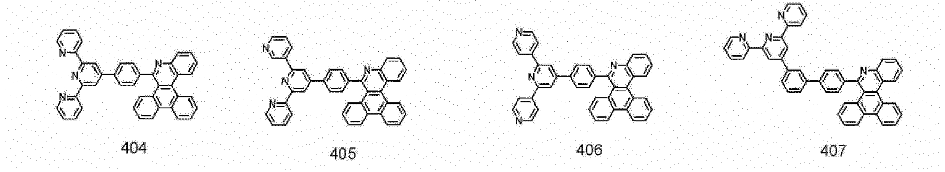

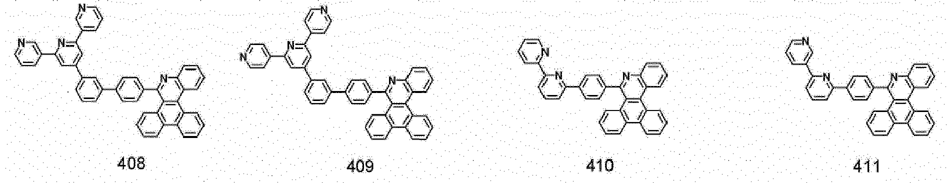

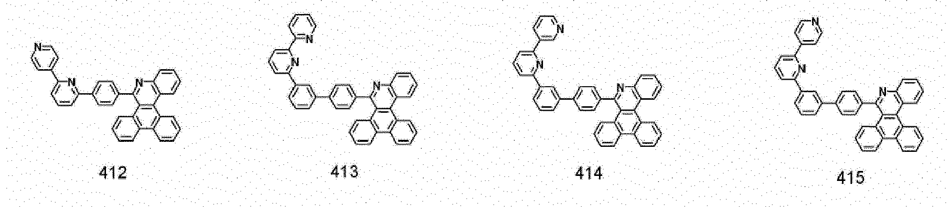

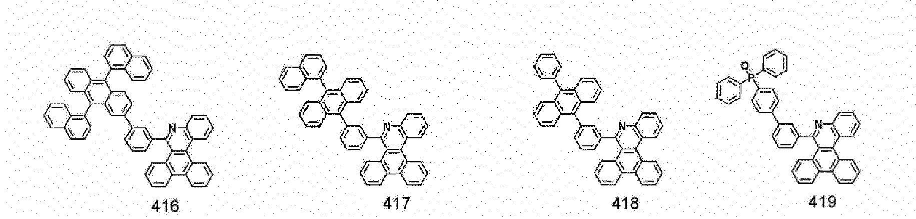

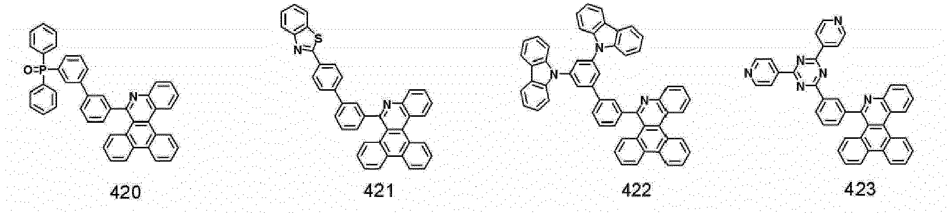

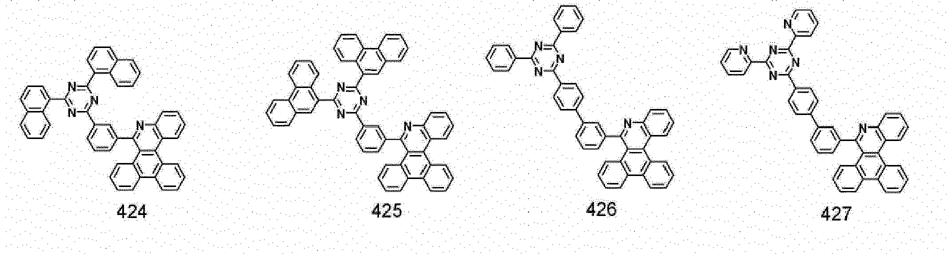

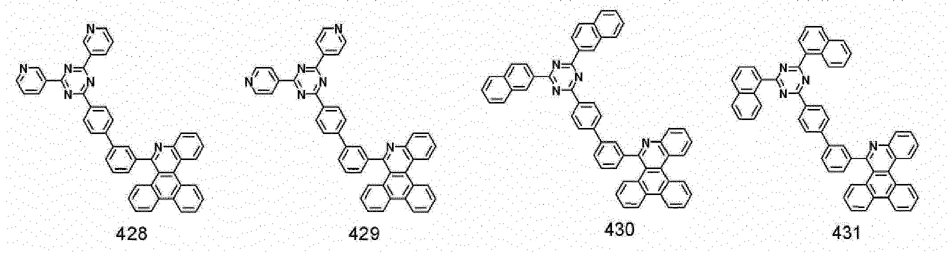

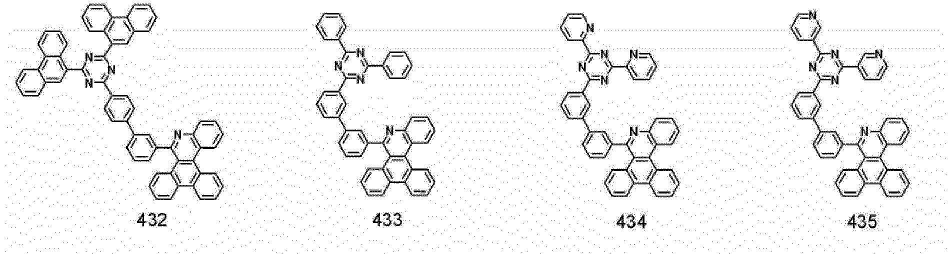

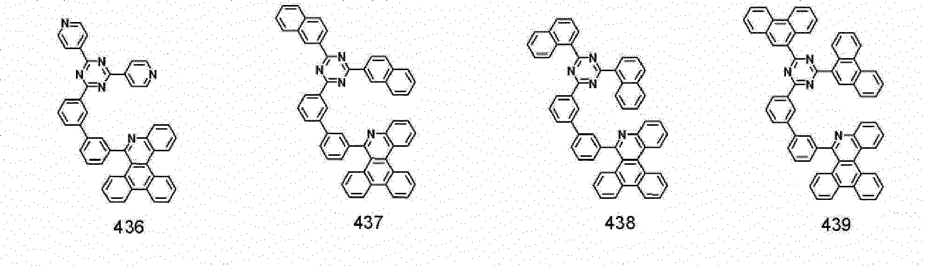

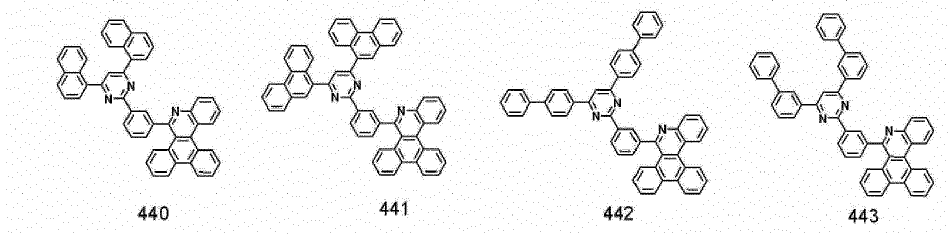

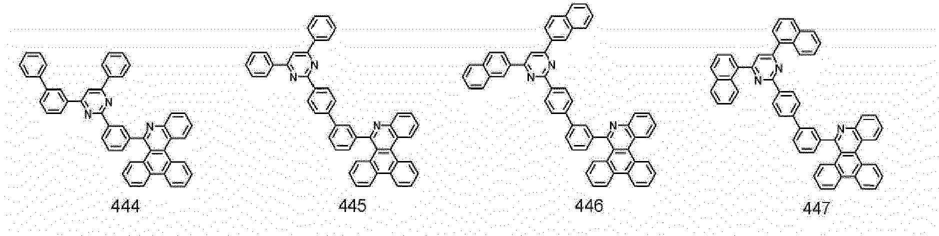

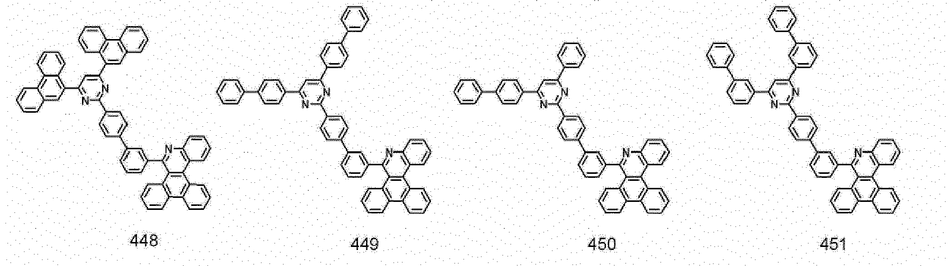

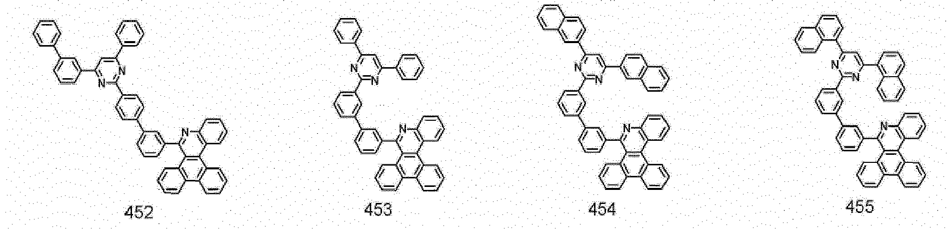

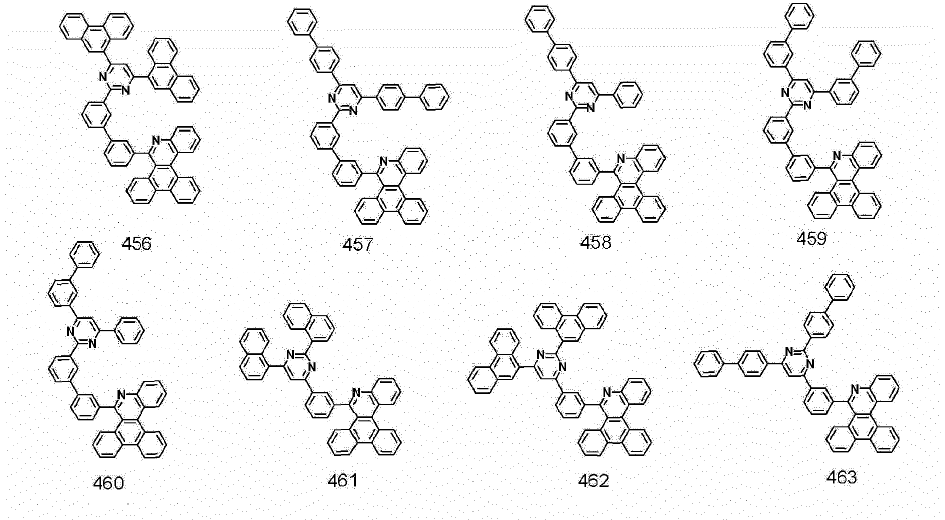

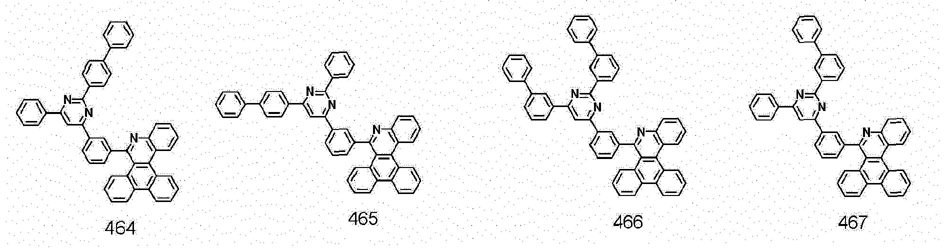

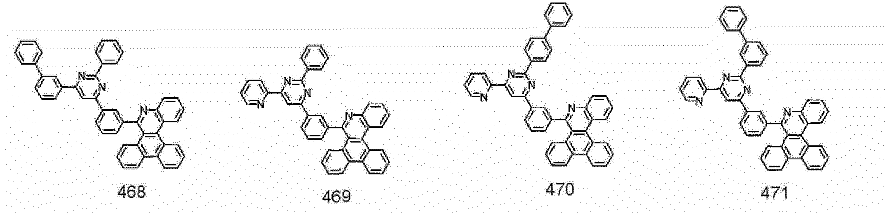

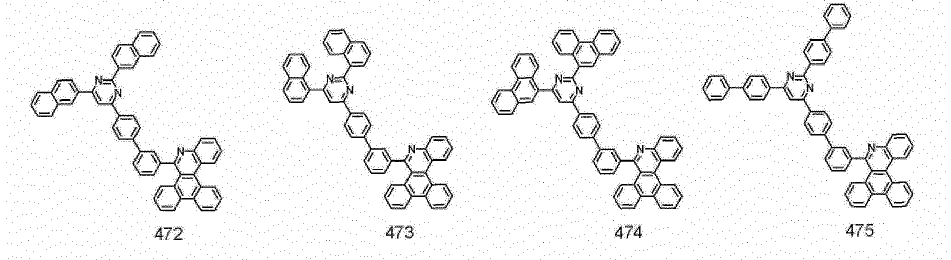

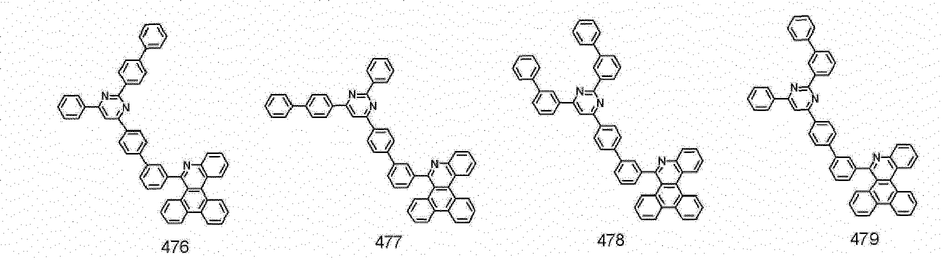

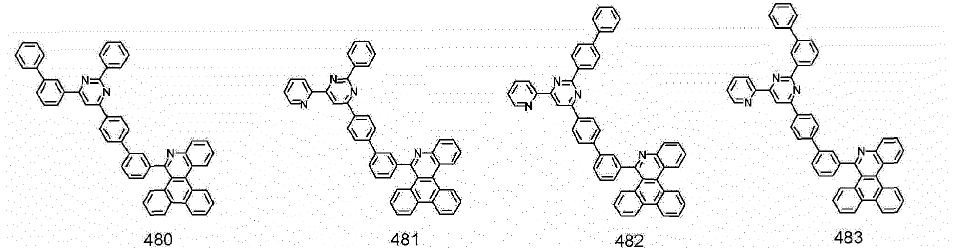

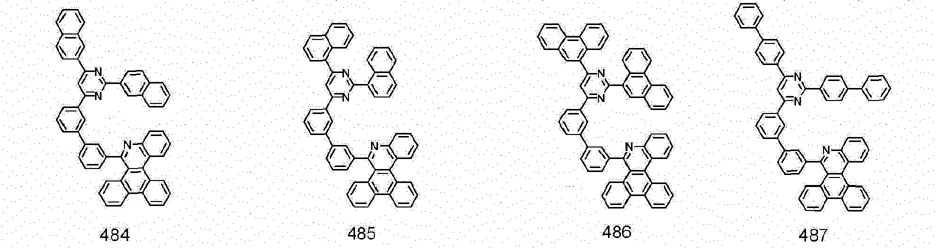

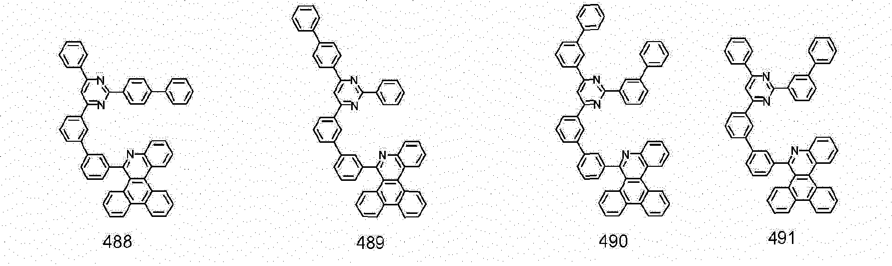

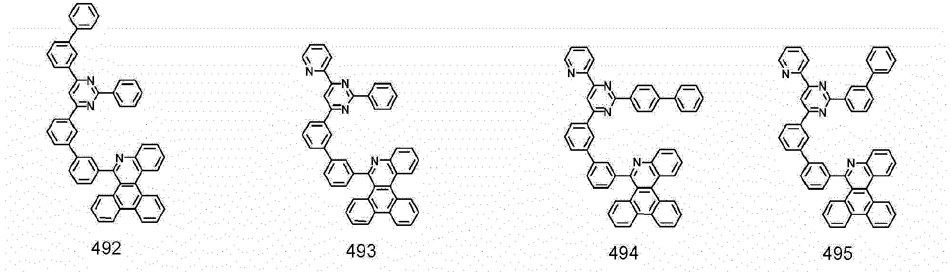

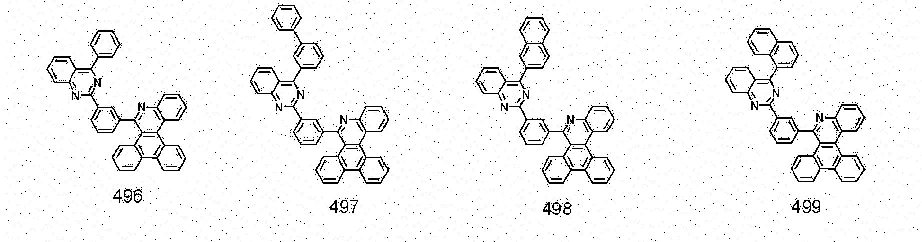

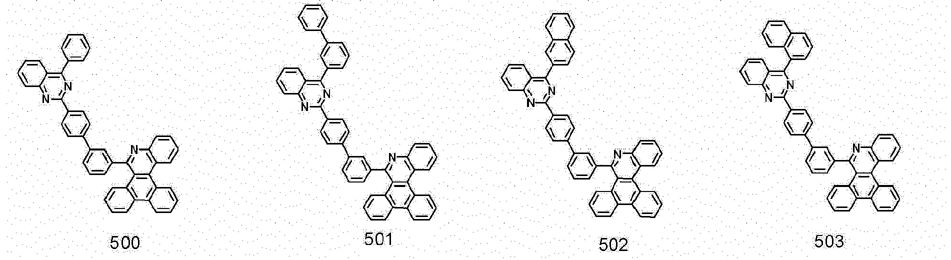

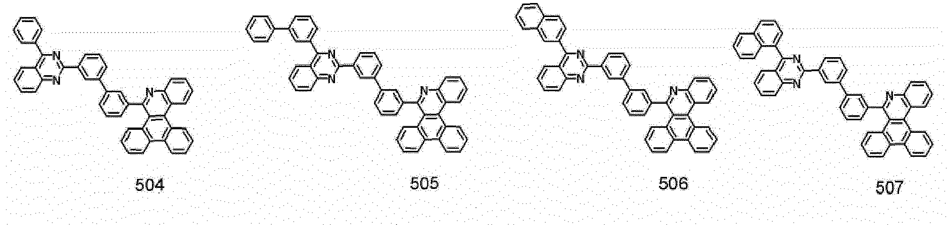

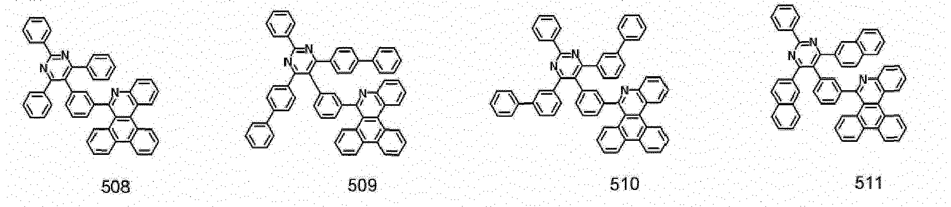

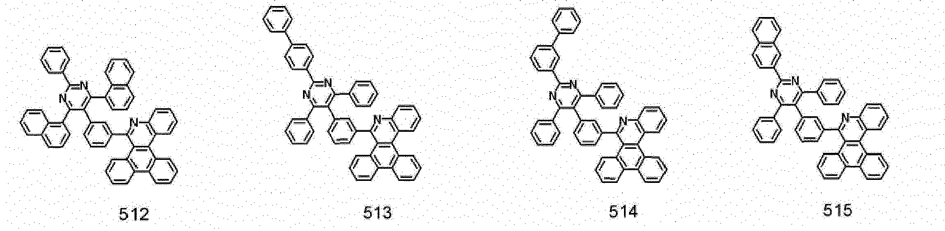

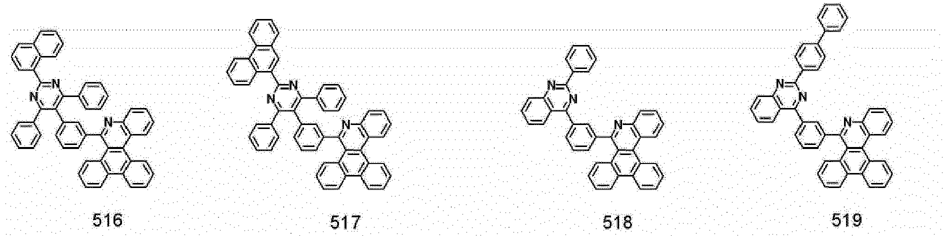

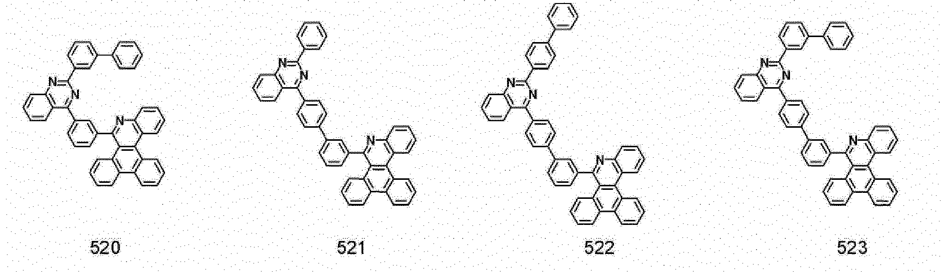

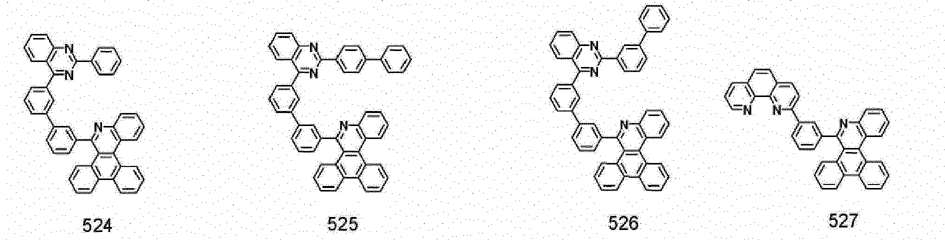

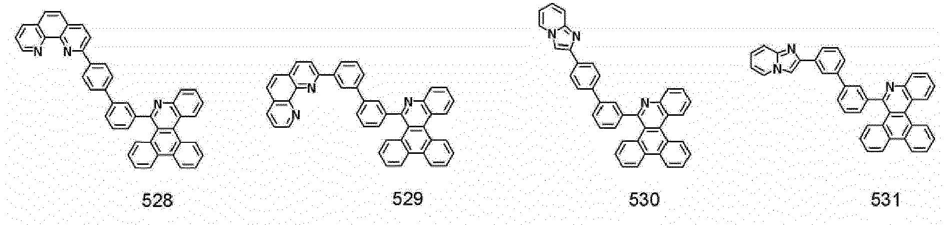

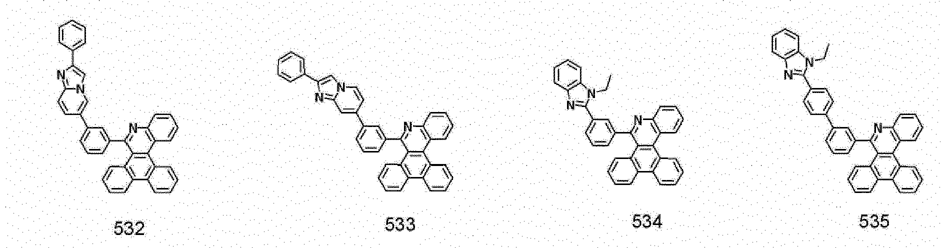

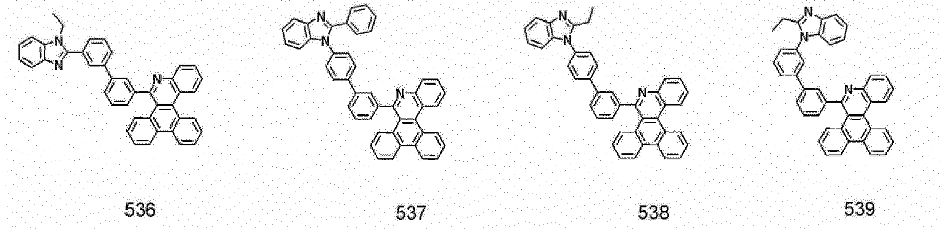

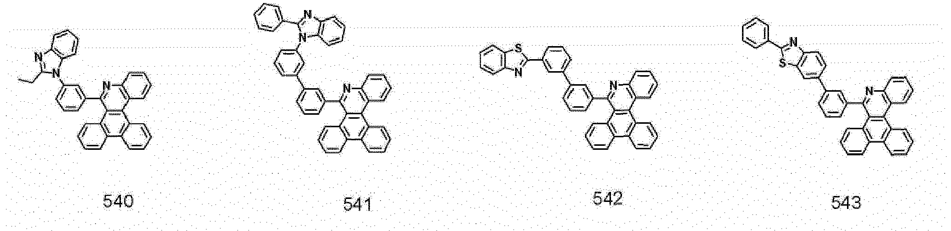

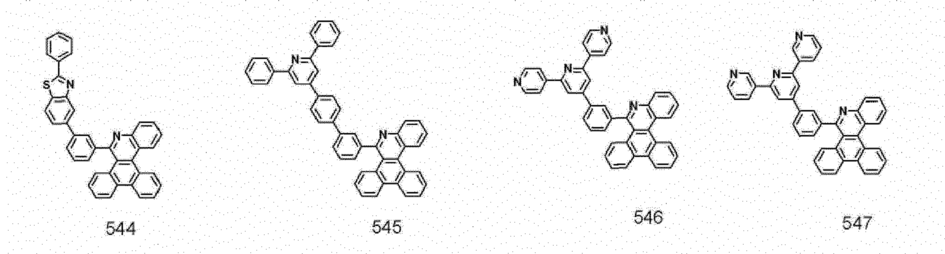

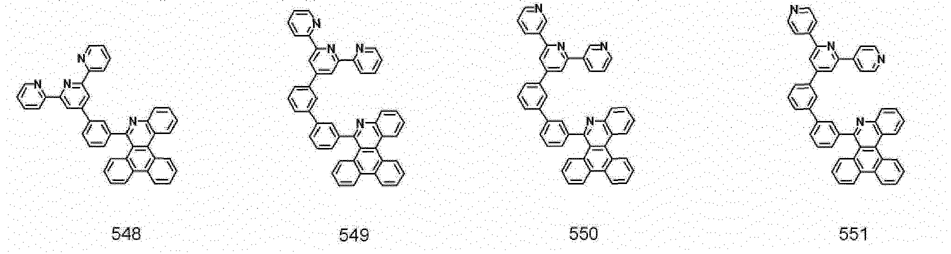

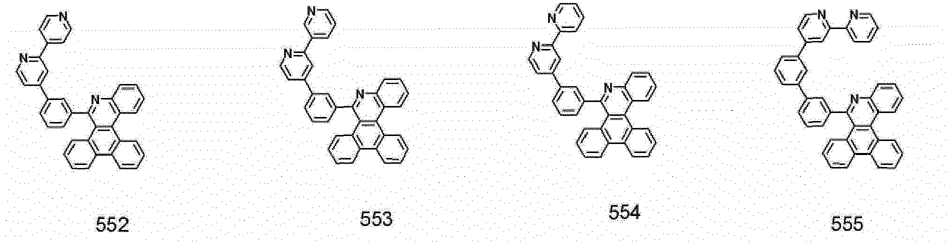

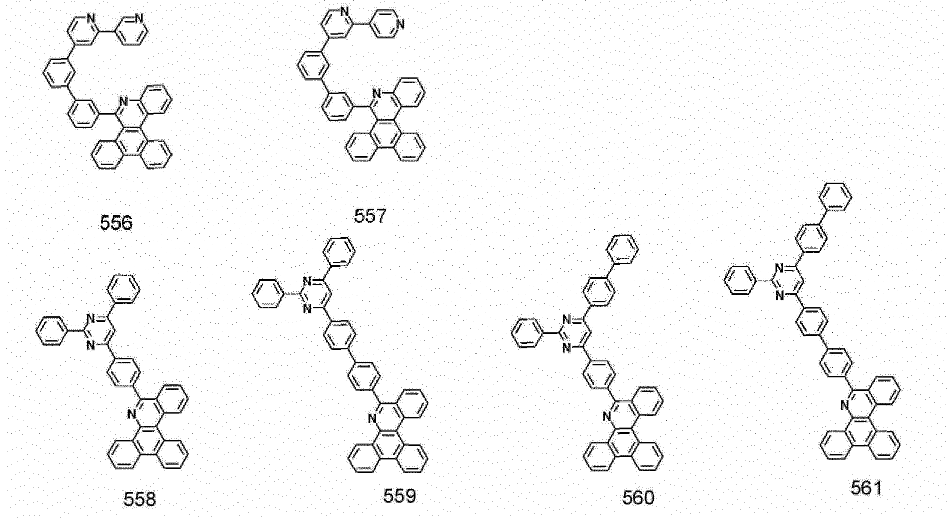

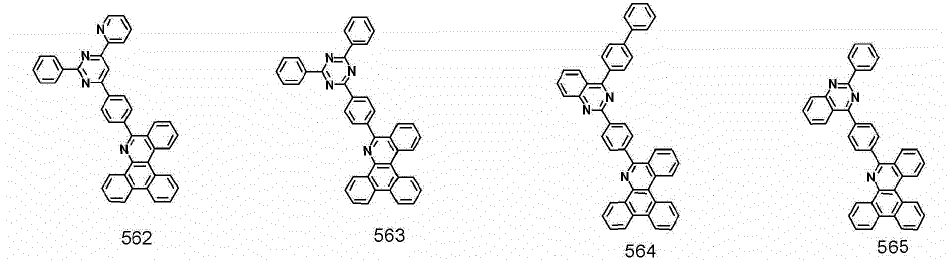

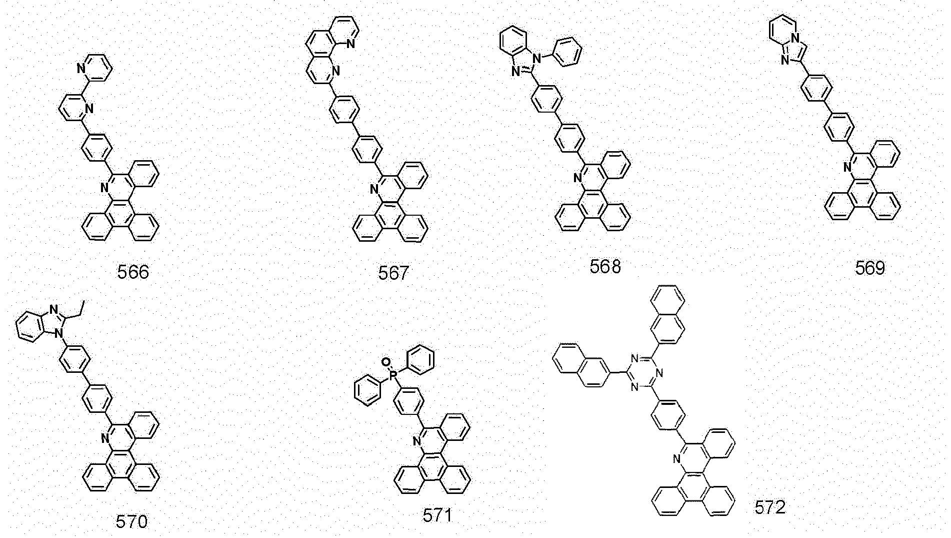

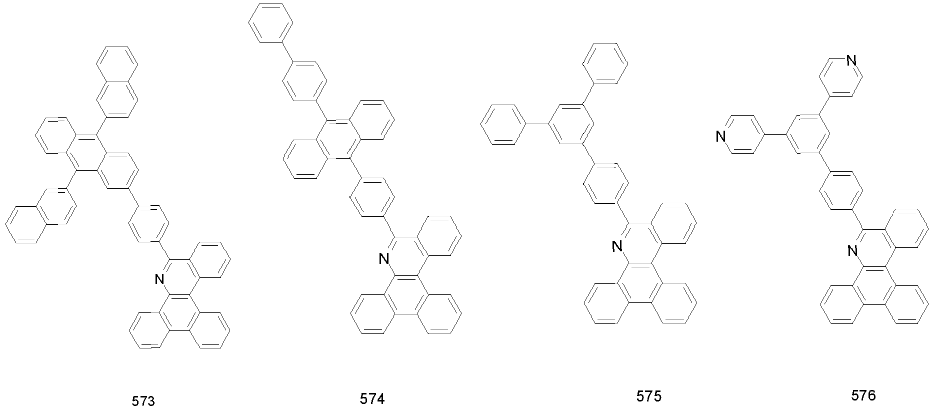

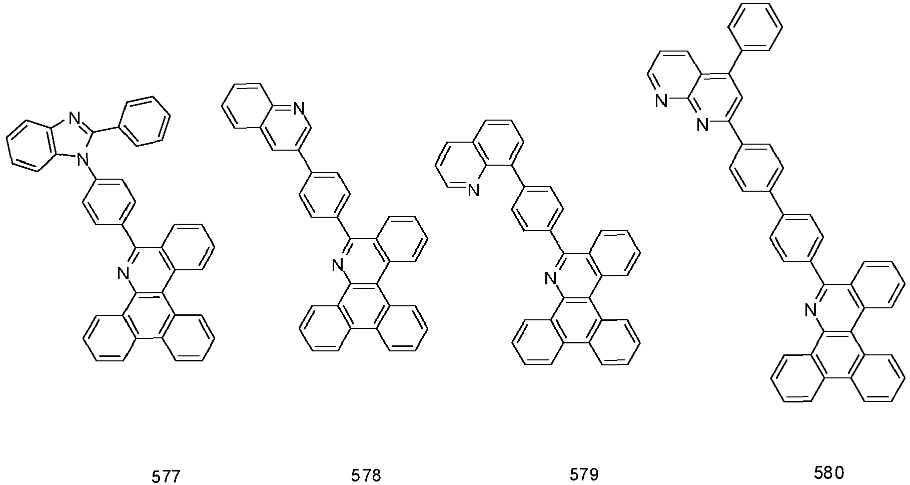

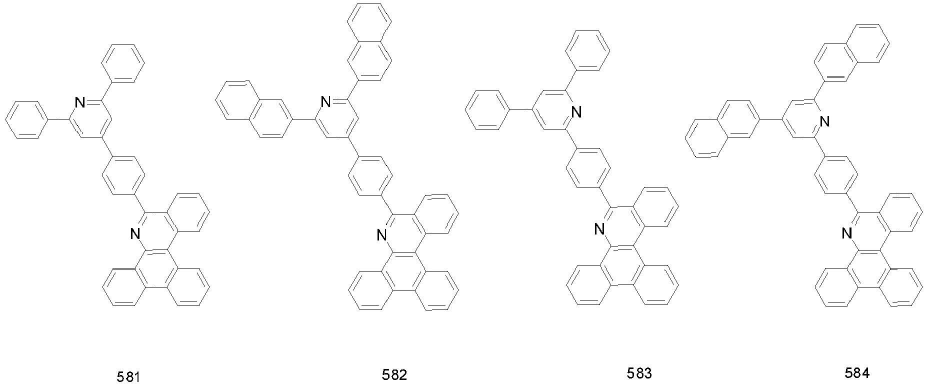

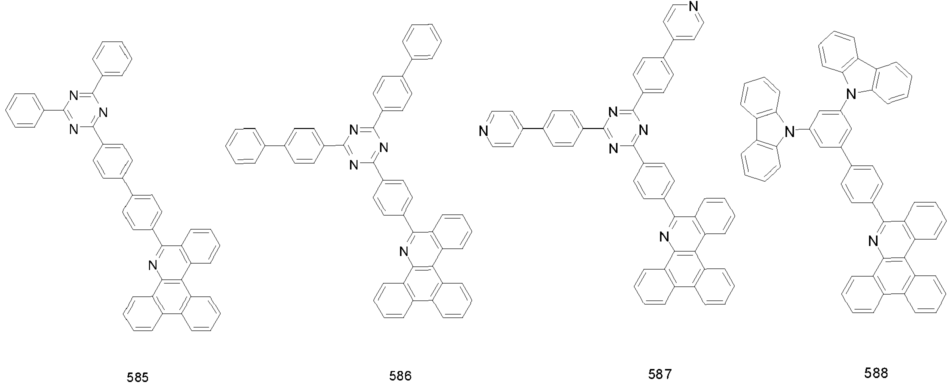

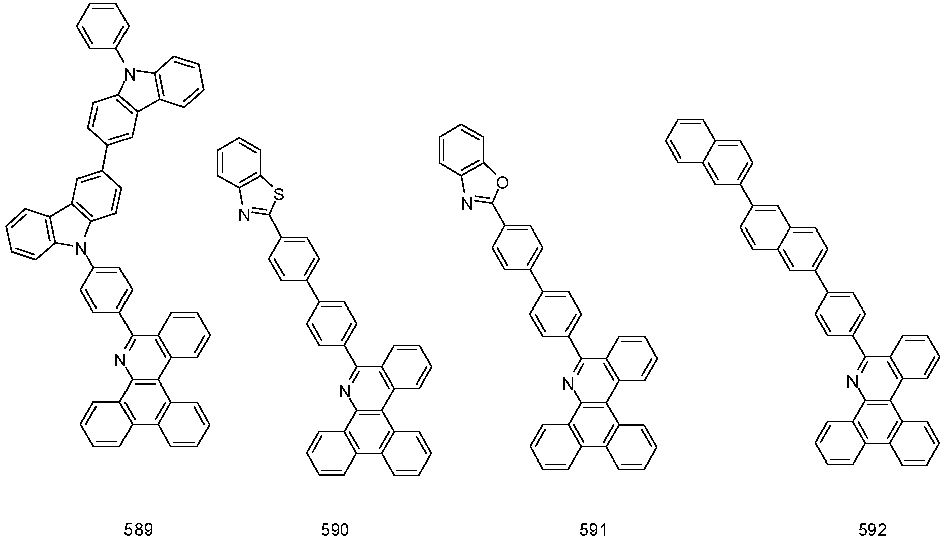

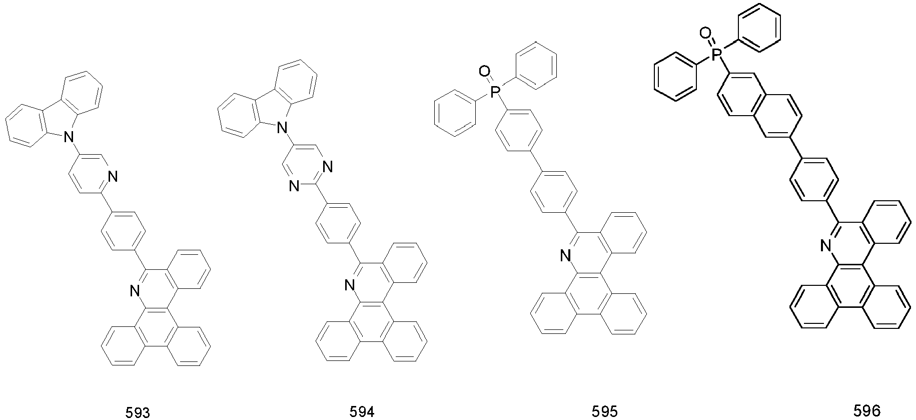

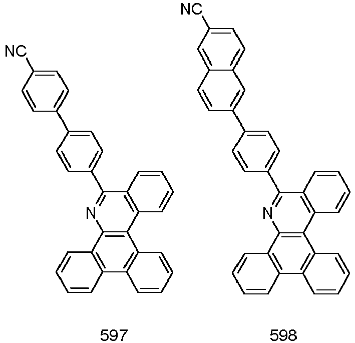

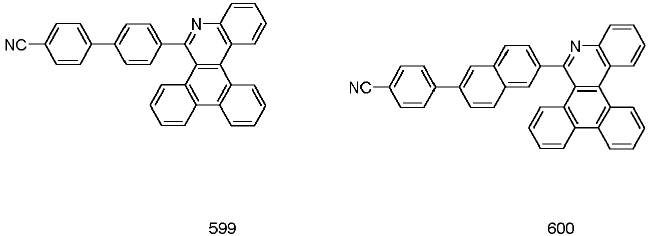

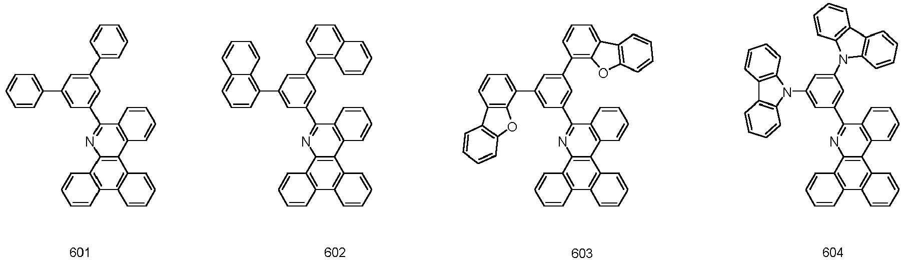

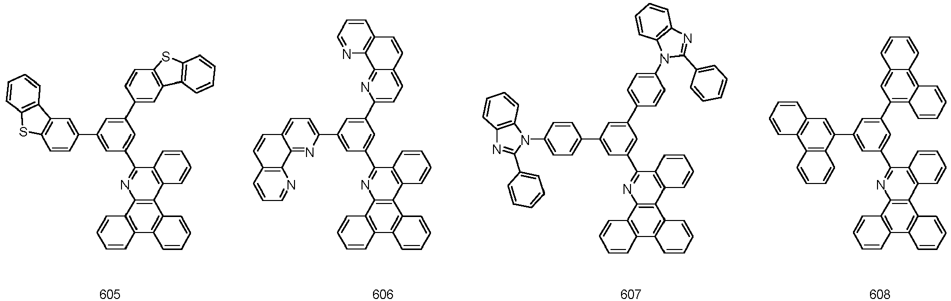

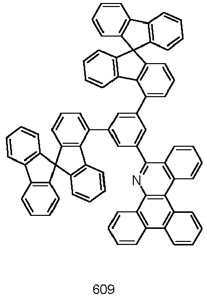

The compounds described above may be prepared based on the preparation examples described below. The following preparation examples are representative examples, but if necessary, substituents may be added or excluded and positions of substituents may be changed. Further, based on technologies known in the art, starting materials, reactants, and reaction conditions may be changed. If necessary, kinds or positions of the substituents at the other positions may be modified by those skilled in the art using technologies known in the art.

For example, the compound of the chemical formula 2 (not according to the invention) may be manufactured into a core structure of the following general formula 1.

A substituent can be bonded by a method known in the art, and a position of a substituent or the number of substitutents can be modified according to technologies known in the art.

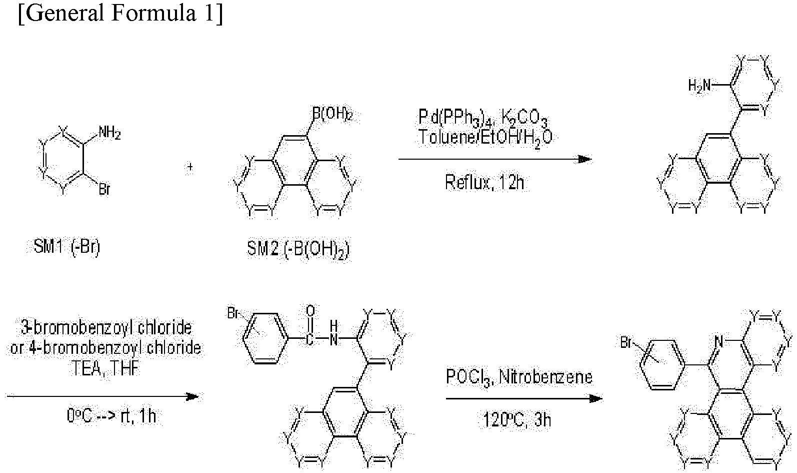

In the general formula 1, Y₁ to Y₁₂ are the same as definedabove.

The general formula 1 is an example of an intermediate reaction for bonding a substituent at a position of R₁ in a core structure of the chemical formula 2. To be specific, the final compound of the general formula 1 is phenyl of which R₁ is substituted with Br in the chemical formula 2. The Br may be changed to another substituent as necessary, and the phenyl can also be changed to another substituent by changing benzoyl chloride as a reactant.

For example, the compound of the chemical formula 3 may be manufactured into a core structure of the following general formula 2.

A substituent can be bonded by a method known in the art, and a position of a substituent or the number of substitutents can be modified according to technologies known in the art.

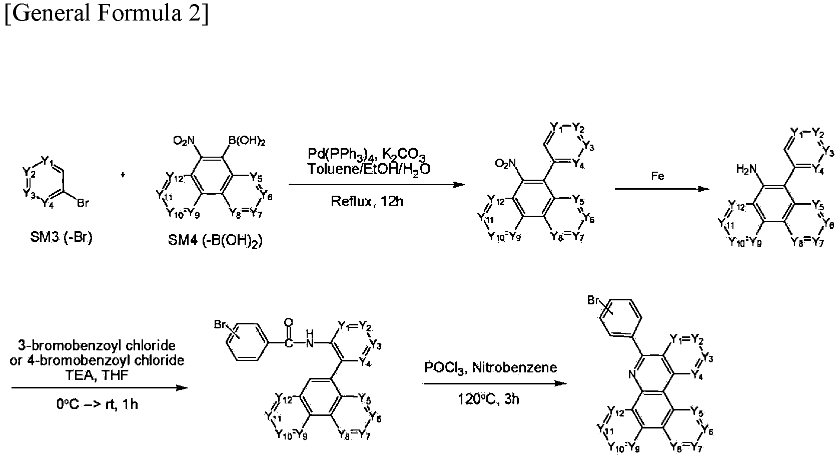

In the general formula 2, Y₁ to Y₁₂ are the same as definedabove.

The general formula 2 is an example of an intermediate reaction for bonding a substituent at a position of R₁ in a core structure of the chemical formula 3. To be specific, the final compound of the general formula 2 is phenyl of which R₁ is substituted with Br in the chemical formula 3. The Br may be changed to another substituent as necessary, and the phenyl can also be changed to another substituent by changing benzoyl chloride as a reactant.

Another exemplary embodiment of the present invention provides an organic light emitting device including the compound of the invention. To be specific, the organic light emitting device includes an anode, a cathode, and one or more organic material layers provided between the anode and the cathode, wherein one or more layers of the organic material layers include the compound of the invention.

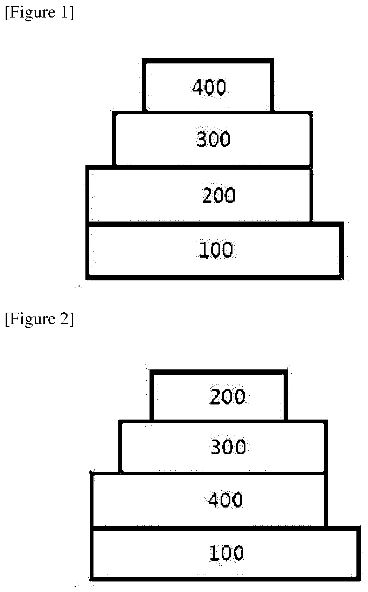

FIGS. 1 to 3 illustrate examples of laminating order of electrodes and organic material layers of an organic light emitting device according to exemplary embodiments of the present invention. However, these drawings are not provided for limiting the scope of the present invention, and the structure of the organic light emitting device known in the art can also be applied to the present invention.

Referring to FIG. 1, an organic light emitting device in which an anode 200, an organic material layer 300, and a cathode 400 are laminated in sequence on a substrate 100 is illustrated by the diagram. However, the structure of the organic light emitting device is not limited to this structure only, and as illustrated in FIG. 2, an organic light emitting device in which a cathode, an organic material layer, and an anode are laminated in sequence on a substrate may also be included.

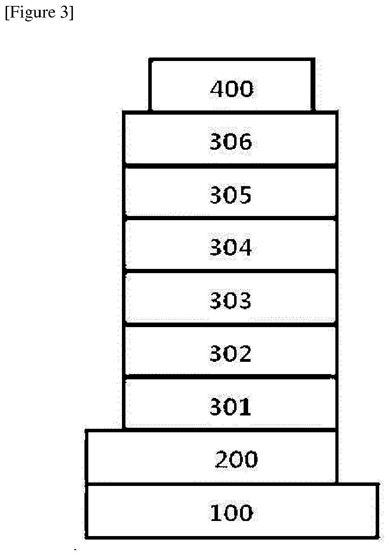

FIG. 3 illustrates the case where the organic material layer is a multilayer. An organic light emitting device illustrated in FIG. 3 includes a hole injection layer 301, a hole transport layer 302, a light emitting layer 303, a hole blocking layer 304, an electron transport layer 305, and an electron injection layer 306. However, the scope of the present invention is not limited to this laminated structure, and when necessary, other layers except the light emitting layer may not be included, and other necessary layers having other functions may be added.

An organic light emitting device according to the present invention may be prepared using materials and methods known in the art except that the compound of the invention is included in one or more layers of the organic material layers.

The compound of the invention may form one or more layers of the organic material layers alone in an organic light emitting device. However, when necessary, the compound of the invention may be mixed with other materials to form the organic material layers.

The compound of the invention may be used as a hole injection material, a hole transport material, a light emitting material, a hole blocking material, an electron transport material, or an electron injection materialin an organic light emitting device.

For example, the compound according to the exemplary embodiment of the present invention may be used as a material of an electron injection layer, an electron transport layer, or a layer that simultaneously injects and transports an electron in an organic light emitting device.

Further, the compound according to the exemplary embodiment of the present invention may be used as a material of a light emitting layer in an organic light emitting device. To be specific, the compound may be used alone as a light emitting material, or may be used as a host material or a dopant material of a light emitting layer.

Furthermore, the compound according to the exemplary embodiment of the present invention may be used as a phosphorescent host material in an organic light emitting device. In this case, the compound according to an exemplary embodiment of the present invention is included together with a phosphorescent dopant.

Moreover, the compound according to the exemplary embodiment of the present invention may be used as a material of a hole blocking layer in an organic light emitting device.

In the organic light emitting device according to the present invention, other materials than the compound of the invention are illustrated below, but they are for illustrative purposes only, and are not intended to limit the scope of the present invention, and can be substituted with materials known in the art.

As the anode material, materials having relatively large work function may be used, and transparent conductive oxides, metals, or conductive polymers may be used.

As the cathode material, materials having relatively small work function may be used, and metals, metal oxides, or conductive polymers may be used.

As the hole injection material, hole injection materials known in the art may be used, and for example, phthalocyanine compounds such as copper phthalocyanine disclosed in U.S. Pat. No. 4,356,429, or starbust-type amine derivatives disclosed in a literature [Advanced Material, 6, p. 677 (1994)], such as TCTA, m-MTDATA, m-MTDAPB, Pani/DBSA (polyaniline/dodecylbenzenesulfonic acid) or PEDOT/PSS (poly(3,4-ethylenedioxythiophene)/poly(4-styrenesulfonate)), Pani/CSA (polyaniline/camphor sulfonic acid) or PANI/PSS (polyaniline/poly(4-styrene-sulfonate), which is a conductive polymer having solubilitymay be used.

As the hole transport material, a pyrazoline derivative, an arylamine-based derivative, a stilbene derivative, a triphenyldiamine derivative, or the like may be used, and a low molecular or high molecular material may also be used.

As the electron transport material, an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, 8-hydroxyquinoline and a metal complex of a derivative thereofmay be used, and a high molecular material as well as a low molecular material may also be used.

As the electron injection material, for example, LiF is typically used in the related industry. However, the present invention is not limited thereto.

As the light emitting material, a red, green, or blue light emitting material may be used, and when necessary, two or more light emitting materials may be mixed and used. Further, as the light emitting material, a fluorescent material may be used and a phosphorescent material may also be used. As the light emitting material, materials that emit light alone by bonding the holes and the electrons injected from an anode and a cathode, respectively, may be used. However, materials in which a host material and a dopant material are both involved in light emitting may also be used.

If the compound according to the present invention is used as a phosphorescent host material, a phosphorescent dopant material to be used together may employ those known in the art.

For example, phosphorescent dopant materials as expressed by LL'MX, LL'L"M, LMXX', L₂MX, and L₃M may be used, but the present invention is not limited thereto.

Herein, L, L', L", X, and X' are not equivalent, bidentate ligands, and M is a metal that forms octahedral complexes.

M may be iridium, platinum, or osmium.

L is an anionic bidentate ligand which coordinates to M via an sp² hybridized carbon and a heteroatom, and X functions to trap electrons or holes. Non-limiting examples of the L may include 2-(1-naphthyl)benzoxazole, (2-phenylbenzoxazole), (2-phenylbenzothiazole), (2- phenylbenzothiazole), (7,8-benzoquinoline), (thienylpyridine), phenylpyridine, benzothienylpyridine, 3-methoxy-2-phenylpyridine, thienylpyridine, and tolylpyridine. Non-limiting examples of the X may include acetylacetonate (acac), hexafluoroacetylacetonate, salicylidene, picolinate, and 8-hydroxyquinolinate.

More specific examples will be described below, but the present invention is not limited thereto.

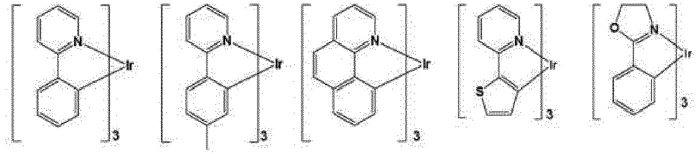

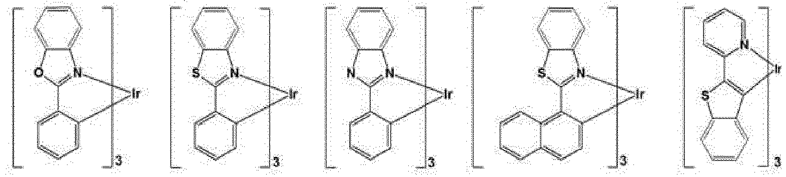

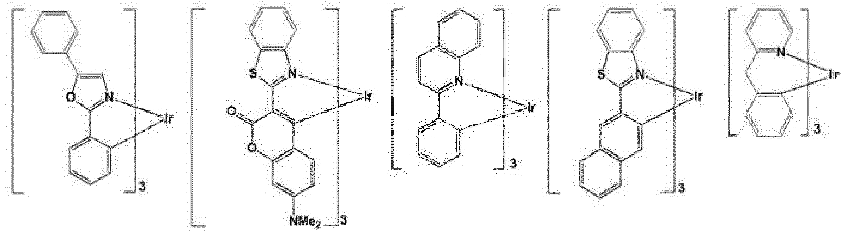

Hereinafter, the present invention will be described in more detail with reference to examples, however, it is to be understood that these are for illustrative purposes only, and are not intended to limit the scope of the present invention.

### [Preparation Example 1] Preparation of Compound 2 (not according to the invention)

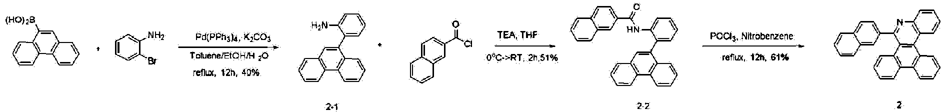

### Preparation of Compound 2-1

A mixed solution of 50.6 g (0.294 mol) of 2-bromoaniline, 71.8 g (0.324 mol) of phenanthren-9-yl boronic acid, K₂CO₃ (121.7 g, 0.882 mmol), Pd(PPh₃)₄ (17.0 g, 0.0147 mol), and toluene, EtOH, H₂O (500 ml/200 ml/100 ml) was put into a two neck round bottom flask (two neck r.b.f) and refluxed and stirred therein under nitrogen for 12 hours. A reaction mixture cooled to room temperature was extracted with MC (methylene chloride)/H₂O and dried using MgSO₄ and then filtered. After being concentrated, the resultant reaction product was separated by column chromatography (SiO₂, hexane : MC (methylene chloride) = 2 : 1). As a result, an ivory solid compound 2-1 (32 g, 40%) was obtained.

### Preparation of Compound 2-2

Compound 2-1 (10.0 g, 0.0371 mol) was dissolved in 100 ml of THF (tetrahydrofuran) within a one neck round bottom flask (one neck r.b.f) under nitrogen, and triethylamine (15.4 ml, 0.111 mol) was added dropwise thereto at room temperature. After the reaction mixture was cooled to 0°C, 2-naphthoyl chloride was dissolved in 50 ml of THF and slowly added dropwise thereto at a low temperature. Then, the resultant reaction product was stirred at room temperature for 2 hours and added with H₂O to terminate the reaction. The solvent was distilled under reduced pressure, and then MC (methylene chloride)/H₂O was extracted. The resultant reaction product was dried using MgSO₄ and then filtered. After distillation under reduced pressure and concentration, the resultant reaction product was dissolved in a small amount of MC (methylene chloride) and added with methanol to precipitate a solid. As a result, an ivory solid compound 2-2 (8.01 g, 51%) was obtained.

### Preparation of Compound 2

Compound 2-2 (8.01 g, 0.0189 mol) was dissolved in nitrobenzene (80 ml) within a one neck round bottom flask (one neck r.b.f), and POCl₃ (phosphorous oxychloride) (0.35 ml, 3.78 mmol) was added dropwise thereto at room temperature and then refluxed and stirred for 12 hours. After the reaction was terminated, the mixed solution was cooled to room temperature and neutralized with NaHCO₃ aqueous solution. Then, MC (methylene chloride)/H₂O was extracted. The resultant reaction product was dried using MgSO₄ and then filtered. After the solvent was distilled under reduced pressure and concentrated, a small amount of MC (methylene chloride) and an excessive amount of hexane were added to precipitate a solid. As a result, a yellow solid compound 2 (4.67 g, 61%) was obtained.

### [Preparation Example 2] Preparation of Compound 18

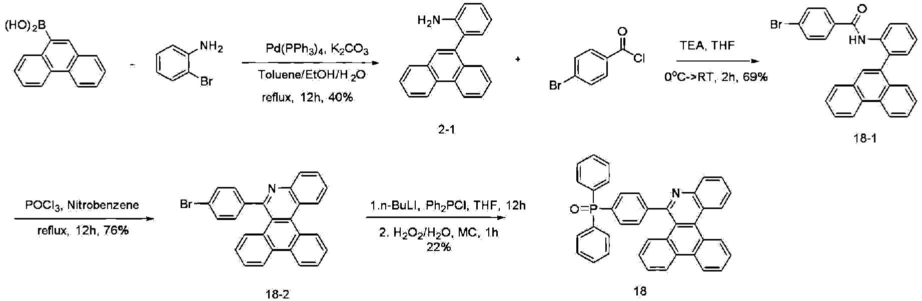

### Preparation of Compound 18-1

Compound 2-1 (32 g, 0.119 mol) was dissolved in THF (200 ml) within a one neck round bottom flask (one neck r.b.f) under nitrogen, and triethylamine (49.4 ml, 0.356 mol) was added dropwise thereto at room temperature. After the reaction mixture was cooled to 0°C, 39.2 g (0.179 mol) of 4-bromobenzoyl chloride was dissolved in 50 ml of THF and slowly added dropwise thereto at a low temperature. Then, the resultant reaction product was stirred at room temperature for 2 hours and added with H₂O to terminate the reaction. The solvent was distilled under reduced pressure, and then MC (methylene chloride)/H₂O was extracted. The resultant reaction product was dried using MgSO₄ and then filtered. After distillation under reduced pressure and concentration, the resultant reaction product was dissolved in a small amount of MC (methylene chloride) and added with methanol to precipitate a solid. As a result, an ivory solid compound 18-1 (37.1 g, 69%) was obtained.

### Preparation of Compound 18-2

Compound 18-1 (20g, 0.0442 mol) was dissolved in nitrobenzene (150 ml) within a one neck round bottom flask (one neck r.b.f), and POCl₃ (phosphorous oxychloride) (0.82 ml, 8.84 mmol) was added dropwise thereto at room temperature and then refluxed and stirred for 12 hours. After the reaction was terminated, the mixed solution was cooled to room temperature and neutralized with NaHCO₃ aqueous solution. Then, MC (methylene chloride)/H₂O was extracted. The resultant reaction product was dried using MgSO₄ and then filtered. After the solvent was distilled under reduced pressure and concentrated, a small amount of MC (methylene chloride) and an excessive amount of hexane were added to precipitate a solid. As a result, a pale yellow solid compound 18-2 (14.5 g, 76%) was obtained.

### Preparation of Compound 18

Compound 18-2 (10 g, 0.023 mol) was dissolved in anhydrous THF (50 ml) within a one neck round bottom flask (one neck r.b.f) under nitrogen and then cooled to -78°C. Then, n-butyllithium (2.5 M in hexane) (12 ml, 0.0299 mol) was slowly added dropwise thereto and stirred for 1 hour. 5.37 ml (0.0299 mol) of chlorodiphenylphosphine was added dropwise to the above solution and stirred at room temperature for 12 hours. The reaction mixture was extracted with MC (methylene chloride)/H₂O and distilled under reduced pressure. The reaction mixture was dissolved in 250 ml of MC (methylene chloride) and then stirred with 20 ml of 30% H₂O₂ aqueous solution at room temperature for 1 hour. The reaction mixture was extracted with MC (methylene chloride)/H₂O, and then the concentrated mixture was separated by column chromatography (SiO₂, MC (methylene chloride): methanol = 25 : 1). As a result, a yellow solid compound 18 (2.81 g, 22%) was obtained.

### [Preparation Example 3] Preparation of Compound 22

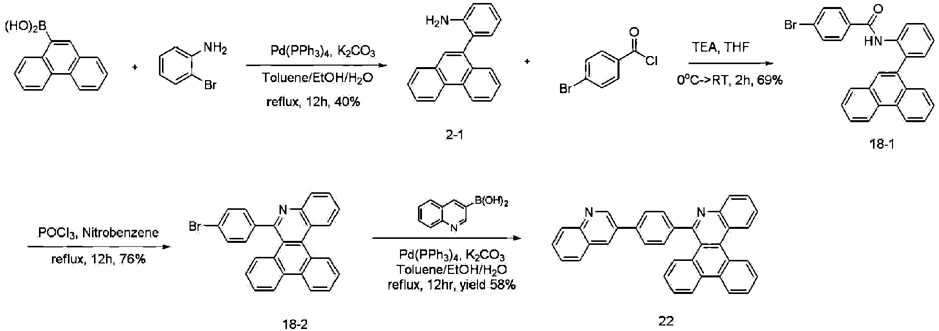

### Preparation of Compound 22

A mixed solution of Compound 18-2 (10.0 g, 0.023 mol), 4.38 g (0.0253 mol) of quinolin-3-yl boronic acid, K₂CO₃ (9.52 g, 0.069 mmol), Pd(PPh₃)₄ (1.33 g, 1.15 mmol), and toluene, EtOH, H₂O (200 ml/30 ml/30 ml) was put into a two neck round bottom flask (two neck r.b.f) and refluxed and stirred therein under nitrogen for 12 hours. A reaction mixture cooled to room temperature was extracted with MC (methylene chloride)/H₂O and dried using MgSO₄ and then filtered. After being concentrated, the resultant reaction product was separated by column chromatography (SiO₂, hexane : ethyl acetate = 2 : 1). As a result, a yellow solid compound 22 (6.4 g, 58%) was obtained.

### [Preparation Example 4] Preparation of Compound 37

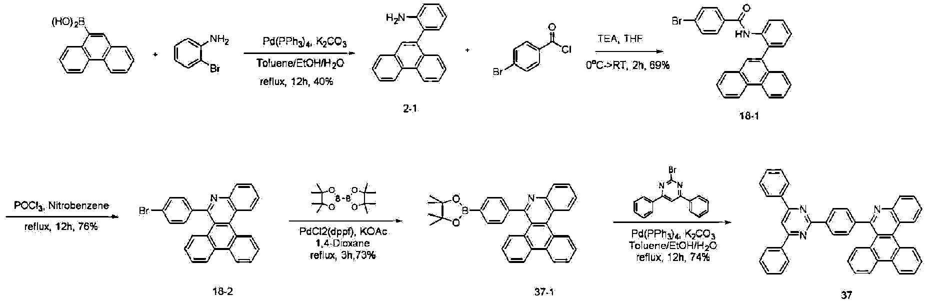

### Preparation of Compound 37-1

A mixed solution of Compound 18-2 (13.6 g, 0.0313 mol), 10.3 g (0.0407 mol) of bis(pinacolato)diboron, 9.21 g (0.0939 mol) of KOAc(potassium acetate), 1.14 g (1.56 mmol) of PdCl₂(dppf), and 250 ml of 1,4-dioxane was put into a two neck round bottom flask (two neck r.b.f) and refluxed and stirred therein under nitrogen for 3 hours. A reaction mixture cooled to room temperature was extracted with MC (methylene chloride)/H₂O and dried using MgSO₄ and then filtered. After being concentrated, a solid is produced with hexane and filtered. As a result, an ivory solid compound 37-1 (11.2 g, 73%) was obtained.

### Preparation of Compound 37

A mixed solution of Compound 37-1 (10.0 g, 0.021 mol), 7.11 g (0.023 mol) of 2-bromo-4,6-diphenylpyrimidine, K₂CO₃ (8.69 g, 0.063 mmol), Pd(PPh₃)₄ (1.21 g, 1.05 mmol), and toluene, EtOH, H₂O (100 ml/20 ml/20 ml) was put into a two neck round bottom flask (two neck r.b.f) and refluxed and stirred therein under nitrogen for 12 hours. The solvent of the reaction mixture was filtered and the resultant solid was washed with 200 ml of toluene, 300 ml of hexane, and 300 ml of methanol in sequence. As a result, a white solid compound 37 (9.1 g, 74%) was obtained.

### [Preparation Example 5] Preparation of Compound 63

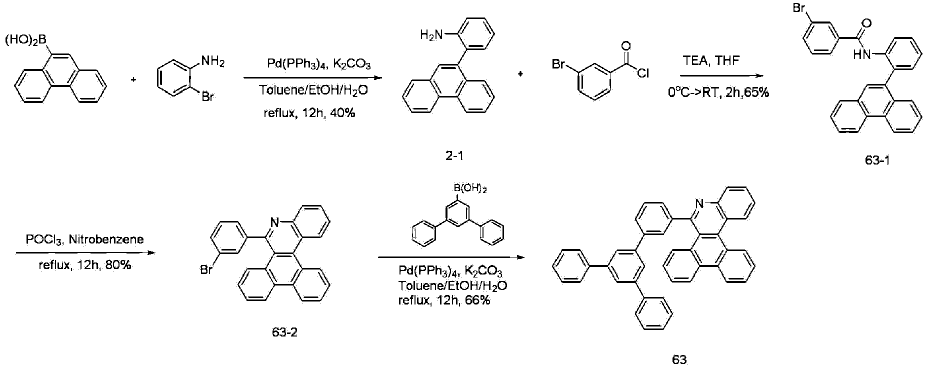

### Preparation of Compound 63-1

Compound 2-1 (32 g, 0.119 mol) was dissolved in 200 ml of THF within a one neck round bottom flask (one neck r.b.f) and then triethylamine (49.4 ml, 0.356 mol) was added dropwise thereto at room temperature. The reaction mixture was cooled to 0°C, and 3-bromobenzoyl chloride dissolved in 50 ml of THF was slowly added dropwise thereto. Then, after stirring at room temperature for 2 hours, H₂O was added to terminate the reaction. The solvent was distilled under reduced pressure, and MC (methylene chloride)/H₂O was extracted and dried using MgSO₄ and then filtered. After distillation under reduced pressure and concentration, the resultant reaction product was dissolved in a small amount of MC (methylene chloride) and added with methanol to precipitate a solid. As a result, an ivory solid compound 18-1 (35.0g, 65%) was obtained.

### Preparation of Compound 63-2

Compound 63-1 (35.0 g, 0.077 mol) was dissolved in 200 ml of nitrobenzene within a two neck round bottom flask (two neck r.b.f) and then 1.4 ml (0.015 mol) of POCl₃ (phosphorous oxychloride) was added dropwise thereto at room temperature and refluxed and stirred therein for 12 hours. After the reaction was terminated, the mixed solution was cooled to room temperature and neutralized with NaHCO₃ aqueous solution. Then, MC (methylene chloride)/H₂O was extracted. The resultant reaction product was dried using MgSO₄ and then filtered. After the solvent was distilled under reduced pressure and concentrated, a small amount of MC (methylene chloride) and an excessive amount of hexane were added to precipitate a solid. As a result, a pale yellow solid compound 63-2 (26.7 g, 80%) was obtained.

### Preparation of Compound 63

A mixed solution of Compound 63-2 (10.0 g, 0.023 mol), 6.94 g (0.0253 mol) of(3, 5-diphenylphenyl)boronic acid, K₂CO₃ (9.52 g, 0.069 mol), Pd(PPh₃)₄ (1.32 g, 1.15 mol), and toluene, ethanol, H₂O (200 ml/30 ml/30 ml) was put into a two neck round bottom flask (two neck r.b.f) and refluxed and stirred therein under nitrogen for 12 hours. A reaction mixture cooled to room temperature was extracted with MC (methylene chloride)/H₂O and dried using MgSO₄ and then filtered. After being concentrated, the resultant reaction product was separated by column chromatography (SiO₂, hexane : MC (methylene chloride) = 2 : 1). As a result, a white solid compound 63 (8.86 g, 66%) was obtained.

### [Preparation Example 6] Preparation of Compound 73

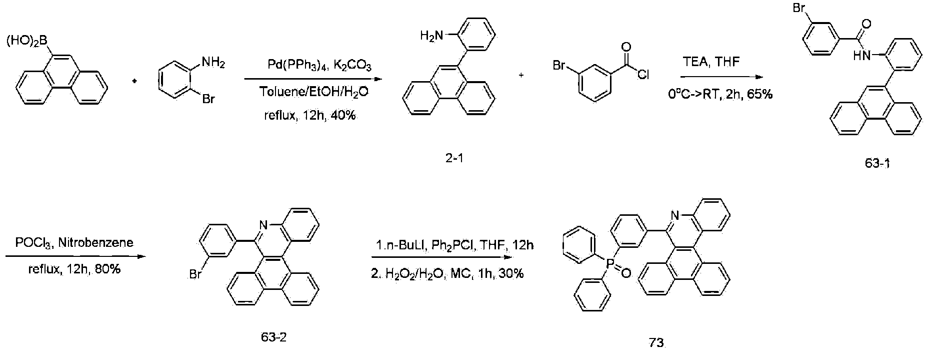

### Preparation of Compound 73

Compound 63-2 (10.0 g, 0.023 mol) was dissolved in anhydrous THF (50 ml) within a one neck round bottom flask (one neck r.b.f) under nitrogen and then cooled to -78°C. Then, 12.0 ml (0.0299 mol) of n-butyllithium (2.5 M in hexane) was slowly added dropwise thereto and stirred for 1 hour. 5.37 ml (0.0299 mol) of chlorodiphenylphosphine was added dropwise to the above solution and stirred at room temperature for 12 hours. The reaction mixture was extracted with MC (methylene chloride)/H₂O and distilled under reduced pressure. The reaction mixture was dissolved in 50 ml of MC (methylene chloride) and then stirred with 20 ml of 30% H₂O₂ aqueous solution at room temperature for 1 hour. The reaction mixture was extracted with MC (methylene chloride)/H₂O, and then the concentrated mixture was separated by column chromatography (SiO₂, MC (methylene chloride): methanol = 25 : 1). As a result, a yellow solid compound 73 (3.83 g, 30%) was obtained.

### [Preparation Example 7] Preparation of Compound 92

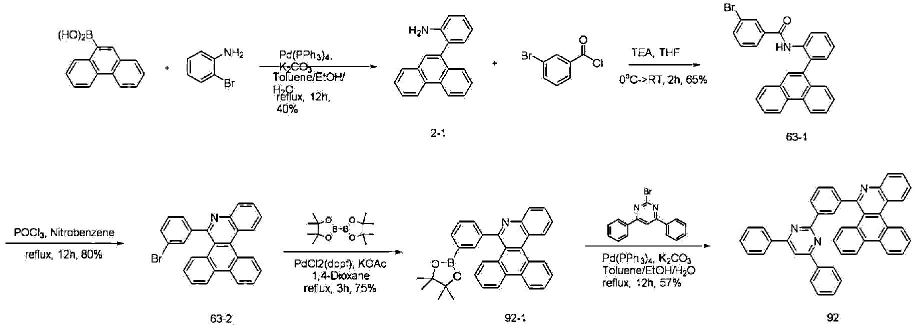

### Preparation of Compound 92-1

A mixed solution of Compound 63-2 (14 g, 0.0322 mol), bispinacolate diboron) (9.82 g, 0.0386 mol), 9.48 g (0.0966 mol) of KOAc(potassium acetate), 1.18 g (1.61 mmol) of PdCl₂(dppf), and 1,4-dioxane (250 ml) was put into a two neck round bottom flask (two neck r.b.f) and refluxed and stirred therein under nitrogen for 3 hours. A reaction mixture cooled to room temperature was extracted with MC (methylene chloride)/H₂O and dried using MgSO₄ and then filtered. After being concentrated, a solid is produced with hexane and filtered. As a result, an ivory solid compound 92-1 (11.6 g, 75%) was obtained.

### Preparation of Compound 92

A mixed solution of Compound 92-1 (11.6 g, 0.024 mol), 8.25 g (0.0265 mol) of 2-bromo-4,6-diphenylpyrimidine, K₂CO₃ (9.94 g, 0.072 mmol), Pd(PPh₃)₄ (1.39 g, 1.2 mmol), and toluene, ethanol, H₂O (200 ml/30 ml/30 ml) was put into a two neck round bottom flask (two neck r.b.f) and refluxed and stirred therein under nitrogen for 12 hours. The solvent of the reaction mixture was filtered and the resultant solid was washed with 200 ml of toluene, 300 ml of hexane, and 300 ml of methanol in sequence. As a result, a white solid compound 92 (8.04 g, 57%) was obtained.

### [Preparation Example 8] Preparation of Compound 111

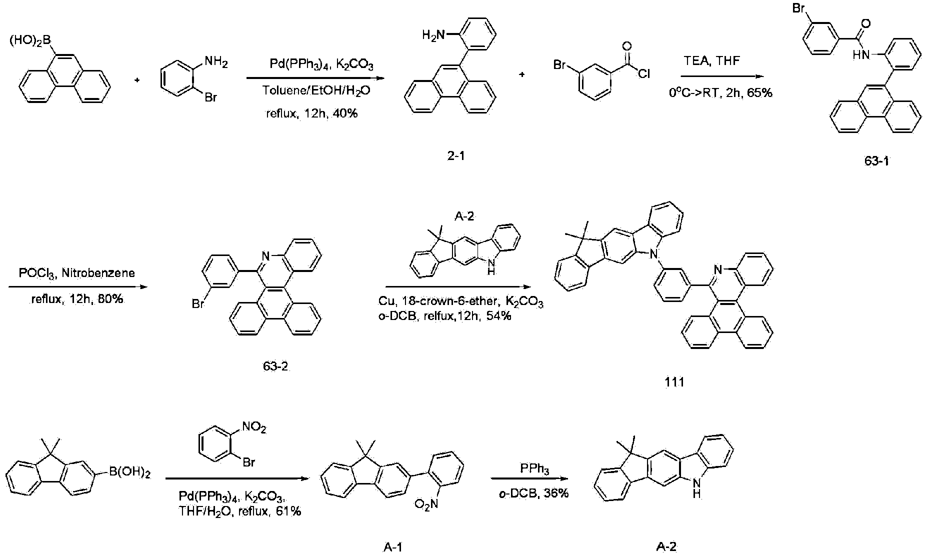

### Preparation of Compound A-1

A mixture of 25.9 g (0.108 mol) of (9,9-dimethyl-9H-fluoren-2-yl)boronic acid, 20 g (0.099 mol) of 1-bromo-2-nitrobenzene, Pd(PPh₃)₄ (5.7 g, 4.95 mmol), K₂CO₃ (27.3 g, 0.198 mol), and THF (250 ml)/H₂O (50 ml) was put into a one neck round bottom flask (one neck r.b.f) and refluxed and stirred for 24 hours. After a water layer was removed, an organic layer was dried using MgS04. After being concentrated, the resultant reaction product was separated by column chromatography (SiO₂, hexane : MC (methylene chloride) = 2 : 1). As a result, a yellow solid compound A-1 (21 g, 61%) was obtained.

### Preparation of Compound A-2

A mixture of Compound A-1 (20 g, 0.0634 mmol), PPh₃ (49.8 g, 0.190 mol), and *o-*dichlorobenzene (o-DCB) (300 ml) was put into a one neck round bottom flask (one neck r.b.f) and refluxed and stirred under nitrogen for 18 hours. After o-dichlorobenzene (*o-*DCB) was distilled under reduced pressure and removed, the resultant reaction product was separated by column chromatography (SiO₂, hexane : MC (methylene chloride) = 3 : 1). As a result, a white solid compound A-2 (6.6 g, 36%) was obtained.

### Preparation of Compound 111

A mixture of Compound 63-2 (8.0 g, 0.0184 mol), Compound A-2 (4.74 g, 0.0167 mol), Cu (0.58 g, 9.2 mol), 0.97 g(3.68 mmol) of 18-crown-6-ether, K₂CO₃ (12.7 g, 0.092 mol), and o-dichlorobenzene (o-DCB) (80 ml) was put into a one neck round bottom flask (one neck r.b.f) and refluxed and stirred under nitrogen for 12 hours. After *o-*dichlorobenzene (o-DCB) was distilled under reduced pressure and removed, the resultant reaction product was separated by column chromatography (SiO₂, hexane : MC (methylene chloride) = 4 : 1). As a result, a white solid compound 111 (6.3 g, 54%) was obtained.

### [Preparation Example 9] Preparation of Compound 130

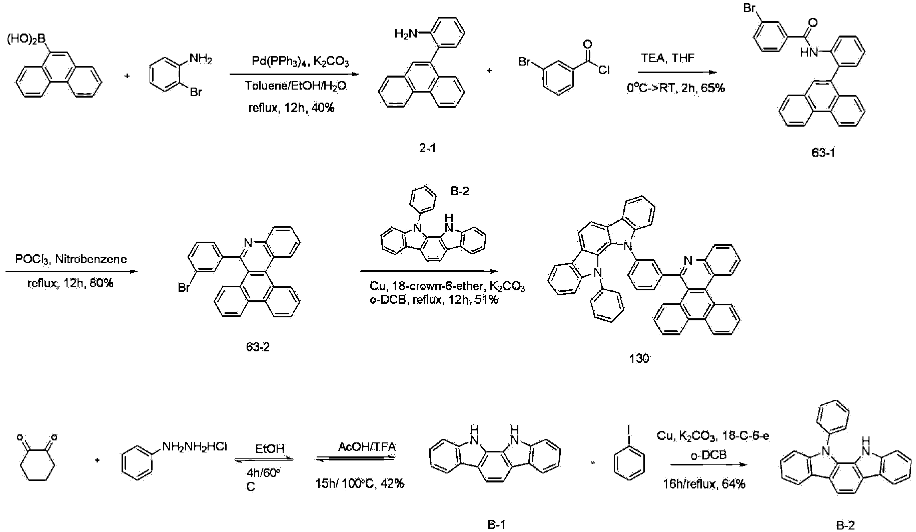

### Preparation of Compound B-1

Sulfuric acid (1.4 mL, 0.0374 mol) was slowly added dropwise to a mixture of 30.0 g (0.374 mol) of 1,2-dicyclohexanone, 77.37 g (0.749 mol) of phenylhydrazine hydrochloride, and ethanol (1000 ml) in a one neck round bottom flask (one neck r.b.f) under nitrogen and stirred at 60°C for 4 hours. The mixed solution cooled to room temperature was filtered, and a light brown solid (69 g, 93%) was obtained. Trifluoroacetic acid (46.5 mL, 0.6 mol) was added to a mixture of the above solid (68.9 g, 0.25 mol) and acetic acid (700 ml) within a one neck round bottom flask (one neck r.b.f) and stirred at 100°C for 15 hours. The mixed solution cooled to room temperature was washed with acetic acid and hexane and filtered. As a result, an ivory solid B-1 (27.3 g, 42%) was obtained.

### Preparation of Compound B-2

A mixture of Compound B-1 (2.1 g, 0.0082 mol), iodobenzene (2.5 g, 0.013 mol), Cu (0.312 g, 0.0049), 18-crown-6-ether (0.433 g, 0.0016 mol), K₂CO₃ (3.397 g, 0.0246 mol), and o-dichlorobenzene (o-DCB) (20ml) was refluxed and stirred in a two neck round bottom flask (two neck r.b.f) under nitrogen for 16 hours. The mixed solution cooled to room temperature was extracted with MC (methylene chloride)/H₂O and concentrated and separated by column chromatography (SiO₂, hexane : ethyl acetate = 10 : 1). As a result, a white solid compound B-2 was obtained (1.76 g, 64%).

### Preparation of Compound 130

A mixture of Compound 63-2 (5.0 g, 0.0115 mmol), Compound B-2 (3.48 g, 0.01 mmol), Cu (0.32 g, 5.0 mmol), 18-crown-6-ether (0.53 g, 2.0 mmol), K₂CO₃ (6.9 g, 0.05 mol), and o-dichlorobenzene (o-DCB) (30 ml) was refluxed and stirred in a two neck round bottom flask (two neck r.b.f) under nitrogen for 12 hours. After o-dichlorobenzene (o-DCB) was distilled under reduced pressure and removed, the resultant reaction product was separated by column chromatography (SiO₂, hexane : MC (methylene chloride) = 3 : 1). As a result, a solid compound 130 (3.5 g, 51%) was obtained.

### [Preparation Example 10] Preparation of Compound 181

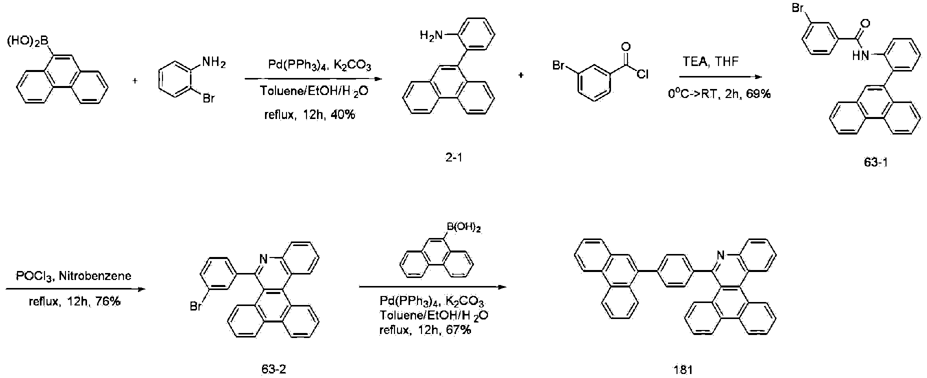

### Preparation of Compound 181

A mixed solution of Compound 63-2 (10.0 g, 0.023 mol), phenanthrene-9-yl boronic acid (5.6 g, 0.0253 mol), K₂CO₃ (9.5 g, 0.069 mol), Pd(PPh₃)₄ (1.33 g, 1.15 mmol), and toluene, ethanol, H₂O (200 ml/30 ml/30 ml) was put into a two neck round bottom flask (two neck r.b.f) and refluxed and stirred therein for 12 hours. A reaction mixture cooled to room temperature was extracted with MC (methylene chloride)/H₂O and dried using MgSO₄ and then filtered. After being concentrated, the resultant reaction product was separated by column chromatography (SiO₂, hexane : MC (methylene chloride) = 1 : 1). As a result, a white solid compound 181 (8.19 g, 67%) was obtained.

### [Preparation Example 11] Preparation of Compound 209

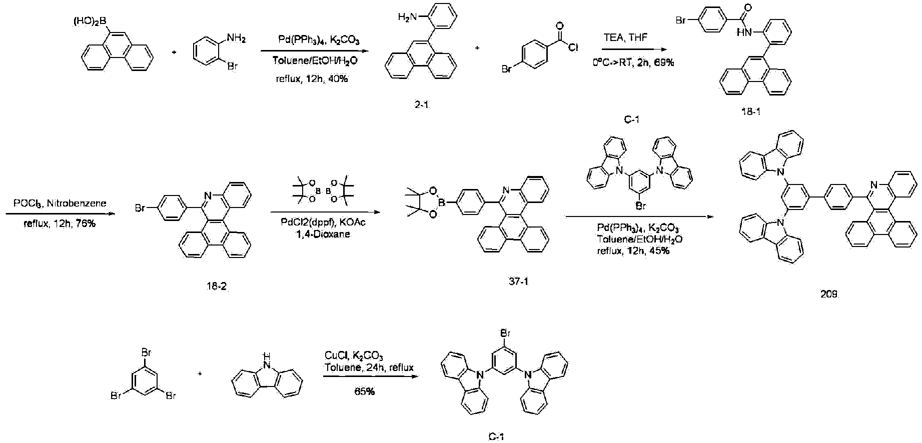

### Preparation of Compound C-1

A mixture of 1,3,5-tribromobenzene (10.0 g, 0.031 mol), 9H-carbazole (10.37 g, 0.062 mol), CuCl (0.3 g, 3.1 mmol), K₂CO₃ (8.56 g, 0.062 mol), and toluene (400 ml) was refluxed and stirred in a two neck round bottom flask (two neck r.b.f) under nitrogen for 24 hours. The mixed solution cooled to room temperature was extracted with MC (methylene chloride)/H₂O and dried using MgSO₄ and then filtered. After being concentrated, the resultant reaction product was separated by column chromatography (SiO₂, hexane : MC (methylene chloride) = 1 : 1). As a result, a white solid compound C-1 was obtained (9.75 g, 65%).

### Preparation of Compound 209

A mixed solution of Compound 37-1 (10.0 g, 0.021 mol), Compound C-1 (9.2 g, 0.019 mol), K₂CO₃ (8.7 g, 0.063 mol), Pd(PPh₃)₄ (1.21 g, 1.05 mmol), and toluene, ethanol, H₂O (200 ml/30 ml/30 ml) was refluxed and stirred in a two neck round bottom flask (two neck r.b.f) under nitrogen for 12 hours. The mixed solution cooled to room temperature was extracted with MC (methylene chloride)/H₂O and dried using MgSO₄ and then filtered. After being concentrated, the resultant reaction product was separated by column chromatography (SiO₂, hexane : MC (methylene chloride) = 1 : 1). As a result, a white solid compound 209 (7.2 g, 45%) was obtained.

### [Preparation Example 12] Preparation of Compounds 220, 224, 228, 232, 236, 240, and 244

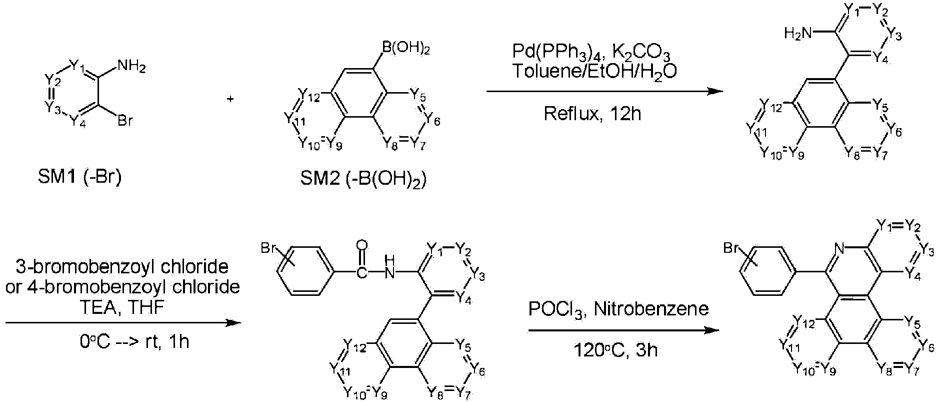

### [Preparation Example 13] Preparation of Compounds 248, 252, 256, 260, 264, 268, and 272

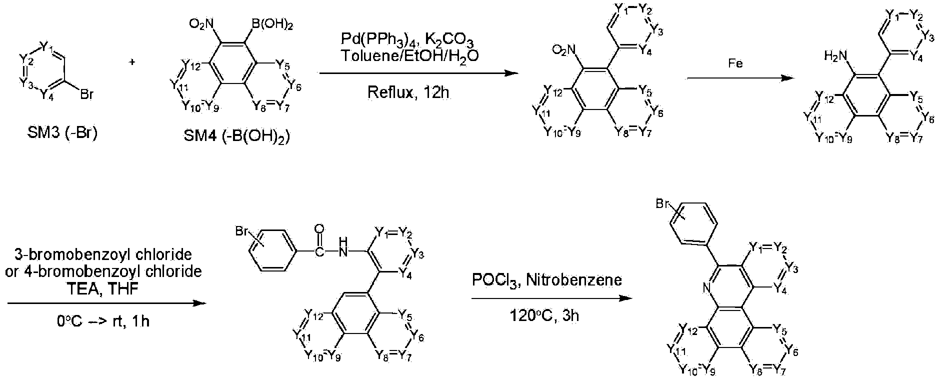

In the preparation examples 12 and 13, Compounds 220 to 275 of the present invention can be prepared by modifying a starting material 1 (SM1(-Br)), a starting material 2 (SM2 (-B(OH)₂), a starting material 3 (SM3(-Br)), and a starting material 4 (SM4 (-B(OH)₂). As the starting materials 1 to 4, compounds respectively having the strucures as listed in the following Table 1 were used to prepare Compounds 220, 224, 228, 232, 236, 240, 244, 248, 252, 256, 260, 264, 268, and 272. Herein, Y₁ to Y₁₂ are -CH, but Y₁ to Y₁₂ in the starting materials 1 to 4 can be modified.

**[Table 1]**

| Compound | SM1 (-Br) | SM2 (-B(OH)₂) | Compound | SM3 (-Br) | SM4 (-B(OH)₂) |
|---|---|---|---|---|---|
| 220 | 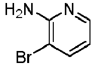 | 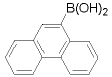 | 248 | 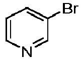 | 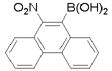 |
| 224 | 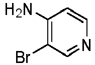 | 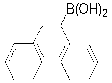 | 252 | 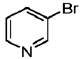 | 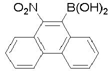 |
| 228 | 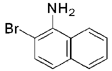 | 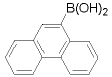 | 256 | 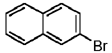 | 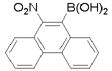 |
| 232 | 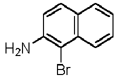 | 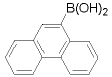 | 260 | 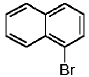 | 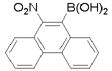 |
| 236 | 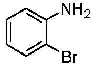 | 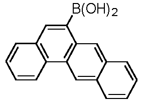 | 264 | 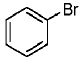 | 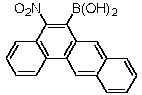 |
| 240 | 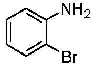 | 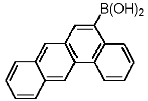 | 268 | 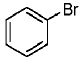 | 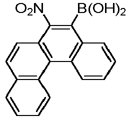 |
| 244 | 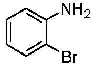 | 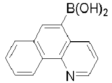 | 272 | 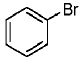 | 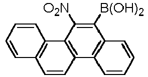 |

### [Preparation Example 14] Preparation of Compound 324

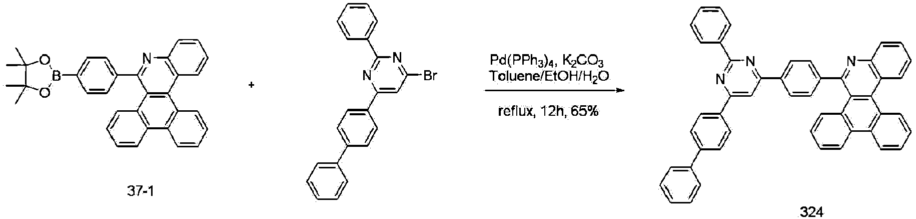

### Preparation of Compound 324

A mixed solution of Compound 37-1 (10.0 g, 0.0208 mol), 4-([1,1'-biphenyl]-4-yl)-6-bromo-2-phenylpyrimidine (9.65 g, 0.249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. A precipitated solid was filtered with the reaction mixture cooled to room temperature and washed with toluene and methanol. The filtered solid was dissolved in an excessive amount of hot toluene and allowed to pass through silica gel and then concentrated. The resultant reaction product was dissolved in a small amount of EA and in a small amount of 1, 2-dichloroethane and then filtered. As a result, a white solid compound 324 (8.95 g, 65%) was obtained.

### [Preparation Example 15] Preparation of Compound 337

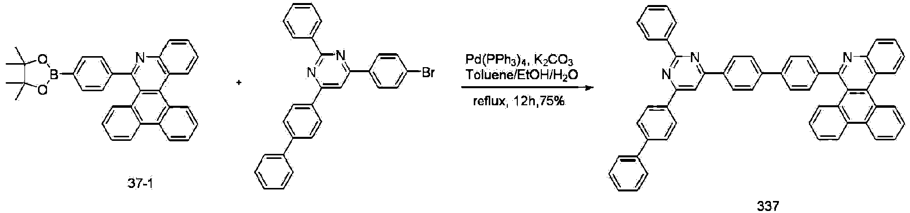

### Preparation of Compound 337

A mixed solution of Compound 37-1 (10.0 g, 0.0208 mol), 4-([1,1'-biphenyl]-4-yl)-6-(4-bromophenyl)-2-phenylpyrimidine (11.54 g, 0.249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. A precipitated solid was filtered with the reaction mixture cooled to room temperature and washed with toluene and methanol. The filtered solid was dissolved in an excessive amount of hot toluene and allowed to pass through silica gel and then concentrated. The resultant reaction product was dissolved in a small amount of EA and in a small amount of 1,2-dichloroethane and then filtered. As a result, a white solid compound 337 (11.5 g, 75%) was obtained.

### [Preparation Example 16] Preparation of Compound 376

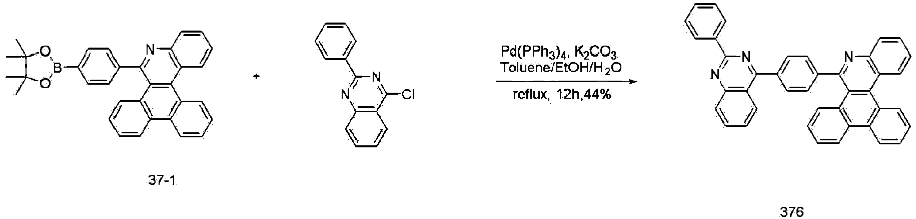

### Preparation of Compound 376

A mixed solution of Compound 37-1 (10.0 g, 0.0208 mol), 4-chloro-2-phenylquinazoline (5.99 g, 0.0249 mol), K₂CO₃ (8.61 g, 0.062 mmol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then filtered. After being concentrated, the resultant reaction product was separated by column chromatography (SiO₂, hexane : MC = 2 : 1). As a result, a white solid compound 376 (8.61 g, 44%) was obtained.

### [Preparation Example 17] Preparation of Compound 386

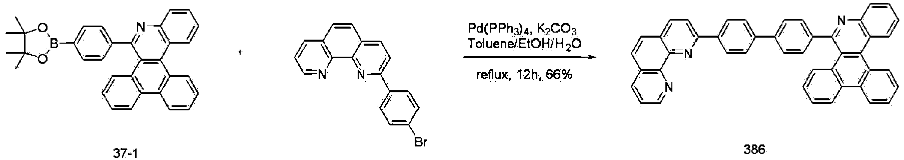

### Preparation of Compound 386

A mixed solution of Compound 37-1 (10.0 g, 0.0208 mol), 2-(4-bromophenyl)-1, 10-phenanthroline (8.35 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. A precipitated solid was filtered with the reaction mixture cooled to room temperature and washed with toluene and methanol. The filtered solid was dissolved in an excessive amount of hot toluene and allowed to pass through silica gel and then concentrated. The resultant reaction product as being hot was stirred for 3 or more hours in EA and 1, 2-dichloroethane and then filtered. As a result, a white solid compound 386 (8.37 g, 66%) was obtained.

### [Preparation Example 18] Preparation of Compound 388

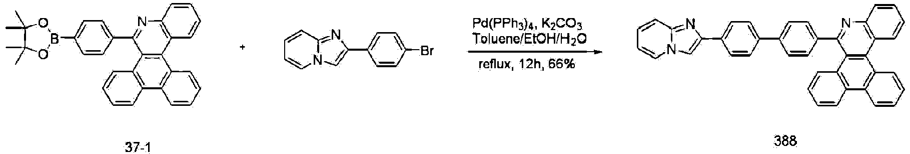

### Preparation of Compound 388

A mixed solution of Compound 37-1 (10.0 g, 0.0208 mol), 2-(4-bromophenyl)imidazo[1,2-a]pyridine (6.80 g, 0.0249 mol), K₂CO₃ (8.61 g, 0.062 mmol), Pd(PPh₃)₄ (1.2 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and allowed to pass through silica gel. After being concentrated, the resultant reaction product was dissolved in a small amount of EA, in a small amount of 1, 2-dichloroethane, and in a small amount of methanol and stirred for 3 or more hours and then filtered. As a result, a white compound 388 (5.92 g, 66%) was obtained.

### [Preparation Example 19] Preparation of Compound 396

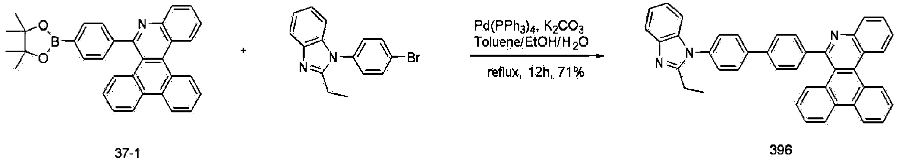

### Preparation of Compound 396

A mixed solution of Compound 37-1 (10.0 g, 0.0208 mol), 1-(4-bromophenyl)-2-ethyl-lH-benzo[d]imidazole (7.50 g, 0.0249 mol), K₂CO₃ (8.61 g, 0.062 mmol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and filtered. After being concentrated, the resultant reaction product was separated by column chromatography (SiO₂, hexane : MC = 2 : 1). As a result, a white solid compound 396 (8.50 g, 71%) was obtained.

### [Preparation Example 20] Preparation of Compound 421

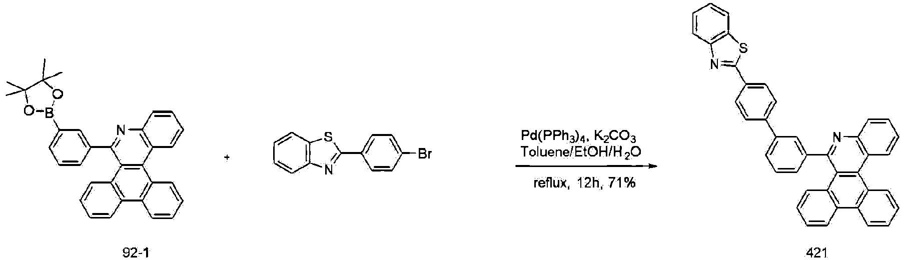

### Preparation of Compound 421

A mixed solution of Compound 92-1 (10.0 g, 0.0208 mol), 2-(4-bromophenyl)benzo[d]thiazole) (7.22 g, 0.0249 mol), K₂CO₃ (8.61 g, 0.062 mmol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and allowed to pass through silica gel. After being concentrated, the resultant reaction product was dissolved in a small amount of EA, in a small amount of 1, 2-dichloroethane, and in a small amount of methanol and stirred for 3 or more hours and then filtered. As a result, a white compound 421 (8.22 g, 70%) was obtained.

### [Preparation Example 21] Preparation of Compound 422

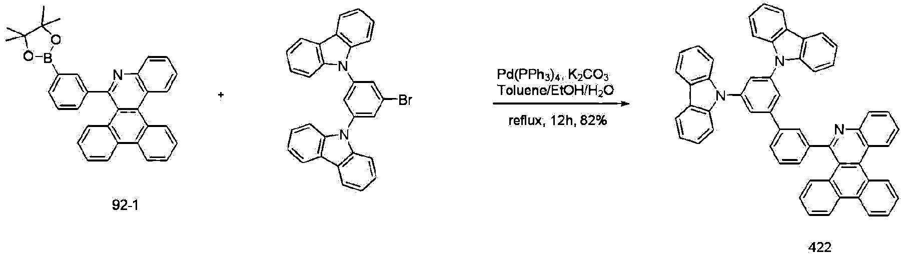

### Preparation of Compound 422

A mixed solution of Compound 92-1 (10.0 g, 0.0208 mol), 9,9'-(5-bromo-1,3-phenylene)bis(9H-carbazole) (12.1 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. A precipitated solid was filtered with the reaction mixture cooled to room temperature and washed with toluene and methanol. The filtered solid was dissolved in an excessive amount of hot toluene and allowed to pass through silica gel and then concentrated. The resultant reaction product was dissolved in a small amount of EA and in a small amount of 1, 2-dichloroethane and then filtered. As a result, a white solid compound 422 (12.98 g, 82%) was obtained.

### [Preparation Example 22] Preparation of Compound 465

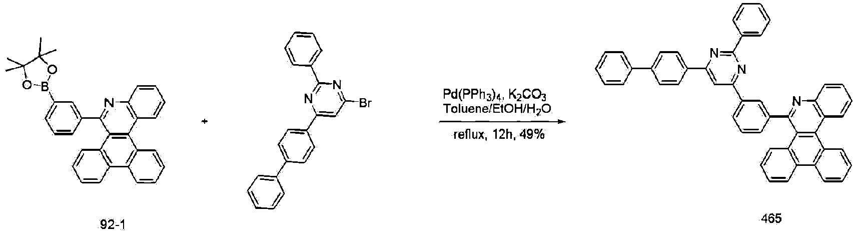

### Preparation of Compound 465

A mixed solution of Compound 92-1 (10.0 g, 0.0208 mol), 4-([1,1'-biphenyl]-4-yl)-6-bromo-2-phenylpyrimidine (9.64 g, 0.0249 mol), K₂CO₃ (8.61 g, 0.062 mmol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then filtered. After being concentrated, the resultant reaction product was separated by column chromatography (SiO₂, hexane : MC = 2 : 1). As a result, a white solid compound 465 (6.75 g, 49%) was obtained.

### [Preparation Example 23] Preparation of Compound 477

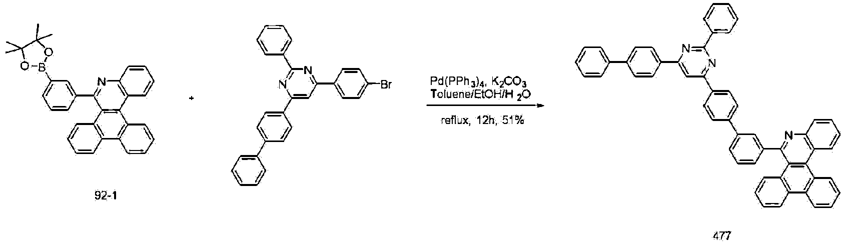

### Preparation of Compound 477

A mixed solution of Compound 92-1 (10.0 g, 0.0208 mol), 4-([1,1'-biphenyl]-4-yl)-6-(4-bromophenyl)-2-phenylpyrimidine (11.5 g, 0.0249 mol), K₂CO₃ (8.61 g, 0.062 mmol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and allowed to pass through silica gel. After being concentrated, the resultant reaction product was dissolved in a small amount of EA, in a small amount of 1, 2-dichloroethane, and in a small amount of methanol and stirred for 3 or more hours and then filtered. As a result, a white compound 477 (7.83 g, 51%) was obtained.

### [Preparation Example 24] Preparation of Compound 518

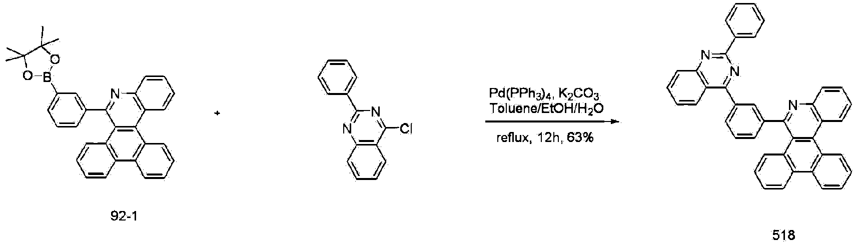

### Preparation of Compound 518

A mixed solution of Compound 92-1 (10.0 g, 0.0208 mol), 4-chloro-2-phenylquinazoline (5.99 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then filtered. After being concentrated, the resultant reaction product was separated by column chromatography (SiO₂, hexane : MC = 2 : 1). As a result, a white solid compound 518 (7.33 g, 63%) was obtained.

### [Preparation Example 25] Preparation of Compound 528

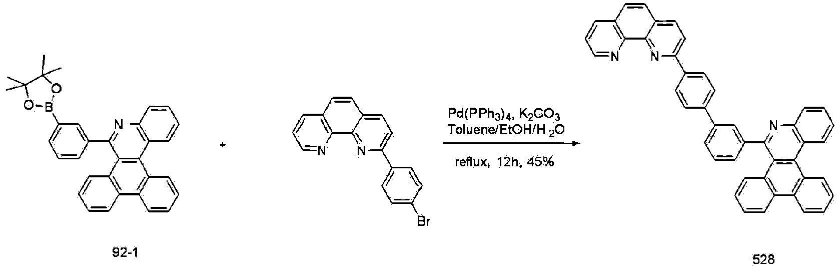

### Preparation of Compound 528

A mixed solution of Compound 92-1 (10.0 g, 0.0208 mol), 2-(4-bromophenyl)-1, 10-phenanthroline (6.94 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. A precipitated solid was filtered with the reaction mixture cooled to room temperature and washed with toluene and methanol. The filtered solid was dissolved in an excessive amount of hot toluene and allowed to pass through silica gel and then concentrated. The resultant reaction product was dissolved in a small amount of EA and in a small amount of 1, 2-dichloroethane and then filtered. As a result, a white solid compound 528 (5.70 g, 45%) was obtained.

### [Preparation Example 26] Preparation of Compound 530

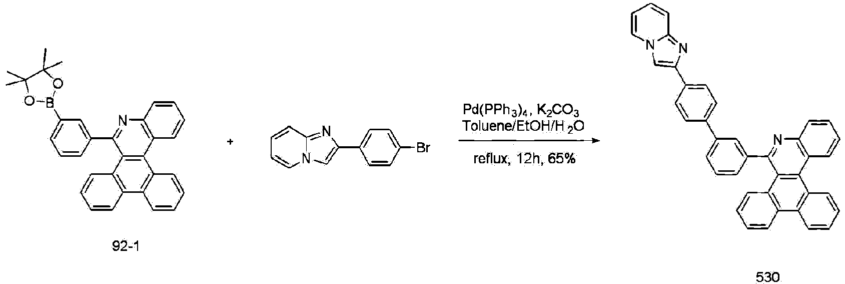

### Preparation of Compound 530

A mixed solution of Compound 92-1 (10.0 g, 0.0208 mol), 2-(4-bromophenyl)imidazo[1,2-a]pyridine (6.80 g, 0.0249 mol), K₂CO₃ (8.61 g, 0.062 mol), Pd(PPh₃)₄ (1.20 g, 0.0104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then filtered. After being concentrated, the resultant reaction product was separated by column chromatography (SiO₂, hexane : MC = 2 : 1). As a result, a white solid compound 530 (7.40 g, 65%) was obtained.

### [Preparation Example 27] Preparation of Compound 538

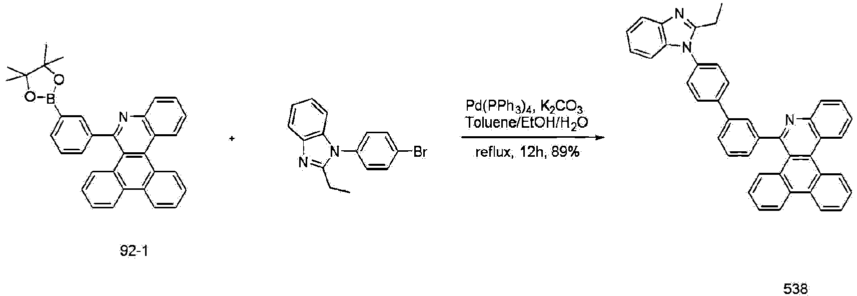

### Preparation of Compound 538

A mixed solution of Compound 92-1 (10.0 g, 0.0208 mol), 1-(4-bromophenyl)-2-ethyl-lH-benzo[d]imidazole (7.50 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then filtered. After being concentrated, the resultant reaction product was separated by column chromatography (SiO₂, hexane : MC = 2 : 1). As a result, a white solid compound 538 (10.66 g, 89%) was obtained.

### [Preparation Example 28] Preparation of Compound 558

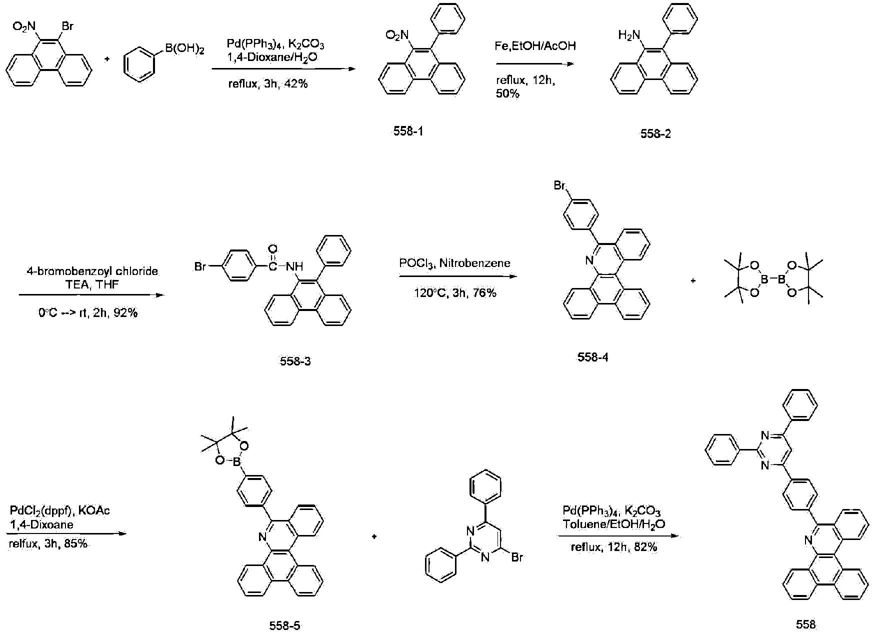

### Preparation of Compound 558-1

A mixed solution of 9-bromo-10-nitrophenanthrene (100 g, 0.33 mol), phenyl boronic acid (60.3 g, 0.495 mol), K₃PO₄ (251.4 g, 0.99 mmol), Pd(PPh₃)₄ (19.0 g, 0.0165 mol), and 1,4-dioxane)/H₂O (2000 ml/400 ml) was refluxed and stirred in a two neck round bottom flask (two neck r.b.f) under nitrogen for 3 hours. The reaction mixture cooled to room temperature was extracted with MC (methylene chloride)/H₂O and dried using MgSO₄ and then filtered. After being concentrated, the resultant reaction product was separated by column chromatography (SiO₂, hexane : MC = 2 : 1). As a result, a solid compound 558-1 (41.5 g, 42%) was obtained.

### Preparation of Compound 558-2

Compound 558-1 (40 g, 0.133 mol), iron (Fe) (8.17 g, 0.146 mol), 150 ml of acetic acid, and 800 ml of ethanol were mixed and then refluxed and stirred in a two neck round bottom flask (two neck r.b.f) for 12 hours. The reaction mixture cooled to room temperature was extracted with MC (methylene chloride)/H₂O and dried using MgSO₄ and then filtered. After being concentrated, the resultant reaction product was separated by column chromatography (SiO₂, hexane : MC = 2 : 1). As a result, a solid compound 558-2 (18 g, 50%) was obtained.

### Preparation of Compound 558-3

Compound 558-2 (18 g, 0.0668 mol) was dissolved in THF (100 ml) within a one neck round bottom flask (one neck r.b.f), and triethylamine (27.9 ml, 0.20 mol) was added dropwise thereto at room temperature. After the reaction mixture was cooled to 0°C, 4-bromobenzoyl chloride (16.1 g, 0.0735 mol) was dissolved in 25 ml of THF and slowly added dropwise thereto at a low temperature. Then, the resultant reaction product was stirred at room temperature for 2 hours and added with H₂O to terminate the reaction. The solvent was distilled under reduced pressure, and then MC (methylene chloride)/H₂O was extracted. The resultant reaction product was dried using MgSO₄ and then filtered and concentrated. After the resultant reaction product was dissolved in a small amount of MC (methylene chloride), methanol was added to precipitate a solid. As a result, an ivory solid compound 558-3 (27.8 g, 92%) was obtained.

### Preparation of Compound 558-4

Compound 558-3 (27 g, 0.0597 mol) was dissolved in nitrobenzene (150 ml) within a one neck round bottom flask (one neck r.b.f), and POCl₃ (phosphorous oxychloride) (3.33ml, 0.0358 mmol) was added dropwise thereto at room temperature and then refluxed and stirred for 12 hours. After the reaction was terminated, the mixed solution was cooled to room temperature and neutralized with NaHCO3 aqueous solution. Then, MC (methylene chloride)/H₂O was extracted. The resultant reaction product was dried using MgSO₄ and then filtered. After the solvent was distilled under reduced pressure and concentrated, a small amount of MC (methylene chloride) and an excessive amount of hexane were added to precipitate a solid. As a result, a pale yellow solid compound 558-4 (19.7g, 76%) was obtained.

### Preparation of Compound 558-5

A mixed solution of Compound 558-4 (15 g, 0.0345 mol), bis(pinacolato) diboron (13.1 g, 0.0518 mol), KOAc(potassium acetate) (10.2 g, 0.104 mol), PdCl₂(dppf) (1.58 g, 0.00173 mol), and 250 ml of 1,4-dioxane was put into a two neck round bottom flask (two neck r.b.f) and refluxed and stirred therein under nitrogen for 3 hours. A reaction mixture cooled to room temperature was extracted with MC (methylene chloride)/H₂O and dried using MgSO₄ and then filtered. After being concentrated, a solid is produced with hexane and filtered. As a result, a solid compound 558-5 (14.1 g, 85%) was obtained.

### Preparation of Compound 558

A mixed solution of Compound 558-5 (10.0 g, 0.0208 mol), 4-bromo-2,6-diphenylpyrimidine (7.75 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then allowed to pass through silica gel. After being concentrated, the resultant reaction product was dissolved in a small amount of EA, in a small amount of 1, 2-dichloroethane, and in a small amount of methanol and stirred for 3 or more hours and then filtered. As a result, a white compound 558 (9.99 g, 82%) was obtained.

### [Preparation Example 29] Preparation of Compound 559

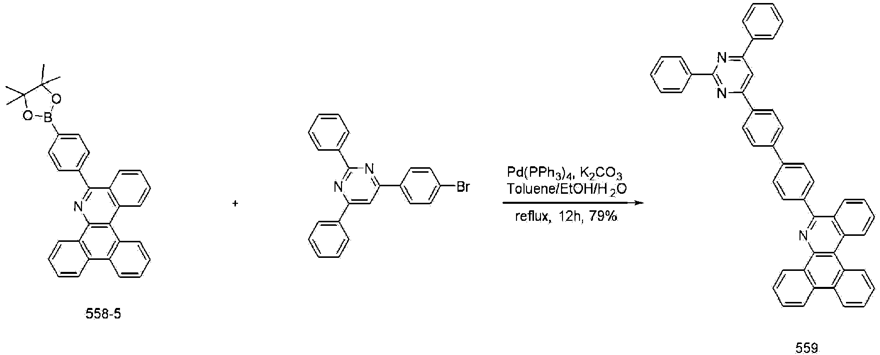

### Preparation of Compound 559

A mixed solution of Compound 558-5 (10.0 g, 0.0208 mol), 4-(4-bromophenyl)-2, 6-diphenylpyrimidine (9.64 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. A precipitated solid was filtered with the reaction mixture cooled to room temperature and washed with toluene and methanol. The filtered solid was dissolved in an excessive amount of hot toluene and allowed to pass through silica gel and then concentrated. The resultant reaction product was dissolved in a small amount of EA and in a small amount of 1, 2-dichloroethane and then filtered. As a result, a white solid compound 559 (10.87 g, 79%) was obtained.

### [Preparation Example 30] Preparation of Compound 560

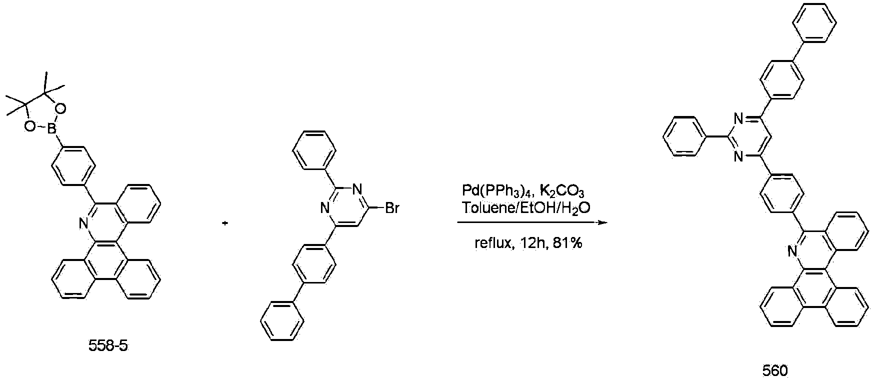

### Preparation of Compound 560

A mixed solution of Compound 558-5 (10.0 g, 0.0208 mol), 4-([1,1'-biphenyl]-4-yl)-6-bromo-2-phenylpyrimidine (9.64 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. A precipitated solid was filtered with the reaction mixture cooled to room temperature and washed with toluene and methanol. The filtered solid was dissolved in an excessive amount of hot toluene and allowed to pass through silica gel and then concentrated. The resultant reaction product was dissolved in a small amount of EA and in a small amount of 1, 2-dichloroethane and then filtered. As a result, a white solid compound 560 (11.15 g, 81%) was obtained.

### [Preparation Example 31] Preparation of Compound 561

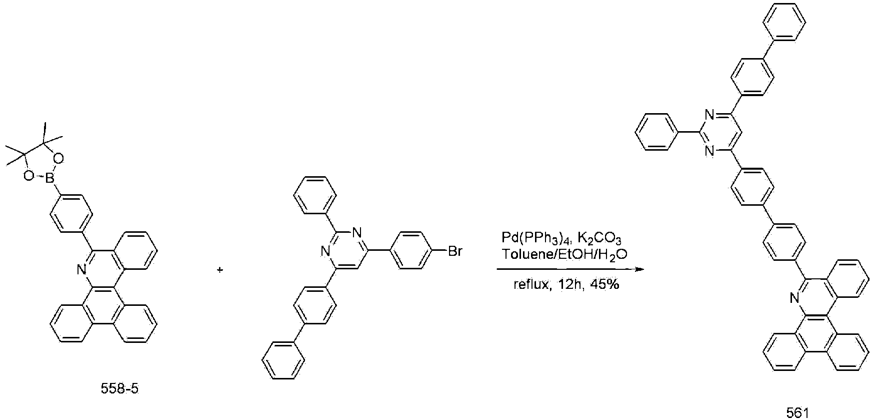

### Preparation of Compound 561

A mixed solution of Compound 558-5 (10.0 g, 0.0208 mol), 4-([1,1'-biphenyl]-4-yl)-6-(4-bromophenyl)-2-phenylpyrimidine (11.54 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. A precipitated solid was filtered with the reaction mixture cooled to room temperature and washed with toluene and methanol. The filtered solid was dissolved in an excessive amount of hot toluene and allowed to pass through silica gel and then concentrated. The resultant reaction product was dissolved in a small amount of EA and in a small amount of 1, 2-dichloroethane and then filtered. As a result, a white solid compound 561 (6.90 g, 45%) was obtained.

### [Preparation Example 32] Preparation of Compound 562

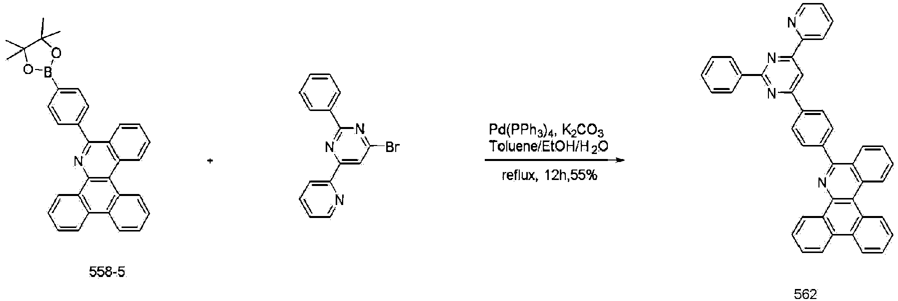

### Preparation of Compound 562

A mixed solution of Compound 558-5 (10.0 g, 0.0208 mol), 4-bromo-2-phenyl-6-(pyridin-2-yl)pyrimidine (7.77 g, 0.0249 mol), K₂CO₃ (8.62 g, 0.062 mol), Pd(PPh₃)₄ (1.20 g, 0.0104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then filtered. After being concentrated, the resultant reaction product was separated by column chromatography (SiO₂, hexane : MC = 2 : 1). As a result, a white solid compound 562 (6.71 g, 55%) was obtained.

### [Preparation Example 33] Preparation of Compound 563

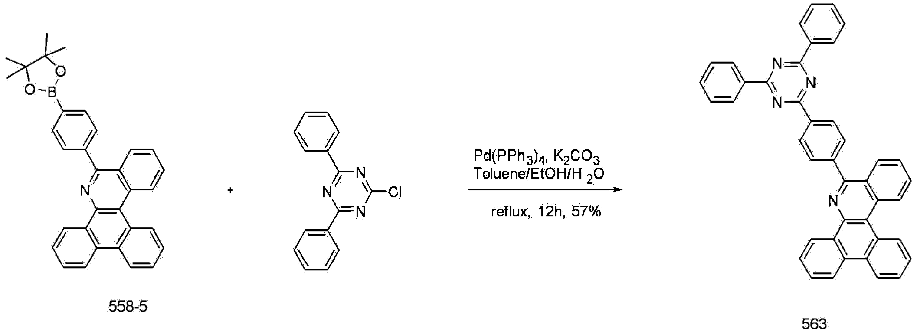

### Preparation of Compound 563

A mixed solution of Compound 558-5 (10.0 g, 0.0208 mol), 2-chloro-4,6-diphenyl-1,3,5-triazine (6.66 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. A precipitated solid was filtered with the reaction mixture cooled to room temperature and washed with toluene and methanol. The filtered solid was dissolved in an excessive amount of hot toluene and allowed to pass through silica gel and then concentrated. The resultant reaction product was dissolved in a small amount of EA and in a small amount of 1, 2-dichloroethane and then filtered. As a result, a white solid compound 563 (6.95 g, 57%) was obtained.

### [Preparation Example 34] Preparation of Compound 564

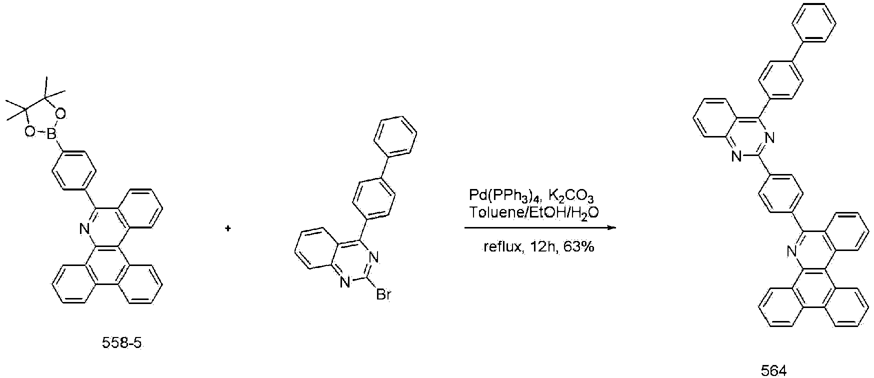

### Preparation of Compound 564

A mixed solution of Compound 558-5 (10.0 g, 0.0208 mol), 4-([1,1'-biphenyl]-4-yl)-2-bromoquinazoline (8.99 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then allowed to pass through silica gel. After being concentrated, the resultant reaction product was dissolved in a small amount of EA, in a small amount of 1, 2-dichloroethane, and in a small amount of methanol and stirred for 3 or more hours and then filtered. As a result, a white compound 564 (8.33 g, 63%) was obtained.

### [Preparation Example 35] Preparation of Compound 565

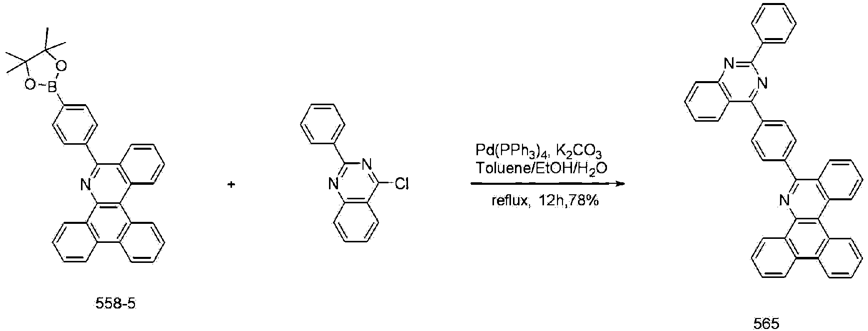

### Preparation of Compound 565

A mixed solution of Compound 558-5 (10.0 g, 0.0208 mol), 4-chloro-2-phenylquinazoline (5.99 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then allowed to pass through silica gel. After being concentrated, the resultant reaction product was dissolved in a small amount of EA, in a small amount of 1, 2-dichloroethane, and in a small amount of methanol and stirred for 3 or more hours and then filtered. As a result, a white compound 565 (9.08 g, 78%) was obtained.

### [Preparation Example 36] Preparation of Compound 566

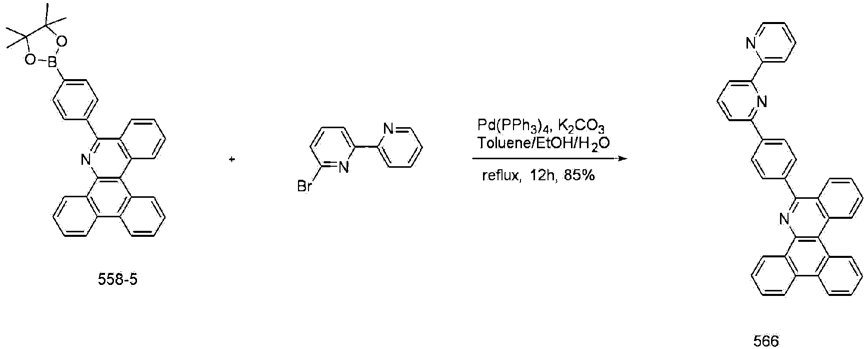

### Preparation of Compound 566

A mixed solution of Compound 558-5 (10.0 g, 0.0208 mol), 6-bromo-2, 2'-bipyridine (5.85 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then filtered. After being concentrated, the resultant reaction product was separated by column chromatography (SiO₂, hexane : MC = 2 : 1). As a result, a white solid compound 566 (9.0 g, 85%) was obtained.

### [Preparation Example 37] Preparation of Compound 567

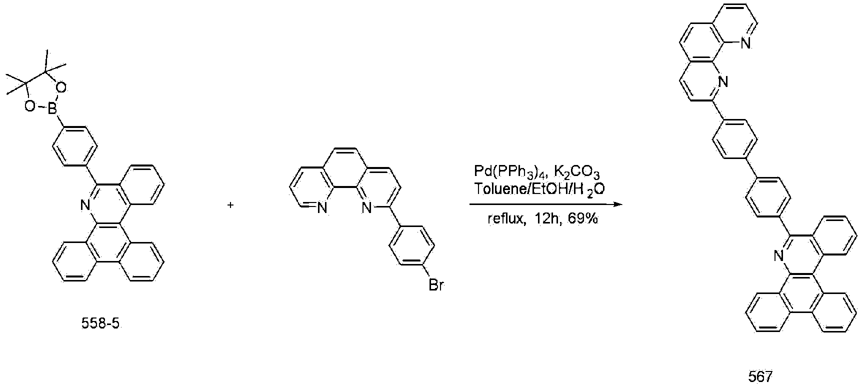

### Preparation of Compound 567

A mixed solution of Compound 558-5 (10.0 g, 0.0208 mol), 2-(4-bromophenyl)-1, 10-phenanthroline (8.35 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. A precipitated solid was filtered with the reaction mixture cooled to room temperature and washed with toluene and methanol. The filtered solid was dissolved in an excessive amount of hot toluene and allowed to pass through silica gel and then concentrated. The resultant reaction product was dissolved in a small amount of EA and in a small amount of 1, 2-dichloroethane and then filtered. As a result, a white solid compound 567 (8.75 g, 69%) was obtained.

### [Preparation Example 38] Preparation of Compound 568

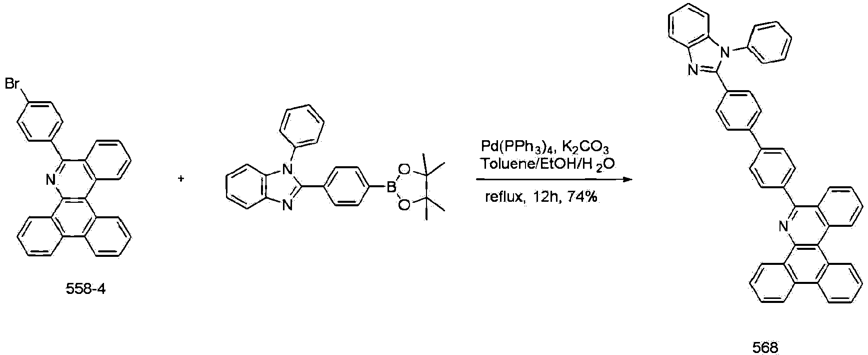

### Preparation of Compound 568

A mixed solution of Compound 558-4 (10.0 g, 0.023 mol), 1-phenyl-2-(4-(4,4,5,5-tetramethyl-1,3,2-dioxaborolan-2-yl)phenyl)-1H-benzo[d]imidazole (10.95 g, 0.0276 mol), K₂CO₃ (9.52 g, 0.069 mmol), Pd(PPh₃)₄ (1.33 g, 0.00115 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then allowed to pass through silica gel. After being concentrated, the resultant reaction product was dissolved in a small amount of EA, in a small amount of 1, 2-dichloroethane, and in a small amount of methanol and stirred for 3 or more hours and then filtered. As a result, a white compound 568 (9.60 g, 74%) was obtained.

### [Preparation Example 39] Preparation of Compound 569

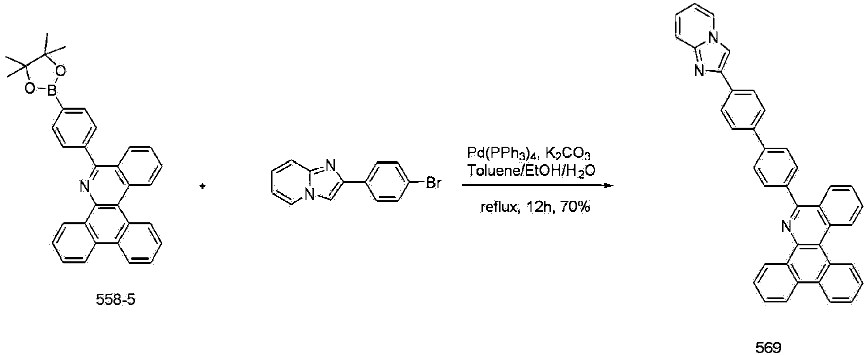

### Preparation of Compound 569

A mixed solution of Compound 558-5 (10.0 g, 0.0208 mol), 2-(4-bromophenyl)imidazo[1,2-a]pyridine (6.80 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then allowed to pass through silica gel. After being concentrated, the resultant reaction product was dissolved in a small amount of EA, in a small amount of 1, 2-dichloroethane, and in a small amount of methanol and stirred for 3 or more hours and then filtered. As a result, a white compound 569 (7.97 g, 70%) was obtained.

### [Preparation Example 40] Preparation of Compound 570

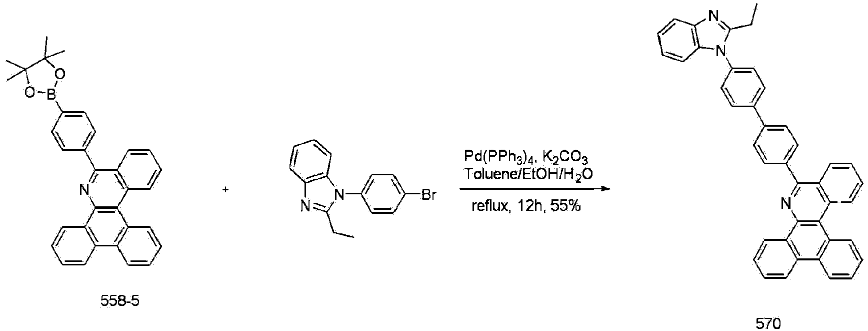

### Preparation of Compound 570

A mixed solution of Compound 558-5 (10.0 g, 0.0208 mol), 1-(4-bromophenyl)-2-ethyl-1H-benzo[d]imidazole (7.50 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. The reaction mixture cooled to room temperature was extracted with MC (methylene chloride)/H₂O and dried using MgSO₄ and then filtered. After being concentrated, the resultant reaction product was separated by column chromatography (SiO₂, hexane : MC = 2 : 1). As a result, a white solid compound 570 (6.59 g, 55%) was obtained.

### [Preparation Example 41] Preparation of Compound 571

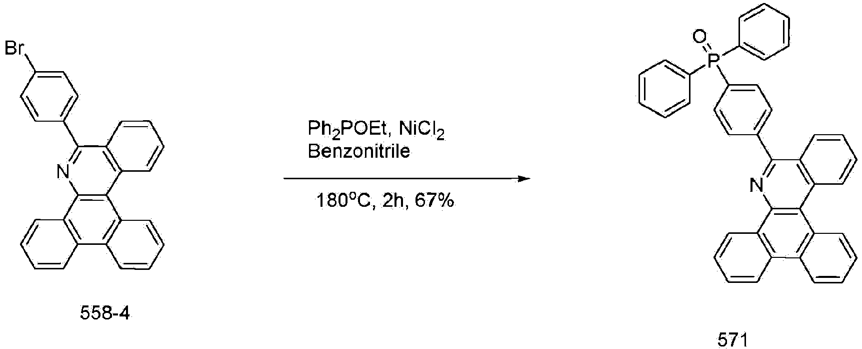

### Preparation of Compound 571

A mixed solution of Compound 558-4 (10.0 g, 0.023 mol), nickel(II) chloride (1.79 g, 0.0138 mol), and benzonitrile was heated to 180°C and stirred for 1 hour in a two neck round bottom flask (two neck r.b.f) under nitrogen. Ph₂POEt (ethyl diphenylphosphinite) (8.48 g, 0.0368 mol) was slowly added dropwise to the above mixture and then stirred for 2 hours. The reaction mixture cooled to room temperature was extracted with MC (methylene chloride)/H₂O and dried using MgSO₄ and then filtered. After being concentrated, the resultant reaction product was separated by column chromatography (SiO₂, hexane : MC = 2 : 1). As a result, a white solid compound 571 (6.4 g, 58%) was obtained.

### [Preparation Example 42] Preparation of Compound 572

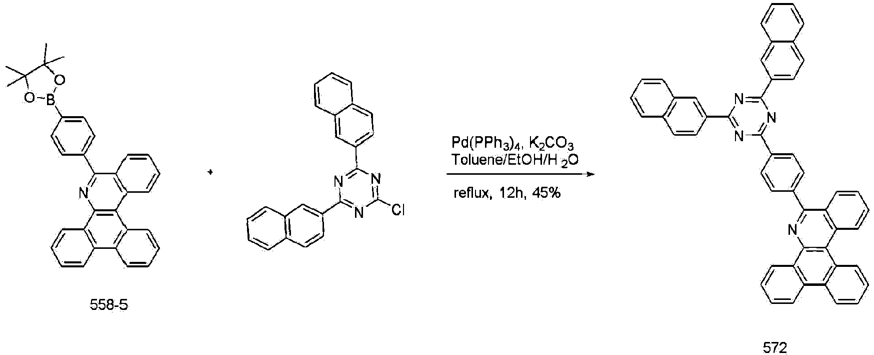

### Preparation of Compound 572

A mixed solution of Compound 558-5 (10.0 g, 0.0208 mol), 2-chloro-4,6-di(naphthalen-2-yl)-1,3,5-triazine (9.16 g, 0.0249 mol), K₂CO₃ (8.61 g, 0.062 mol), Pd(PPh₃)₄ (1.20 g, 0.00104 mol), and toluene, ethanol, H₂O (200 ml/30 ml/30 ml) was refluxed and stirred in a two neck round bottom flask (two neck r.b.f) under nitrogen for 12 hours. A precipitated solid was filtered with the reaction mixture cooled to room temperature and washed with toluene and methanol. The filtered solid was dissolved in an excessive amount of hot toluene and allowed to pass through silica gel and then concentrated. The resultant reaction product was dissolved in a small amount of EA and in a small amount of 1, 2-dichloroethane and then filtered. As a result, a white solid compound 572 (6.43 g, 45%) was obtained.

### [Preparation Example 43] Preparation of Compound 577

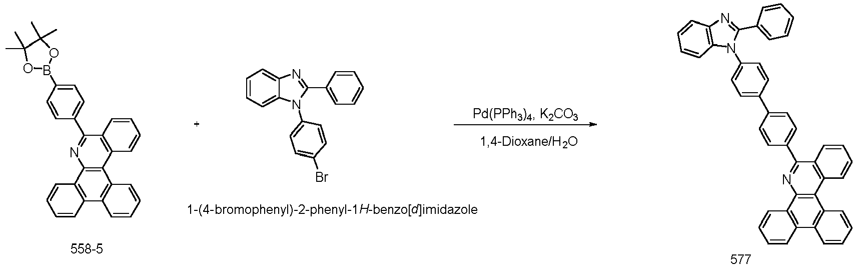

### Preparation of Compound 577

The title compound was prepared in the same manner as in the preparation method of Compound 558, except that 1-(4-bromophenyl)-2-phenyl-1H-benzo[d] imidazole was used instead of 4-bromo-2,6-diphenylpyrimidine (10.2 g, 79%).

### [Preparation Example 44] Preparation of Compound 590

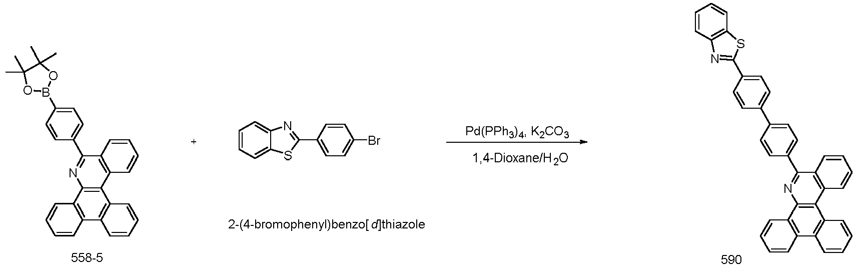

### Preparation of Compound 590

The title compound was prepared in the same manner as in the preparation method of Compound 558, except that 2-(4-bromophenyl)benzo[d]thiazole was used instead of 4-bromo-2,6-diphenylpyrimidine (6.1 g, 52%).

### [Preparation Example 45] Preparation of Compound 597

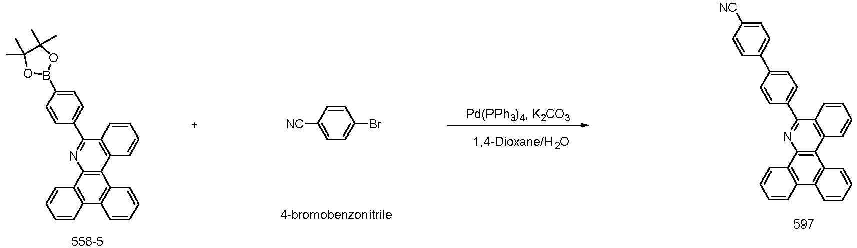

### Preparation of Compound 597

The title compound was prepared in the same manner as in the preparation method of Compound 558, except that 4-bromobenzonitrile was used instead of 4-bromo-2,6-diphenylpyrimidine (6.26 g, 66%).

### [Preparation Example 46] Preparation of Compound 598

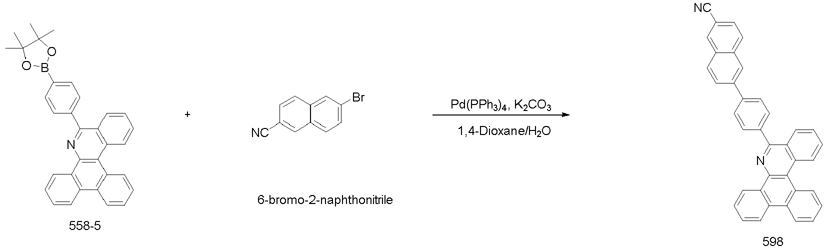

### Preparation of Compound 598

The title compound was prepared in the same manner as in the preparation method of Compound 558, except that 4-bromobenzonitrile was used instead of 4-bromo-2,6-diphenylpyrimidine (5.68 g, 54%).

### [Preparation Example 47] Preparation of Compound 599

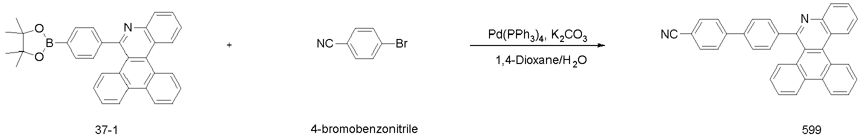

### Preparation of Compound 599

The title compound was prepared in the same manner as in the preparation method of Compound 37, except that 4-bromobenzonitrile was used instead of 2-bromo-4,6-diphenylpyrimidine (6.26 g, 66%).

### [Preparation Example 48] Preparation of Compound 600

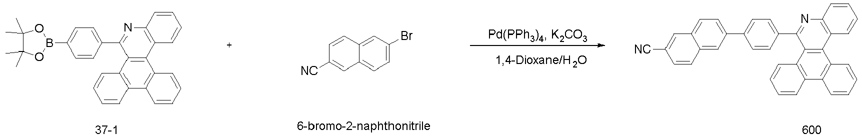

The title compound was prepared in the same manner as in the preparation method of Compound 37, except that 6-bromo-2-naphthonitrile was used instead of 2-bromo-4,6-diphenylpyrimidine (5.68 g, 54%).

### [Preparation Example 49] Preparation of Compound 600

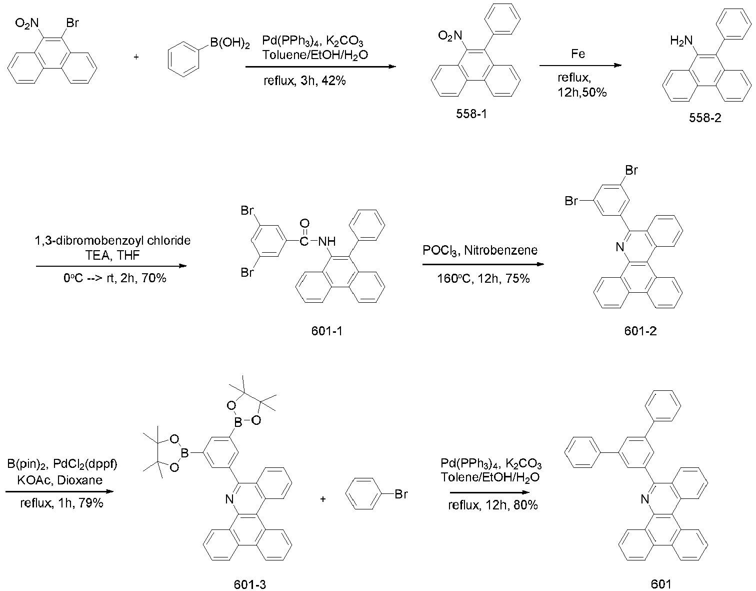

### Preparation of Compound 601-1

Compound 558-2 (60 g, 0.223 mol) was dissolved in THF (1,000 ml) in a one neck round bottom flask, and then trimethylamine (92.6 ml, 0.668 mol) was added dropwise thereto at room temperature. The reaction mixture was cooled to 0°C, and then 1,3-dibromobenzoyl chloride (73.1 g, 0.245 mol) was dissolved in 80 ml of THF, and the resulting solution was slowly added dropwise to the mixture at low temperature. The resulting mixture was then stirred at room temperature for 2 hours, and then H₂O was added thereto to terminate the reaction. The solvent was distilled under reduced pressure, and then the resulting product was extracted with methylene chloride (MC)/H₂O and dried over MgSO₄, and then resulting product was filtered and concentrated. The product was dissolved in a small amount of methylene chloride (MC), and then methanol was added to the solution to precipitate a solid, thereby obtaining an ivory solid Compound 601-1 (82.8 g, 70%).

### Preparation of Compound 601-2

Compound 601-1 (82 g, 0.154 mol) was dissolved in nitrobenzene (1,600 ml) in a one neck round bottom flask, and then phosphorous oxychloride (POCl₃, 8.6 ml, 0.0924 mol) was added dropwise thereto at room temperature, and the resulting mixture was stirred under reflux for 12 hours. The mixed solution, in which the reaction had been terminated, was cooled to room temperature, was neutralized with a NaHCO₃ aqueous solution, and then the resulting product was extracted with methylene chloride (MC)/H₂O, dried over MgSO₄, and then filtered. The solvent was distilled under reduced pressure and concentrated, and then, a small amount of methylene chloride (MC) and an excessive amount of methanol were added to the solution to precipitate a solid, thereby obtaining a pale yellow solid Compound 601-2 (59.4 g, 75%).

### Preparation of Compound 601-3

A 300 ml-mixed 1,4-dioxane solution of Compound 601-2 (15 g, 0.0292 mol), bis(pinacolato) diboron (22.2 g, 0.0876 mol), potassium acetate (KOAc, 14.3 g, 0.146 mol), and PdCl₂(dppf) (2.1 g, 0.00292 mol) was stirred under reflux under nitrogen for 3 hours in a two neck round bottom flask. The reaction mixture cooled to room temperature was extracted with methylene chloride (MC)/H₂O, dried over MgSO₄, and then filtered. After concentration, a small amount of EA slurry was post-filtered to obtain solid Compound 601-3 (14.1 g, 79%).

### Preparation of Compound 601

A toluene/ethanol/H₂O (250 ml/150 ml/50 ml) mixed solution of Compound 601-3 (14.0 g, 0.023 mol), bromobenzene (10.8 g, 0.069 mol), K₂CO₃ (15.87 g, 0.115 mol), and Pd(PPh₃)₄ (2.6 g, 0.0023 mol) was stirred under reflux under nitrogen for 12 hours in a two neck round bottom flask. The reaction mixture cooled to room temperature was extracted with methylene chloride (MC)/H₂O, dried over MgSO₄, and then allowed to pass through silica gel. After concentration, a small amount of the resulting product was dissolved in EA, tetrahydrofuran (THF), and methanol, respectively, and then the resulting mixture was stirred for 3 hours or more and filtered to obtain white Compound 601 (9.36 g, 80%).

### [Preparation Example 50] Preparation of Compound 602

A toluene/ethanol/H₂O (250 ml/150 ml/50 ml) mixed solution of Compound 601-3 (14.0 g, 0.023 mol), 1-bromonaphthalene (14.2 g, 0.069 mol), K₂CO₃ (15.87 g, 0.115 mol), and Pd(PPh₃)₄ (2.6 g, 0.0023 mol) was stirred under reflux under nitrogen for 12 hours in a two neck round bottom flask. The reaction mixture cooled to room temperature was extracted with methylene chloride (MC)/H₂O, dried over MgSO₄, and then allowed to pass through silica gel. After concentration, a small amount of the resulting product was dissolved in EA, tetrahydrofuran (THF), and methanol, respectively, and then the resulting mixture was stirred for 3 hours or more and filtered to obtain white Compound 602 (9.09 g, 65%).

### [Preparation Example 51] Preparation of Compound 603

A toluene/ethanol/H₂O (250 ml/150 ml/50 ml) mixed solution of Compound 601-2 (11.8 g, 0.023 mol), dibenzo[b,d]furan-4-ylboronic acid (14.6 g, 0.069 mol), K₂CO₃ (15.87 g, 0.115 mol), and Pd(PPh₃)₄ (2.6 g, 0.0023 mol) was stirred under reflux under nitrogen for 12 hours in a two neck round bottom flask. The reaction mixture cooled to room temperature was extracted with methylene chloride (MC)/H₂O, dried over MgSO₄, and then allowed to pass through silica gel. After concentration, a small amount of the resulting product was dissolved in EA, tetrahydrofuran (THF), and methanol, respectively, and then the resulting mixture was stirred for 3 hours or more and filtered to obtain white Compound 603 (12.9 g, 82%).

### [Preparation Example 52] Preparation of Compound 604

A 250-ml toluene mixed solution of Compound 601-2 (11.8 g, 0.023 mol), 9H-carbazole (11.5 g, 0.069 mol), sodium tert-butoxide (11.1 g, 0.115 mol), Pd₂(dba)₃ (2.1 g, 0.0023 mol), and tri-tert-butyl phosphine (1.1 ml, 0.0046 mol) was stirred under reflux under nitrogen for 12 hours in a two neck round bottom flask. The reaction mixture cooled to room temperature was extracted with methylene chloride (MC)/H₂O, dried over MgSO₄, and then allowed to pass through silica gel. After concentration, a small amount of the resulting product was dissolved in EA, tetrahydrofuran (THF), and methanol, respectively, and then the resulting mixture was stirred for 3 hours or more and filtered to obtain white Compound 604 (12.9 g, 82%).

### [Preparation Example 53] Preparation of Compound 605

A toluene/ethanol/H₂O (250 ml/150 ml/50 ml) mixed solution of Compound 601-3 (14.0 g, 0.023 mol), 2-bromodibenzo[b,d]thiophene (18.2 g, 0.069 mol), K₂CO₃ (15.87 g, 0.115 mol), and Pd(PPh₃)₄ (2.6 g, 0.0023 mol) was stirred under reflux under nitrogen for 12 hours in a two neck round bottom flask. The reaction mixture cooled to room temperature was extracted with methylene chloride (MC)/H₂O, dried over MgSO₄, and then allowed to pass through silica gel. After concentration, a small amount of the resulting product was dissolved in EA, tetrahydrofuran (THF), and methanol, respectively, and then the resulting mixture was stirred for 3 hours or more and filtered to obtain white Compound 605 (11.6 g, 70%).

### [Preparation Example 54] Preparation of Compound 606

A toluene/ethanol/H₂O (250 ml/150 ml/50 ml) mixed solution of Compound 601-3 (14.0 g, 0.023 mol), 2-bromo-9,10-phenanthroline (17.9 g, 0.069 mol), K₂CO₃ (15.87 g, 0.115 mol), and Pd(PPh₃)₄ (2.6 g, 0.0023 mol) was stirred under reflux under nitrogen for 12 hours in a two neck round bottom flask. The reaction mixture cooled to room temperature was extracted with methylene chloride (MC)/H₂O, dried over MgSO₄, and then allowed to pass through silica gel. After concentration, a small amount of the resulting product was dissolved in EA, tetrahydrofuran (THF), and methanol, respectively, and then the resulting mixture was stirred for 3 hours or more and filtered to obtain white Compound 606 (7.7 g, 47%).

### [Preparation Example 55] Preparation of Compound 607

A toluene/ethanol/H₂O (250 ml/150 ml/50 ml) mixed solution of Compound 601-3 (14.0 g, 0.023 mol), 1-(4-bromophenyl)-2-phenyl-1H-benzo[d]imidazole (24.1 g, 0.069 mol), K₂CO₃ (15.87 g, 0.115 mol), and Pd(PPh₃)₄ (2.6 g, 0.0023 mol) was stirred under reflux under nitrogen for 12 hours in a two neck round bottom flask. The reaction mixture cooled to room temperature was extracted with methylene chloride (MC)/H₂O, dried over MgSO₄, and then allowed to pass through silica gel. After concentration, a small amount of the resulting product was dissolved in EA, tetrahydrofuran (THF), and methanol, respectively, and then the resulting mixture was stirred for 3 hours or more and filtered to obtain white Compound 607 (20.5 g, 66%).

### [Preparation Example 56] Preparation of Compound 608

A toluene/ethanol/H₂O (250 ml/150 ml/50 ml) mixed solution of Compound 601-2 (11.8 g, 0.023 mol), phenanthren-9-yl boronic acid (15.3 g, 0.069 mol), K₂CO₃ (15.87 g, 0.115 mol), and Pd(PPh₃)₄ (2.6 g, 0.0023 mol) was stirred under reflux under nitrogen for 12 hours in a two neck round bottom flask. The reaction mixture cooled to room temperature was extracted with methylene chloride (MC)/H₂O, dried over MgSO₄, and then allowed to pass through silica gel. After concentration, a small amount of the resulting product was dissolved in EA, tetrahydrofuran (THF), and methanol, respectively, and then the resulting mixture was stirred for 3 hours or more and filtered to obtain white Compound 603 (11.6 g, 71%).

### [Preparation Example 57] Preparation of Compound 609

A toluene/ethanol/H₂O (250 ml/150 ml/50 ml) mixed solution of Compound 601-3 (14.0 g, 0.023 mol), 4-bromo-9,9'-spirobi[fluorene] (27.3 g, 0.069 mol), K₂CO₃ (15.87 g, 0.115 mol), and Pd(PPh₃)₄ (2.6 g, 0.0023 mol) was stirred under reflux under nitrogen for 12 hours in a two neck round bottom flask. The reaction mixture cooled to room temperature was extracted with methylene chloride (MC)/H₂O, dried over MgSO₄, and then allowed to pass through silica gel. After concentration, a small amount of the resulting product was dissolved in EA, tetrahydrofuran (THF), and methanol, respectively, and then the resulting mixture was stirred for 3 hours or more and filtered to obtain white Compound 607 (12.4 g, 55%).

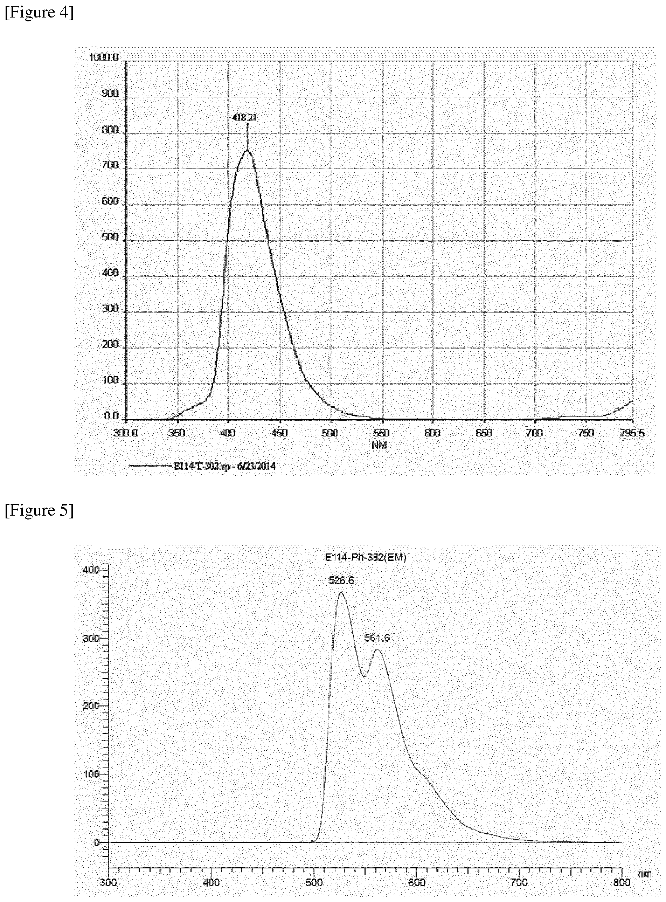

The compounds were prepared according to the above-described preparation examples. Synthesis thereof was checked,and the check results were as listed in Table 2 and illustrated in FIG. 4 to FIG. 25.

The following Table 2 lists ¹H NMR(CDCl₃, 200 Mz) measurement data and measurement data obtained by an FD-spectrometer (FD-MS: Field desorption mass spectrometry).

**[Table 2]**

| Compound | ¹H NMR(CDCl₃, 200Mz) | MS/FAB | |
|---|---|---|---|
| | | found | calculated |
| **2** | 9.08(2H,d), 8.17(1H,s), 8.37(1H,d), 8.19(3H,m), 8.06(2H,m), 8.00(1H,m), 7.94(1H,d), 7.85(1H,t) | 405.50 | 405.15 |
| **4** | 9.08(2H,d), 8.95(1H,d), 8.69(2H,d), 8.50(1H,d), 8.19(4H,m), 8.09(1H,d), 7.94(1H,d), 7.85(1H,t), 7.77-7.62(6H,m), 7.52(1H,t), 7.39(1H,t), 7.25(2H,d) | 481.60 | 481.18 |
| **6** | 9.08(2H,d), 8.69(4H,d), 8.37(1H,d), 8.19(3H,m), 7.94(1H,d), 7.85(5H,m), 7.70-7.62(5H,m), 7.38(1H,t), 7.14(1H,d), 6.90(1H,t) | 508.62 | 508.19 |
| **7** | 9.08(2H,d), 8.69(2H,d), 8.35(3H,m), 8.19(3H,m), 7.94(1H,d), 7.85(3H,m), 7.73-7.61(7H,m), 7.38(1H,t), 7.14(1H,d), 6.90(1H,t) | 508.62 | 508.19 |
| **8** | 9.08(2H,d), 8.69(2H,d), 8.19(3H,m), 8.04(3H,s), 7.94(1H,d), 7.85(3H,m), 7.73(7H,m), 7.62(2H,m), 7.49(4H,m), 7.41(2H,m) | 583.73 | 583.13 |
| **9** | 9.08(2H,d), 8.70(6H,m), 8.19(3H,m), 8.04(3H,s), 7.98(5H,m), 7.85(3H,m), 7.70(3H,t), 7.62(2H,d) | 585.71 | 585.22 |
| **12** | 9.08(2H,d), 8.99(1H,s), 8.69(2H,d), 8.42(1H,d), 8.19(5H,m), 8.08(4H,m), 7.97(3H,m), 7.85(3H,m), 7.70-7.55(11H,m), 7.47-7.32 (5H,m) | 783.97 | 783.29 |
| **16** | 9.08(2H,d), 8.69(2H,d), 8.56(1H,d,), 8.19(3H,m), 7.95(3H,m), 7.84(4H,m), 7.70(3H,m), 7.62(3H,m), 7.50(3H,m), 7.38(2H,m), 7.26(3H,m) | 623.76 | 623.24 |
| **18** | 9.08(2H,d), 8.36(2H,d), 8.19(3H,m), 7.95(3H,m), 7.85(1H,t), 7.77(4H,m), 7.65(5H,m), 7.51(6H,m) | 555.62 | 555.18 |
| **22** | 9.27(2H,d), 8.89(1H,d), 8.76(2H,t), 8.62(1H,d), 8.39(1H,d), 8.32(1H,d), 8.16(1H,d), 7.92(4H,m), 7.82(3H,m), 7.76(3H,m), 7.60(3H,m), 7.29(1H,t) | 482.59 | 482.18 |
| **32** | 9.08(2H,d), 8.70(10H,m), 8.19(5H,m), 8.00(4H,d), 7.94(1H,d), 7.85(7H,m), 7.70(3H,m), 7.62(2H,t) | 738.89 | 738.28 |
| **35** | 9.08(2H,d), 8.70(10H,m), 8.19(5H,m), 8.00(4H,d), 7.94(1H,d), 7.85(3H,m), 7.70(3H,t), 7.62(2H,t), 7.25(4H,m) | 738.89 | 738.28 |
| **37** | 9.08(2H,d), 8.69(2H,d), 8.20(4H,m), 7.95(7H,m), 7.85(1H,t), 7.70(3H,m), 7.62-7.49(8H,m) | 585.71 | 585.22 |
| **38** | 9.08(2H,d), 8.69(2H,d), 8.30(2H,d), 8.20(4H,m), 7.95(5H,m), 7.85(3H,m), 7.70-7. 41(13H,m) | 661.81 | 661.25 |
| **41** | 9.08(2H,d), 8.69(2H,d), 8.33(4H,m), 8.20(4H,m), 7.94(3H,m), 7.85(1H,t), 7.70-7.49(11H,m) | 585.71 | 585.22 |
| **43** | 9.08(2H,d), 8.69(2H,d), 8.30(2H,d), 8.17(4H,m), 7.95(3H,m), 7.85(5H,m), 7.75-7.70(5H,m), 7.62-7.58(3H,m), 7.49(2H,m), 7.41(1H,m) | 635.77 | 635.24 |
| **44** | 9.08(2H,d), 8.69(2H,d), 8.36(4H,m), 8.19(3H,m), 7.95(3H,m), 7.85(1H,t), 7.70(3H,t), 7.62(2H,t), 7.50(6H,m) | 586.70 | 586.22 |
| **46** | 9.08(2H,d), 8.69(2H,d), 8.19(3H,m), 7.95(7H,m), 7.85(1H,t), 7.75-7.62(9H,m), 7.49(4H,t), 7.41(2H,m), 7.25(4H,d) | 738.89 | 738.28 |
| **54** | 9.08(2H,d), 8.69(2H,d), 8.20-8.13(4H,m), 8.06(2H,m), 7.95(3H,m), 7.84(5H,m), 7.70(3H,t), 7.62-7.49(6H,m) | 674.81 | 674.25 |
| **55** | 9.08(2H,d), 8.69(4H,d), 8.19(6H,m), 7.94(2H,d), 7.85(6H,m), 7.70(6H,m), 7.62(4H,m) | 708.86 | 708.26 |
| **57** | 9.08(2H,d), 8.33(2H,m), 8.19(3H,m), 8.08(2H,m), 7.97(2H,m), 7.85(1H,t), 7.73-7.55(10H,m), 7.38(1H,d) | 481.60 | 481.18 |
| **59** | 9.08(2H,d), 8.33(2H,m), 8.19(3H,m), 7.94(2H,m), 7.85(1H,t), 7.75-7.61(12H,m), 7.49(2H,t), 7.41(1H,m) | 507.64 | 507.20 |
| **60** | 9.08(2H,d), 8.71(2H,d), 8.33(2H,m), 8.19(2H,d), 8.00(2H,d), 7.94(2H,m), 7.85(1H,t), 7.73-7.61(10H,m) | 508.62 | 508.19 |
| **61** | 9.08(2H,d), 8.69(2H,d), 8.35(3H,m), 8.49(3H,m), 7.94(1H,d), 7.85(3H,m), 7.71(4H,m), 7.62(3H,m), 7.38(1H,t), 7.14(1H,d), 6.90(1H,t) | 508.62 | 508.19 |
| **63** | 9.08(2H,d), 8.33(2H,m), 8.19(3H,m), 8.04(3H,s), 7.94(1H,d), 7.85(1H,t), 7.73(8H,m), 7.62(3H,m), 7.49(4H,m), 7.41(2H,m) | 583.73 | 583.23 |
| **67** | 9.08(2H,d), 8.99(1H,s), 8.42(1H,d), 8.33(2H,m), 8.19(5H,m), 8.08(4H,m), 7.97(3H,m), 7.85(1H,t), 7.73-7.55(13H,m), 7.45(3H,m), 7.38(2H,m) | 783.97 | 738.29 |
| **70** | 9.08(2H,d), 8.33(2H,m), 8.19(3H,m), 8.09(1H,d), 7.94-7.55(15H,m), 7.45(2H,m), 7.38(1H,d),7.28(5H,m) | 669.83 | 669.25 |
| **71** | 9.08(2H,d), 8.56(1H,d), 8.33(2H,m), 8.19(3H,m), 7.95(3H,m), 7.83(2H,m),7.72(4H,m), 7.62(4H,m), 7.50(3H,m), 7.38(2H,m), 7.27(3H,m) | 623.76 | 623.24 |
| **73** | 9.08(2H,d), 8.50(2H,m), 8.19(3H,m), 7.94(1H,d), 7.86(2H,m), 7.74(8H,m), 7.62(2H,t), 7.51(6H,m) | 555.62 | 555.18 |
| **77** | 9.08(2H,d), 8.33(2H,m), 8.26(1H,s), 8.19(3H,m), 8.08(2H,m), 7.94(1H,d), 7.85(1H,t), 7.73-7.61(9H,m) | 482.59 | 482.18 |
| **87** | 9.08(2H,d), 8.70(8H,m), 8.33(2H,m), 8.20(5H,m), 8.00(4H,d), 7.44(1H,d), 7.85(5H,m), 7.71(4H,m), 7.62(3H,m) | 738.89 | 738.28 |
| **90** | 9.08(2H,d), 8.71(7H,m), 8.33(2H,m), 8.19(5H,m), 8.00(4H,d), 7.94(1H,d), 7.85(3H,m), 7.71(4H,m), 7.62(2H,t), 7.25(4H,d) | 738.89 | 738.28 |
| **92** | 9.08(2H,d), 8.35(3H,m), 8.20(4H,m), 7.94(5H,m), 7.85(1H,t), 7.73-7.49(12H,m) | 585.71 | 585.22 |
| **93** | 9.08(2H,d), 8.33(5H,m), 8.20(4H,m), 7.94(3H,m), 7.85(3H,m), 7.73(6H,m), 7.62-7.41(8H,m) | 661.81 | 661.25 |
| **96** | 9.08(2H,d), 8.34(4H,m), 8.20(4H,m), 7.94(4H,m), 7.85(1H,t), 7.73-7.49(12H,m) | 585.71 | 585.22 |
| **98** | 9.08(2H,d), 8.35(5H,m), 8.17(4H,m), 7.94(1H,d), 7.85(5H,t), 7.73-7.58(7H,m), 7.49(2H,m), 7.41(1H,m) | 635.77 | 635.24 |
| **99** | 9.08(2H,d), 8.35(7H,m), 8.19(3H,m), 7.94(1H,d),7.85(1H,t), 7.71(4H,m), 7.62(2H,t), 7.50(6H,m) | 586.70 | 586.22 |
| **101** | 9.08(2H,d), 8.58(2H,m), 8.35(3H,m), 8.20(5H,m), 7.94(1H,d), 7.85(3H,m), 7.70(6H,m), 7.40(2H,t) | 738.89 | 738.28 |
| **109** | 9.08(2H,d), 8.33(2H,m), 8.15(4H,m), 8.06(2H,m), 7.95(3H,m), 7.84(2H,m), 7.73-7.54(11H,m), 7.25(2H,d), 6.53(1H,s) | 674.81 | 674.25 |
| **110** | 9.08(4H,d), 8.69(2H,d), 8.33(2H,m), 8.19(6H,m), 7.94(2H,d), 7.85(4H,m), 7.73-7.61(12H,m) | 708.86 | 708.26 |
| **111** | 9.08(2H,d), 9.01(1H,s), 8.60(1H,m), 8.19(5H,m), 7.94(1H,d), 7.85(1H,t), 7.74-7.50(12H,m), 7,38(1H,t), 7.20(1H,t), 1.69(12H,s) | 636.80 | 636.26 |
| **112** | 9.08(2H,d), 9.01(1H,s), 8.60(1H,m), 8.20(6H,m), 7.49(1H,d), 7.85(1H,t), 7.74-7.50(12H,m), 7.38(1H,t), 7.26-7.18(7H,m), 7.10(4H,m) | 760.94 | 760.29 |
| **113** | 9.08(2H,d), 8.60(1H,m), 8.45(1H,d), 8.19(4H,m), 7.94(2H,m), 7.86(2H,m), 7.78(1H,s), 7.70-7.49(11H,m), 7.20(1H,t) | 626.78 | 626.18 |
| **115** | 9.08(2H,d), 8.60(1H,m), 8.55(1H,d), 8.19(5H,m), 7.94(2H,d), 7.85(1H,t), 7.70-7.50(15H,m), 7.40(1H,s), 7.35(1H,t), 7.18(2H,m) | 685.83 | 685.25 |
| **130** | 9.08(2H,d), 8.60(1H,m), 8.55(1H,d), 8.19(4H,m), 8.12(1H,d), 7.94(3H,d), 7.85(1H,t), 7.70-7.50(13H,m), 7.35(2H,t), 7.16(2H,t) | 685.83 | 685.25 |
| **131** | 9.08(2H,d), 8.60(1H,m), 8.55(1H,d), 8.20(6H,m), 7.90(4H,m), 7.70-7.54(9H,m), 7.36(2H,m), 7.16(1H,t), 1.69(12H,s) | 636.80 | 636.26 |
| **132** | 9.08(2H,d), 8.60(1H,m), 8.55(1H,d), 8.45(1H,d), 8.19(4H,m), 8.05(1H,d), 7.94(3H,m), 7.85(1H,t), 7.70-7.49(10H,m), 7.35(1H,t), 7.16(1H,t) | 626.78 | 626.18 |
| **134** | 9.08(2H,d), 8.60(1H,m), 8.55(1H,d), 8.19(5H,m), 7.94(2H,m), 7.85(1H,t), 7.70-7.50(15H,m), 7.40(1H,s), 7.35(1H,t), 7.18(2H,m) | 685.83 | 685.25 |
| **135** | 9.08(2H,d), 8.60(1H,m), 8.45(1H,d), 8.19(5H,m), 7.94(2H,m), 7.86(2H,m), 7.78(1H,s), 7.70-7.50(11H,m), 7.20(1H,t) | 626.78 | 626.18 |
| **137** | 9.08(2H,d), 8.60(1H,m), 8.19(7H,m), 7.94(1H,d), 7.87(2H,m), 7.74-7.50(11H,m), 7.38(1H,t), 7.26-7.10(11H,m) | 760.94 | 760.29 |
| **146** | 9.08(2H,d), 8.55(1H,d), 8.30(1H,d), 8.24(2H,m), 8.19(3H,m), 7.93(4H,m), 7.85(1H,t), 7.72(5H,m), 7.60(3H,m), 7.36(2H,m), 7.16(1H,t), 1.69(12H,s) | 636.80 | 636.26 |
| **147** | 9.08(2H,d), 8.55(1H,d), 8.30(2H,d), 8.24(2H,m), 8.19(3H,m), 7.93(4H,m), 7.85(1H,t), 7.74-7.57(8H,m), 7.37(2H,m), 7.26-7.10(11H,m) | 760.94 | 760.29 |
| **148** | 9.08(2H,d), 8.55(1H,d), 8.45(1H,d), 8.30(2H,d), 8.19(3H,m), 7.93(5H,m), 7.85(2H,m), 7.78(1H,s), 7.70-7.49(7H,m), 7.35(1H,t), 7.16(1H,t) | 626.78 | 626.18 |
| **150** | 9.08(2H,d), 8.55(1H,d), 8.30(2H,d), 8.19(4H,m), 7.93(4H,m), 7.85(1H,t), 7.70-7.50(13H,m), 7.40(1H,s), 7.35(1H,t), 7.18(2H,m) | 685.83 | 685.25 |
| **165** | 9.08(2H,d), 8.55(2H,d), 8.30(2H,d), 8.19(3H,m), 8.12(1H,d), 7.93(5H,m), 7.85(1H,t), 7.70-7.57(8H,m), 7.50(2H,d), 7.35(2H,t), 7.16(2H,t) | 685.83 | 685.25 |
| **166** | 9.08(2H,d), 8.55(1H,d), 8.30(2H,d), 8.24-8.17(5H,m), 7.93 (5H,m), 7.85(1H,t), 7.74-7.57(7H,m), 7.37(2H,m), 7.16(1H,t), 1.69 (12H,s) | 636.80 | 636.26 |
| **167** | 9.08(2H,d), 8.55(1H,d), 8.45(1H,d), 8.30(2H,d), 8.19(3H,m), 8.05(1H,d), 7,93(5H,m), 7.85(1H,t), 7.70-7.49(8H,m), 7.35(1H,t), 7.16(1H,t) | 626.78 | 626.18 |
| **169** | 9.08(2H,d), 8.55(1H,d), 8.30(2H,d), 8.19(4H,m), 7.93(4H,m), 7.85(1H,t), 7.70-7.50(13H,m), 7.40(1H,s), 7.35(1H,t), 7.18(2H,m) | 685.83 | 685.25 |
| **170** | 9.08(2H,d), 8.55(1H,d), 8.45(1H,d), 8.30(2H,d), 8.19(3H,m), 7.93(5H,m), 7.86(2H,m), 7.78(1H,s), 7.70-7.49(7H,m), 7.35(1H,t), 7.16(1H,t) | 626.78 | 626.18 |
| **172** | 9.08(2H,d), 8.55(1H,d), 8.30(2H,d), 8.20(4H,m), 7.94-7.85(6H,m), 7.74-7.57(7H,m), 7.49(1H,s), 7.36(2H,m), 7.26-7.16(11H,m) | 760.94 | 760.29 |
| **181** | 9.08(2H,d), 8.84(1H,d), 8.69(2H,d), 8.27(1H,d), 8.19(3H,m), 8.05(1H,s), 7.94-7.85(3H,m), 7.70-7.62(9H,m), 7.25(2H,d) | 531.66 | 531.20 |
| **184** | 9.27(1H,s), 9.08(2H,d), 8.79(1H,d), 8.69(2H,d), 8.69(2H,d), 8.35(4H,m), 8.19(3H,m), 7.94(1H,d),7.85(3H,m), 7.70-7.62(9H,m), 7.52(1H,d) | 581.72 | 581.21 |
| **185** | 9.08(3H,m), 8.84(1H,d), 8.33(2H,m), 8.27(1H,d), 8.19(3H,m), 8.05(1H,s), 7.94-7.85(3H,m), 7.73-7.62(11H,m) | 531.66 | 531.20 |
| **188** | 9.60(1H,d), 9.27(1H,s), 9.08(2H,d), 8.35(5H,m), 8.19(3H,m), 7.94(1H,d), 7.85(1H,t), 7.73-7.61(11H,m), 7.51(2H,d) | 581.72 | 581.21 |
| **189** | 9.08(2H,d), 8.55(1H,d), 8.30(2H,d), 8.19(4H,m), 7.93(4H,m), 7.85(1H,t), 7.60(3H,m), 7.50(1H,t), 7.35(1H,t), 7.18(2H,m) | 520.63 | 520.19 |
| **199** | 9.08(2H,d), 8.60(1H,s), 8.55(1H,d), 8.19(5H,m), 7.94(2H,d), 7.85(1H,t), 7.70-7.58(8H,m), 7.50(1H,t), 7.35(1H,t), 7.18(2H,m) | 520.63 | 520.19 |
| **209** | 9.08(2H,d), 8.69(2H,d), 8.55(2H,d), 8.19(7H,m), 7.94(3H,d), 7.85(3H,m), 7.70(3H,m), 7.60(5H,m), 7.50(2H,t), 7.35(2H,t), 7.18(4H,m) | 761.93 | 761.28 |
| **210** | 9.08(2H,d), 8.55(1H,d), 8.30(3H,d), 8.20-8.13(5H,m), 7.99-7.85(8H,m), 7.77-7.58(10H,m), 7.50(3H,m), 7.35(1H,t), 7.18(2H,m) | 761.93 | 761.28 |
| **220** | 9.08(2H,d), 8.70(3H,m), 8.39(1H,d), 8.23(1H,s), 8.17(2H,d), 7.95(6H,m), 7.70(2H,t), 7.62-7.41(9H,m) | 586.70 | 586.20 |
| **224** | 9.45(1H,s), 9.08(2H,d), 8.82(1H,d), 8.69(2H,d), 8.23(1H,s), 8.17(2H,d), 7.95(6H,m), 7.70-7.49(11H,m) | 586.70 | 586.20 |
| **228** | 9.08(2H,d), 8.69(2H,d), 8.51(1H,d), 8.23(1H,s), 8.20(1H,d), 8.17(2H,d), 8.11(1H,d), 7.95(6H,m), 7.90(1H,d), 7.72-7.49(12H,m) | 635.77 | 635.24 |
| **232** | 9.08(2H,d), 8.69(2H,d), 8.54(1H,d), 8.23(1H,s), 8.17(2H,d), 8.08(2H,m), 7.99-7.94(7H,m), 7.70-7.49(12H,m) | 635.77 | 635.24 |
| **236** | 9.16(1H,s), 9.09(2H,d), 8.69(2H,d), 8.23(1H,s), 8.20(1H,s), 8.15(3H,m), 7.95(7H,m), 7.85(1H,t), 7.70(2H,t), 7.62(1H,t), 7.55-7.49(8H,m) | 635.77 | 635.24 |
| **240** | 9.16(1H,s), 9.09(2H,d), 8.69(2H,d), 8.23(1H,s), 8.20(1H,d), 8.15(3H,m), 7.95(7H,m), 7.85(1H,t), 7.70(2H,t), 7.62(1H,t), 7.55-7.49(10H,m) | 635.77 | 635.24 |
| **244** | 8.94(1H,d), 8.69(2H,d), 8.54-8.46(3H,m), 8.23(1H,s), 8.20(1H,d), 7.95(7H,m), 7.85(1H,t), 7.72-7.49(10H,m) | 586.70 | 586.20 |
| **248** | 9.08(1H,d), 9.00(2H,m), 8.69(2H,d), 8.23(1H,s), 8.17(2H,d), 8.11(1H,d), 7.95(6H,m), 7.70-7.49(11H,m) | 586.70 | 586.20 |
| **252** | 9.39(1H,s), 9.08(1H,d), 8.98(1H,d), 8.75(1H,d), 8.69(2H,d), 8.23(1H,s), 8.17(1H,d), 8.11(1H,d), 7.95(6H,m), 7.78(1H,d), 7.70-7.49(10H,m) | 586.70 | 586.20 |
| **256** | 9.08(1H,d), 8.98(1H,d), 8.69(2H,d), 8.54(1H,d), 8.23(1H,s), 8.17(1H,d), 8.11(1H,d), 7.99-7.94(7H,m), 7.83(1H,d), 7.70-7.49(13H,m) | 635.77 | 635.24 |
| **260** | 9.08(1H,d), 8.98(1H,d), 8.69(2H,d), 8.54(1H,d), 8.23(1H,s), 8.17(1H,d), 8.11(1H,d), 7.99-7.94(8H,m), 7.70-7.49(13H,m) | 635.77 | 635.24 |
| **264** | 9.16(1H,s), 9.10(1H,s), 8.98(1H,d), 8.69(2H,d), 8.23(1H,s), 8.12(3H,m), 7.99-7.94(7H,m), 7.83(1H,d), 7.68-7.49(12H,m) | 635.77 | 635.24 |
| **268** | 8.98(2H,d), 8.69(2H,d), 8.23(1H,s), 8.17(1H,d), 7.99-7.90(9H,m), 7.83(1H,d), 7.70-7.49(13H,m) | 635.77 | 635.24 |
| **272** | 9.08(1H,d), 8.98(1H,d), 8.69(2H,d), 8.23(1H,s), 8.17(1H,d), 7.99-7.90(9H,m), 7.83(1H,d), 7.70-7.49(13H,m) | 635.77 | 635.24 |
| **324** | 9.08(2H,d), 8.69(2H,d), 8.33(6H,m), 8.20(4H,m), 7.94(1H,d), 7.85(3H,m), 7.75-7.62 (7H,m), 7.50(5H,m), 7.41(1H,t) | 661.81 | 661.25 |
| **337** | 9.08(2H,d), 8.69(2H,d), 8.33(6H,m), 8.20(4H,m), 7.94(1H,d), 7.85(7H,m), 7.75-7.62 (7H,m), 7.50(5H,m), 7.41(1H,t) | 737.91 | 737.28 |
| **376** | 9.08(2H,d), 8.69(2H,d), 8.33(4H,m), 8.15(4H,m), 7.94(1H,d), 7.85(3H,m), 7.70(3H,m), 7.60(3H,m), 7.50(3H,m) | 559.67 | 559.20 |
| **385** | 9.08(2H,d), 8.80(1H,d), 8.70(5H,d), 8.45(1H,d), 8.19(4H,t), 7.94(1H,d), 7.90(1H,d), 7.85(1H,t), 7.70(3H,m), 7.60(3H,m), 7.29(1H,d) | 533.63 | 533.19 |
| **388** | 9.08(2H,d), 8.69(2H,d), 8.48(1H,d), 8.28(3H,m), 8.19(3H,m), 7.94(1H,d), 7.85(4H,m), 7.70(3H,t), 7.62(2H,t), 7.54(1H,d), 7.21(1H,t), 7.16(1H,t) | 547.66 | 547.20 |
| **396** | 9.08(2H,d), 8.69(2H,d), 8.56(1H,d), 8.19(3H,m), 7.94(1H,d), 7.85-7.62(12H,m), 7.54(1H,d), 7.25(2H,m), 2.85(2H,q), 1.30(3H,t) | 575.71 | 575.24 |
| **421** | 9.08(2H,d), 8.33(2H,m), 8.19(4H,m), 8.19(4H,m), 8.02-7.94(4H,m), 7.85(1H,t), 7.72(4H,m), 7.62(3H,m), 7.52(2H,m), 7.25(2H,d) | 564.71 | 564.17 |
| **422** | 9.08(2H,d), 8.55(2H,d), 8.33(2H,m), 8.18(7H,m), 7.94(3H,d), 7.85(1H,t), 7.72(4H,m), 7.60(6H,m), 7.50(2H,m), 7.35(2H,m), 7.18(4H,m) | 761.93 | 761.28 |
| **465** | 9.08(2H,d), 8.33(6H,m), 8.19(4H,m), 7.94(2H,d), 7.85(3H,m), 7.75-7.62(8H,m), 7.50(5H,m), 7.41(1H,m) | 661.81 | 661.25 |
| **477** | 9.08(2H,d), 8.33(8H,m), 8.20(4H,m), 7.94(1H,d), 7.85(5H,m), 7.73(6H,m), 7.62(3H,m), 7.50(5H,m), 7.41(1H,t) | 737.91 | 737.28 |
| **515** | 9.08(2H,d), 8.35(4H,m), 8.15(4H,m), 7.94(1H,d), 7.85(4H,m), 7.73-7.58(7H,m), 7.50(3H,m) | 559.67 | 559.20 |
| **528** | 9.08(2H,d), 8.80(1H,d), 8.70(3H,m), 8.45(1H,d), 8.33(2H,m), 8.19(4H,m), 7.94(1H,d), 7.85(4H,m), 7.73-7.56(8H,m), 7.29(1H,d) | 609.73 | 609.22 |
| **530** | 9.08(2H,d), 8.48(1H,d), 8.30(5H,m), 8.19(3H,m), 7.94(1H,d), 7.85(3H,m), 7.71(4H,m), 7.61(3H,m), 7.54(1H,d), 7.21(1H,t), 6.86(1H,t) | 547.66 | 547.20 |
| **538** | 9.08(2H,d), 8.56(1H,d), 8.33(2H,m), 8.19(3H,m), 7.94(1H,m), 7.85-7.70(9H,m), 7.61(3H,m), 7.54(1H,d), 7.28(1H,t), 7.21(1H,t), 2.85(2H,q), 1.30(3H,t) | 575.71 | 575.24 |
| **557** | 9.08(1H,d), 8.98(1H,d), 8.69(2H,d), 8.33(4H,m), 8.23(1H,s), 8.17(1H,d), 8.11(1H,d), 7.96(3H,m), 7.83(1H,d), 7.70-7.49(12H,m) | 585.71 | 585.22 |
| **558** | 9.08(1H,d), 8.98(1H,d), 8.69(2H,d), 8.33(4H,m), 8.23(1H,s), 8.17(1H,d), 8.11(1H,d), 7.96(3H,m), 7.83(1H,d), 7.70-7.49(12H,m) | 585.71 | 585.22 |
| **559** | 9.08(1H,d), 8.98(1H,d), 8.69(2H,d), 8.33(4H,m), 8.23(1H,s), 8.17(1H,d), 8.11(1H,d), 7.96(3H,m), 7.84(5H,m), 7.70-7.49(12H,m) | 661.81 | 661.25 |
| **560** | 9.08(1H,d), 8.98(1H,d), 8.69(2H,d), 8.33(6H,m), 8.23(1H,s), 8.17(1H,d), 8.11(1H,d), 7.99(1H,d), 7.85(2H,m), 7.75-7.61(9H,m), 7.50(5H,m), 7.41(1H,t) | 661.82 | 661.25 |
| **561** | 9.08(1H,d), 8.98(1H,d), 8.69(2H,d), 8.33(4H,m), 8.23(1H,s), 8.17(1H,d), 8.11(1H,d), 7.99(1H,d), 7.85(7H,m), 7.75-7.61(12H,m), 7.50(5H,m), 7.41(1H,t) | 737.91 | 737.28 |
| **562** | 9.19(1H,s) 9.08(1H,d), 8.98(1H,d), 8.74(1H,d), 8.69(2H,d), 8.35(5H,m), 8.17(1H,d), 8.11(1H,d), 8.00(2H,m), 7.83(1H,d), 7.70-7.59(7H,m) | 586.70 | 586.22 |
| **563** | 9.08(1H,d), 8.98(1H,d), 8.69(2H,d), 8.36(4H,m), 8.17(1H,d), 8.11(1H,d), 7.96(3H,m), 7.83(1H,d), 7.70-7.61(6H,m), 7.50(6H,m) | 586.70 | 586.22 |
| **564** | 9.08(1H,d), 8.98(1H,d), 8.69(2H,d), 8.15(3H,m), 7.98(3H,m), 7.82(5H,m), 7.70-7.58(9H,m), 7.49(1H,t) | 635.77 | 635.54 |
| **565** | 9.08(1H,d), 8.98(1H,d), 8.69(2H,d), 8.33(4H,m), 8.15(3H,m), 7.99(1H,d), 7.84(3H,m), 7.70-7.58(7H,m), 7.50(3H,m) | 559.67 | 559.20 |
| **566** | 9.18(1H,d), 9.08(1H,d), 8.98(1H,d), 8.78(1H,d), 8.69(4H,s), 8.55(1H,d), 8.17(1H,d), 8.11(1H,d), 7.99(1H,d), 7.83(1H,d), 7.74-7.61(7H,m), 7.37(1H,t), 7.23(1H,t), 6.88(1H,d) | 509.61 | 509.19 |
| **567** | 9.08(1H,d), 8.98(1H,d), 8.80(1H,d), 8.70(5H,m), 8.45(1H,d), 8.20(1H,d), 8.17(1H,d), 8.11(1H,d), 7.99(1H,d), 7.88(6H,m), 7.70-7.56(7H,m), 7.29(1H,d) | 609.73 | 609.22 |
| **568** | 9.08(1H,d), 8.98(1H,d), 8.69(2H,d), 8.56(1H,d), 8.17(1H,d), 8.11(1H,d), 7.99(3H,m), 7.83(4H,m), 7.70-7.48(10H,m), 7.38(2H,m), 7.27(3H,m) | 623.76 | 623.24 |
| **569** | 9.08(1H,d), 8.98(1H,d), 8.69(2H,d), 8.48(1H,d), 8.28(3H,m), 8.17(1H,d), 8.11(1H,d), 7.99(1H,d), 7.84(5H,m), 7.70-7.61(6H,m), 7.54(1H,d), 7.21(1H,t), 6.86(1H,d) | 547.66 | 547.20 |
| **570** | 9.08(1H,d), 8.98(1H,d), 8.69(2H,d), 8.56(1H,d), 8.17(1H,d), 8.11(1H,d), 7.99(1H,d), 7.85-7.61(13H,m), 7.54(1H,d), 7.28(1H,t), 7.21(1H,t), 2.85(2H,q), 1.30(3H,t) | 575.71 | 575.24 |
| **571** | 9.08(1H,d), 8.98(1H,d), 8.36(1H,d), 8.17(1H,d), 8.11(1H,d), 7.99(1H,d), 7.96(2H,d), 7.83-7.61(11H,m), 7.51(6H,m) | 555.62 | 555.18 |
| **572** | 9.08(1H,d), 8.98(1H,d), 8.69(2H,d), 8.17(1H,d), 8.11(1H,d), 7.99(1H,d), 7.96(4H,m), 7.85-7.61(13H,m), 7.51(6H,m) | 686.82 | 686.25 |
| **573** | 9.08(1H,d), 8.98(1H,d), 8.69(2H,d), 8.42(1H,d), 8.18(3H,m), 8.11-8.06(5H,m), 7.99(3H,d), 7.84(3H,m), 7.70-7.38(17H,m) | 783.97 | 783.29 |
| **574** | 9.08(1H,d), 8.98(1H,d), 8.69(2H,d), 8.21(4H,m), 8.17(1H,d), 8.11(1H,d), 7.99(1H,d), 7.83(1H,d), 7.75-7.61(8H,m), 7.49(2H,t), 7.39(5H,m), 7.25(6H,m) | 683.85 | 683.26 |
| **575** | 9.08(1H,d), 8.98(1H,d), 8.69(2H,d), 8.17(1H,d), 8.11(1H,d), 8.04(3H,s), 7.99(1H,d), 7.85(3H,m), 7.75-7.61(10H,m), 7.49(4H,m), 7.41(2H,m) | 583.73 | 583.23 |
| **576** | 9.08(1H,d), 8.98(1H,d),8.70(6H,m), 8.17(1H,d), 8.11(1H,d), 8.02(8H,m), 7.84(3H,m), 7.68-7.61(6H,m) | 585.71 | 585.22 |
| **577** | 9.08(1H,d), 8.98(1H,d), 8.56(1H,d), 8.28(2H,m), 8.17-8.11(4H,m), 7.99(1H,d), 7.81(4H,m), 7.70-7.50(10H,m), 7.28(1H,t) | 547.66 | 547.20 |
| **578** | 9.08(1H,d), 8.98(1H,d), 8.69(2H,d), 8.26(1H,s), 8.20-8.06(4H,m), 7.99(1H,d), 7.83(1H,d), 7.71-7.58(8H,m), 7.25(2H,m) | 482.59 | 482.18 |
| **579** | 9.08(1H,d), 8.98(1H,d), 8.87(1H,d), 8.69(2H,d), 8.55(1H,d), 8.17(1H,d), 8.11(1H,d), 8.05(1H,d), 7.97(2H,m), 7.83(1H,d), 7.70-7.61(7H,m), 7.49(1H,t), 7.25(2H,m) | 482.59 | 482.18 |
| **580** | 9.08(1H,d), 8.98(1H,d), 8.70(5H,m), 8.39(1H,d), 8.17(1H,d), 8.11(1H,d), 7.97(2H,m), 7.85-7.79(7H,m), 7.70-7.61(6H,m), 7.46-7.41(4H,m) | 635.77 | 635.24 |
| **581** | 9.08(1H,d), 8.98(1H,d), 8.69(2H,m), 8.29(4H,m), 8.20(2H,s), 8.17(1H,d), 8.11(1H,d), 7.99(1H,d), 7.84(3H,m), 7.70-7.49(12H,m) | 584.72 | 584.23 |
| **582** | 9.08(1H,d), 8.98(1H,d), 8.85(2H,d), 8.69(2H,d), 8.37(2H,d), 8.20(2H,s), 8.17(1H,d), 8.11(1H,d), 8.06-7.99(7H,m), 7.84(3H,m), 7.70-7.59(10H,m) | 684.84 | 684.26 |
| **583** | 9.08(1H,d), 8.98(1H,d), 8.69(4H,s), 8.29(2H,d), 8.20(2H,s), 8.17(1H,d), 8.11(1H,d), 7.99(1H,d), 7.83(1H,d), 7.75-7.49(14H,m) | 584.72 | 584.23 |
| **584** | 9.08(1H,d), 8.98(1H,d), 8.85(1H,m), 8.69(4H,s), 8.37(1H,d), 8.20(2H,s), 8.17(1H,d), 8.11-7.99(7H,m), 7.93(1H,d), 7.83(1H,d), 7.72-7.59(12H,m) | 685.84 | 684.26 |
| **585** | 9.08(1H,d), 8.98(1H,d), 8.69(2H,d), 8.36(4H,m), 8.17(1H,d), 8.11(1H,d), 7.98(3H,m), 7.84(3H,m), 7.70-7.61(6H,m), 7.50(6H,m), 7.25(2H,d) | 662.80 | 662.25 |
| **586** | 9.08(1H,d), 8.98(1H,d), 8.69(2H,d), 8.17(1H,d), 8.11(1H,d), 7.98(7H,m), 7.83(1H,d), 7.75-7.61(10H,m), 7.49(4H,m), 7.41(2H,m), 7.25(2H,d) | 738.89 | 738.29 |
| **587** | 9.08(1H,d), 8.98(1H,d), 8.70(4H,m), 8.17(1H,d), 8.11(1H,d), 7.98(1H,m), 7.83(1H,d), 7.70-7.61(6H,m), 7.25(4H,m) | 740.87 | 740.27 |
| **588** | 9.08(1H,d), 8.98(1H,d), 8.69(2H,d), 8.55(2H,d), 8.18(5H,m), 8.11(1H,d), 7.97(3H,m), 7.84(3H,m), 7.70-7.58(9H,m), 7.50(2H,t), 7.35(2H,t), 7.18(4H,m) | 761.93 | 761.28 |
| **589** | 9.08(1H,d), 8.98(1H,d), 8.55(2H,d), 8.30(2H,m), 8.17(1H,d), 8.11(1H,d), 7.99-7.89(9H,m), 7.77-7.61(13H,m), 7.50(2H,m), 7.35(2H,t), 7.16(2H,t) | 761.93 | 761.28 |
| **590** | 9.08(1H,d), 8.98(1H,d), 8.69(2H,d), 8.18(2H,m), 8.11(1H,d), 8.02-7.96(4H,m), 7.84(3H,m), 7.70-7.51(8H,m), 7.25(2H,d) | 564.71 | 564.17 |
| **591** | 9.08(1H,d), 8.98(1H,d), 8.69(2H,d), 8.17(1H,d), 8.11(1H,d), 7.97(3H,m), 7.84(3H,m), 7.74-7.61(8H,m), 7.38(2H,m),7.25(2H,d) | 548.64 | 548.19 |
| **592** | 9.08(1H,d), 8.98(1H,d), 8.69(2H,d), 8.17(1H,d), 8.11-8.06(3H,m), 7.98(4H,m), 7.84(3H,m), 7.70-7.55(11H,m), 7.40(2H,t) | 607.76 | 607.23 |
| **593** | 9.08(1H,d), 8.98(1H,d), 8.69(5H,m), 8.55(1H,d), 8.18(2H,m), 8.11(1H,d), 7.99-7.94(3H,m), 7.85(2H,m), 7.70-7.58(8H,m), 7.50(1H,t), 7.35(1H,t), 7.18(2H,m) | 597.72 | 597.72 |
| **594** | 9.08(1H,d), 8.98(1H,d), 8.74(2H,s), 8.69(2H,s), 8.55(1H,d), 8.18(2H,m), 8.11(1H,d), 7.99-7.94(4H,m), 7.83(1H,d), 7.70-7.58(7H,m), 7.50(1H,t), 7.35(1H,t), 7.18(2H,m) | 598.71 | 598.22 |
| **595** | 9.08(1H,d), 8.98(1H,d), 8.69(2H,m), 8.17(1H,d), 8.11(1H,d), 7.98(5H,m), 7.85-7.61(13H,m), 7.51(6H,m) | 631.71 | 631.21 |
| **596** | 9.08(1H,d), 8.98(1H,d), 8.69(2H,d), 8.43(1H,s), 8.17(2H,m), 8.11(1H,d), 8.00(3H,m), 7.85-7.61(13H,m), 7.53(7H,m), 7.38(1H,d) | 681.77 | 681.22 |
| **597** | 9.08 (1H,d), 8.98(1H,d), 8.68(2H,d),8.56(1H,d), 8.28(2H,m), 8.17(1H,d), 8.11(1H,d), 7.99(1H,d), 7.85-7.50(18H,m), 7.28(1H,t) | 456.55 | 456.16 |
| **598** | 9.08(1H,d), 8.98(1H,d), 8.69(1H,d), 8.18(2H,m), 8.11(1H,d), 8.02-7.96(4H,m), 7.84(3H,m), 7.70-7.51(8H,m), 7.25(2H,d) | 506.61 | 506.18 |
| **599** | 9.08(2H,d), 8.69(2H,d), 8.19(3H,m), 7.94(1H,d), 7.85(7H,m), 7.70(3H,m), 7.62(2H,t) | 456.55 | 456.16 |
| **600** | 9.08(2H,d), 8.91(1H,s), 8.39(1H,d), 8.19(3H,m), 7.99-7.91(3H,m), 7.85(5H,m), 7.70(3H,m), 7.62(2H,t), 7.55(1H,s), 7.38(1H,d) | 506.61 | 506.18 |
| **601** | δ = 9.08(1H,d), 8.98(1H,d), 8.43(2H,s), 8.17(1H,d), 8.11(1H,d), 8.04(1H,s), 7.99(1H,d), 7.83(1H,d), 7.75-7.61(10H,m), 7.49-7.41(6H,m) | 507.64 | 507.20 |
| **602** | δ = 9.08(1H,d), 8.97(3H,m), 8.50(2H,d), 8.43(2H,s), 8.19(3H,m), 8.10(3H,m), 8.04(1H,s), 7.99(1H,d), 7.83-7.61(9H,m), 7.52(2H,m), 7.39(2H,m) | 607.76 | 607.23 |
| **603** | δ = 9.08(1H,d), 8.98(1H,d), 8.43(1H,s), 8.17-7.98(10H,m), 7.83(1H,d), 7.70=-7.51(10H,m), 7.35(2H,m), 7.31(2H,m) | 687.80 | 687.22 |
| **604** | δ = 9.08(1H,d), 8.98(1H,d), 8.56(4H,s), 8.18(3H,m), 8.11(1H,d), 7.99(1H,d), 7.94(2H,m), 7.83(1H,d), 7.70-7.58(9H,m), 7.50(2H,t), 7.35(2H,t), 7.18(4H,m) | 685.83 | 685.25 |
| **605** | δ = 9.19(1H,s) 8.98(1H,d), 8.45(4H,m), 8.17-8.11(7H,m), 8.04(1H,s), 7.99(2H,m), 7.93(2H,m), 7.83(1H,d), 7.70-7.49(10H,m) | 719.92 | 719.17 |
| **606** | δ = 9.08(1H,d), 8.98(1H,d), 8.82(3H,s), 8.80(2H,d), 8.71(2H,d), 8.45(2H,d), 8.19(3H,m), 8.11(1H,d), 7.99(2H,d), 7.90(2H,d), 7.83(1H,d), 7.70-7.56(8H,m), 7.29(2H,d) | 711.83 | 711.24 |
| **607** | δ = 9.08(1H,d), 8.98(1H,d), 8.56(2H,d), 8.43(2H,s), 8.28(4H,m), 8.17(1H,d), 8.11(1H,d), 8.04(1H,s), 7.99(1H,d), 7.83-7.77(10H,m), 7.68-7.61(7H,m), 7.53-7.50(8H,m), 7.28(2H,t) | 892.08 | 891.34 |
| **608** | δ = 9.08(1H,d), 8.98(1H,d), 8.84(2H,d), 8.43(2H,s), 8.27(2H,d), 8.17(1H,d), 8.11(1H,d), 8.05(3H,s), 7.99(1H,d), 7.90(2H,d), 7.83(1H,d), 7.70-7.61(14H,m) | 707.88 | 707.26 |
| **609** | δ = 9.08(1H,d), 8.98(1H,d), 8.43(2H,s), 8.17(1H,d), 8.11(1H,d), 8.04(1H,s), 7.99(1H,d), 7.90-7.78(8H,m), 7.70-7.55(10H,m), 7.47-7.28(17H,m) | 984.21 | 983.36 |

Further, FIG. 4 to FIG. 31 are graphs each illustrating a PL (Photoluminescence) or LTPL (Low Temperature Photoluminescence) emission/absorption spectrum of a compound in a specific UV wavelength region.

PL was measured at room temperature using an LS55 luminescent spectrometer manufactured by Perkin Elmer, and LTPL was measured using an F7000 luminescent spectrometer manufactured by HITACHI, and analyzed using liquid nitrogen under lowtemperature conditions of -196°C (77K).

FIG. 4 is a graph illustrating a PL spectrum of a compound 16 at a wavelength of 302 nm.

FIG. 5 is a graph illustrating an LTPL spectrum of the compound 16 at a wavelength of 382 nm.

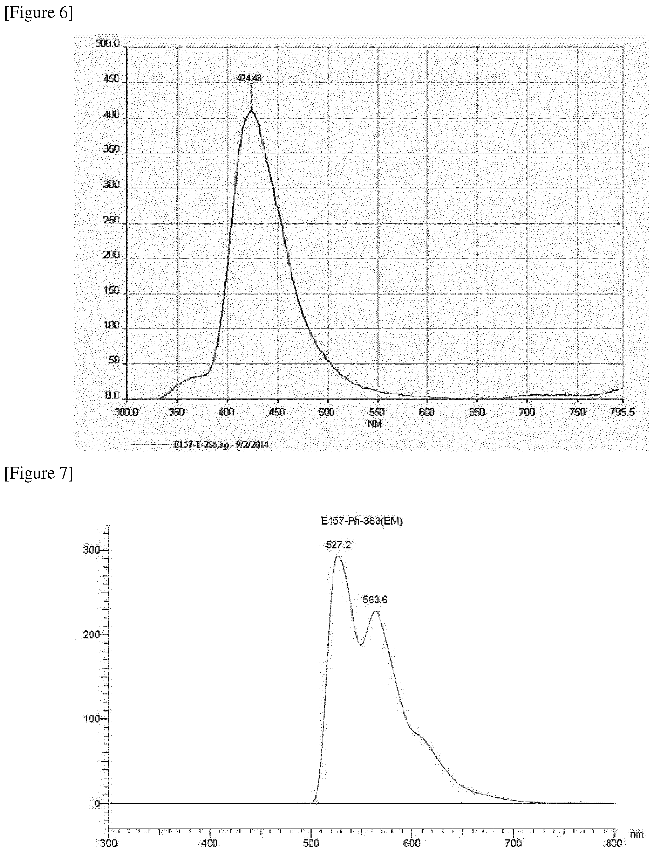

FIG. 6 is a graph illustrating a PL spectrum of a compound 18 at a wavelength of 286 nm.

FIG. 7 is a graph illustrating an LTPL spectrum of the compound 18 at a wavelength of 383 nm.

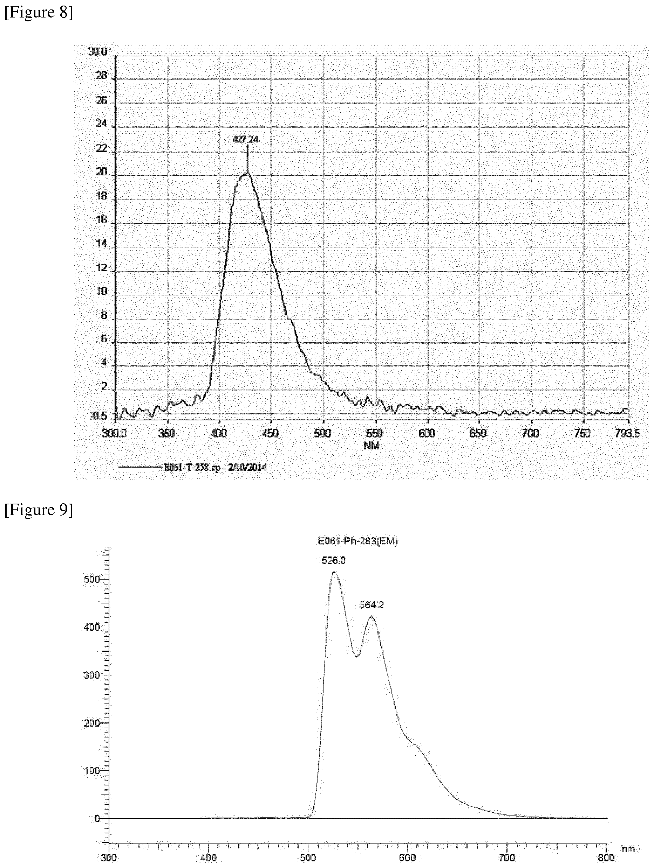

FIG. 8 is a graph illustrating a PL spectrum of a compound 22 at a wavelength of 258 nm.

FIG. 9 is a graph illustrating an LTPL spectrum of the compound 22 at a wavelength of 283 nm.

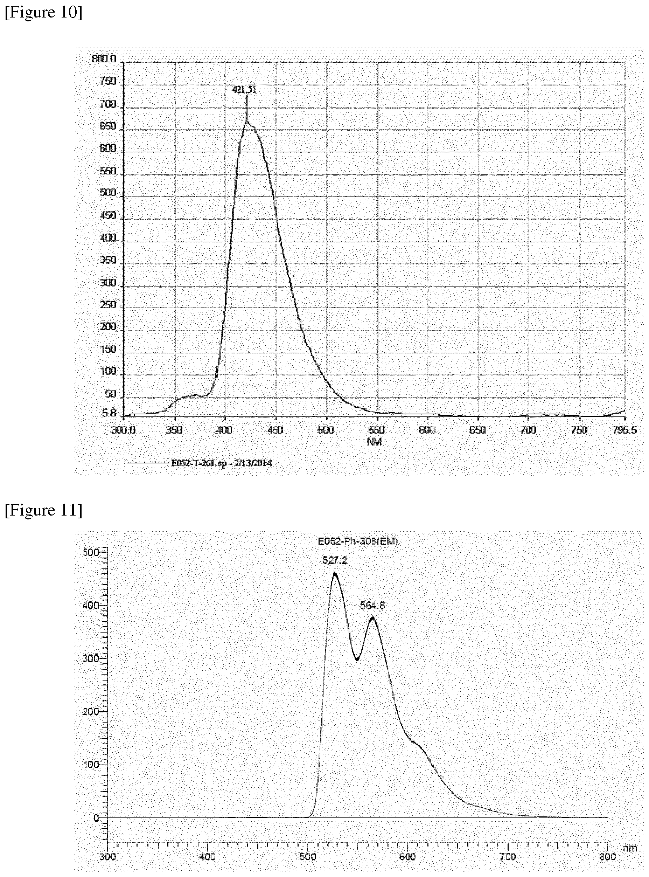

FIG. 10 is a graph illustrating a PL spectrum of a compound 37 at a wavelength of 261 nm.

FIG. 11 is a graph illustrating an LTPL spectrum of the compound 37 at a wavelength of 308 nm.

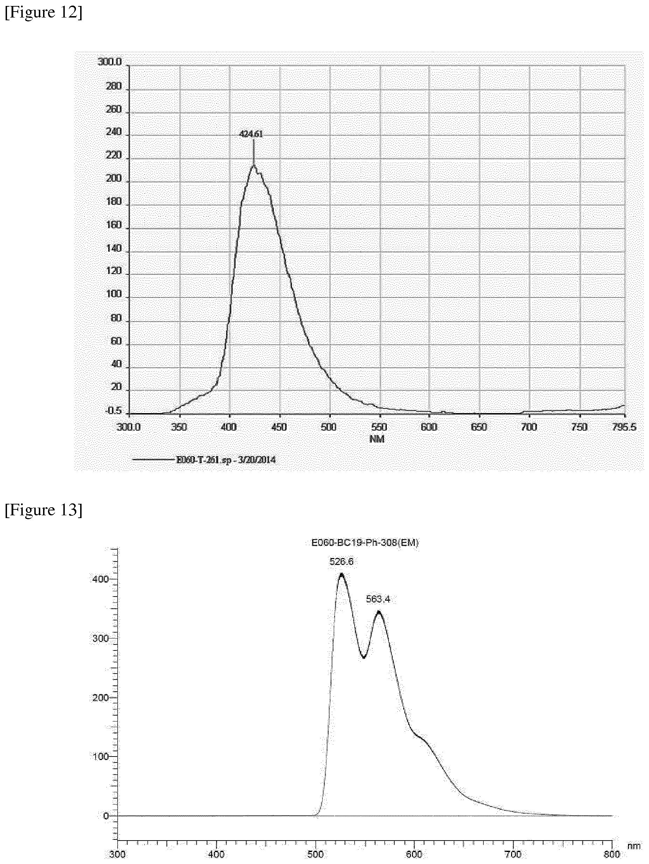

FIG. 12 is a graph illustrating a PL spectrum of a compound 41 at a wavelength of 261 nm.

FIG. 13 is a graph illustrating an LTPL spectrum of the compound 41 at a wavelength of 308 nm.

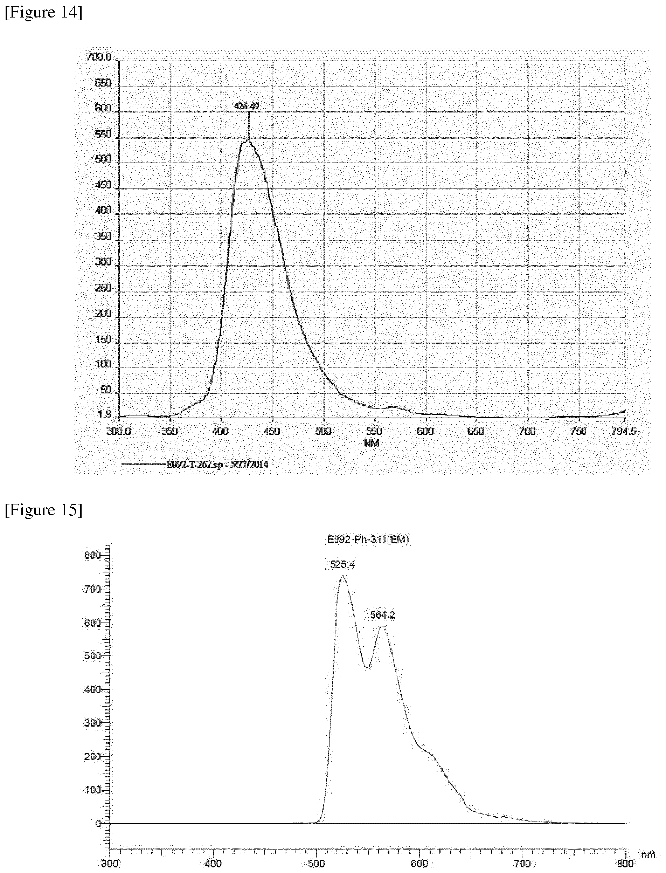

FIG. 14 is a graph illustrating a PL spectrum of a compound 43 at a wavelength of 262 nm.

FIG. 15 is a graph illustrating an LTPL spectrum of the compound 43 at a wavelength of 311 nm.

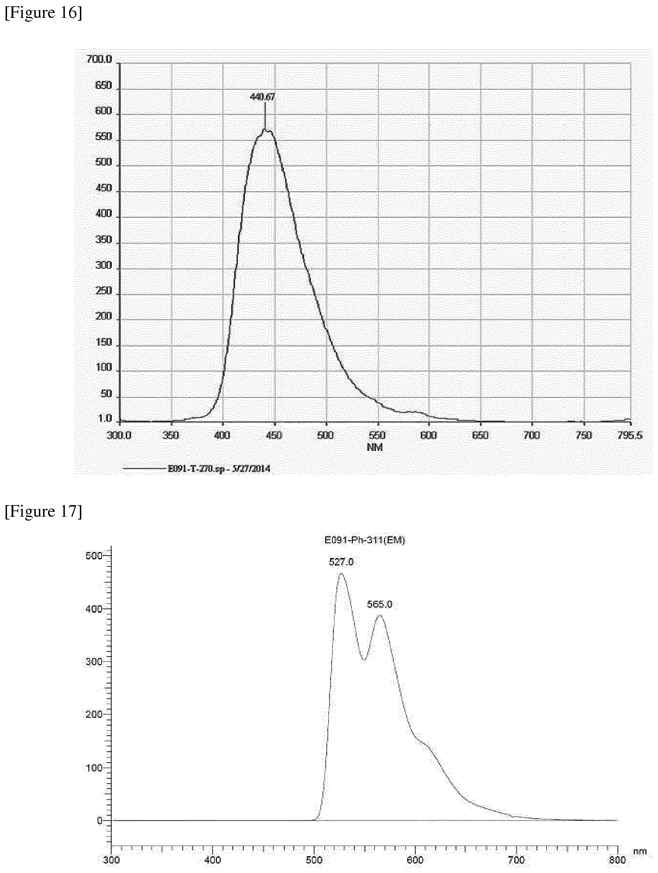

FIG. 16 is a graph illustrating a PL spectrum of a compound 44 at a wavelength of 270 nm.

FIG. 17 is a graph illustrating an LTPL spectrum of the compound 44 at a wavelength of 311 nm.

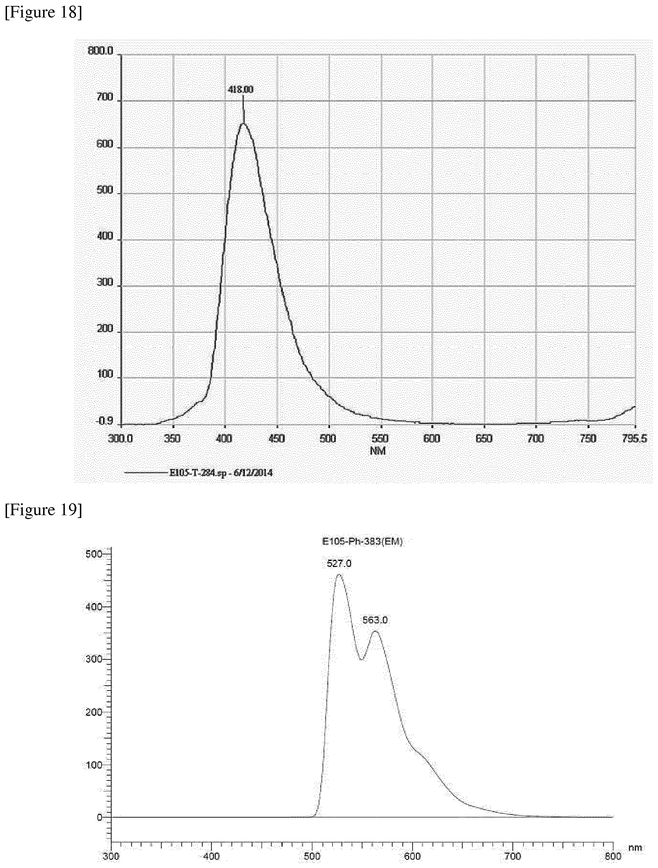

FIG. 18 is a graph illustrating a PL spectrum of a compound 73 at a wavelength of 284 nm.

FIG. 19 is a graph illustrating an LTPL spectrum of the compound 73 at a wavelength of 383 nm.

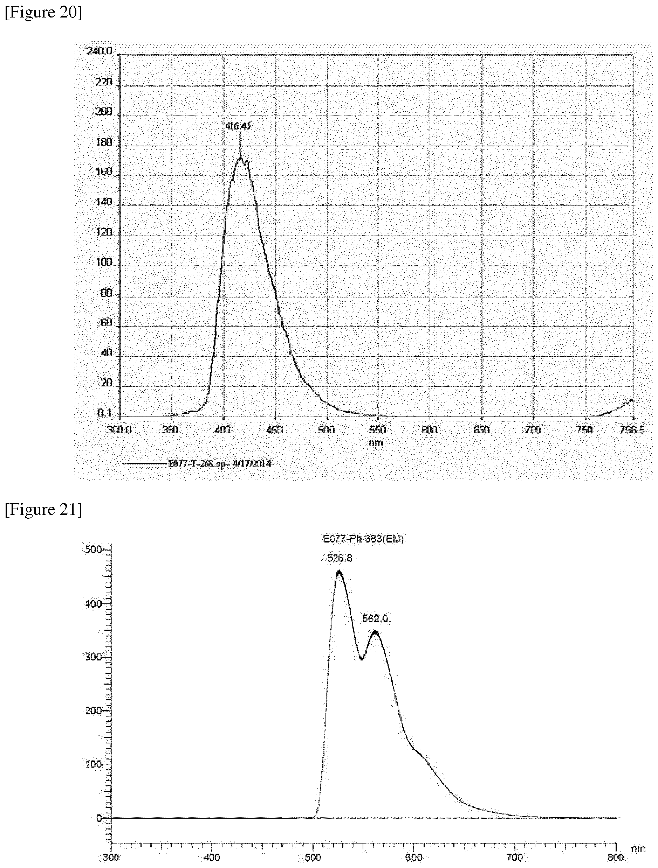

FIG. 20 is a graph illustrating a PL spectrum of a compound 92 at a wavelength of 268 nm.

FIG. 21 is a graph illustrating an LTPL spectrum of the compound 92 at a wavelength of 383 nm.

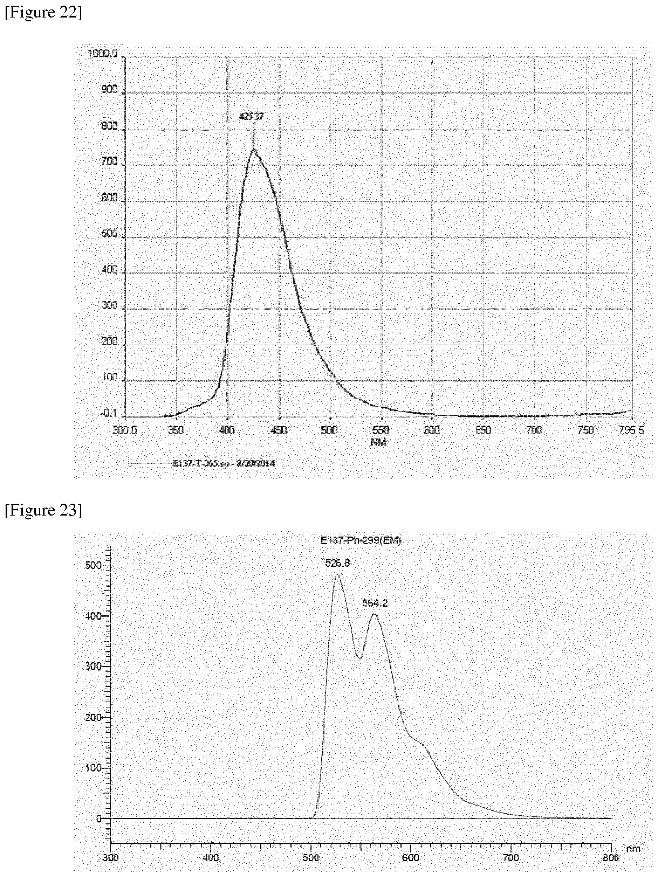

FIG. 22 is a graph illustrating a PL spectrum of a compound 294 at a wavelength of 265 nm.

FIG. 23 is a graph illustrating an LTPL spectrum of the compound 294 at a wavelength of 299 nm.

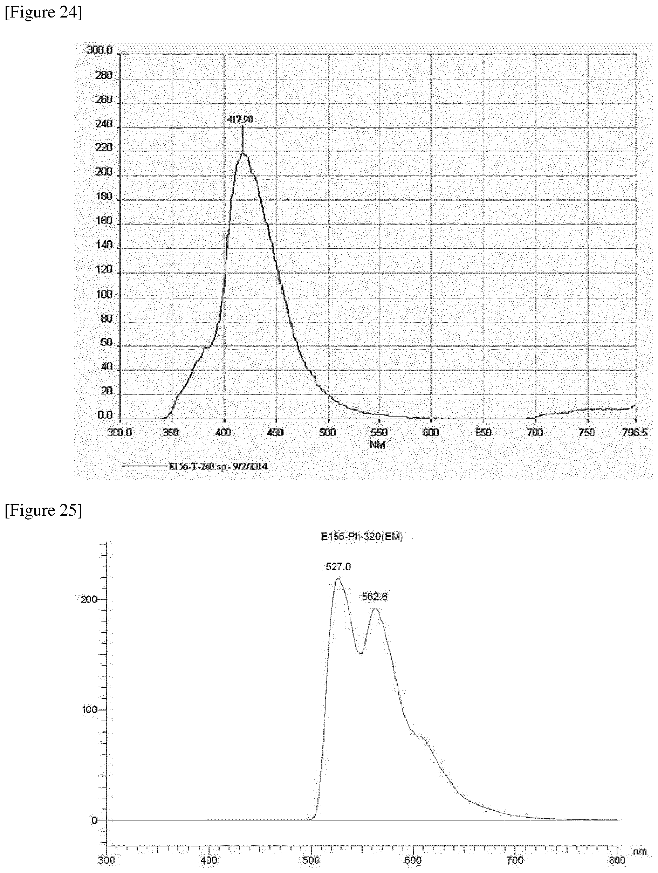

FIG. 24 is a graph illustrating a PL spectrum of a compound 324 at a wavelength of 260 nm.

FIG. 25 is a graph illustrating an LTPL spectrum of the compound 324 at a wavelength of 320 nm.

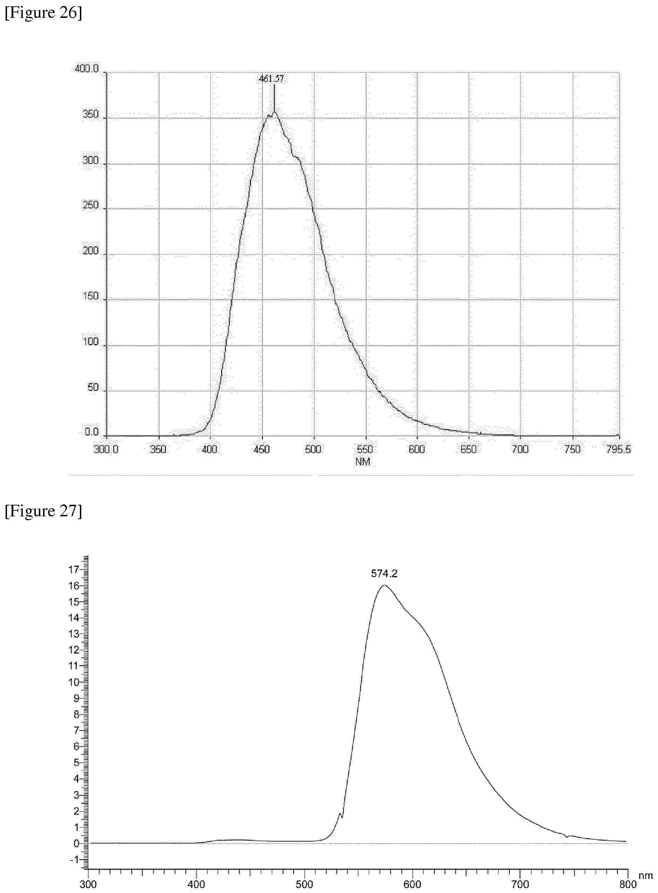

FIG. 26 is a graph illustrating a PL spectrum of a compound 560 at a wavelength of 273 nm.

FIG. 27 is a graph illustrating an LTPL spectrum of the compound 560 at a wavelength of 353 nm.

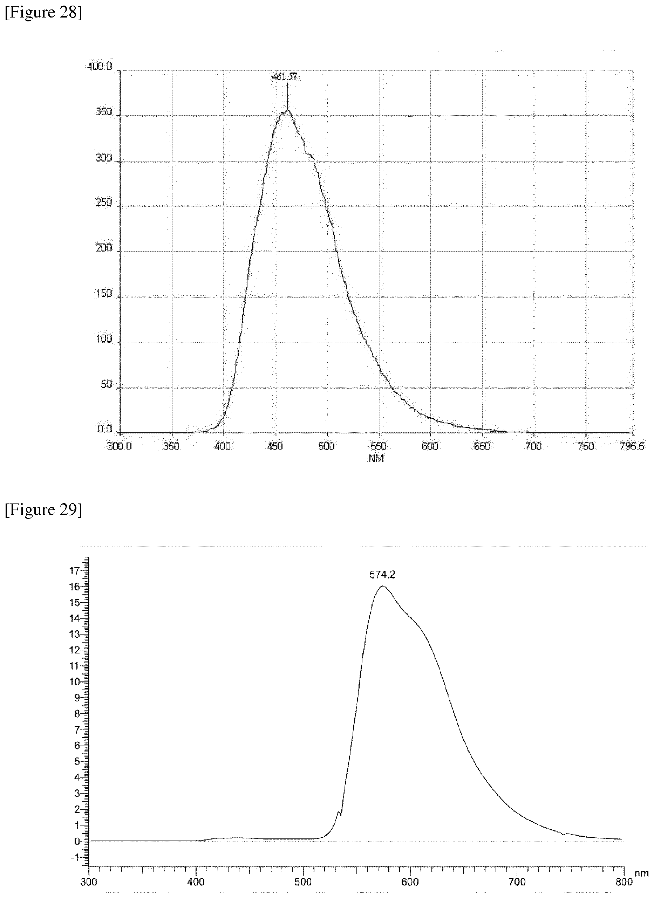

FIG. 28 is a graph illustrating a PL spectrum of a compound 563 at a wavelength of 273 nm.

FIG. 29 is a graph illustrating an LTPL spectrum of the compound 563 at a wavelength of 353 nm.

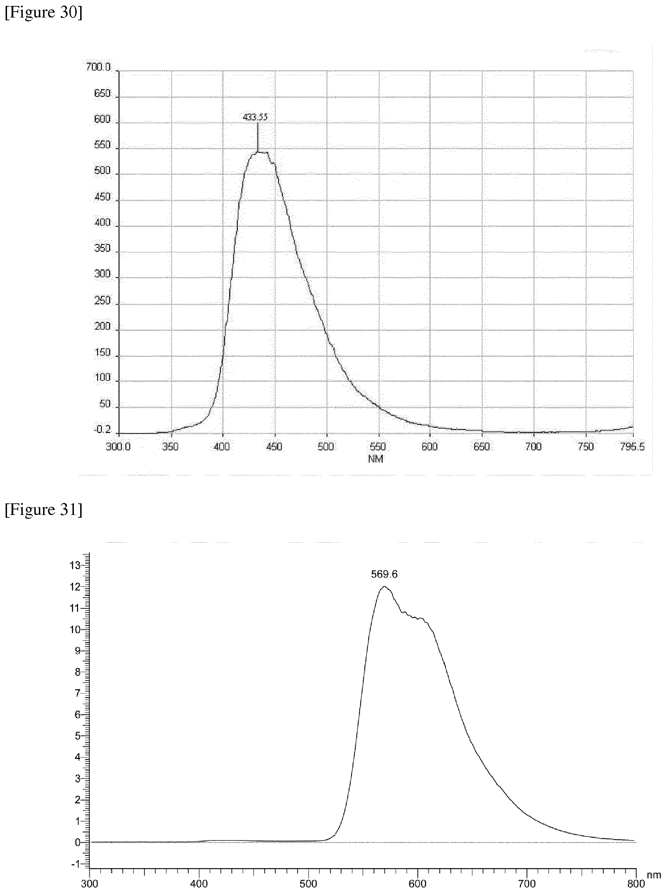

FIG. 30 is a graph illustrating a PL spectrum of a compound 568 at a wavelength of 253 nm.

FIG. 31 is a graph illustrating an LTPL spectrum of the compound 568 at a wavelength of 348 nm.

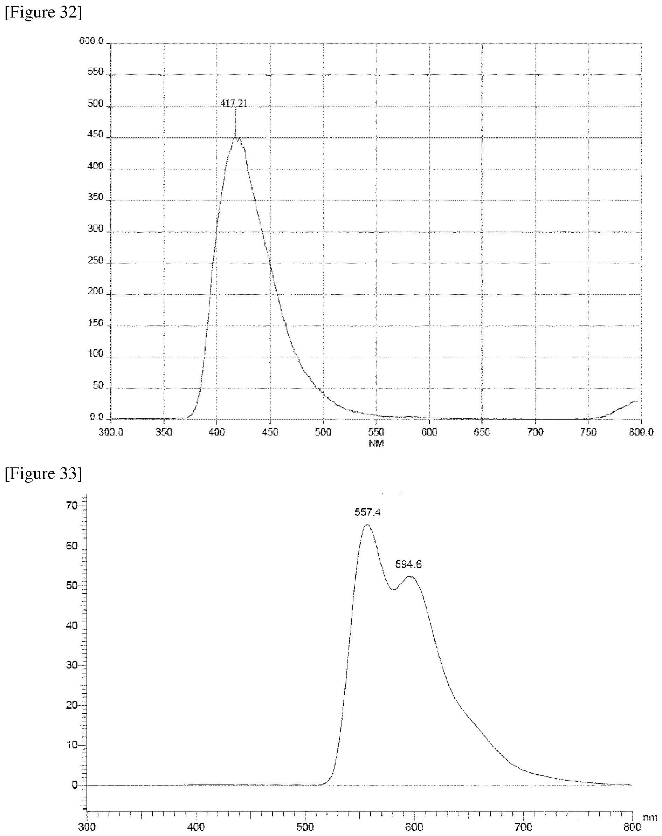

FIG. 32 is a graph illustrating a PL spectrum of a compound 603.

FIG. 33 is a graph illustrating an LTPL spectrum of the compound 603.

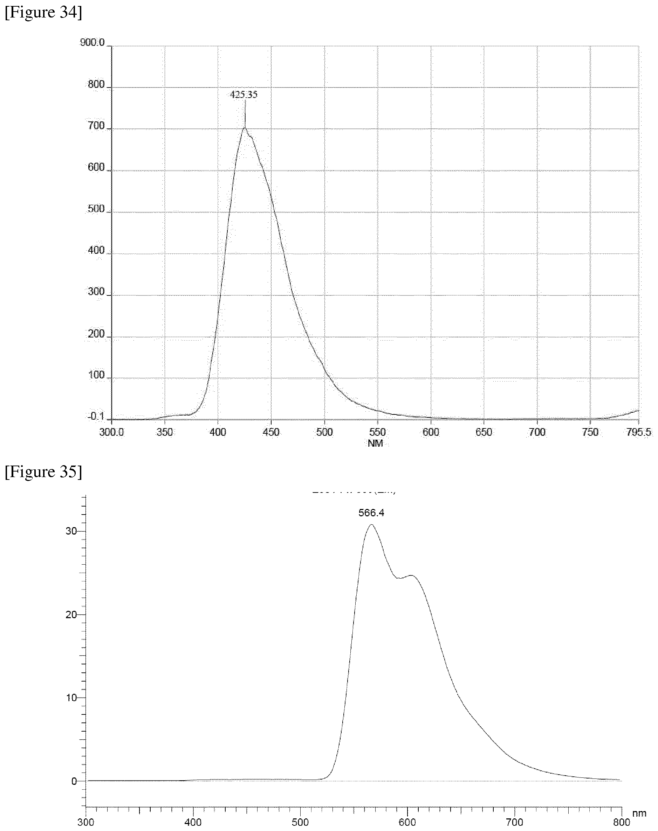

FIG. 34 is a graph illustrating a PL spectrum of a compound 604.

FIG. 35 is a graph illustrating an LTPL spectrum of the compound 604.

In the PL/LTPL graphs as illustrated in FIG. 4 to FIG. 35, the y-axis represents intensity and the x-axis represents a wavelength (unit: nm).

### [Comparative Example 1] Manufacturing of Organic Light Emitting Device

An organic electroluminescent device was manufactured by the following method.

A glass substrate coated with an ITO thin film to a thickness of 1500 Å was ultrasonic cleaned using distilled water. After cleaning with distilled water, the substrate was ultrasonic cleaned using solvents such as acetone, ethanol, or isopropyl alcohol and dried, and then UVO-treated for 5 minutes using UV in a UV cleaner. Then, the substrate was transferred to a plasma cleaner (PT) and plasma-treated in a vacuum to remove ITO work function and a remaining film. Then, the substrate was transferred to a thermal deposition apparatus for depositing an organic substance.

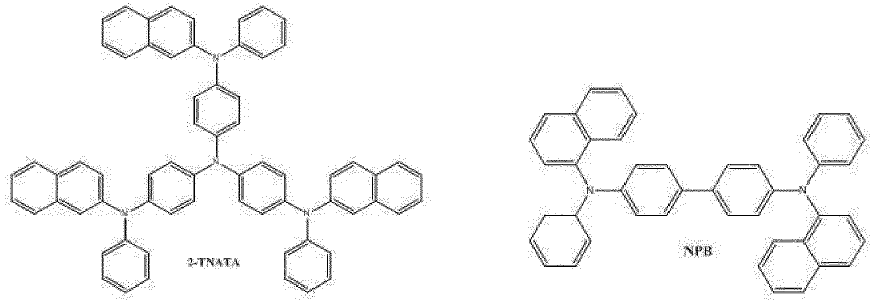

On the ITO transparent electrode (anode) prepared as described above, 4,4', 4"-tris(N,N-(2-naphthyl)-phenylamino)triphenyl amine (2-TNATA) was vacuum-deposited to a thickness of 600 Å so as to form a hole injection layer. Then, N, N'-bis(a-naphthyl)-N,N'-diphenyl-4,4'-diamine (NPB) was vacuum-deposited to a thickness of 300 Å so as to form a hole transport layer.

After the hole injection layer and the hole transport layer as common layers are formed, a light emitting layer was vacuum-deposited thereon as described below. The light emitting layer used CBP as a host and Ir(ppy)₃ as a green phosphorescent dopant. Then, Bebq₂ was deposited as an electron transport layer to a thickness of 200 Å, and on the electron transport layer, lithium fluoride (LiF) was deposited as an electron injection layer to a thickness of 10 Å. Then, on the electron injection layer, aluminum (Al) cathode was deposited to a thickness of 1200 Å so as to form a cathode thereby manufacturing an organic electroluminescent device.

Meanwhile, each of all the organic compounds necessary for manufacturing of an OLED device was vacuumed, sublimed, and purified under 10⁻⁶ to 10⁻⁸ torr, and used for manufacturing OLED.

### [Example 1] Manufacturing of Organic Light Emitting Device

An organic electroluminescent device was manufactured in the same manner as the comparative example 1 except that the compounds 1 to 7 and 10 and 11 synthesized in the preparation examples 1 to 7 and 10 and 11 were used instead of Bebq₂ used in forming the electron transport layer in the comparative example 1.

### [Experimental Example 1-1] Driving Voltage and Efficiency of Organic Electroluminescent Device

Driving voltage and efficiency of the organic electroluminescent devices respectively manufactured in the above-described comparative example 1 and example 1 were measured at a luminescent brightness 6000 cd/m², and the results thereof were as listed in the following Table 3.

**[Table 3]**

| | Electron transport layer material | Driving voltage (V) | Efficiency (cd/A) |
|---|---|---|---|
| Preparation Example 1 | 2 | 5.3 | 44 |
| Preparation Example 2 | 18 | 5.8 | 55 |
| Preparation Example 3 | 22 | 5.0 | 51 |
| Preparation Example 4 | 37 | 5.4 | 47 |
| Preparation Example 5 | 63 | 5.3 | 46 |
| Preparation Example 6 | 73 | 5.7 | 56 |
| Preparation Example 7 | 92 | 5.2 | 48 |
| Preparation Example 10 | 181 | 5.4 | 43 |
| Preparation Example 11 | 209 | 5.3 | 41 |
| Comparative Example 1 | Bebq₂ | 6.6 | 35 |

According to the experimental results, it is exhibited that the organic electroluminescent devices using the compounds of the preparation examples 1 to 7 and 10 and 11 of the present invention have a low driving voltage and a high luminescent efficiency, as compared with the organic electroluminescent device of the comparative example 1 using conventional Bebq₂. Accordingly, it can be seen that since the compound according to the present invention is used as a material of an organic electroluminescent device, a driving voltage and a luminescent efficiency of the device can be improved.

### [Experimental Example 1-2] Life Span of Organic Electroluminescent Device

With respect to the organic electroluminescent devices respectively manufactured in the preparation examples 1 to 7 and 10 and 11, as a driving time of an organic electroluminescent device passes at 2000 cd/m², an average time for brightness to be decreased to 90% of an initial brightness when driving of the device is started was as listed in the following Table 4.

**[Table 4]**

| | Electron transport layer material | T=90 %/hr |
|---|---|---|
| Preparation Example 1 | 2 | 48 |
| Preparation Example 2 | 18 | 58 |
| Preparation Example 3 | 22 | 50 |
| Preparation Example 4 | 37 | 46 |
| Preparation Example 5 | 63 | 47 |
| Preparation Example 6 | 73 | 56 |
| Preparation Example 7 | 92 | 49 |
| Preparation Example 10 | 181 | 48 |
| Preparation Example 11 | 209 | 50 |
| Comparative Example 1 | Bebq₂ | 40 |

According to the experimental results, it is exhibited that the organic electroluminescent devices using the compounds of the preparation examples 1 to 7 and 10 and 11 of the present invention have an excellent life span, as compared with the organic electroluminescent device of the comparative example 1 using conventional Bebq₂.

### [Example 2] Manufacturing of Organic Light Emitting Device

An organic electroluminescent device was manufactured in the same manner as the comparative example 1 except that the compounds 8 and 9 synthesized in the preparation examples 8 and 9 were used instead of CBP used in forming the light emitting layer in the comparative example 1.

### [Experimental Example 2-1] Driving Voltage and Efficiency of Organic Electroluminescent Device

Driving voltage and efficiency of the organic electroluminescent device manufactured in the comparative example 1 or the example 2 were measured at a luminescent brightness 6000 cd/m², and the results thereof were as listed in the following Table 5.

**[Table 5]**

| | Host material | Driving voltage (V) | Efficiency (cd/A) |
|---|---|---|---|
| Preparation Example 8 | 111 | 4.7 | 42 |
| Preparation Example 9 | 130 | 4.8 | 40 |
| Comparative Example 1 | CBP | 6.6 | 35 |

According to the experimental results, it is exhibited that the organic electroluminescent devices using the compounds of the preparation examples 8 and 9 of the present invention have a low driving voltage and a high luminescent efficiency, as compared with the organic electroluminescent device of the comparative example 1 using conventional CBP. Accordingly, it can be seen that since the compound according to the present invention is used as a material of an organic electroluminescent device, a driving voltage and a luminescent efficiency of the device can be improved.

### [Experimental Example 2-2] Life Span of Organic Electroluminescent Device

With respect to the organic electroluminescent devices respectively manufactured in the preparation examples 8 and 9, as a driving time of an organic electroluminescent device passes at 2000 cd/m² an average time for brightness to be decreased to 90% of an initial brightness when driving of the device is started was as listed in the following Table 6.

**[Table 6]**

| | Host material | T=90 %/hr |
|---|---|---|
| Preparation Example 8 | Compound 111 | 54 |
| Preparation Example 9 | Compound 130 | 51 |
| Comparative Example 1 | CBP | 40 |

According to the experimental results, it is exhibited that the organic electroluminescent devices using the compounds of the preparation examples 8 and 9 of the present invention have an excellent life span, as compared with the organic electroluminescent device of the comparative example 1 using conventional CBP.

### [Comparative Example 2]

First, a transparent electrode ITO thin film obtained from an OLED glass (manufactured by Samsung Corning Co., Ltd.) was ultrasonic cleaned using trichloroethylene, acetone, ethanol, and distilled water in sequence for each 5 minutes, and was used after being put into isopropanol.

Then, an ITO substrate was installed in a vacuum deposition apparatus. Thereafter, within a vacuum chamber, 4,4', 4"-tris(N, N-(2-naphthyl)-phenylamino)triphenyl amine (2-TNATA) was vacuum-deposited to a thickness of 600 Å on the ITO so as to form a hole injection layer.

Then, N, N'-bis(α-naphthyl)-N,N'-diphenyl-4,4'-diamine (NPB) was vacuum-deposited to a thickness of 300 Å on the hole injection layer so as to form a hole transport layer.

Thereafter, a light emitting layer was vacuum-deposited to a thickness of 200 Å on the hole transport layer with a blue emission host material HI and a blue emission dopant material D1 at a mixing ratio of 95 : 5.

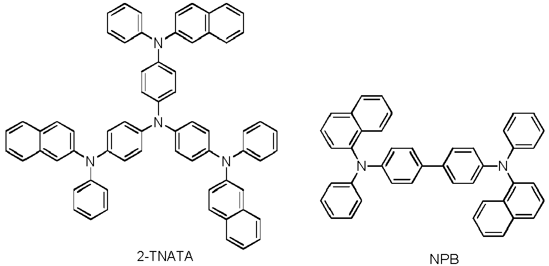

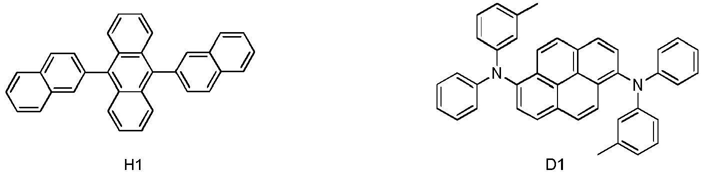

Then, a compound of the following structural formula E1 was deposited to a thickness of 300 Å on the light emitting layer so as to form an electron transport layer.

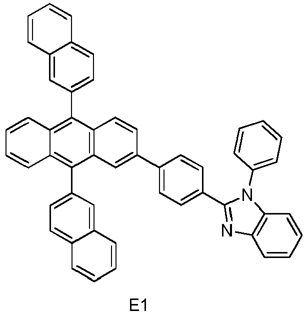

Thereafter, lithium fluoride (LiF) was deposited as an electron injection layer to a thickness of 10 Å on the electron transport layer, and A1 was deposited as a cathode to a thickness of 1000 Å on the electron injection layer, thereby manufacturing an OLED device.

Meanwhile, each of all the organic compound materials necessary for manufacturing of an OLED device was vacuumed, sublimed, and purified under 10⁻⁶ to 10⁻⁸ torr, and used for manufacturing OLED.

### [Example 3]

An organic electroluminescent device was manufactured in the same manner as the comparative example 2 except that the compounds synthesized in the preparation examples 1 to 57 were used instead of E1 used for forming the electron transport layer in the comparative example 2.

### [Experimental Example 3] Evaluation of Organic Electroluminescent Device

Driving voltage, efficiency, color coordinate, and life span of the organic electroluminescent devices respectively manufactured in the above-described comparative example 2 and example 3 were measured at a luminescent brightness 700 cd/m², and the results thereof were as listed in the following Table 7.

Herein, the life span was measured using an M6000PMX manufactured by McScience Co., Ltd.

**[Table 7]**

| Electron transport layer material | Luminescent brightness (cd/m²) | Driving voltage (V) | Efficiency (cd/A) | Color coordinate (x,y) | | Life span (T₅₀) |
|---|---|---|---|---|---|---|
| | | | | x | y | |
| E1 | 700 | 4.70 | 4.50 | 0.150 | 0.180 | 330 |
| Compound 16 | 700 | 5.77 | 3.87 | 0.149 | 0.182 | 355 |
| Compound 18 | 700 | 4.48 | 4.48 | 0.153 | 0.186 | 960 |
| Compound 22 | 700 | 4.22 | 5.59 | 0.150 | 0.182 | 400 |
| Compound 37 | 700 | 4.54 | 5.31 | 0.149 | 0.182 | 456 |
| Compound 41 | 700 | 4.58 | 4.98 | 0.149 | 0.180 | 288 |
| Compound 43 | 700 | 4.48 | 4.94 | 0.148 | 0.182 | 192 |
| Compound 44 | 700 | 5.27 | 3.49 | 0.148 | 0.186 | 672 |
| Compound 45 | 700 | 4.60 | 4.71 | 0.153 | 0.167 | 384 |
| Compound 73 | 700 | 4.69 | 3.45 | 0.150 | 0.177 | 660 |
| Compound 92 | 700 | 5.50 | 4.45 | 0.148 | 0.182 | 380 |
| Compound 100 | 700 | 4.54 | 4.64 | 0.153 | 0.167 | 317 |
| Compound 185 | 700 | 4.48 | 4.86 | 0.153 | 0.170 | 317 |
| Compound 294 | 700 | 4.37 | 4.98 | 0.153 | 0.171 | 576 |
| Compound 324 | 700 | 4.43 | 4.94 | 0.153 | 0.169 | 480 |
| Compound 337 | 700 | 4.43 | 4.56 | 0.153 | 0.166 | 576 |
| Compound 376 | 700 | 4.55 | 4.64 | 0.153 | 0.175 | 499 |
| Compound 385 | 700 | 4.37 | 5.02 | 0.153 | 0.177 | 518 |
| Compound 388 | 700 | 4.45 | 4.86 | 0.153 | 0.180 | 499 |
| Compound 396 | 700 | 4.59 | 4.64 | 0.153 | 0.172 | 509 |
| Compound 404 | 700 | 4.70 | 4.53 | 0.153 | 0.167 | 490 |
| Compound 416 | 700 | 4.69 | 5.09 | 0.153 | 0.167 | 374 |
| Compound 421 | 700 | 4.60 | 4.48 | 0.153 | 0.169 | 432 |
| Compound 422 | 700 | 4.59 | 4.64 | 0.153 | 0.167 | 326 |
| Compound 457 | 700 | 4.53 | 4.79 | 0.153 | 0.168 | 432 |
| Compound 487 | 700 | 4.25 | 4.86 | 0.153 | 0.167 | 461 |
| Compound 489 | 700 | 4.60 | 4.48 | 0.153 | 0.165 | 326 |
| Compound 497 | 700 | 4.51 | 4.71 | 0.153 | 0.170 | 480 |
| Compound 517 | 700 | 4.37 | 5.24 | 0.153 | 0.168 | 653 |
| Compound 518 | 700 | 4.25 | 5.17 | 0.153 | 0.167 | 595 |
| Compound 519 | 700 | 4.13 | 4.71 | 0.153 | 0.169 | 634 |
| Compound 522 | 700 | 4.70 | 4.54 | 0.153 | 0.167 | 672 |
| Compound 530 | 700 | 4.61 | 4.48 | 0.153 | 0.167 | 768 |
| Compound 558 | 700 | 4.5 | 5.3 | 0.150 | 0.180 | 310 |
| Compound 559 | 700 | 4.4 | 5.0 | 0.150 | 0.180 | 340 |
| Compound 560 | 700 | 5.2 | 4.1 | 0.150 | 0.170 | 660 |
| Compound 561 | 700 | 4.7 | 5.1 | 0.150 | 0.180 | 350 |
| Compound 562 | 700 | 5.3 | 4.7 | 0.150 | 0.170 | 700 |
| Compound 563 | 700 | 4.8 | 4.6 | 0.150 | 0.160 | 800 |
| Compound 564 | 700 | 4.6 | 4.7 | 0.150 | 0.170 | 550 |
| Compound 565 | 700 | 4.8 | 4.4 | 0.150 | 0.190 | 480 |
| Compound 566 | 700 | 4.2 | 5.5 | 0.150 | 0.180 | 270 |
| Compound 567 | 700 | 4.7 | 4.5 | 0.150 | 0.170 | 330 |
| Compound 568 | 700 | 4.5 | 4.8 | 0.150 | 0.180 | 400 |
| Compound 569 | 700 | 4.3 | 5.2 | 0.150 | 0.180 | 450 |
| Compound 570 | 700 | 4.0 | 5.8 | 0.150 | 0.170 | 370 |
| Compound 571 | 700 | 5.3 | 4.5 | 0.150 | 0.170 | 900 |
| Compound 572 | 700 | 5.1 | 4.3 | 0.150 | 0.180 | 750 |
| Compound 577 | 700 | 4.6 | 5.1 | 0.150 | 0.170 | 950 |
| Compound 590 | 700 | 4.5 | 5.2 | 0.150 | 0.180 | 380 |
| Compound 597 | 700 | 4.3 | 5.9 | 0.150 | 0.180 | 750 |
| Compound 598 | 700 | 4.5 | 5.5 | 0.150 | 0.180 | 470 |
| Compound 599 | 700 | 4.6 | 5.0 | 0.150 | 0.170 | 430 |
| Compound 600 | 700 | 4.7 | 5.1 | 0.150 | 0.180 | 450 |
| Compound 603 | 700 | 4.2 | 5.1 | 0.150 | 0.170 | 450 |
| Compound 604 | 700 | 4.7 | 5.4 | 0.150 | 0.170 | 550 |
| Compound 605 | 700 | 4.5 | 4.6 | 0.150 | 0.170 | 700 |
| Compound 607 | 700 | 4.4 | 5.1 | 0.150 | 0.170 | 850 |
| Compound 608 | 700 | 4.5 | 5.0 | 0.150 | 0.170 | 400 |

As can be seen from the results listed in Table 7, each organic electroluminescent device using a compound according to the exemplary embodiment of the present application has a low driving voltage and a high luminescent efficiency as compared with the organic electroluminescent device of the comparative example 2 using E1 as an electron transport layer material. Further, it has an excellent durability, i.e., an excellent life span, as compared with the comparative example 2.

## Claims

1. A compound of the following chemical formula 2, 3 or 9; 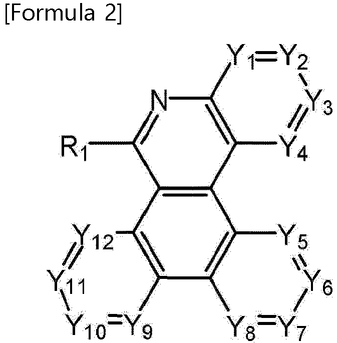 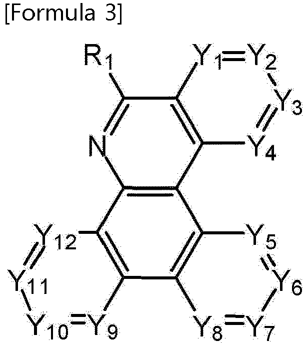 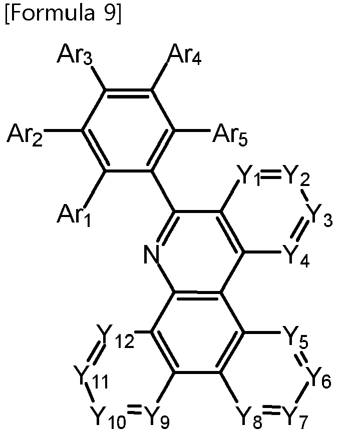 wherein
Y1 to Y12 are the same as or different from each other, and are each independently N or CR,
R1 is -(L)m-(Z)n,
L is substituted or unsubstituted C6 to C60 monocyclic or polycyclic arylene; or substituted or unsubstituted C2 to C60 monocyclic or polycyclic heteroarylene,
Z is one selected from the group consisting of substituted or unsubstituted C6 to C60 monocyclic or polycyclic aryl; substituted or unsubstituted C2 to C60 monocyclic or polycyclic heteroaryl; -SiR11R12R13; and -P(=O)R14R15,
m is an integer of 1 to 5,
n is an integer of 1 to 3,
when m and n are independently integers of 2 or more, multiple L and Z are the same as or different from each other,
R is one selected from the group consisting of hydrogen; or at least two adjacent Rs are bonded to each other to form a substituted or unsubstituted monocyclic or polycyclic aliphatic or aromatic hydrocarbon ring;
wherein "substituted or unsubstituted" refers to a group that may be substituted or may not be further substituted with one or more substituents selected from the group consisting of halogen; -CN; linear or branched C1 to C60 alkyl; C3 to C60 monocyclic or polycyclic cycloalkyl; C6 to C60 monocyclic or polycyclic aryl; C2 to C60 monocyclic or polycyclic heteroaryl; -SiR11R12R13; and - P(=O)R14R15,
R11 to R15 are the same as or different from each other, and are each independently one selected from the group consisting of linear or branched substituted or unsubstituted C1 to C60 alkyl; substituted or unsubstituted C3 to C60 monocyclic or polycyclic cycloalkyl; substituted or unsubstituted C6 to C60 monocyclic or polycyclic aryl; and substituted or unsubstituted C2 to C60 monocyclic or polycyclic heteroaryl, and
at least two of Ar1 to Ar5 are monocyclic or polycyclic substituted or unsubstituted C6 to C60 aryl; or monocyclic or polycyclic substituted or unsubstituted C2 to C60 heteroaryl, and the others are each independently hydrogen; deuterium; halogen; straight-chained or branched substituted or unsubstituted C1 to C60 alkyl; monocyclic or polycyclic substituted or unsubstituted C6 to C60 aryl; or monocyclic or polycyclic substituted or unsubstituted C2 to C60 heteroaryl.

2. The compound of claim 1, wherein all of the Y1 to Y4 are CR, or any one of the Y1 to Y4 is N and the others are CR, and
the R is the same as defined in claim 1.

3. The compound of claim 1, wherein all of the Y5 to Y8 are CR, or any one of the Y5 to Y8 is N and the others are CR, and
the R is the same as defined in claim 1.

4. The compound of claim 1, wherein all of the Y9 to Y12 are CR, or any one of the Y9 to Y12 is N and the others are CR, and
the R is the same as defined in claim 1.

5. The compound of claim 1, wherein the Y1 to Y12 are CR, and the R is the same as defined in claim 1.

6. The compound of claim 1, wherein
L is one selected from the group consisting of C6 to C60 monocyclic or polycyclic arylene unsubstituted or substituted with halogen, linear or branched C1 to C60 alkyl, C3 to C60 monocyclic or polycyclic cycloalkyl, C6 to C60 monocyclic or polycyclic aryl, and C2 to C60 monocyclic or polycyclic heteroaryl; and C2 to C60 monocyclic or polycyclic heteroarylene unsubstituted or substituted with halogen, linear or branched C1 to C60 alkyl, C3 to C60 monocyclic or polycyclic cycloalkyl, C6 to C60 monocyclic or polycyclic aryl, and C2 to C60 monocyclic or polycyclic heteroaryl,
Z is one selected from the group consisting of C6 to C60 monocyclic or polycyclic aryl unsubstituted or substituted with halogen, linear or branched C1 to C60 alkyl, C3 to C60 monocyclic or polycyclic cycloalkyl, C6 to C60 monocyclic or polycyclic aryl, and C2 to C60 monocyclic or polycyclic heteroaryl; C2 to C60 monocyclic or polycyclic heteroaryl unsubstituted or substituted with halogen, linear or branched C1 to C60 alkyl, C3 to C60 monocyclic or polycyclic cycloalkyl, C6 to C60 monocyclic or polycyclic aryl, and C2 to C60 monocyclic or polycyclic heteroaryl; -SiR11R12R13; and -P(=O)R14R15,
R is hydrogen; and the R11 to R15 are the same as or different from each other, and are each independently one selected from the group consisting of hydrogen; linear or branched alkyl unsubstituted or substituted with halogen, linear or branched C1 to C60 alkyl, C3 to C60 monocyclic or polycyclic cycloalkyl, C6 to C60 monocyclic or polycyclic aryl, and C2 to C60 monocyclic or polycyclic heteroaryl; C6 to C60 monocyclic or polycyclic aryl unsubstituted or substituted with halogen, linear or branched C1 to C60 alkyl, C3 to C60 monocyclic or polycyclic cycloalkyl, C6 to C60 monocyclic or polycyclic aryl, and C2 to C60 monocyclic or polycyclic heteroaryl; and C2 to C60 monocyclic or polycyclic heteroaryl unsubstituted or substituted with halogen, linear or branched C1 to C60 alkyl, C3 to C60 monocyclic or polycyclic cycloalkyl, C6 to C60 monocyclic or polycyclic aryl, and C2 to C60 monocyclic or polycyclic heteroaryl, and
if two or more of the Y1 to Y12 are CR, multiple R are the same as or different from each other.

7. The compound of claim 1, wherein Z is substituted or unsubstituted phenyl, substituted or unsubstituted biphenyl, substituted or unsubstituted naphthyl, substituted or unsubstituted chrysenyl, substituted or unsubstituted pyrenyl, substituted or unsubstituted triphenylenyl, substituted or unsubstituted anthracenyl, substituted or unsubstituted phenanthrenyl, substituted or unsubstituted fluorenyl, substituted or unsubstituted benzo chrysenyl, or substituted or unsubstituted spirobifluorenyl.

8. The compound of claim 1, wherein Z is substituted or unsubstituted C2 to C60 monocyclic or polycyclic heteroaryl, and the heteroaryl includes at least one selected from N, O, S, Si, and Se as a heteroatom.

9. The compound of claim 1, wherein Z is 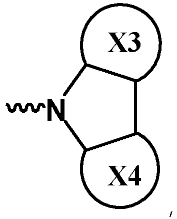 and X3 and X4 are substituted or unsubstituted C6 to C60 monocyclic or polycyclic aromatic hydrocarbon rings; or substituted or unsubstituted C2 to C60 monocyclic or polycyclic aromatic hetero rings.

10. The compound of claim 9, wherein 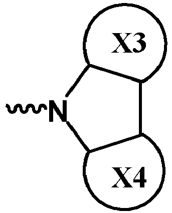 is represented by any one of the following structural formulas: 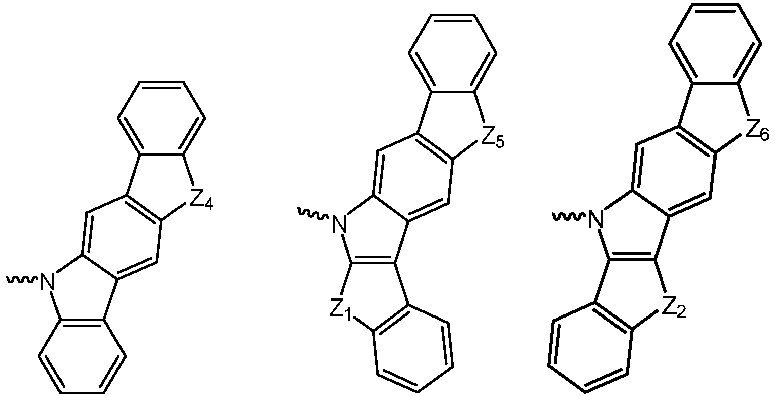 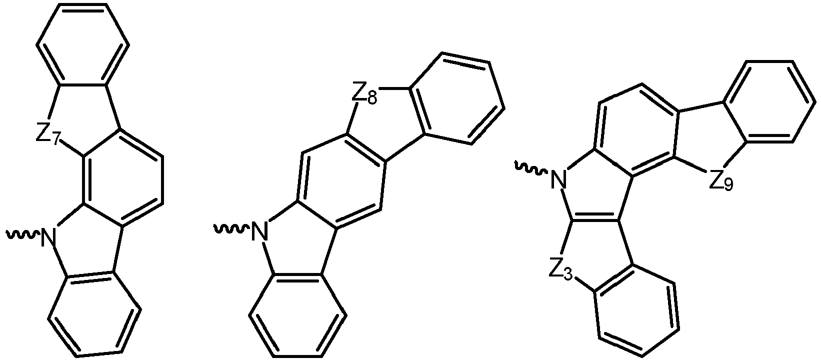
wherein in the structural formulas, Z1 to Z3 are the same as or different from each other, and are each independently S or O,
Z4 to Z9 are the same as or different from each other, and are each independently CR' R", NR', S, or O,
R' and R" are the same as or different from each other, and are each independently hydrogen; linear or branched substituted or unsubstituted C1 to C60 alkyl; or substituted or unsubstituted C6 to C60 monocyclic or polycyclic aryl

11. The compound of claim 1, represented by the following chemical formula 4 or 5: 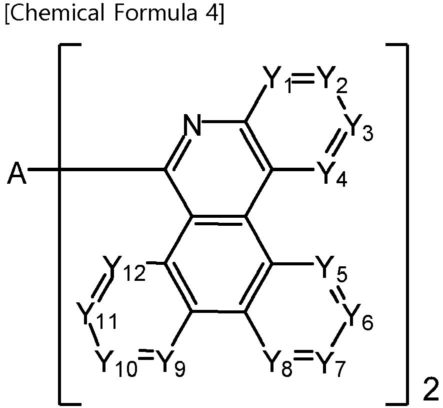 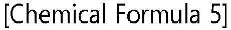 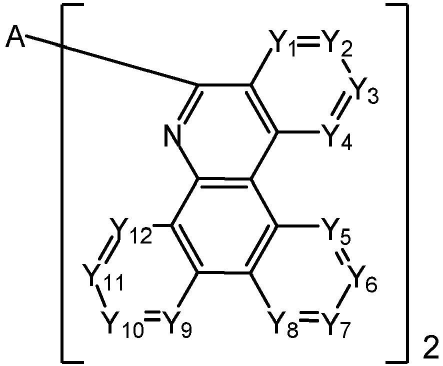 wherein in the chemical formulas 4 and 5,
A is selected from the group consisting of substituted or unsubstituted C6 to C60 monocyclic or polycyclic arylene, and substituted or unsubstituted C2 to C60 monocyclic or polycyclic heteroarylene, and
Y1 to Y12 are the same as defined in claim 1

12. The compound of claim 1, wherein the compound is selected from the following compounds:

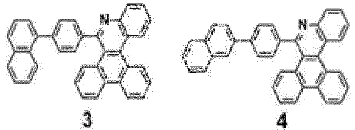

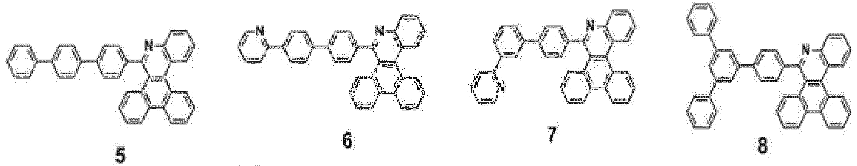

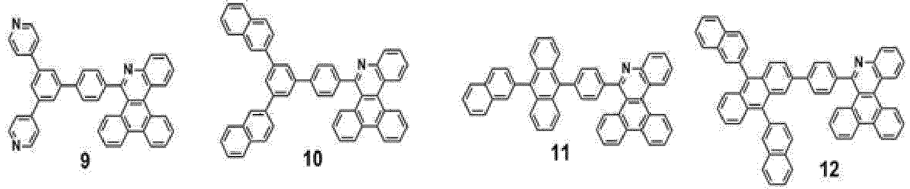

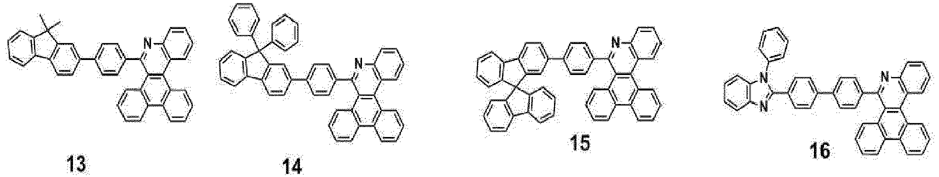

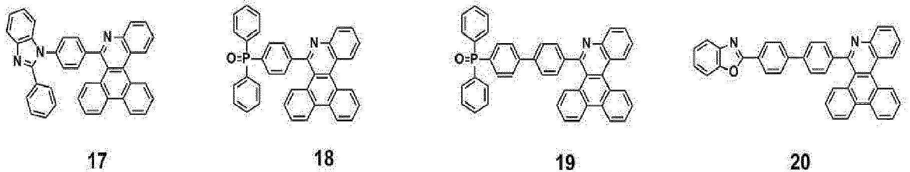

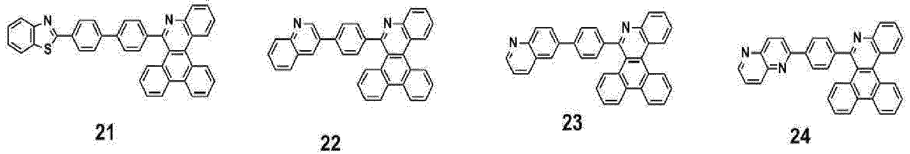

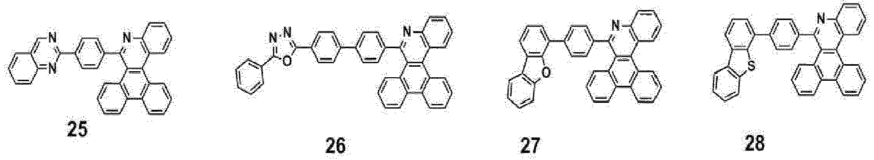

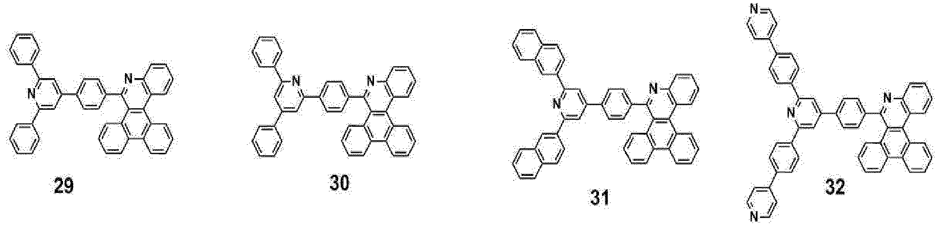

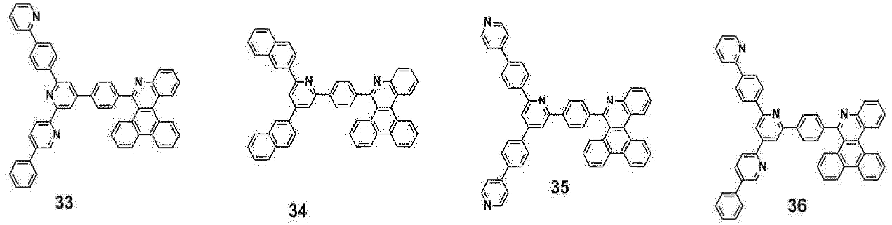

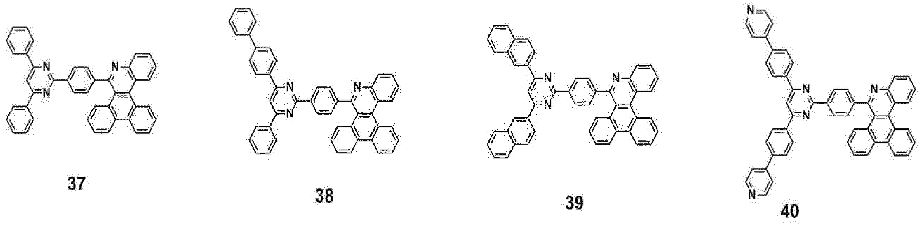

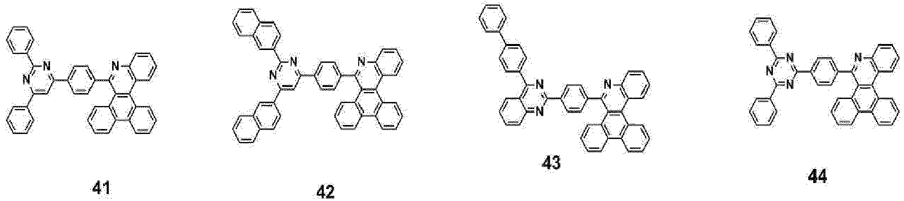

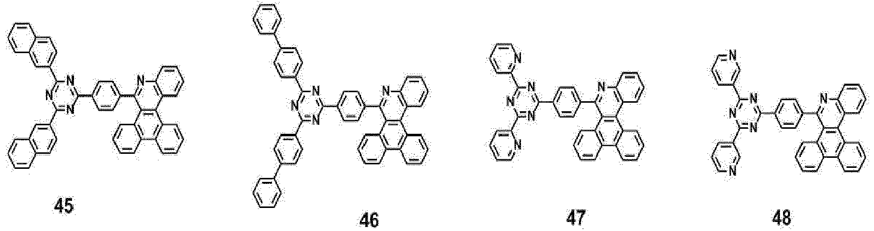

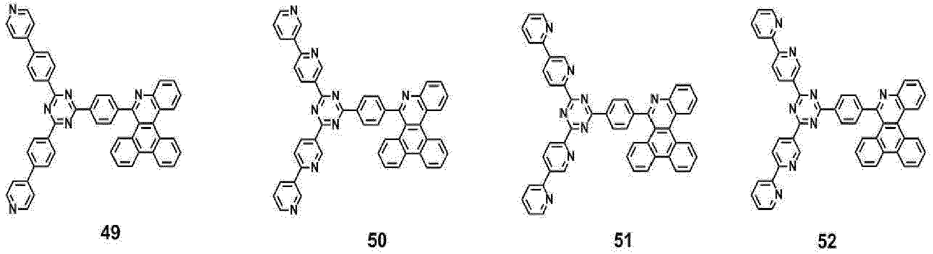

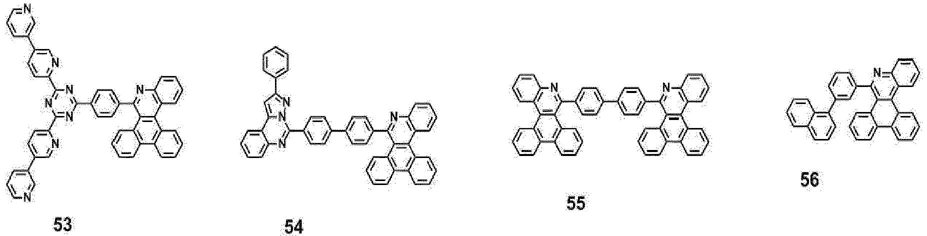

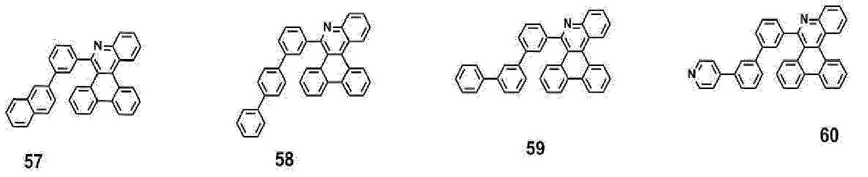

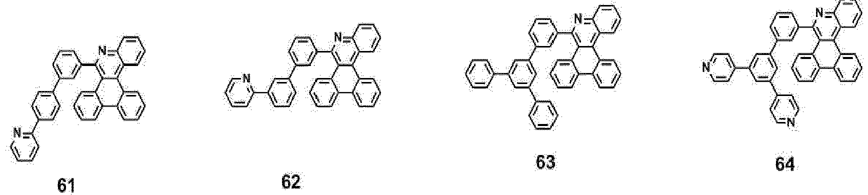

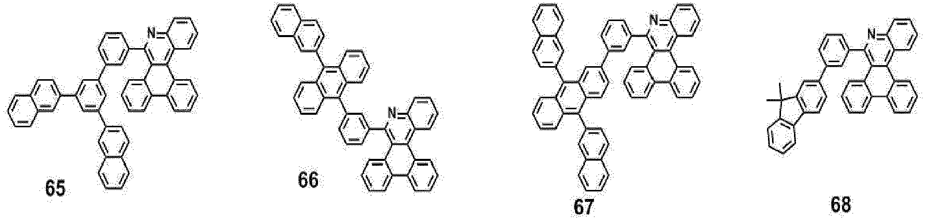

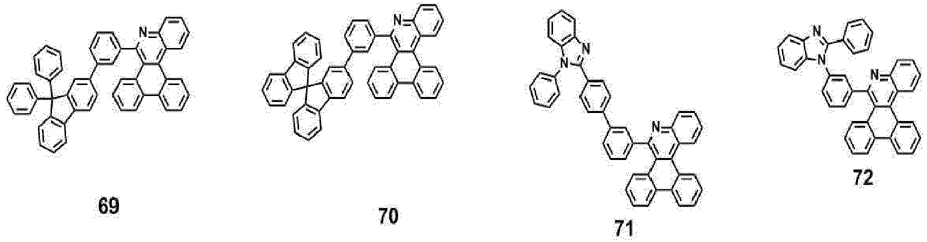

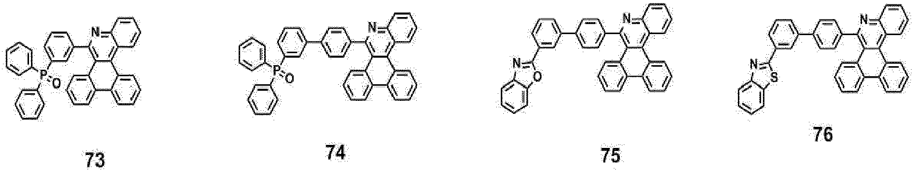

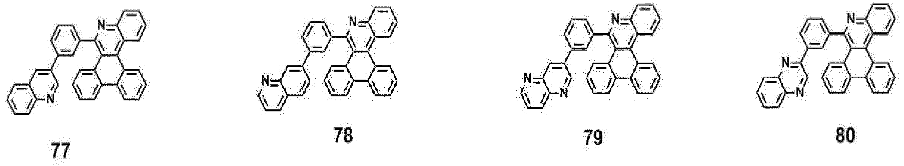

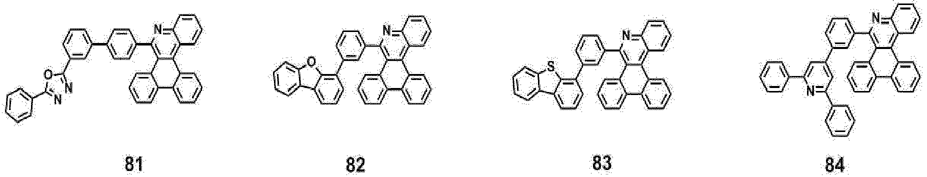

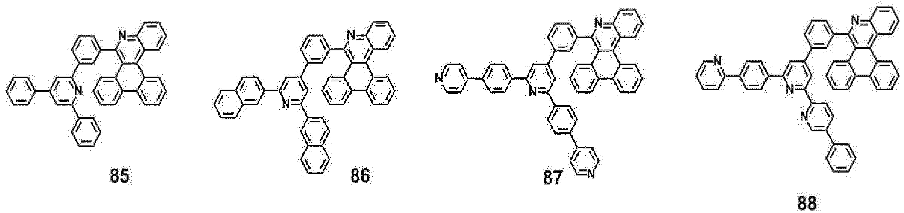

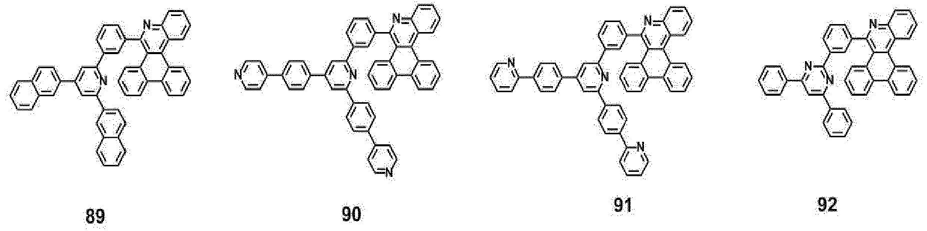

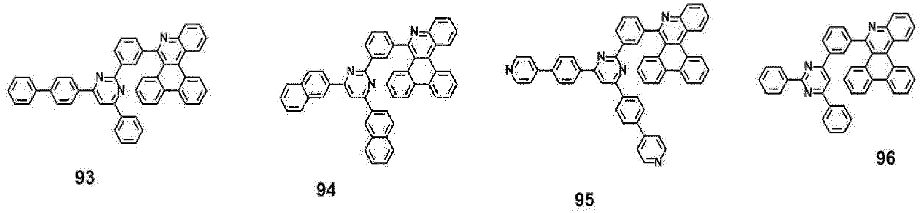

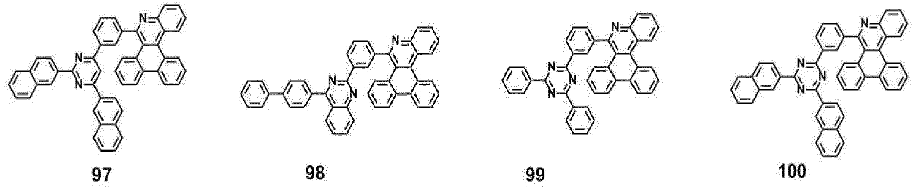

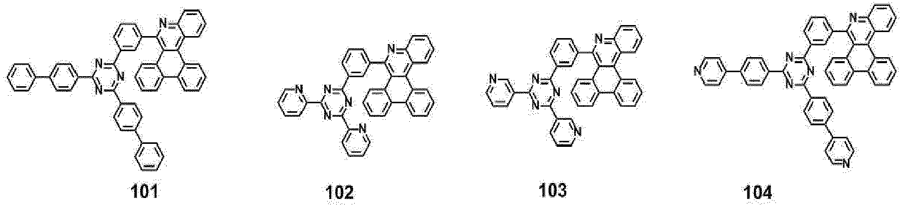

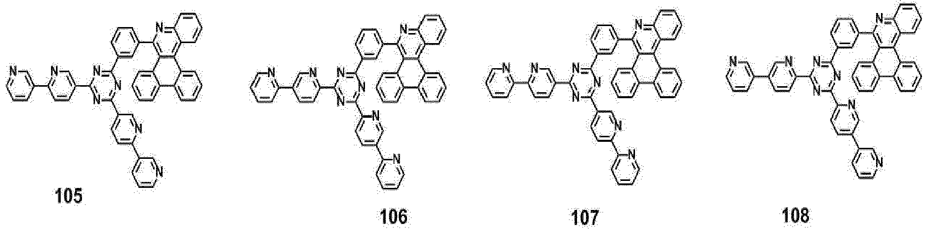

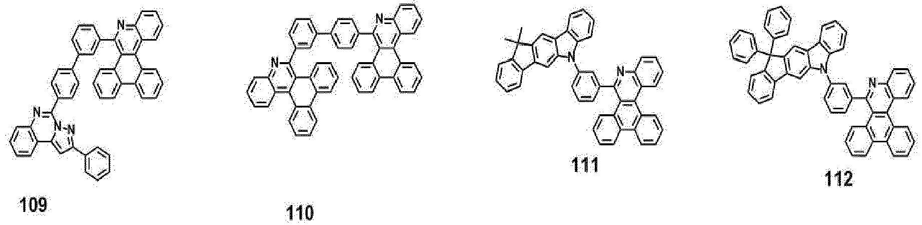

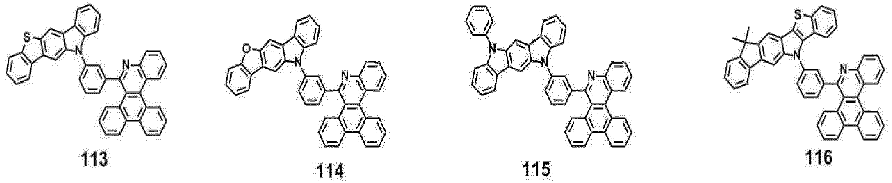

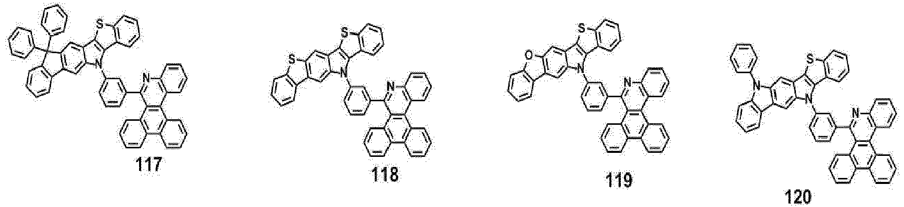

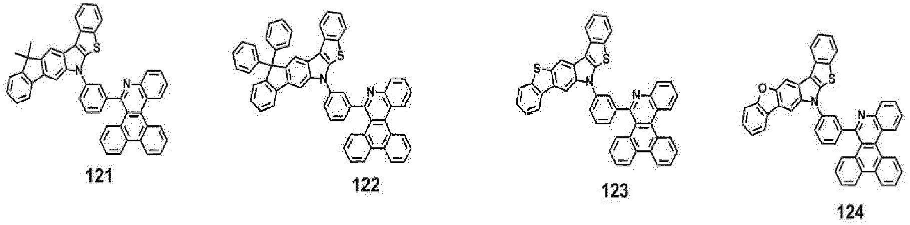

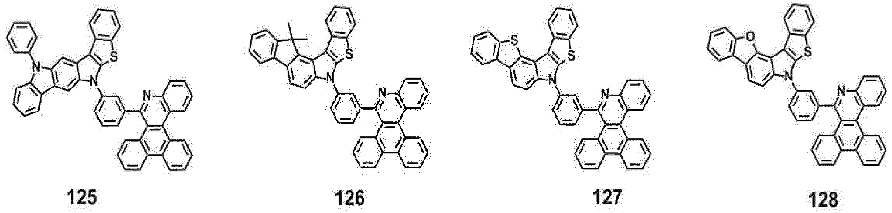

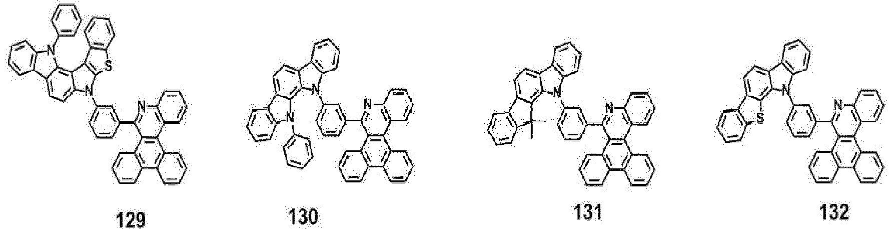

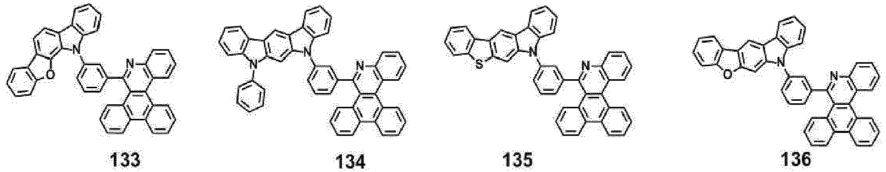

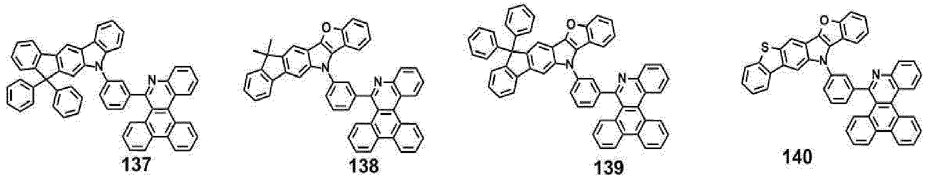

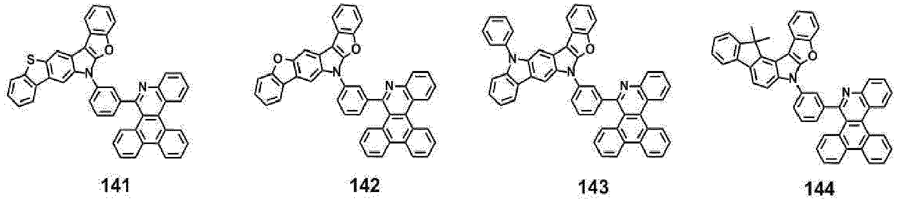

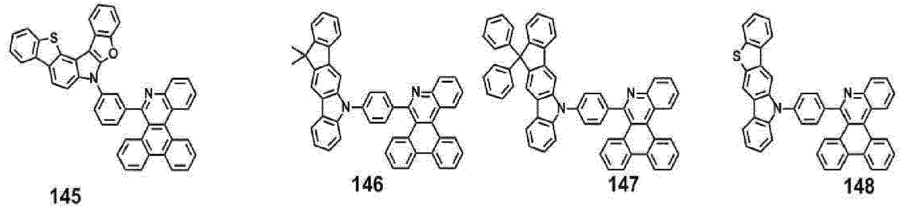

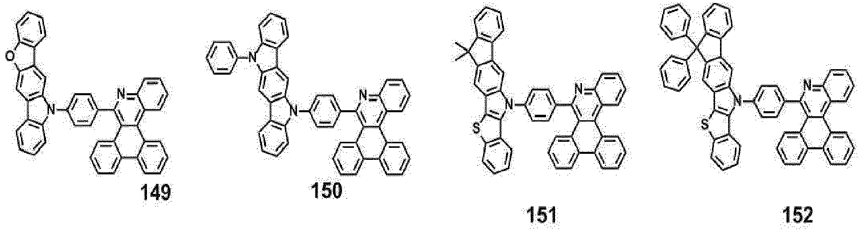

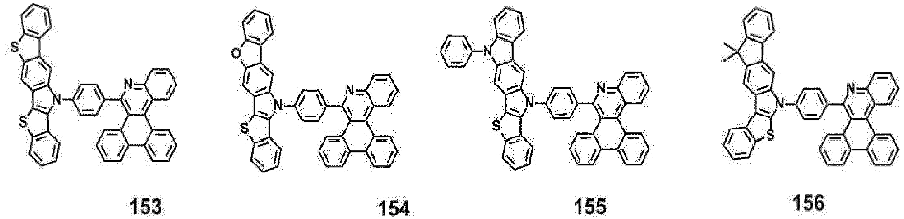

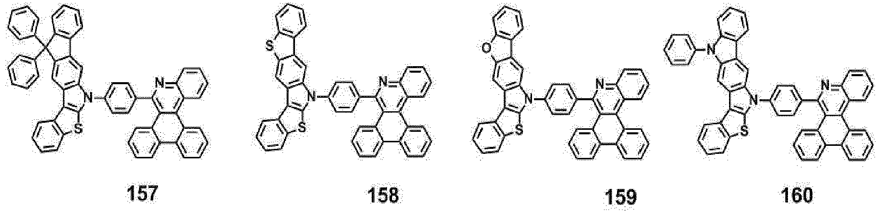

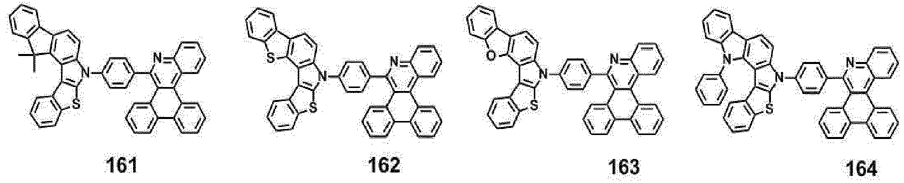

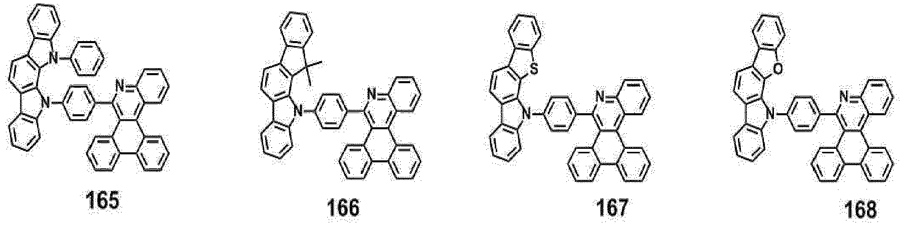

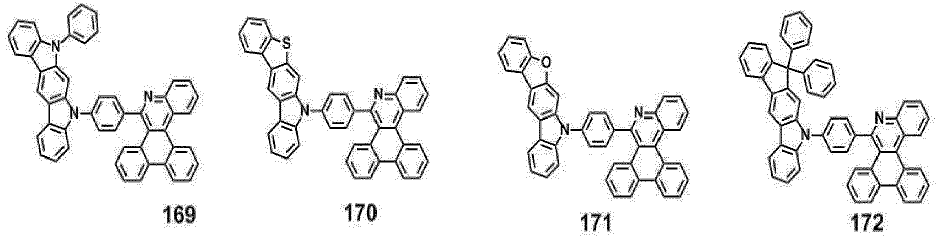

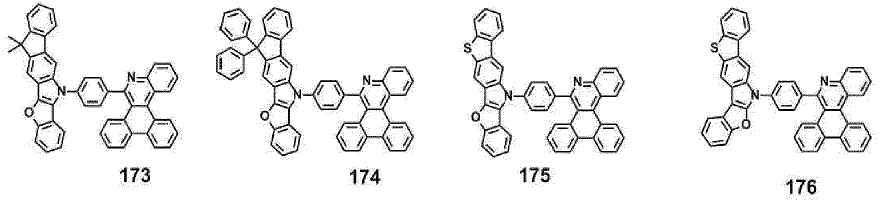

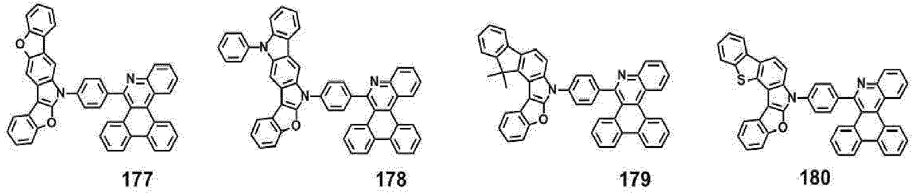

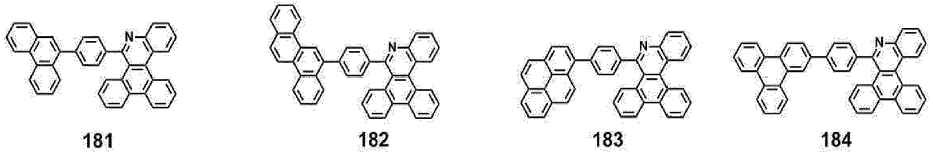

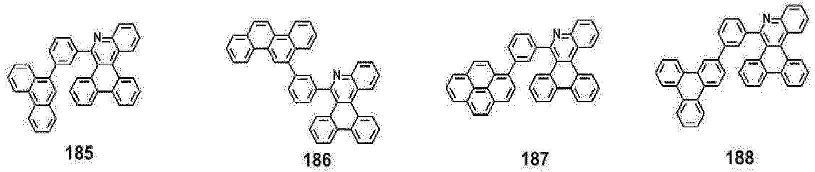

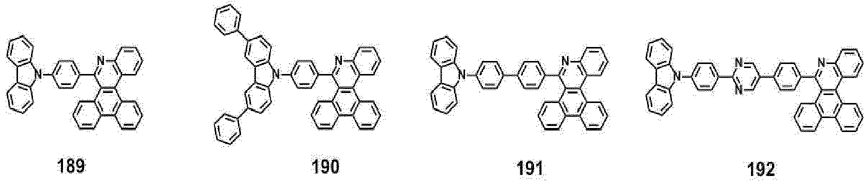

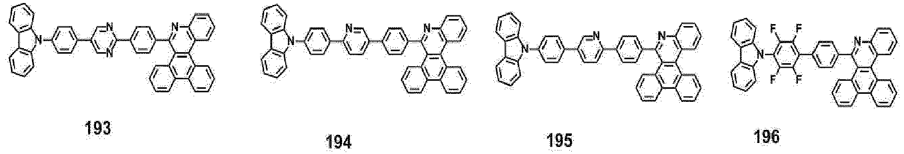

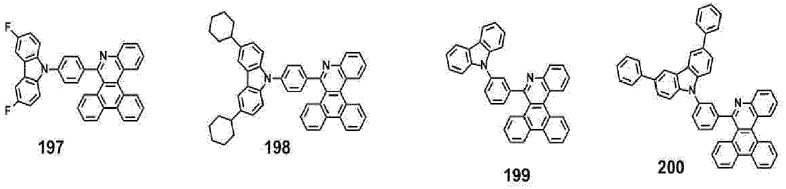

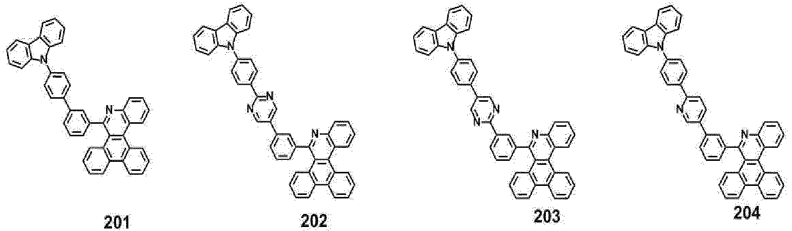

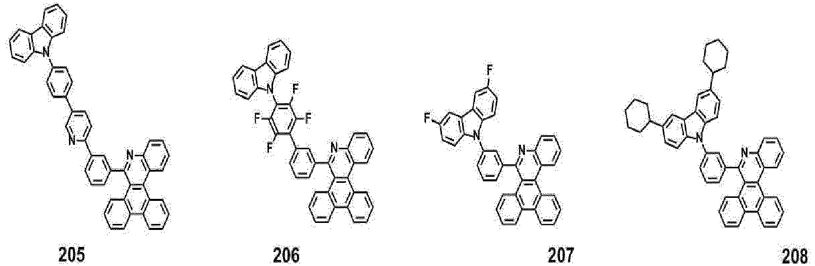

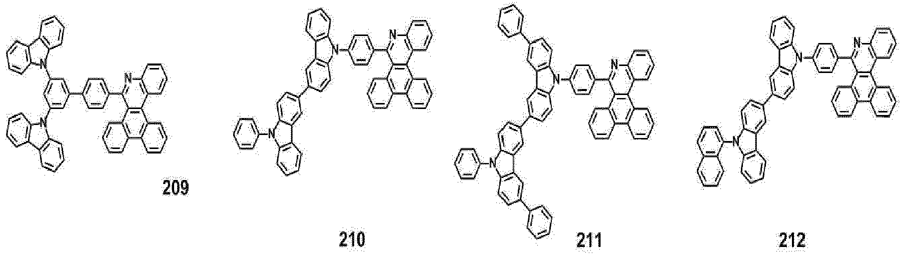

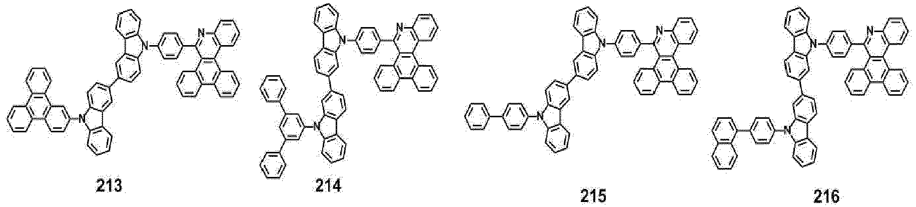

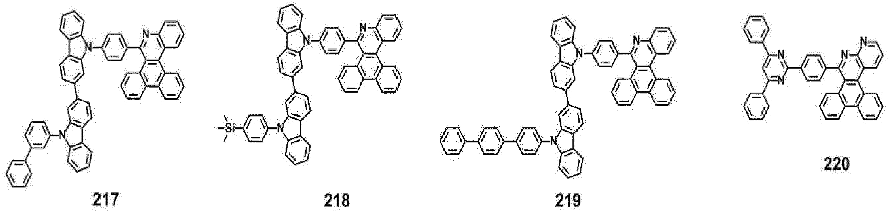

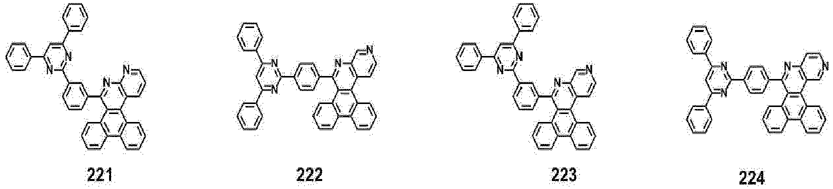

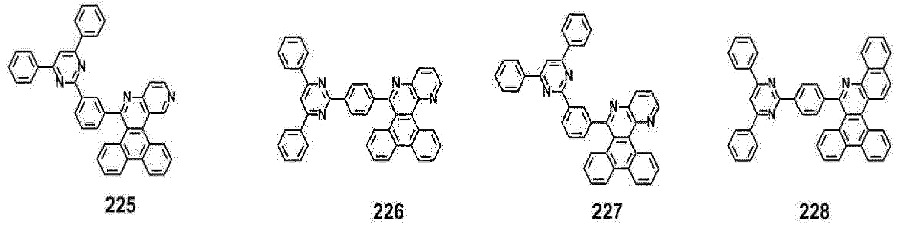

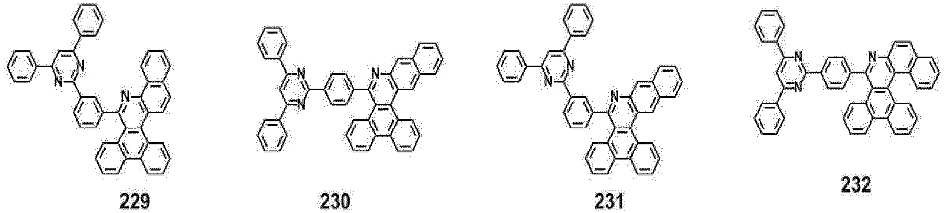

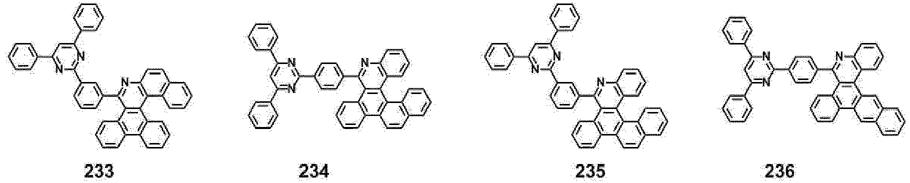

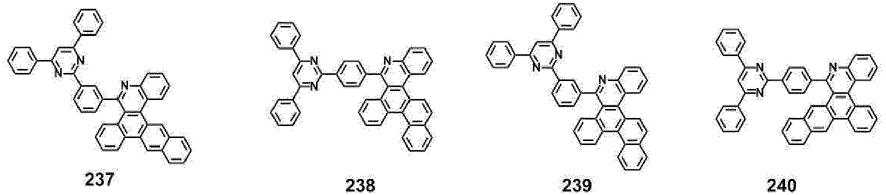

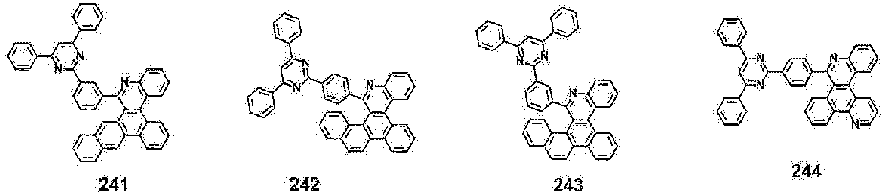

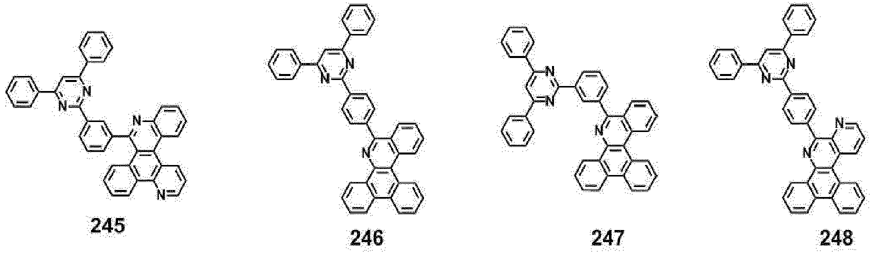

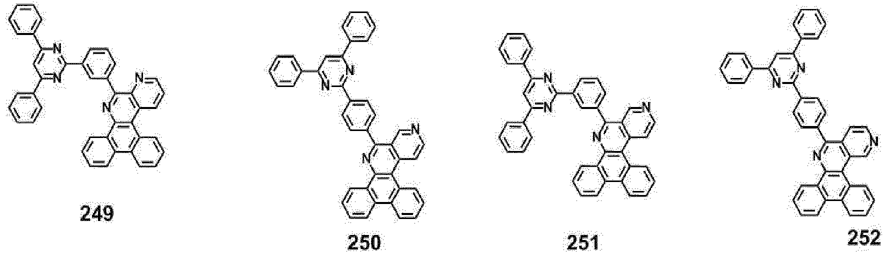

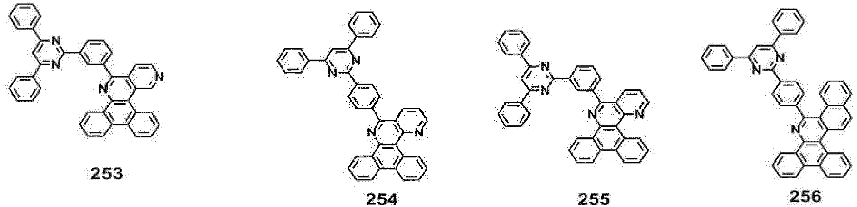

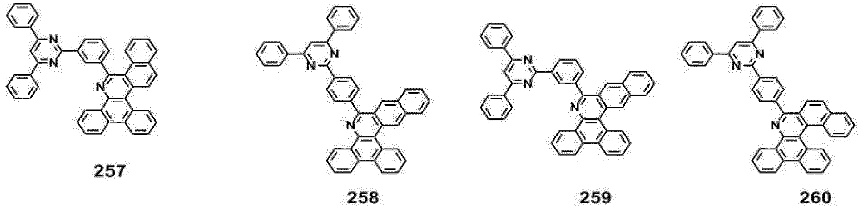

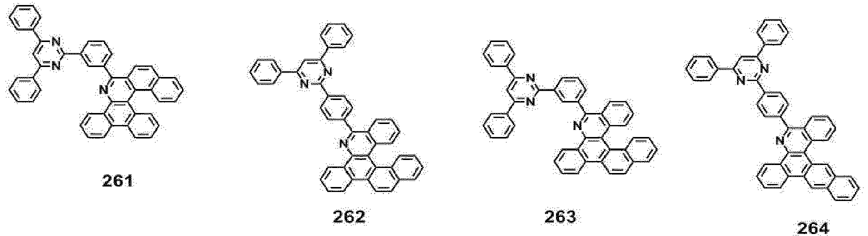

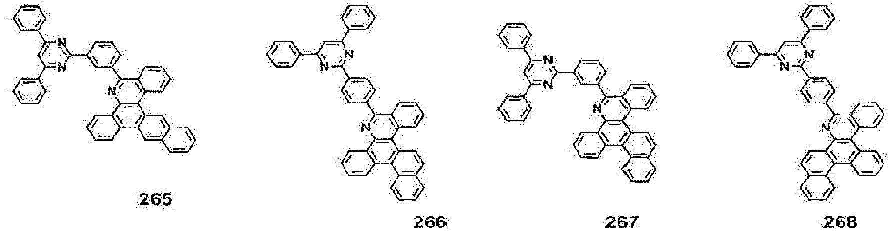

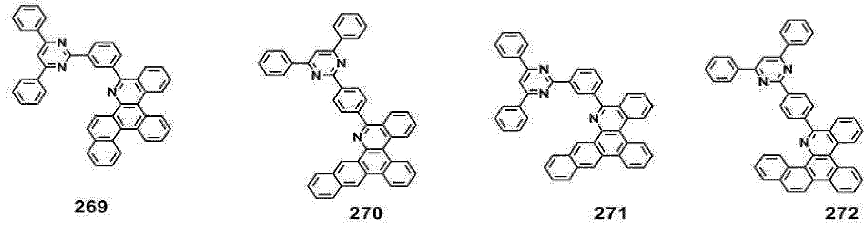

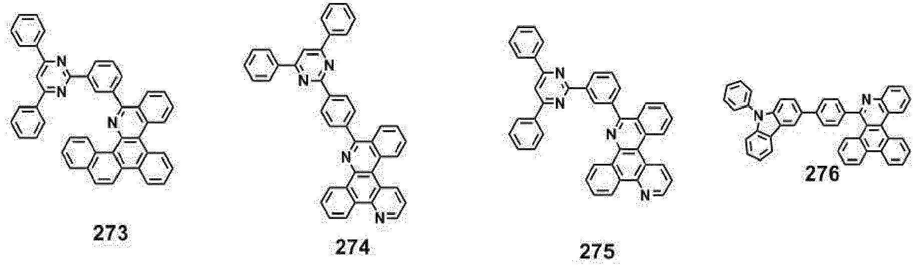

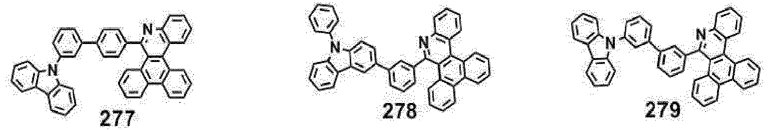

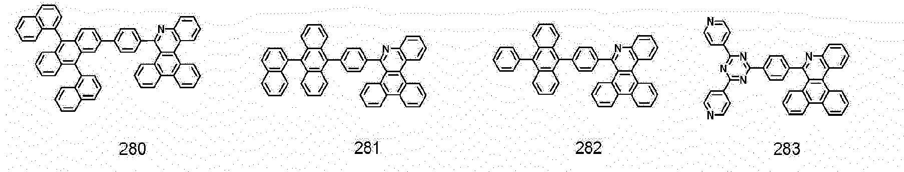

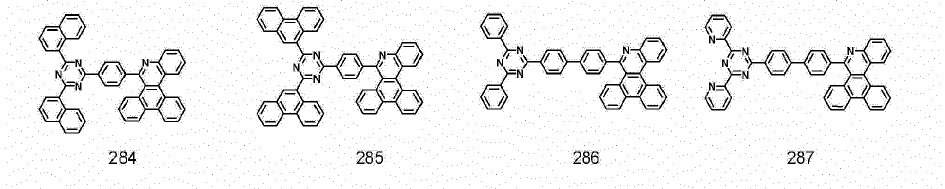

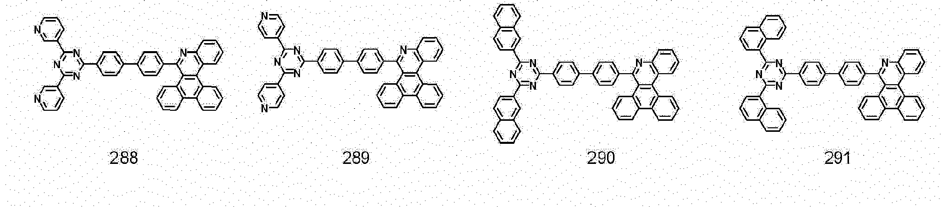

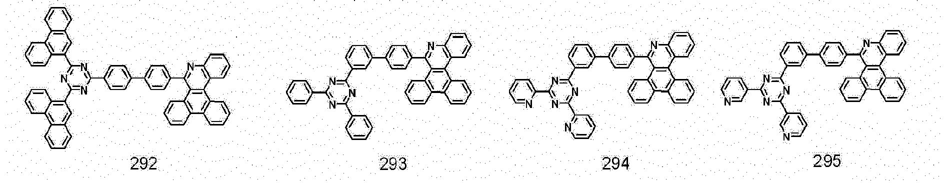

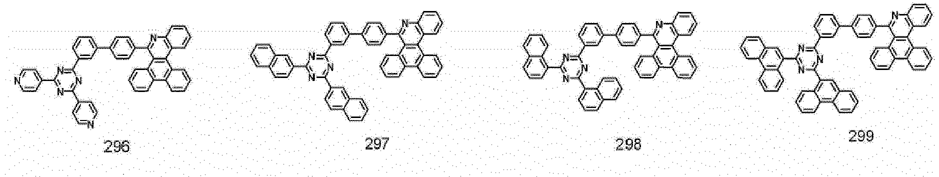

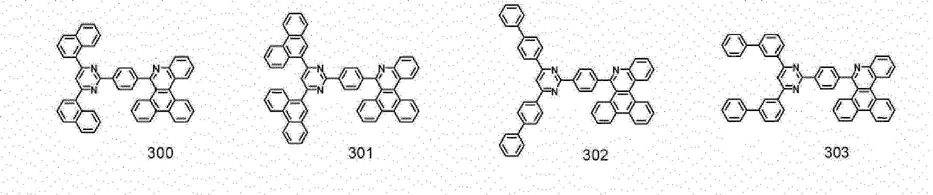

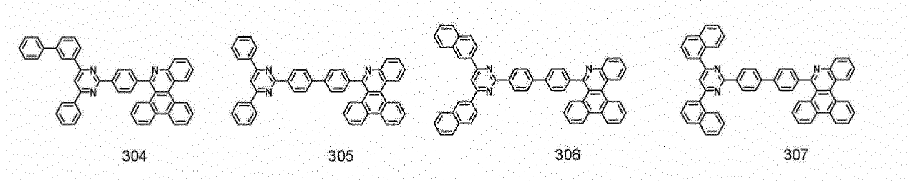

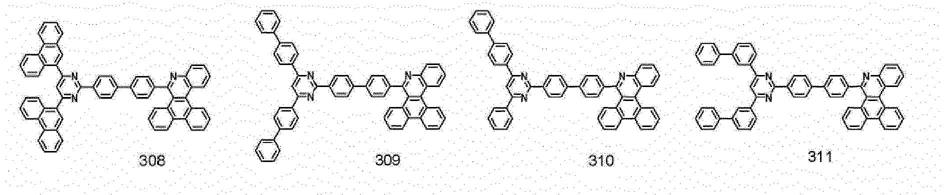

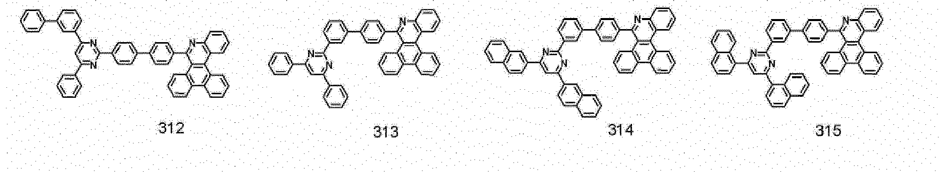

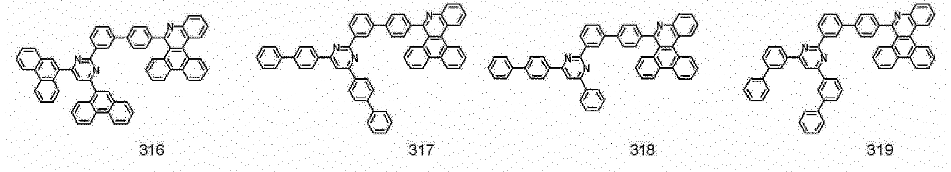

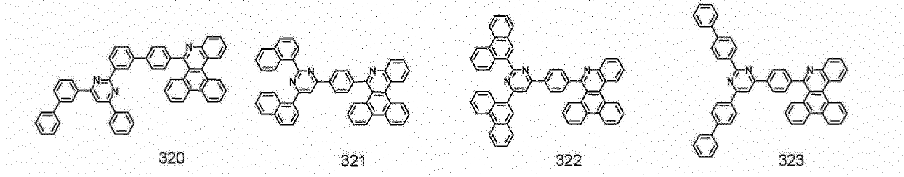

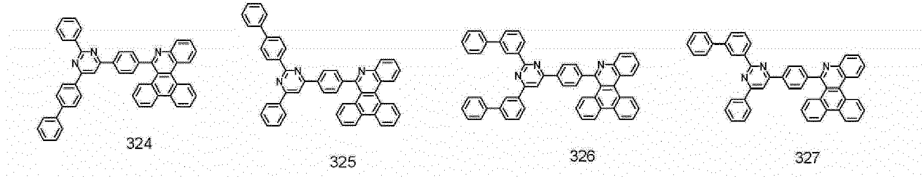

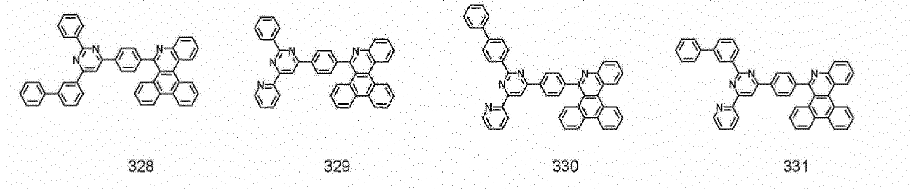

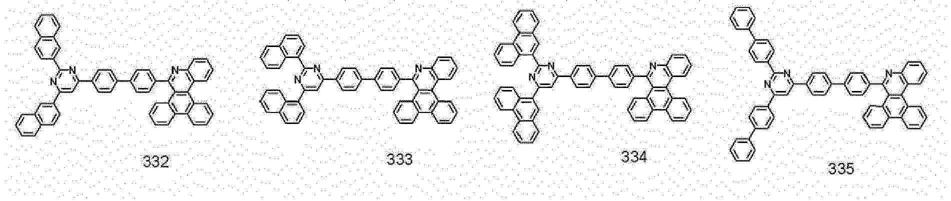

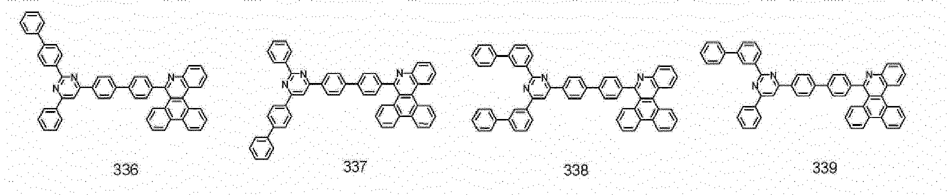

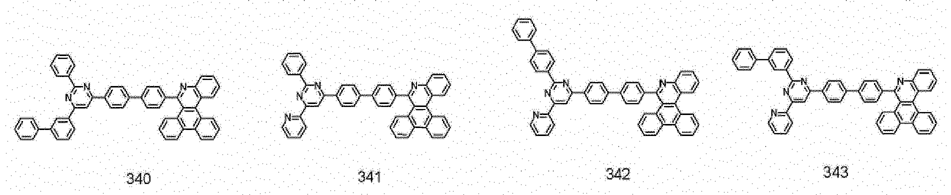

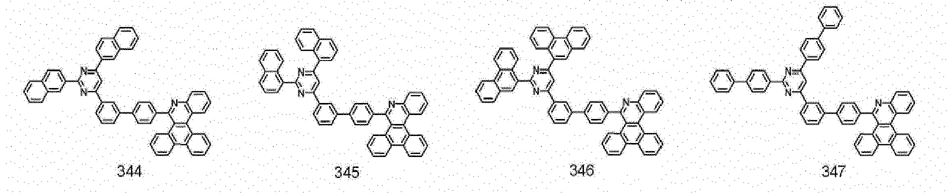

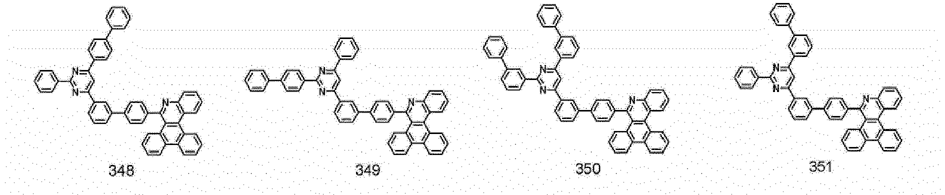

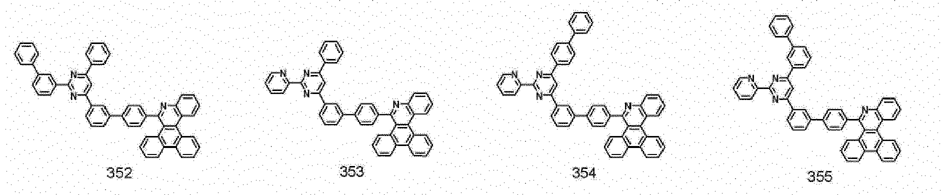

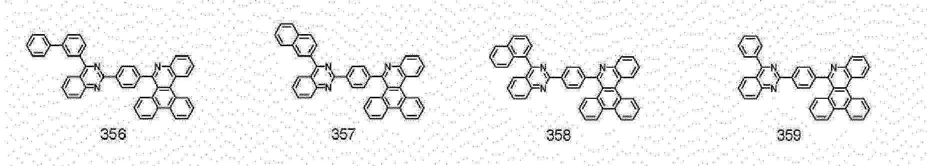

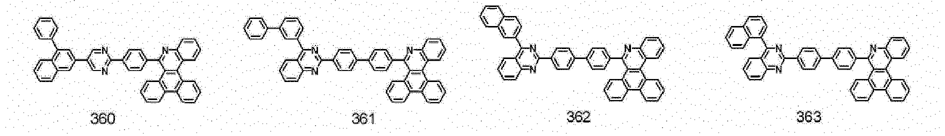

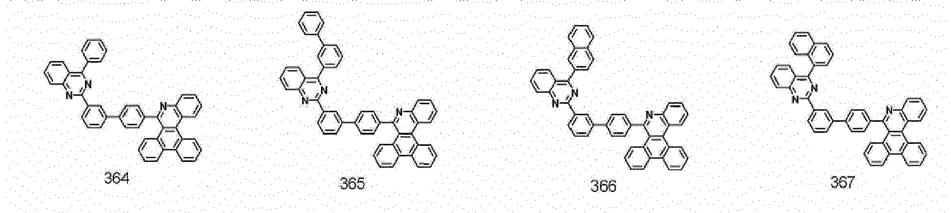

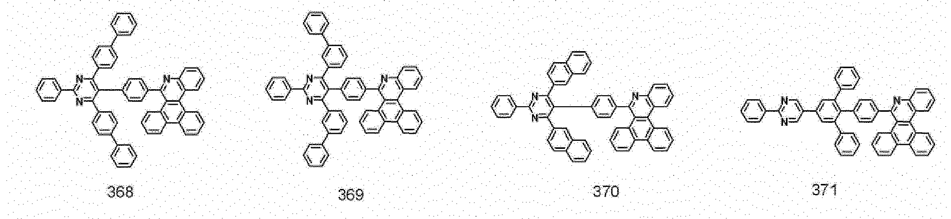

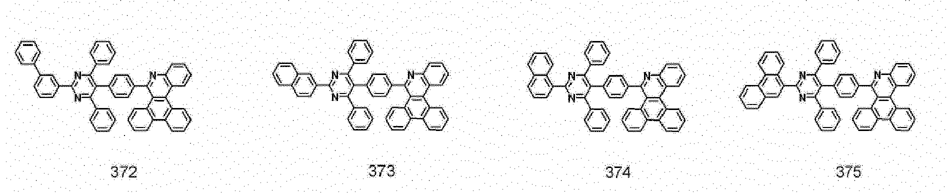

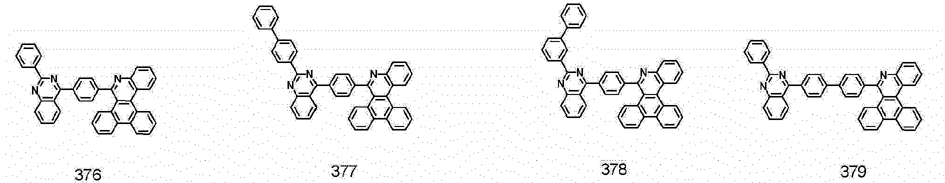

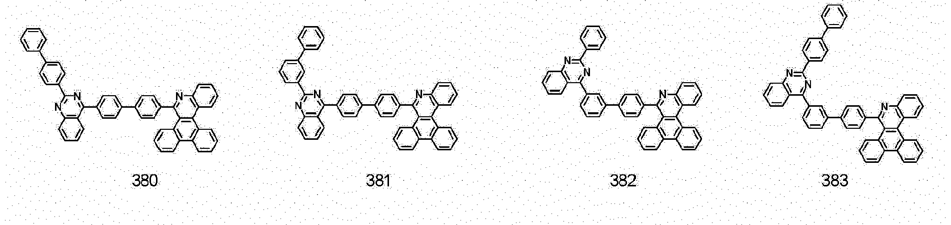

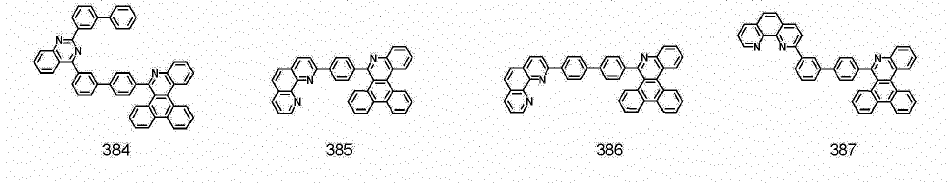

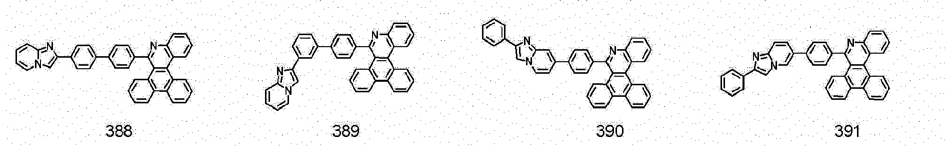

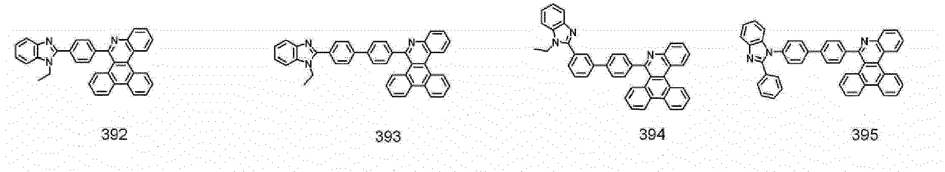

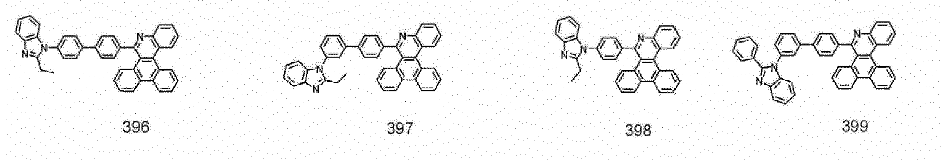

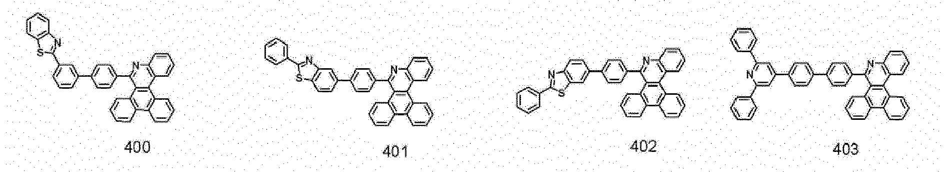

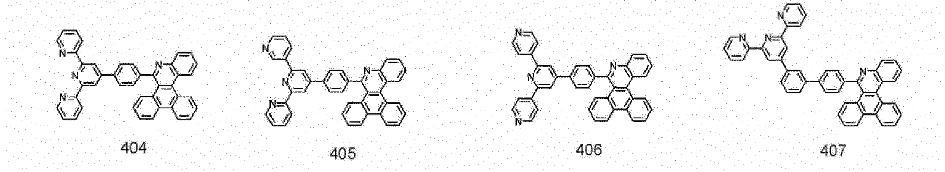

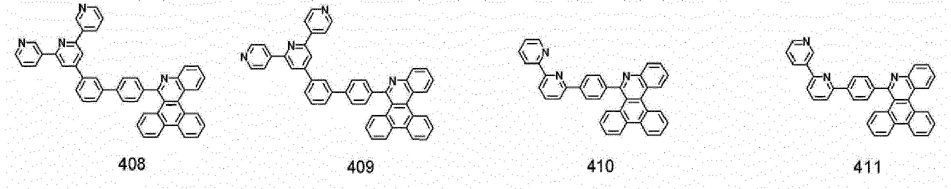

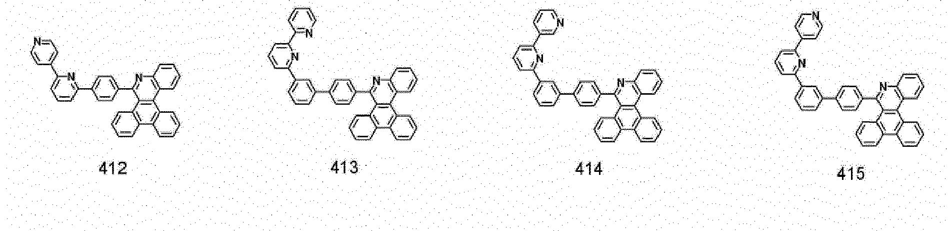

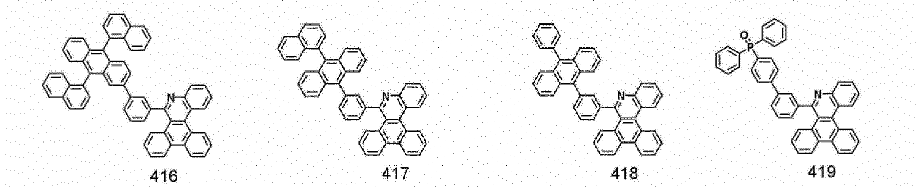

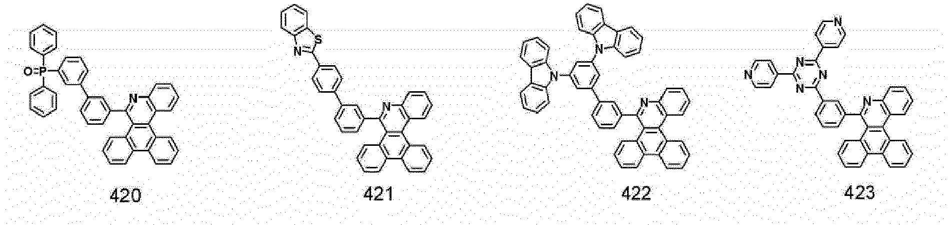

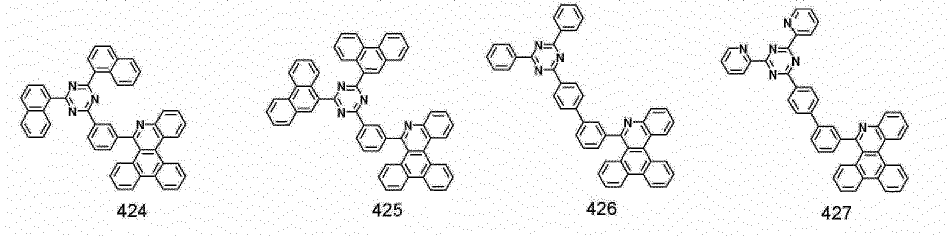

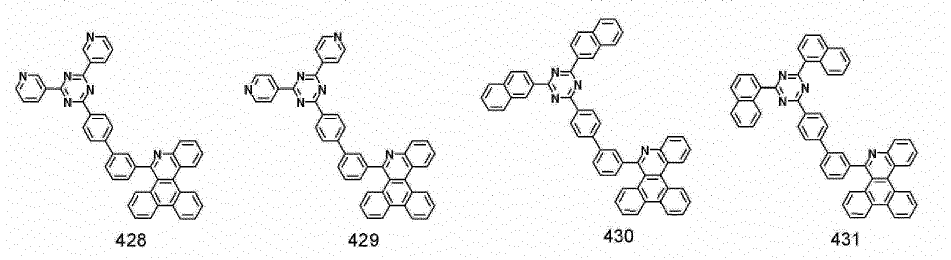

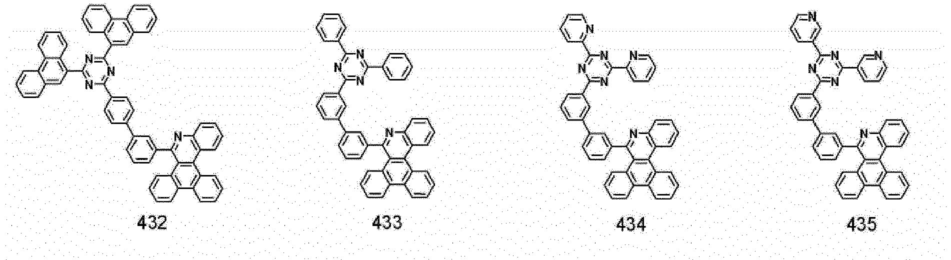

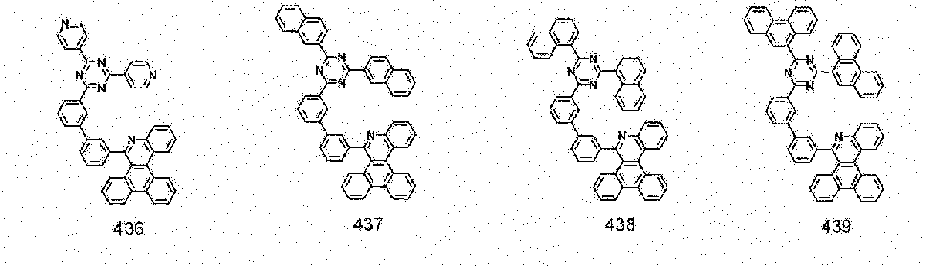

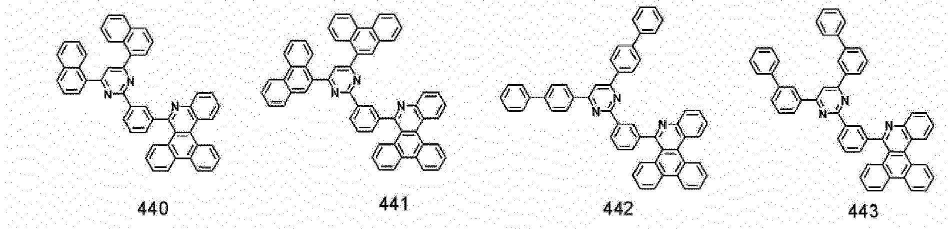

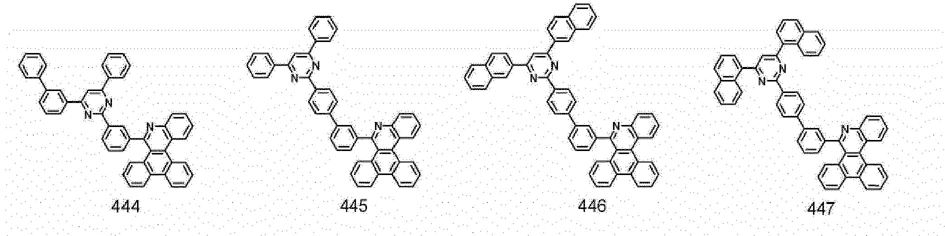

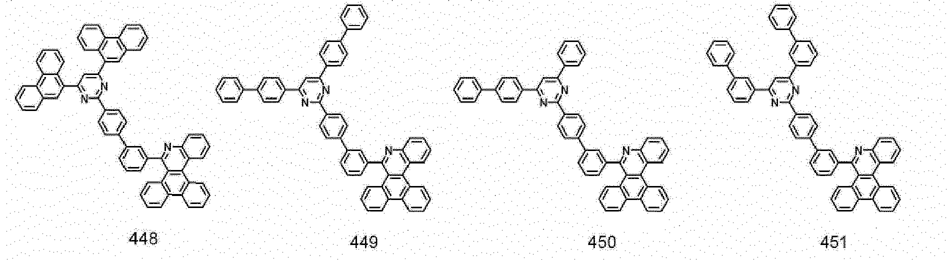

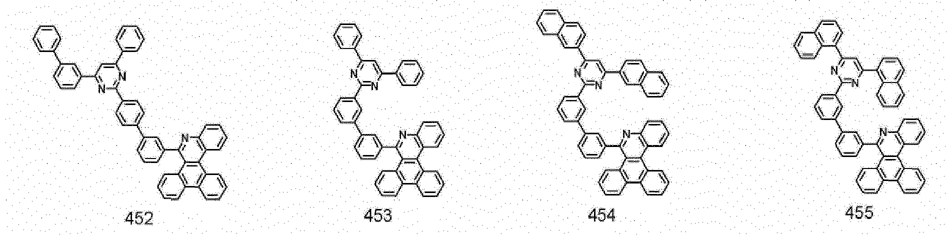

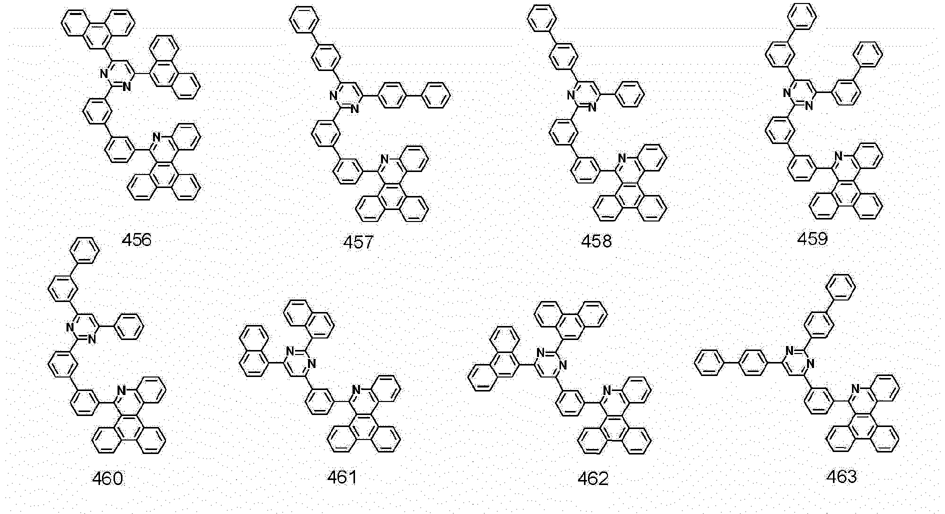

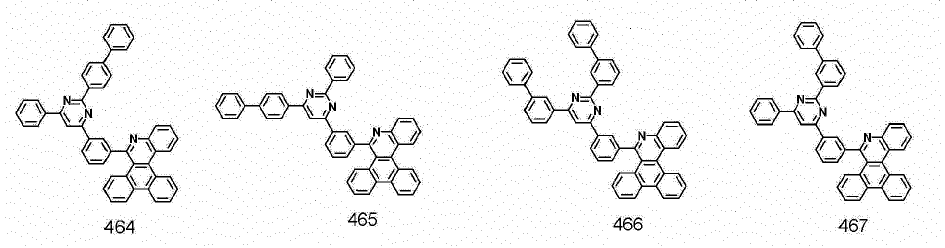

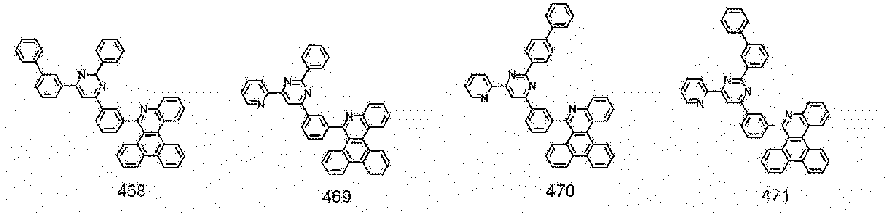

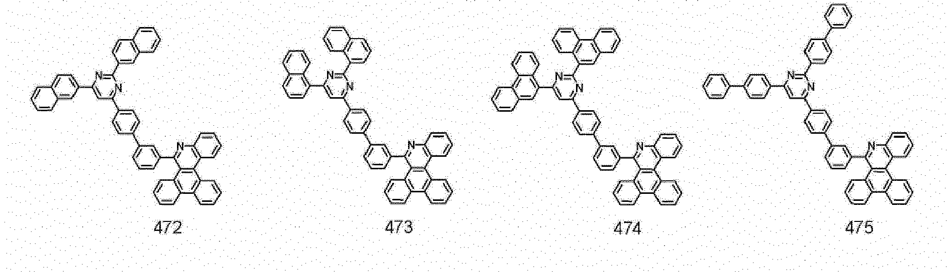

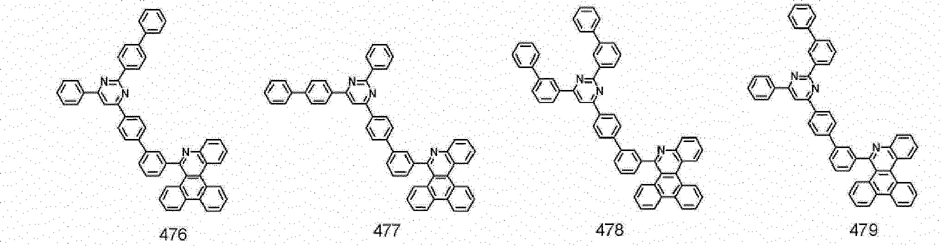

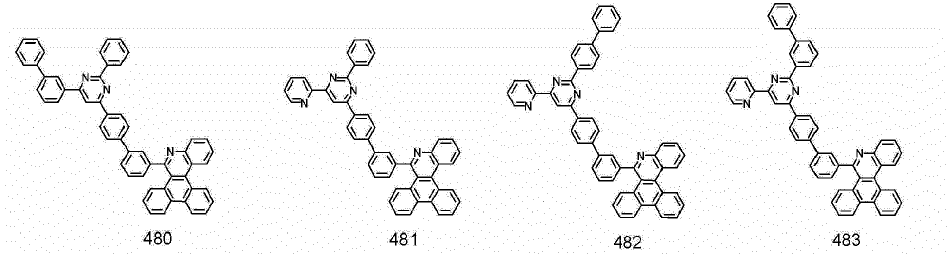

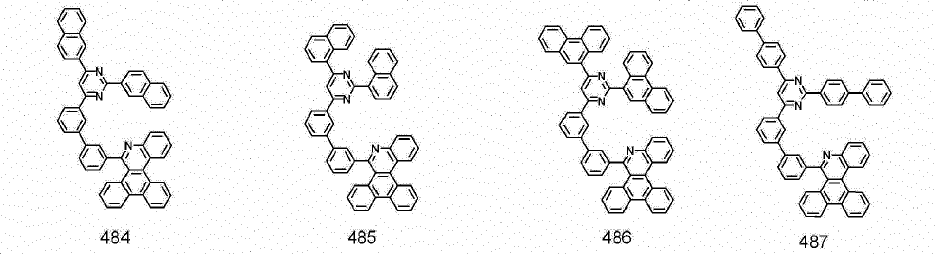

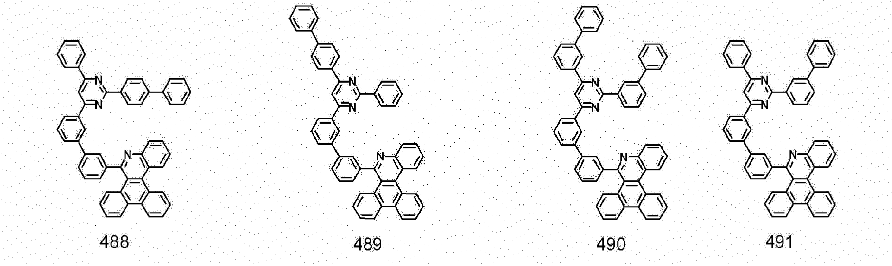

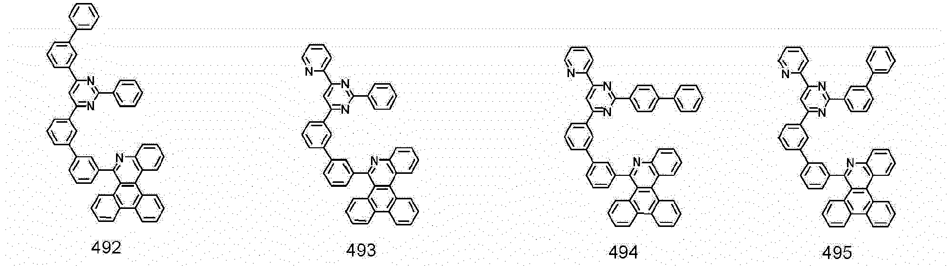

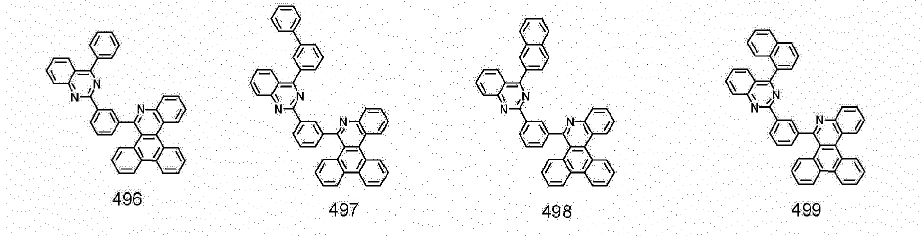

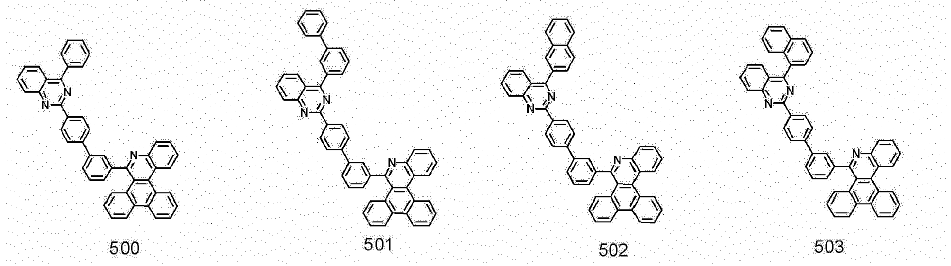

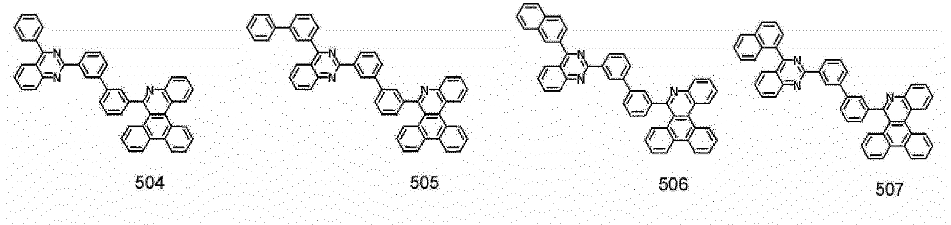

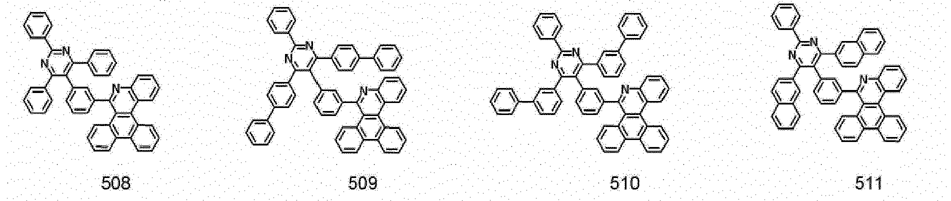

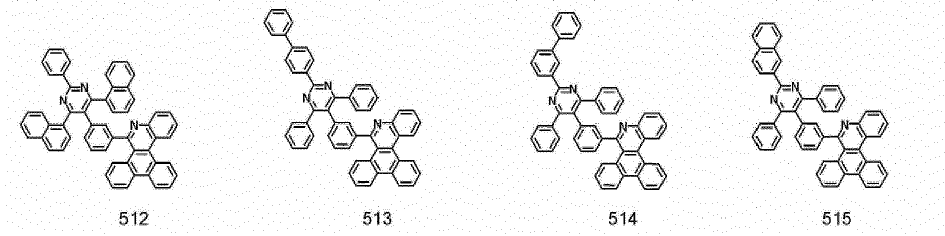

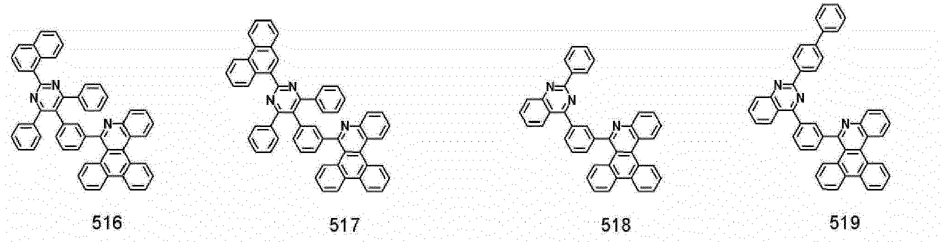

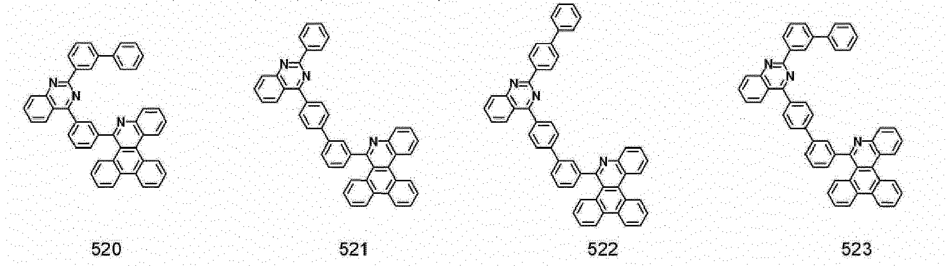

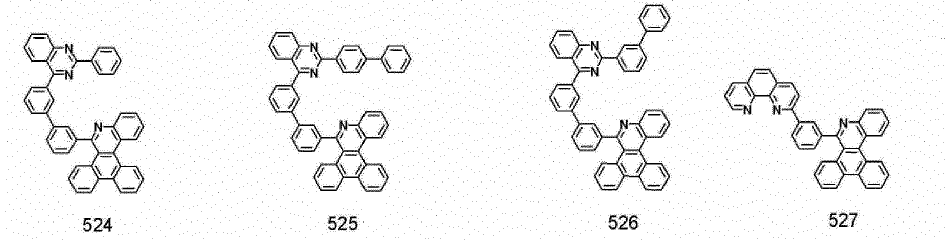

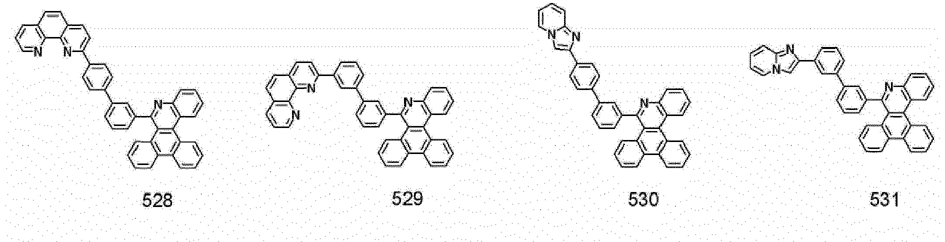

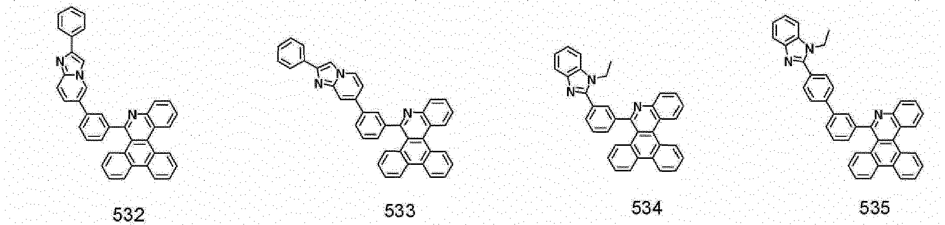

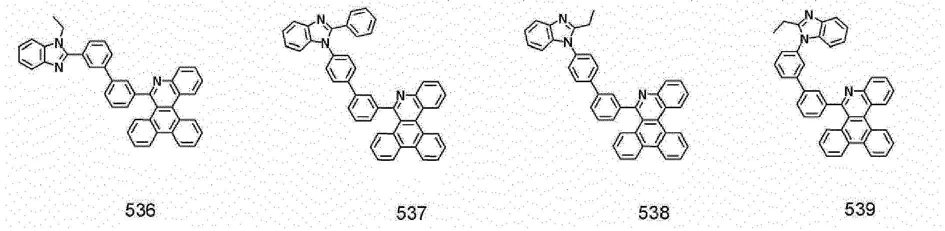

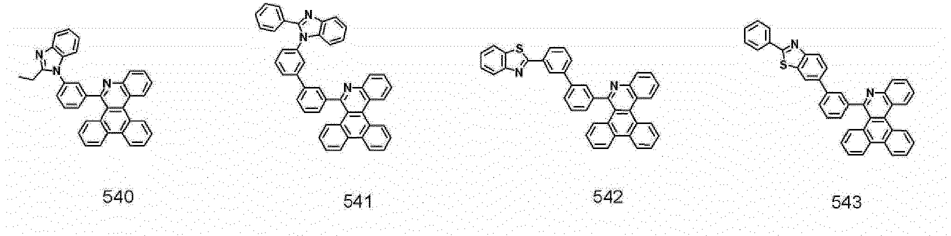

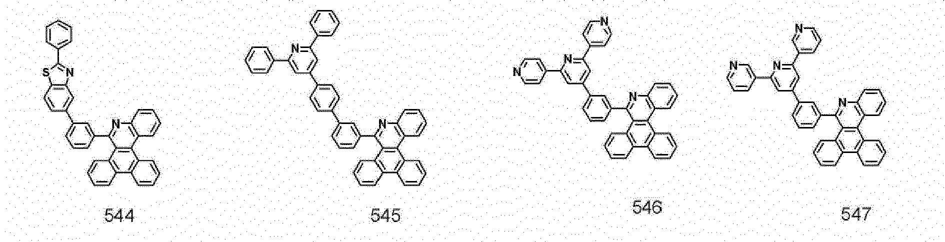

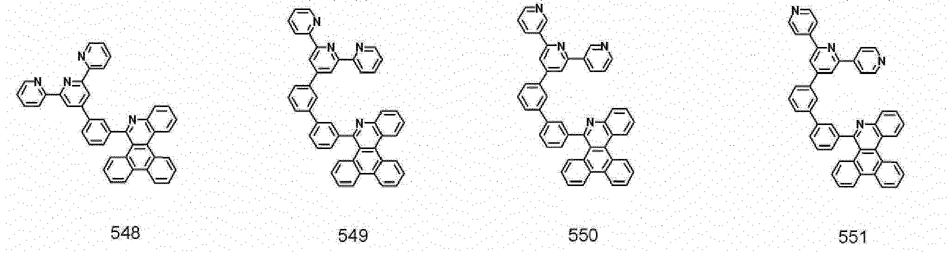

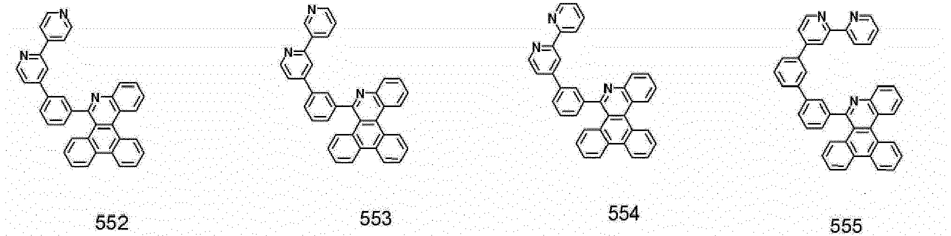

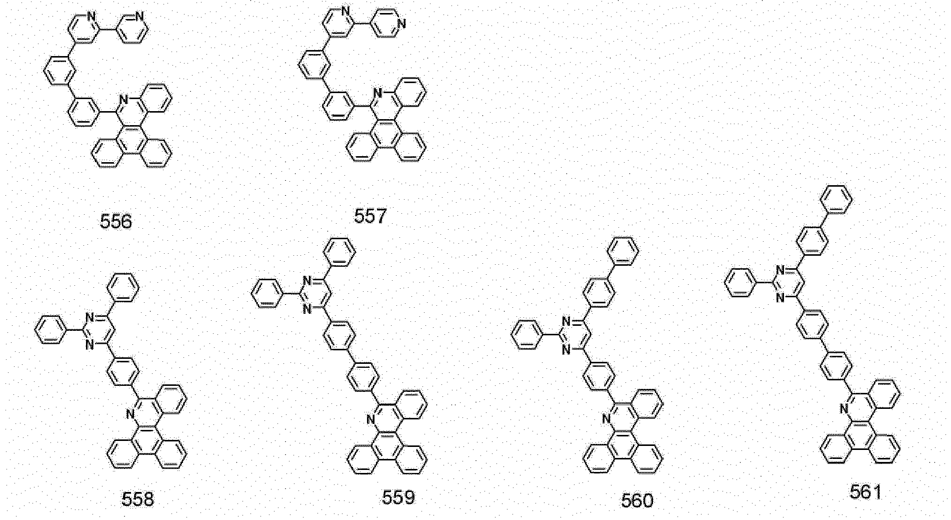

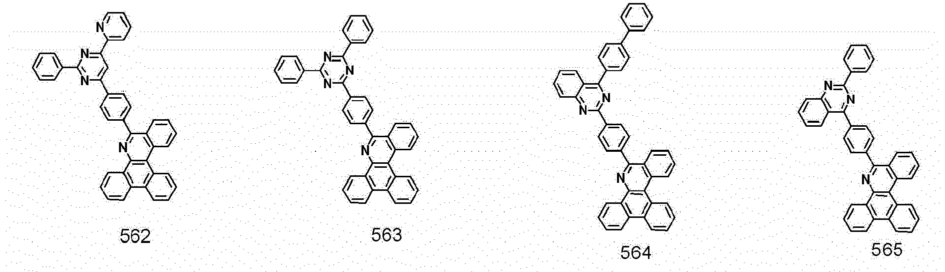

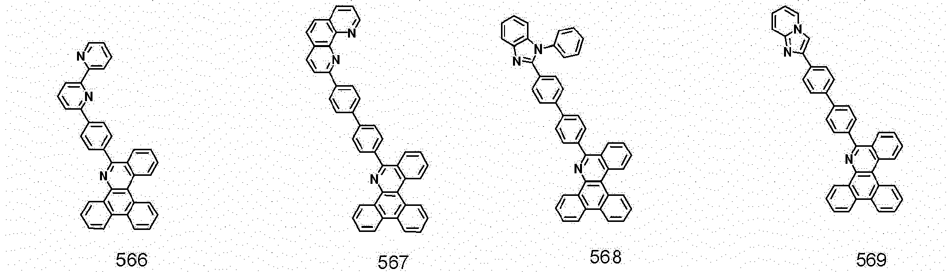

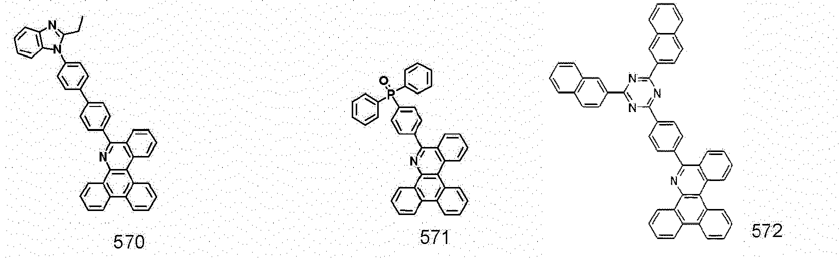

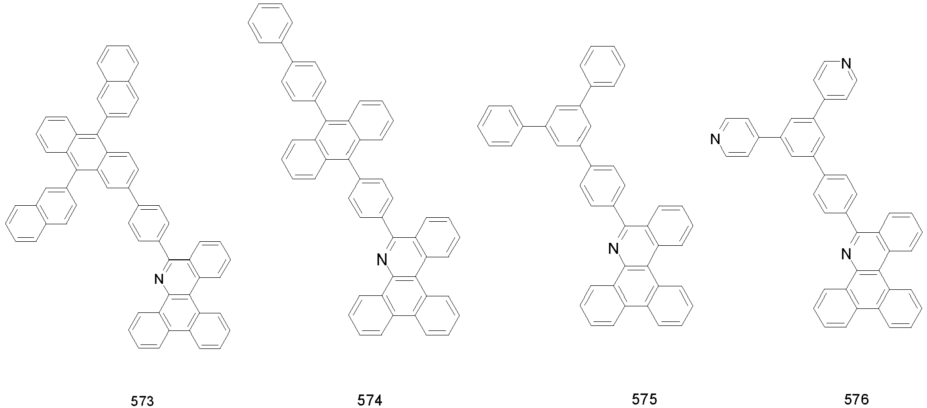

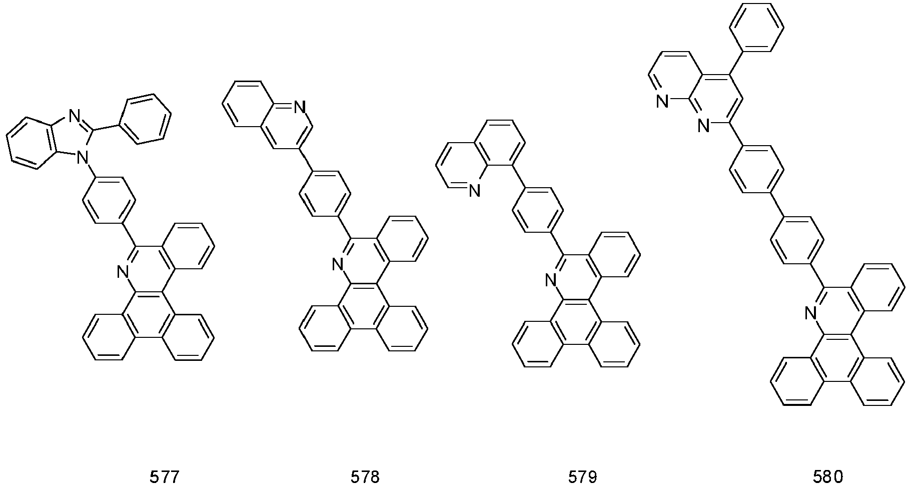

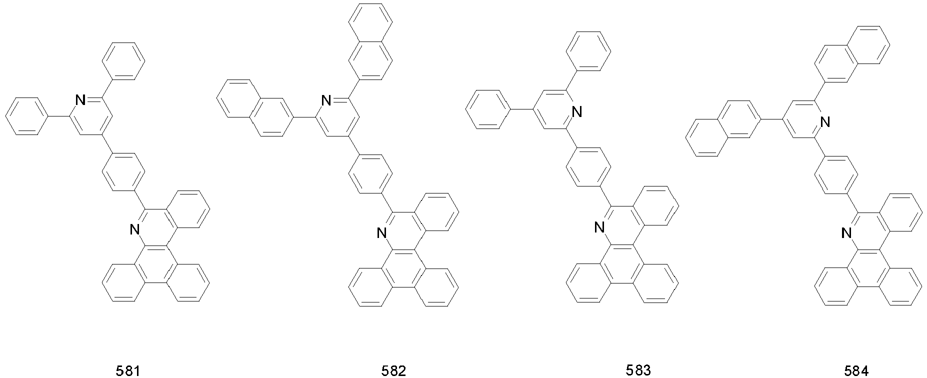

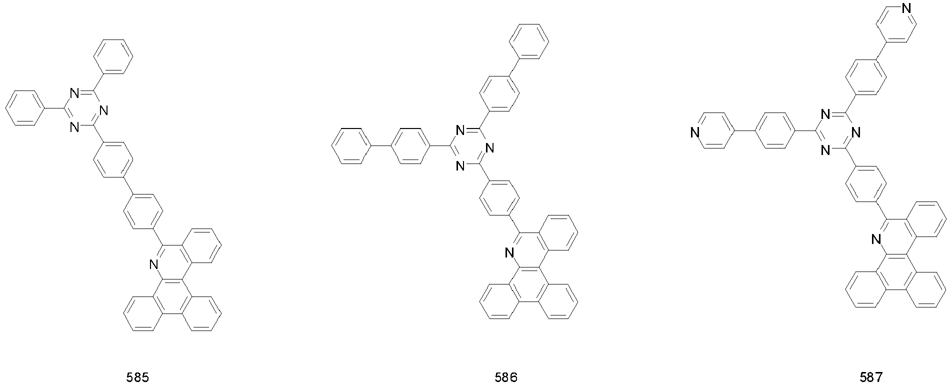

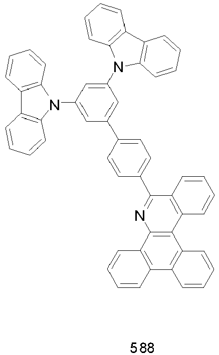

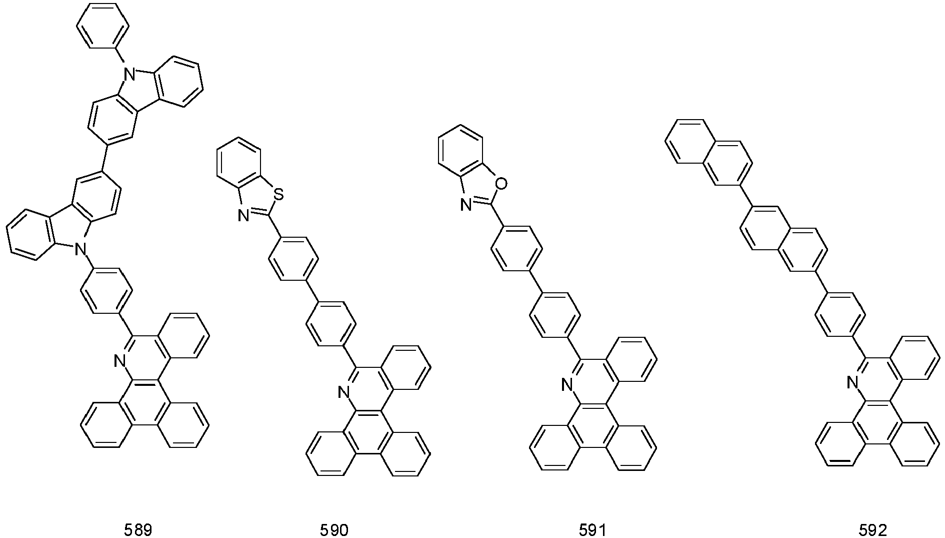

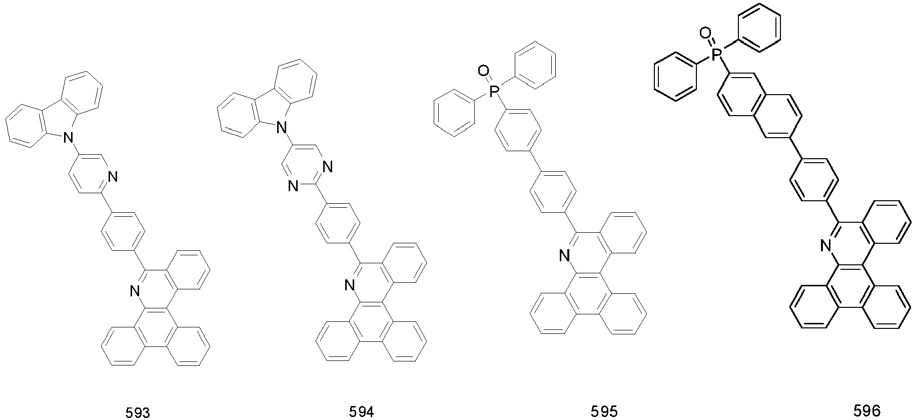

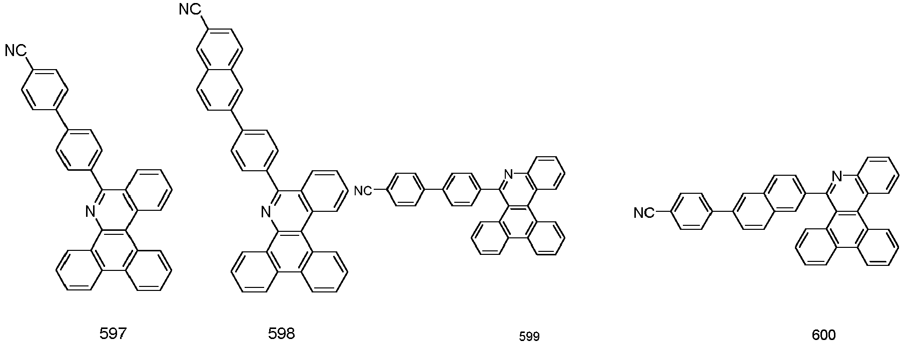

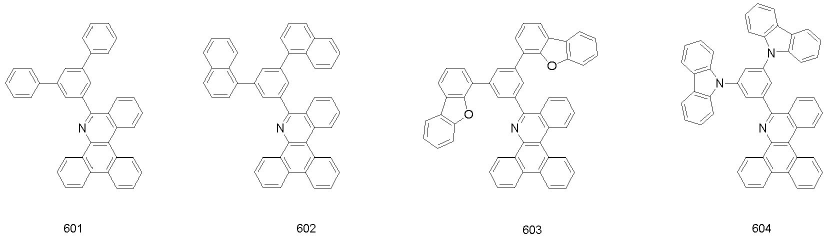

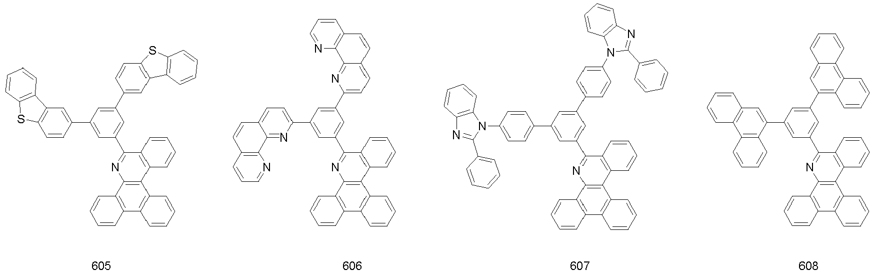

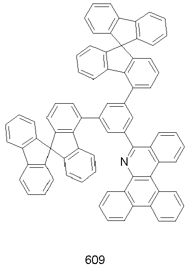

13. An organic light emitting device comprising:
an anode;
a cathode; and
one or more organic material layers provided between the anode and the cathode,
wherein one or more layers of the organic material layers include the compound of any one of claims 1 to 12.

14. The organic light emitting device of claim 13, wherein an organic material layer including the compound of any one of claims 1 to 12 is one or more layers selected from a hole injection layer, a hole transport layer, a light emitting layer, a hole blocking layer, an electron transport layer, and an electron injection layer.

15. The organic light emitting device of claim 13, wherein an organic material layer including the compound of any of claims 1 to 12 is an electron transport layer.

16. The organic light emitting device of claim 13, wherein an organic material layer including the compound of any of claims 1 to 12 is a light emitting layer.

17. The organic light emitting device of claim 13, wherein an organic material layer including the compound of any of claims 1 to 12 is a hole blocking layer.

## Patentansprüche

1. Verbindung der folgenden chemischen Formel 2, 3 oder 9: 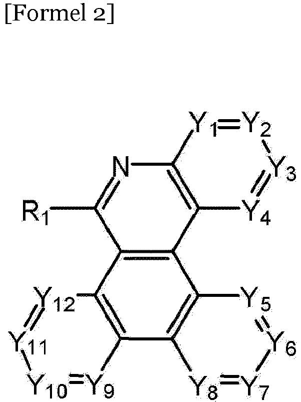 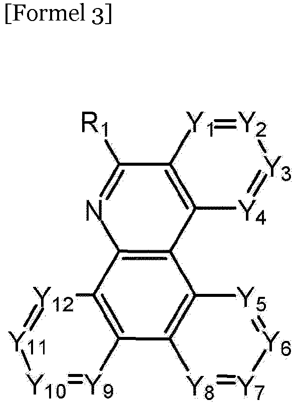 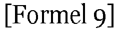 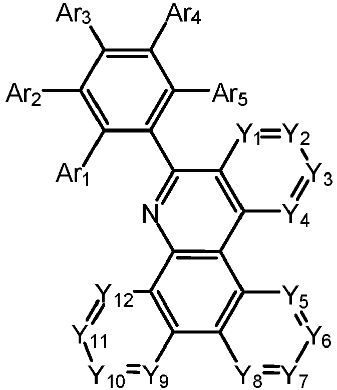 wobei
Y1 bis Y12 gleich oder verschieden voneinander sind und jeweils unabhängig N oder CR sind,
R1 -(L)m-(Z)n ist,
L substituiertes oder unsubstituiertes, moncyclisches oder polycyclisches C6- bis C60-Arylen; oder substituiertes oder unsubstituiertes, monocyclisches oder polycyclisches C2- bis C60-Heteroarylen ist,
Z eines ist, ausgewählt aus der Gruppe bestehend aus substituiertem oder unsubstituiertem, monocyclischem oder polycyclischem C6- bis C60-Aryl; substituiertem oder unsubstituiertem, monocyclischem oder polycyclischem C2-bis C60-Heteroaryl; -SiR11R12R13; und -P(=O)R14R15,
m eine ganze Zahl von 1 bis 5 ist,
n eine ganze Zahl von 1 bis 3 ist,
wobei, wenn m und n unabhängig ganze Zahlen von 2 oder höher sind, mehrere L und Z gleich oder verschieden voneinander sind,
R eines ist, ausgewählt aus der Gruppe bestehend aus Wasserstoff; oder wenigstens zwei benachbarte R miteinander verbunden sind, um einen substituierten oder unsubstituierten, monocyclischen oder polycyclischen, aliphatischen oder aromatischen Kohlenwasserstoffring zu bilden;
wobei "substituiert oder unsubstituiert" sich auf eine Gruppe bezieht, die substituiert sein kann oder nicht weiter substituiert sein kann mit einem oder mehreren Substituenten, ausgewählt aus der Gruppe bestehend aus Halogen; -CN; linearem oder verzweigtem C1- bis C60-Alkyl; monocyclischem oder polycyclischem C3- bis C6o-Cycloalkyl; monocyclischem oder polycyclischem C6- bis C60-Aryl; monocyclischem oder polycyclischem C2- bis C60-Heteroaryl; -SiR11R12R13; und -P(=O)R14R15,
wobei R11 bis R15 gleich oder verschieden voneinander sind und jeweils unabhängig eines sind, ausgewählt aus der Gruppe bestehend aus linearem oder verzweigtem, substituiertem oder unsubstituiertem C1- bis C60-Alkyl; substituiertem oder unsubstituiertem, monocyclischem oder polycyclischem C3-bis C60-Cycloalkyl; substituiertem oder unsubstituiertem, monocyclischem oder polycyclischem C6- bis C60-Aryl; und substituiertem oder unsubstituiertem, monocyclischem oder polycyclischem C2- bis C60-Heteroaryl, und
wenigstens zwei von Ar1 bis Ar5 monocyclisches oder polycyclisches, substituiertes oder unsubstituiertes C6- bis C60-Aryl; oder monocyclisches oder polycyclisches, substituiertes oder unsubstituiertes C2- bis C60-Heteroaryl sind, und die anderen jeweils unabhängig Wasserstoff; Deuterium; Halogen; geradkettiges oder verzweigtes, substituiertes oder unsubstituiertes C1- bis C60-Alkyl; monocyclisches oder polycyclisches, substituiertes oder unsubstituiertes C6- bis C60-Aryl; oder monocyclisches oder polycyclisches, substituiertes oder unsubstituiertes C2- bis C60-Heteroaryl sind.

2. Verbindung nach Anspruch 1, wobei alle der Y1 bis Y4 CR sind, oder irgendeines der Y1 bis Y4 N ist und die anderen CR sind, und
das R das gleiche wie in Anspruch 1 definiert ist.

3. Verbindung nach Anspruch 1, wobei alle der Y5 bis Y8 CR sind, oder irgendeines der Y5 bis Y8 N ist und die anderen CR sind, und
das R das gleiche wie in Anspruch 1 definiert ist.

4. Verbindung nach Anspruch 1, wobei alle der Y9 bis Y12 CR sind, oder irgendeines der Y9 bis Y12 N ist und die anderen CR sind, und
das R das gleiche wie in Anspruch 1 definiert ist.

5. Verbindung nach Anspruch 1, wobei die Y1 bis Y12 CR sind, und das R das gleiche wie in Anspruch 1 definiert ist.

6. Verbindung nach Anspruch 1, wobei
L eines ist, ausgewählt aus der Gruppe bestehend aus monocyclischem oder polycyclischem C6- bis C60-Arylen, unsubstituiert oder substituiert mit Halogen, linearem oder verzweigtem C1- bis C6o-Alkyl, monocyclischem oder polycyclischem C3- bis C60-Cycloalkyl, monocyclischem oder polycyclischem C6- bis C60-Aryl und monocyclischem oder polycyclischem C2- bis C60-Heteroaryl; und monocyclischem oder polycyclischem C2- bis C60-Heteroarylen, unsubstituiert oder substituiert mit Halogen, linearem oder verzweigtem C1- bis C60-Alkyl, monocyclischem oder polycyclischem C3- bis C60-Cycloalkyl, monocyclischem oder polycyclischem C6- bis C60-Aryl und monocyclischem oder polycyclischem C2- bis C60-Heteroaryl,
Z eines ist, ausgewählt aus der Gruppe bestehend aus monocyclischem oder polycyclischem C6- bis C60-Aryl, unsubstituiert oder substituiert mit Halogen, linearem oder verzweigtem C1- bis C6o-Alkyl, monocyclischem oder polycyclischem C3- bis C60-Cycloalkyl, monocyclischem oder polycyclischem C6- bis C60-Aryl und monocyclischem oder polycyclischem C2- bis C60-Heteroaryl; monocyclischem oder polycyclischem C2- bis C60-Heteroaryl, unsubstitziert oder substituiert mit Halogen, linearem oder verzweigtem C1- bis C60-Alkyl, monocyclischem oder polycyclischem C3- bis C60-Cycloalkyl, monocyclischem oder polycyclischem C6- bis C60-Aryl und monocyclischem oder polycyclischem C2- bis C60-Heteroaryl; -SiR11R12R13; und -P(=O)R14R15,
R Wasserstoff ist; und die R11 bis R15 gleich oder verschieden voneinander sind und jeweils unabhängig eines sind, ausgewählt aus der Gruppe bestehend aus Wasserstoff; linearem oder verzweigtem Alkyl, unsubstituiert oder substituiert mit Halogen, linearem oder verzweigtem C1- bis C6o-Alkyl, monocyclischem oder polycyclischem C3- bis C60-Cycloalkyl, monocyclischem oder polycyclischem C6- bis C60-Aryl und monocyclischem oder polycyclischem C2-bis C60-Heteroaryl; monocyclischem oder polycyclischem C6- bis C60-Aryl, unsubstituiert oder substituiert mit Halogen, linearem oder verzweigtem C1- bis C60-Alkyl, monocyclischem oder polycyclischem C3- bis C60-Cycloalkyl, monocyclischem oder polycyclischem C6- bis C60-Aryl und monocyclischem oder polycyclischem C2- bis C60-Heteroaryl; und monocyclischem oder polycyclischem C2- bis C60-Heteroaryl, unsubstituiert oder substituiert mit Halogen, linearem oder verzweigtem C1- bis C60-Alkyl, monocyclischem oder polycyclischem C3- bis C60-Cycloalkyl, monocyclischem oder polycyclischem C6- bis C60-Aryl und monocyclischem oder polycyclischem C2- bis C60-Heteroaryl, und
wenn zwei oder mehr der Y1 bis Y12 CR sind, mehrere R gleich oder verschieden voneinander sind.

7. Verbindung nach Anspruch 1, wobei Z substituiertes oder unsubstituiertes Phenyl, substituiertes oder unsubstituiertes Biphenyl, substituiertes oder unsubstituiertes Naphthyl, substituiertes oder unsubstituiertes Chrysenyl, substituiertes oder unsubstituiertes Pyrenyl, substituiertes oder unsubstituiertes Triphenylenyl, substituiertes oder unsubstituiertes Anthracenyl, substituiertes oder unsubstituiertes Phenanthrenyl, substituiertes oder unsubstituiertes Fluorenyl, substituiertes oder unsubstituiertes Benzochrysenyl oder substituiertes oder unsubstitziertes Spirobifluorenyl ist.

8. Verbindung nach Anspruch 1, wobei Z substituiertes oder unsubstituiertes, monocyclisches oder polycyclisches C2- bis C60-Heteroaryl ist und das Heteroaryl wenigstens eines ausgewählt aus N, O, S, Si und Se als ein Heteroatom einschließt.

9. Verbindung nach Anspruch 1, wobei Z 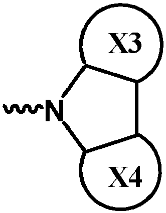 ist, und wobei X3 und X4 substituierte oder unsubstituierte, monocyclische oder polycyclische aromatische C6- bis C60-Kohlenwasserstoffringe; oder substituierte oder unsubstituierte, monocyclische oder polycyclische aromatische C2- bis C6o-Heteroringe sind.

10. Verbindung nach Anspruch 9, wobei 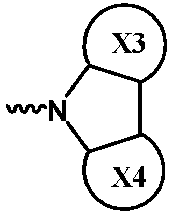 durch eine der folgenden Strukturformeln dargestellt ist: 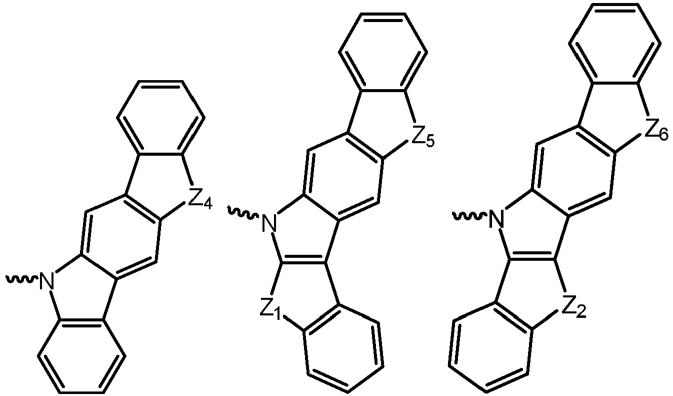 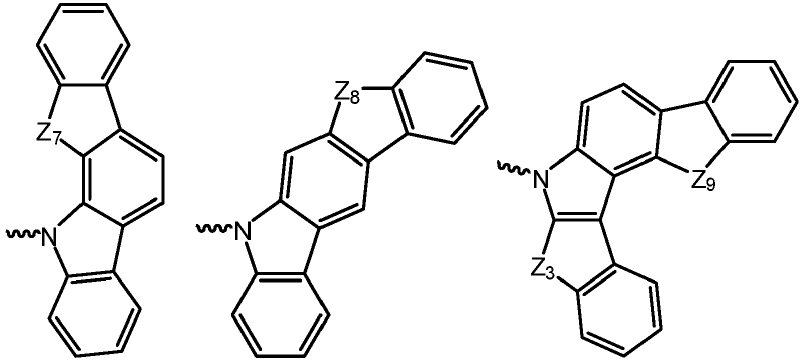
wobei in den Strukturformeln Z1 bis Z3 gleich oder verschieden voneinander sind und jeweils unabhängig S oder O sind,
Z4 bis Z9 gleich oder verschieden voneinander sind und jeweils unabhängig CR'R", NR', S oder O sind,
wobei R' und R" gleich oder verschieden voneinander sind und jeweils unabhängig Wasserstoff; lineares oder verzweigtes, substituiertes C1- bis C60-Alkyl; oder substituiertes oder unsubstituiertes, monocyclisches oder polycyclisches C6- bis C60-Aryl sind.

11. Verbindung nach Anspruch 1, dargestellt durch die folgende chemische Formel 4 oder 5: 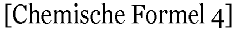 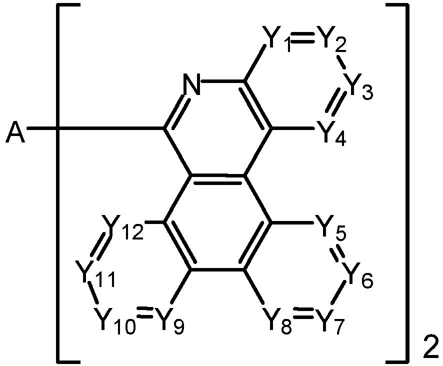 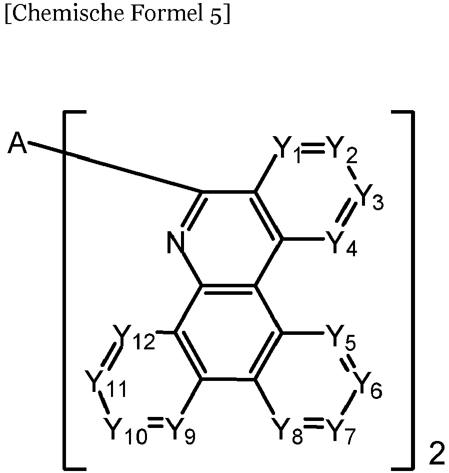 wobei in den chemischen Formeln 4 und 5
A ausgewählt ist aus der Gruppe bestehend aus substituiertem oder unsubstituiertem, monocyclischem oder polycyclischem C6- bis C60-Arylen und substituiertem oder unsubstituiertem, monocyclischem oder polycyclischem C2-bis C60-Heterarylen, und
Y1 bis Y12 das gleiche wie in Anspruch 1 definiert sind.

12. Verbindung nach Anspruch 1, wobei die Verbindung ausgewählt ist aus den folgenden Verbindungen:

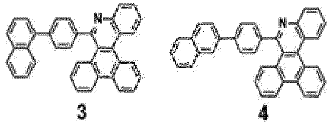

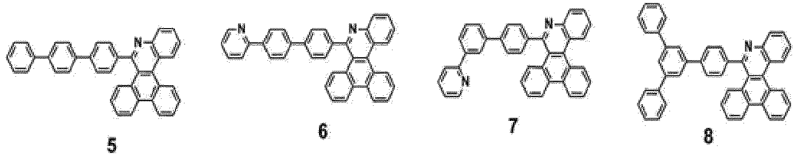

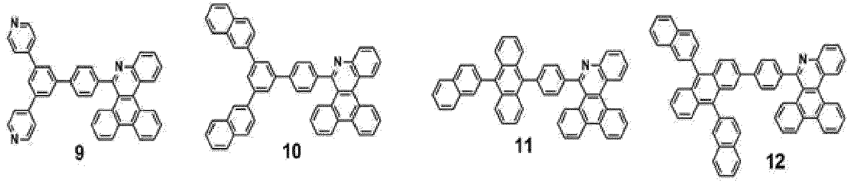

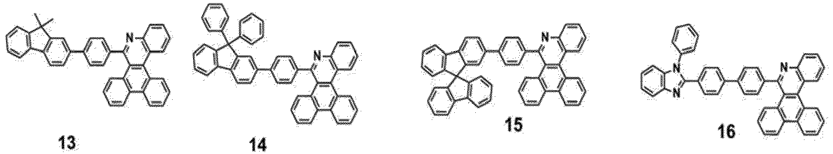

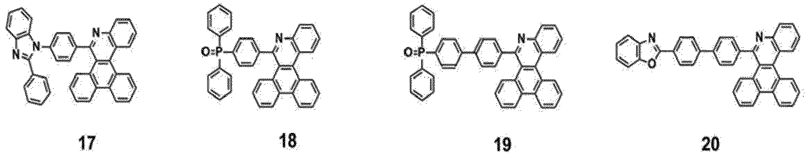

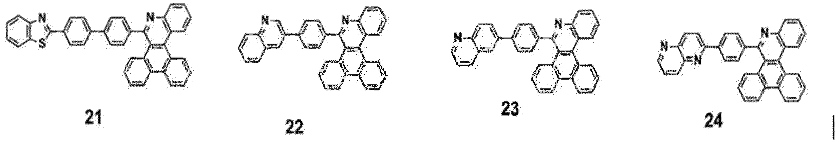

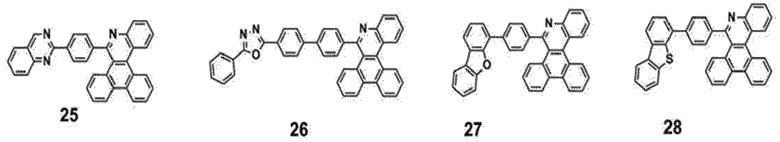

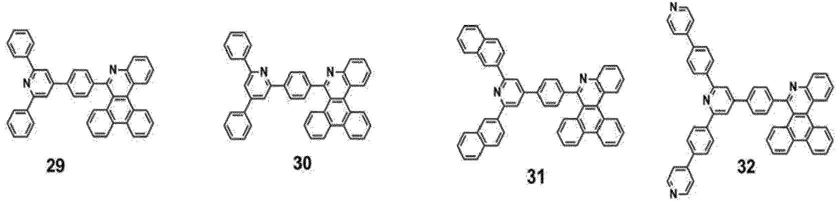

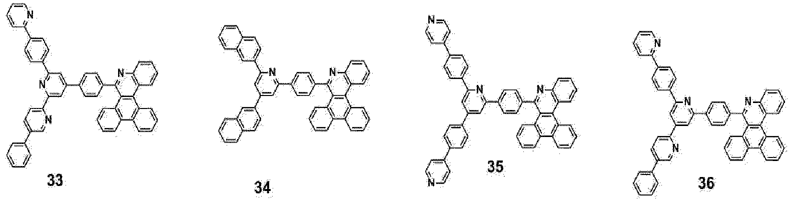

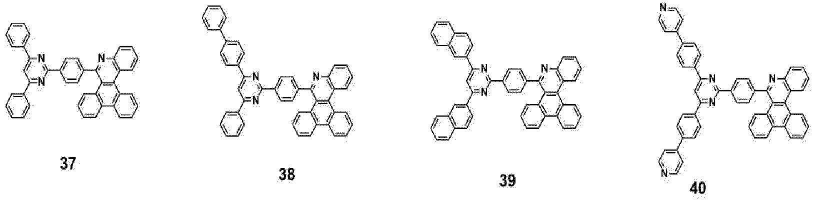

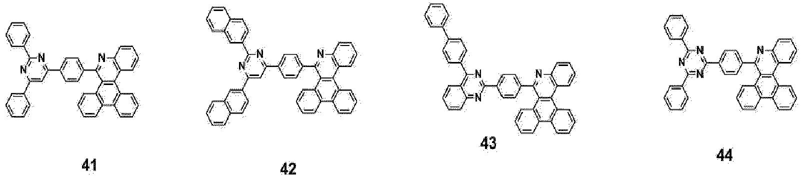

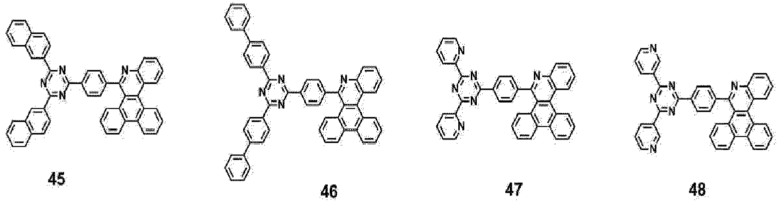

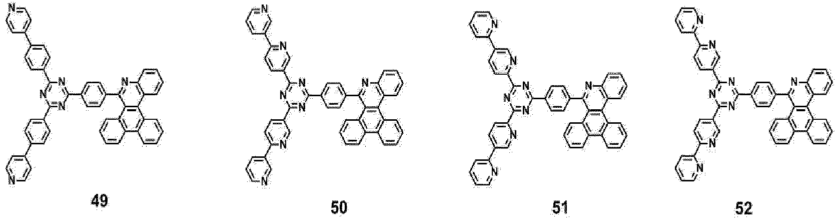

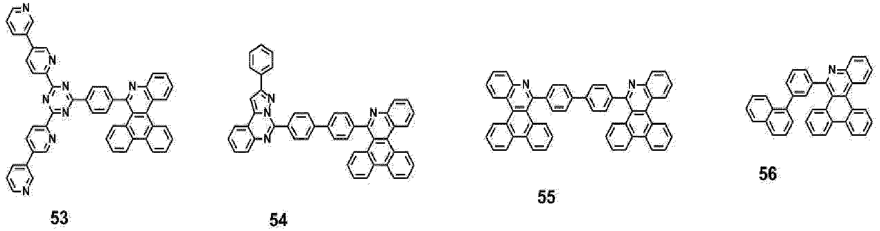

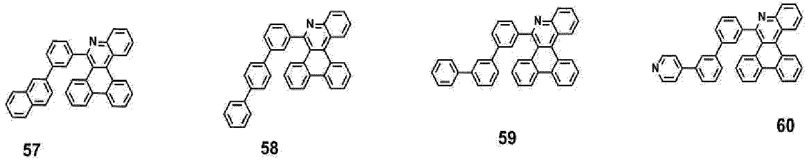

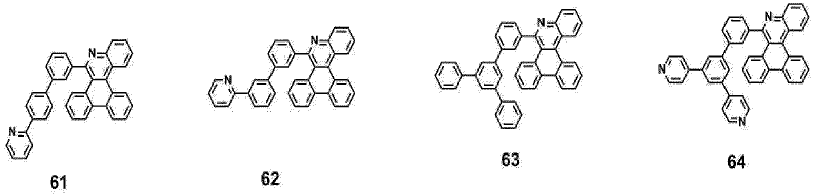

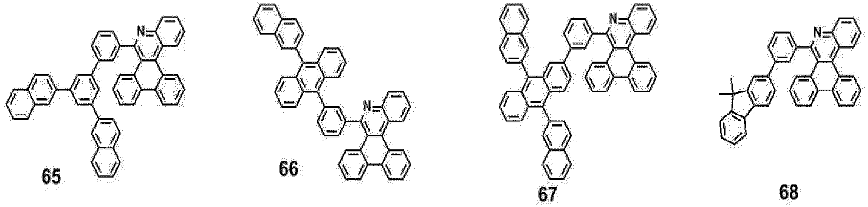

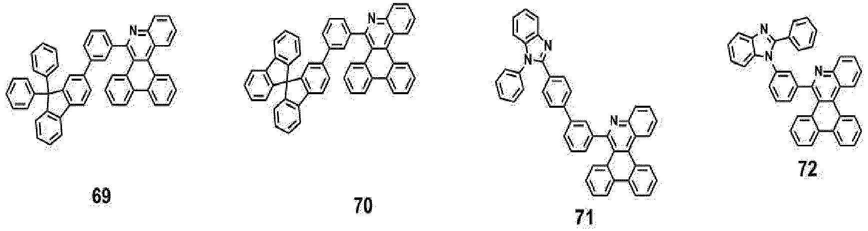

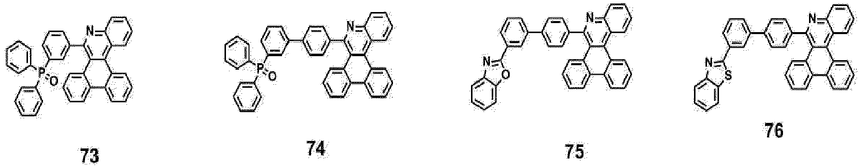

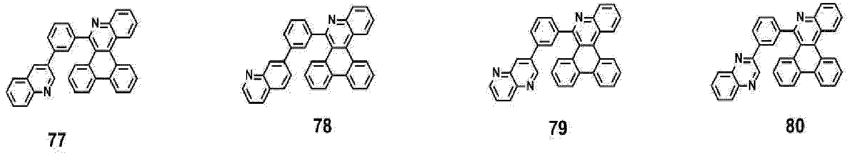

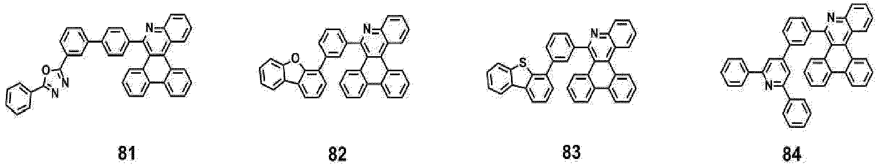

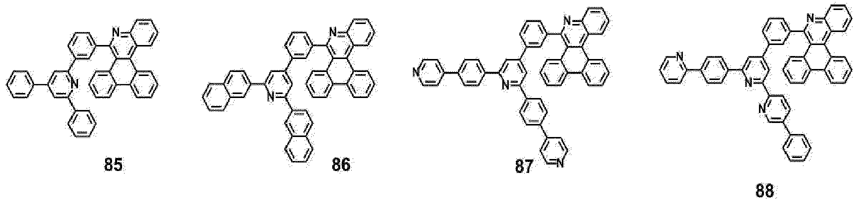

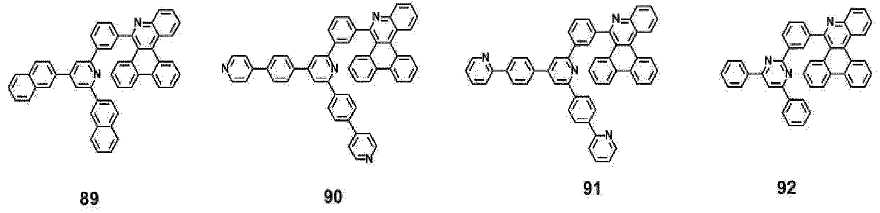

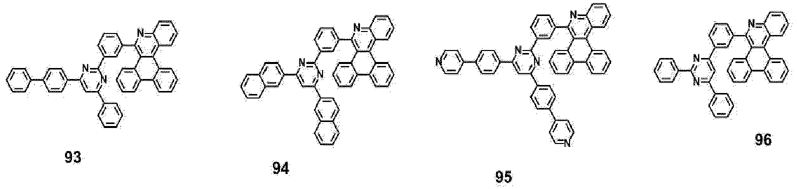

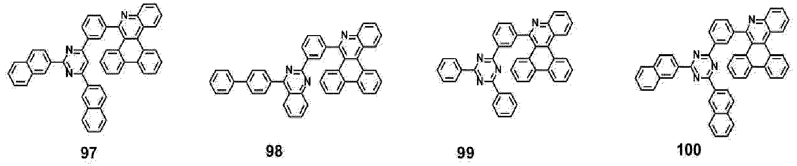

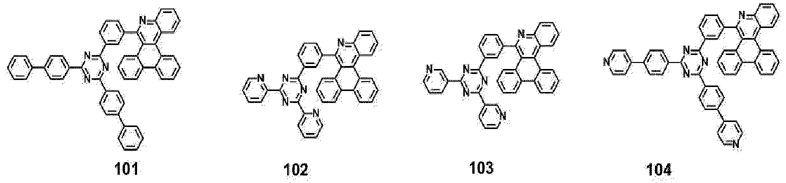

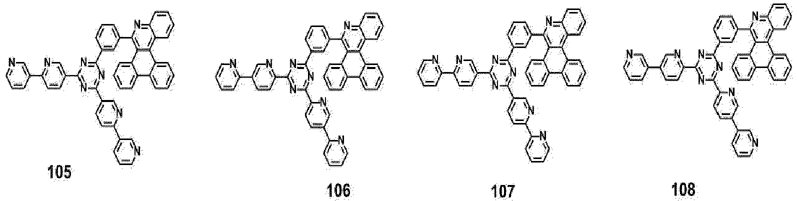

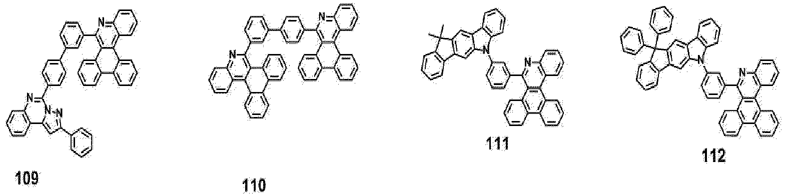

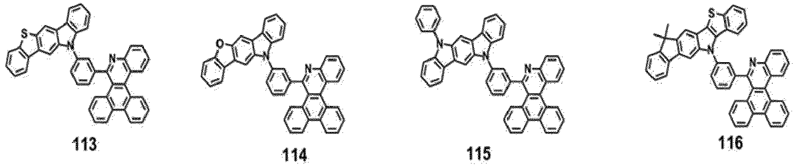

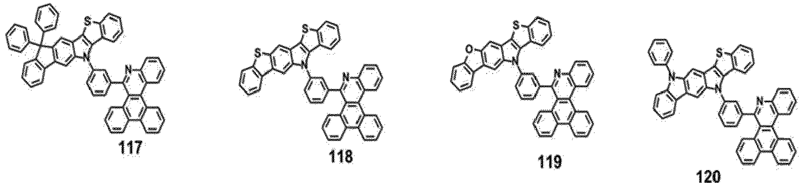

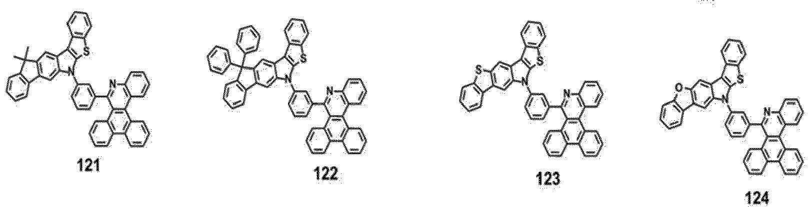

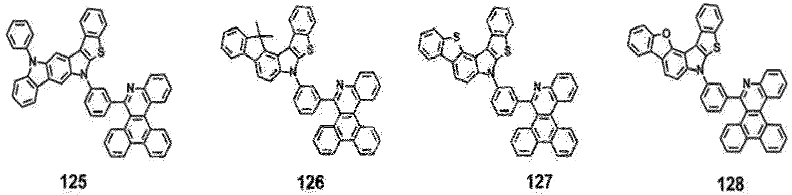

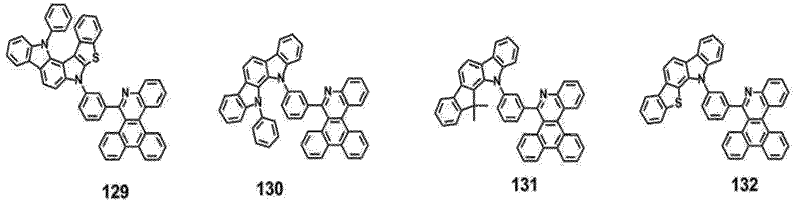

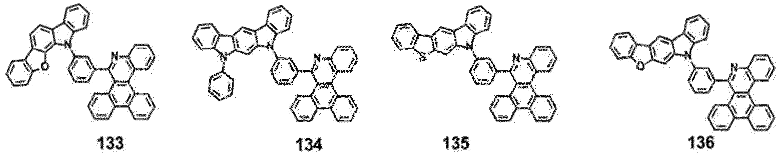

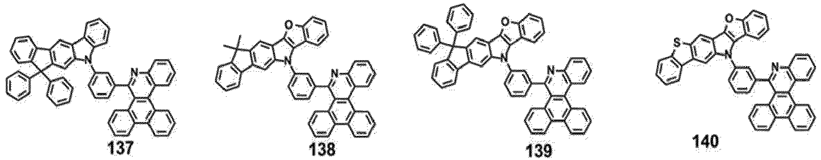

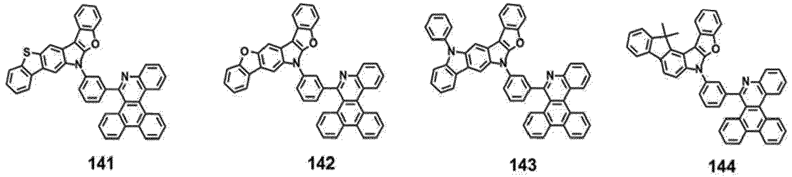

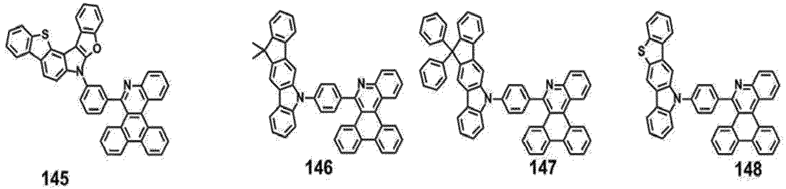

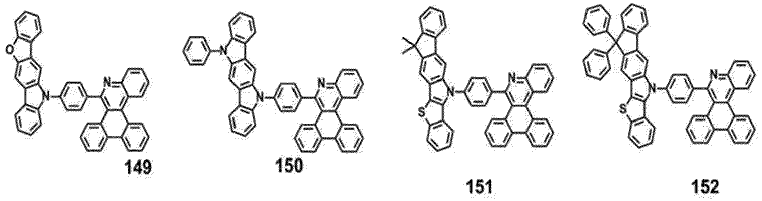

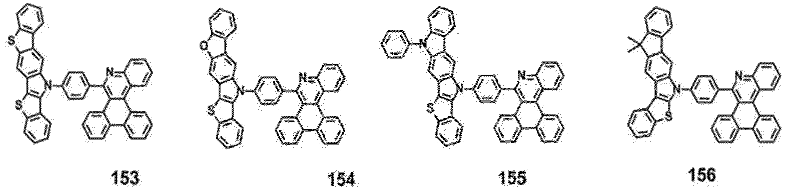

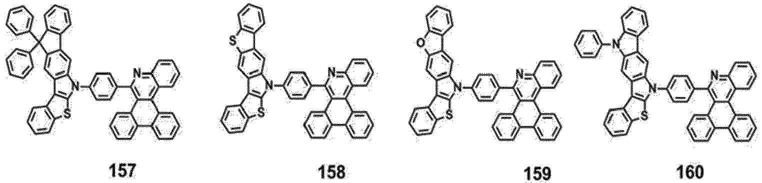

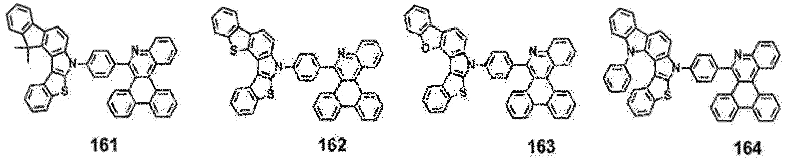

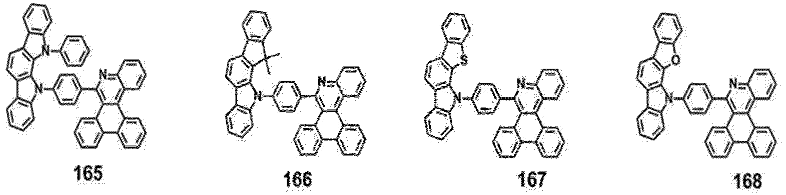

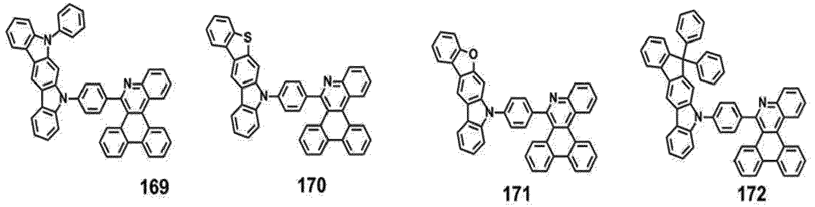

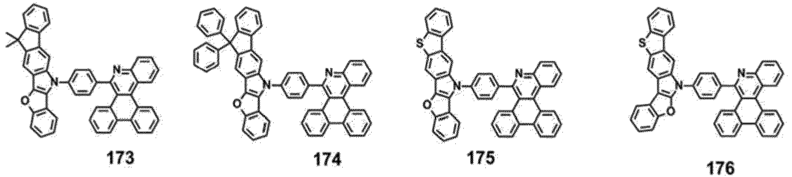

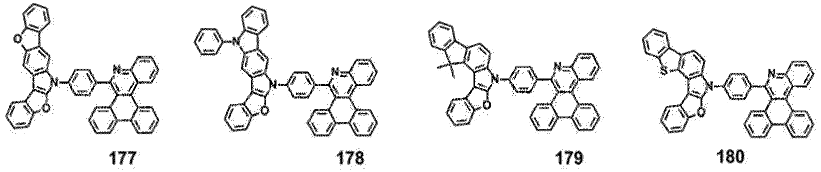

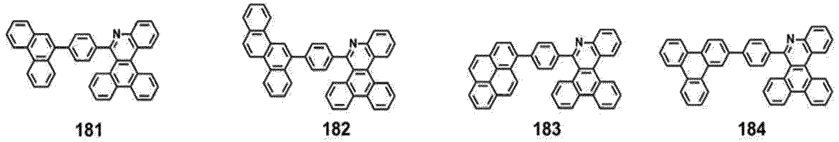

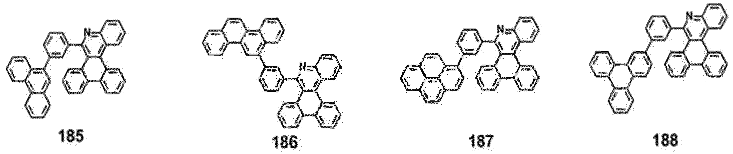

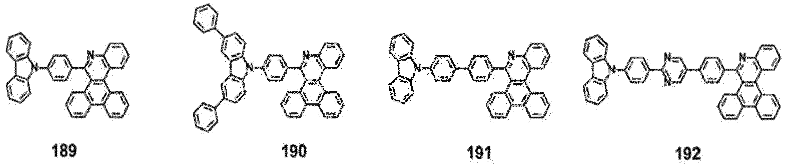

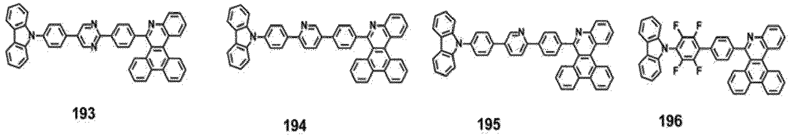

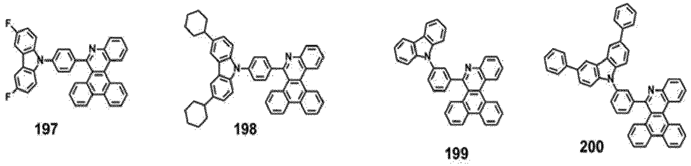

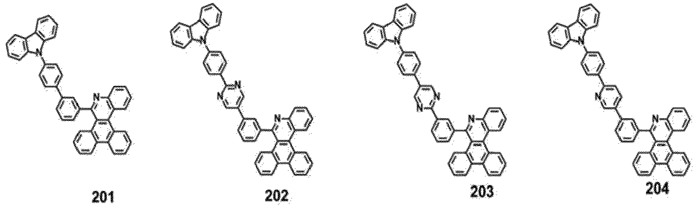

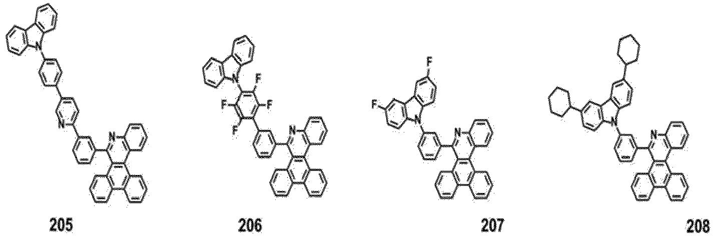

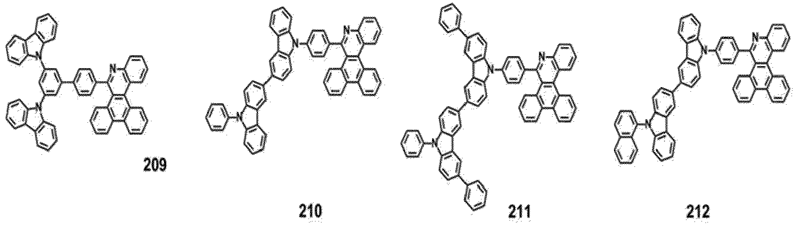

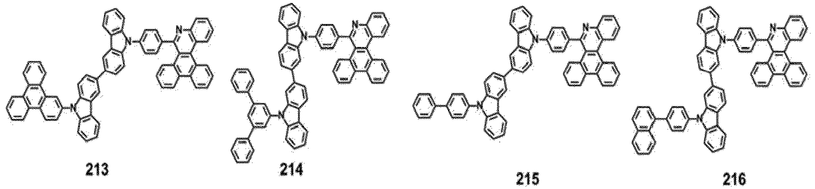

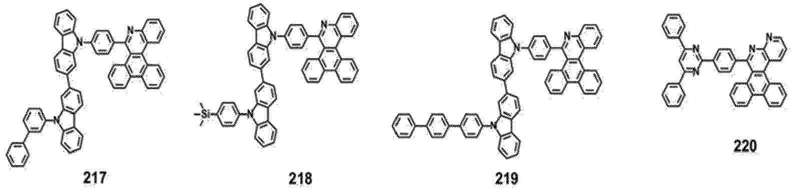

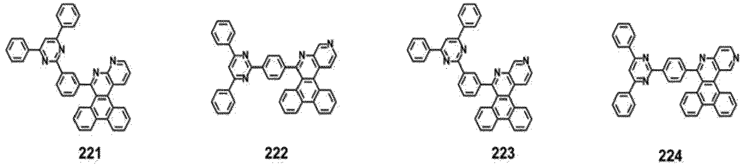

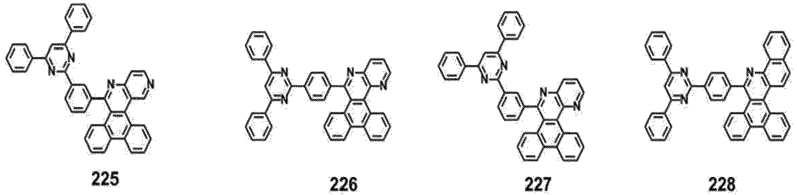

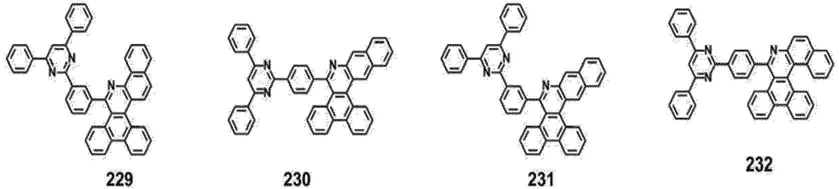

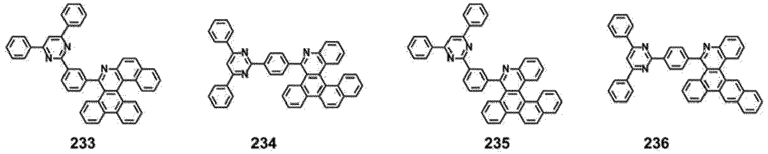

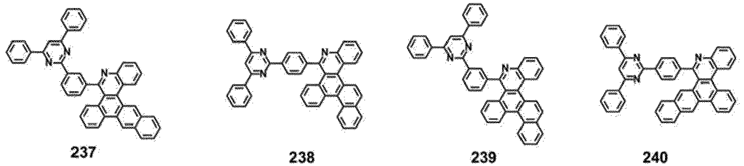

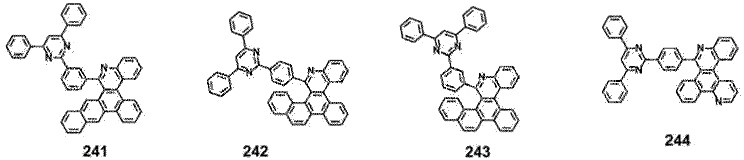

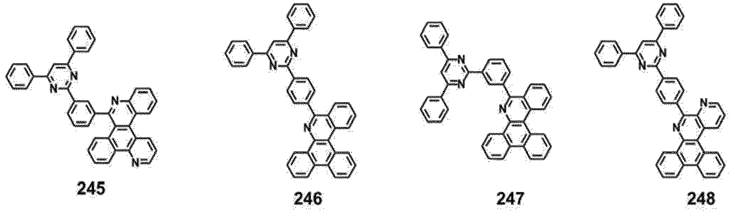

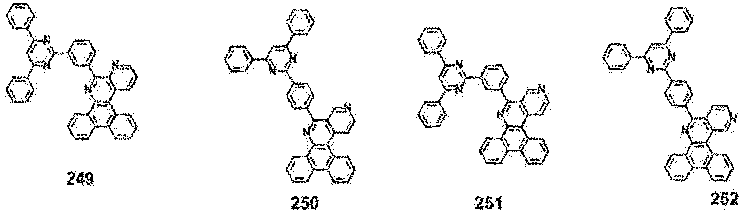

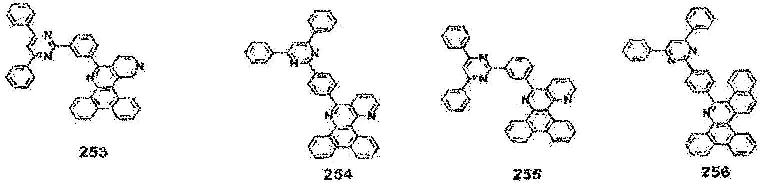

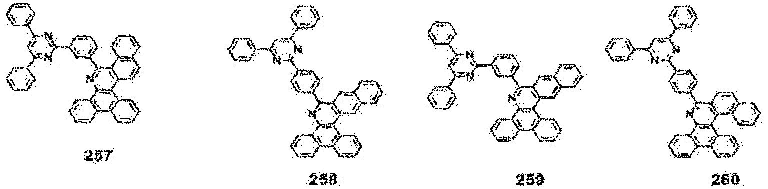

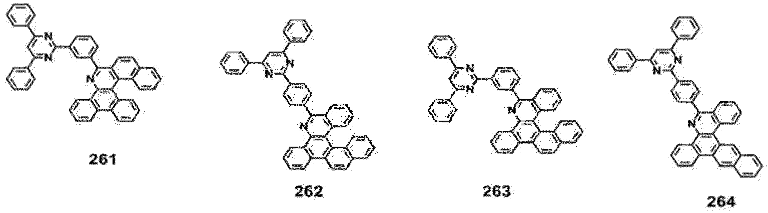

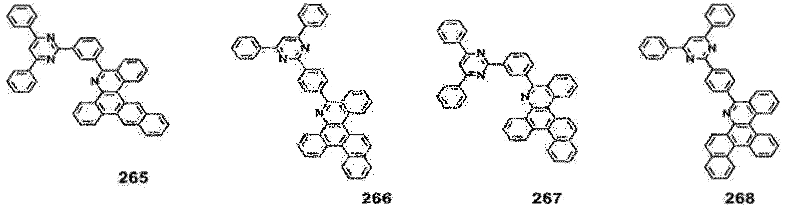

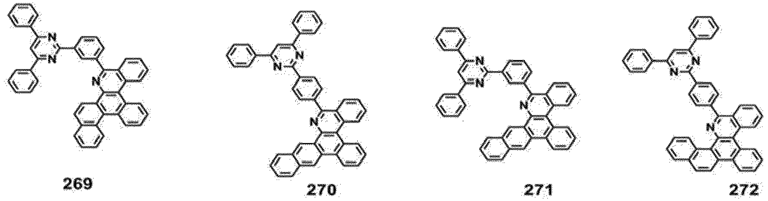

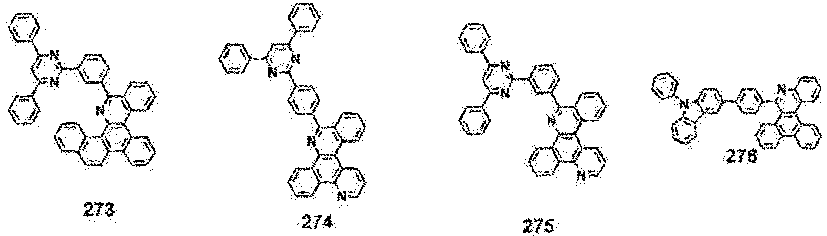

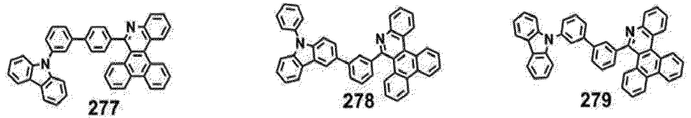

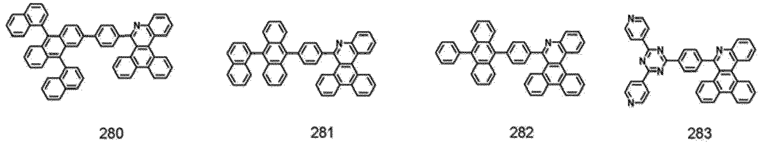

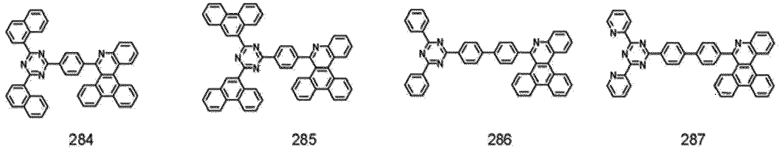

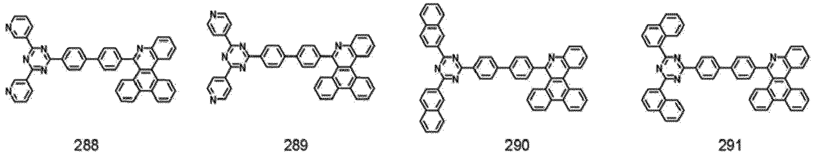

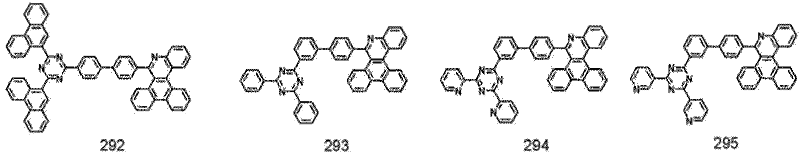

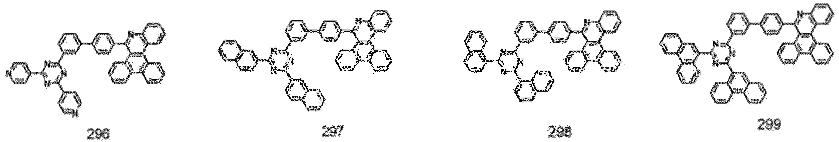

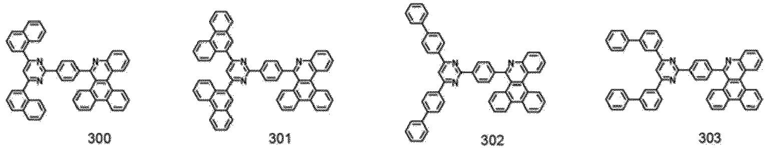

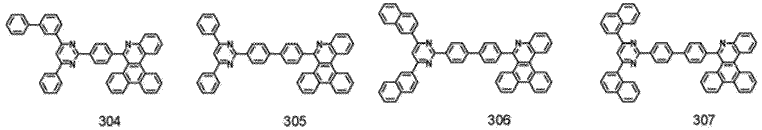

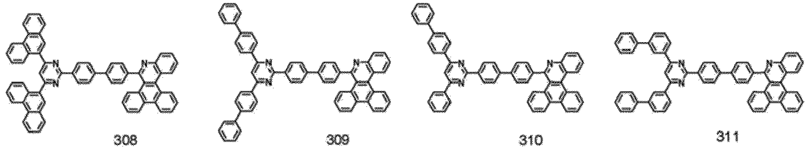

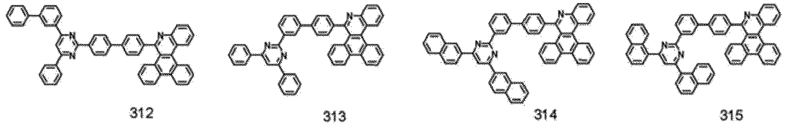

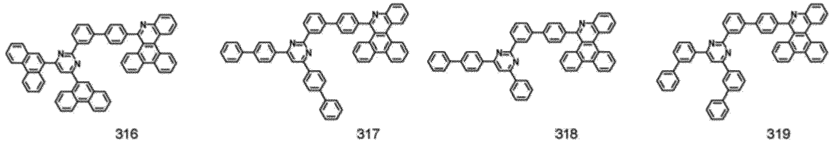

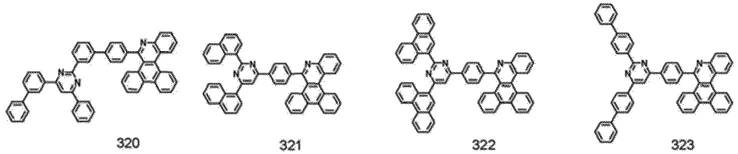

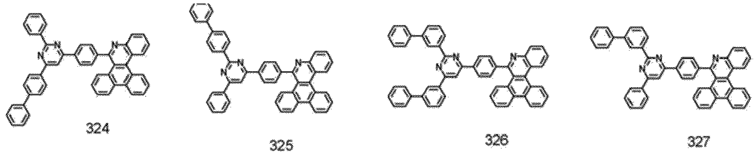

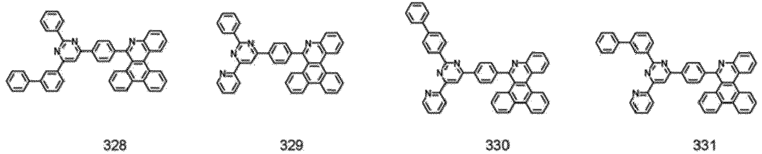

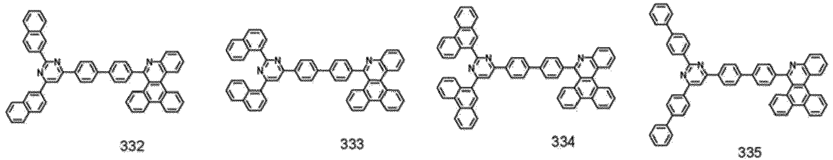

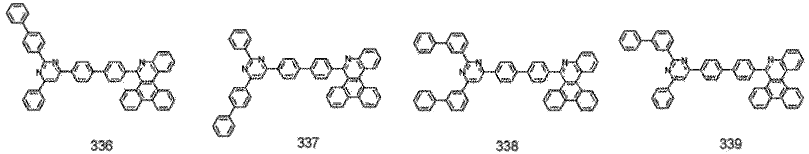

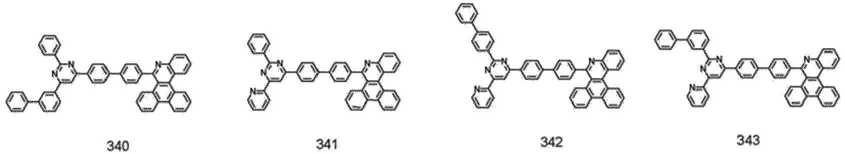

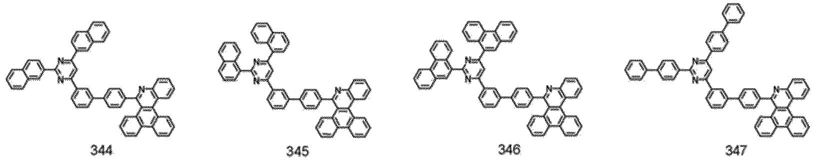

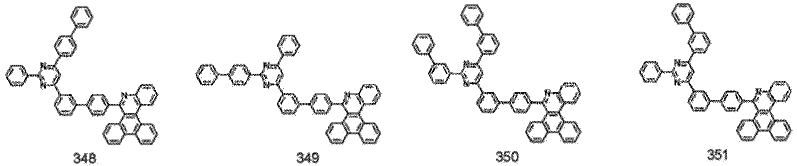

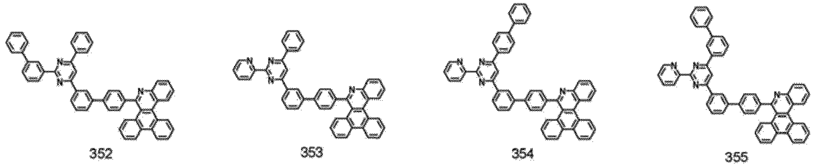

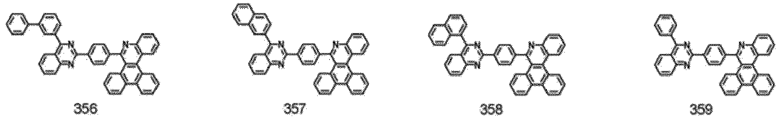

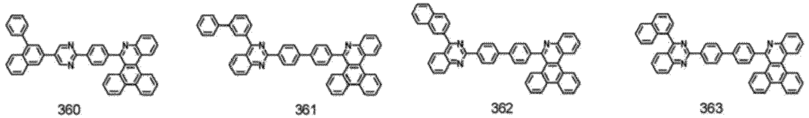

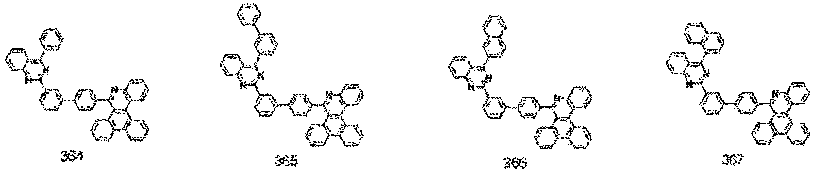

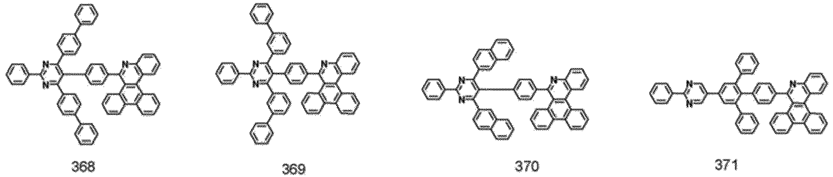

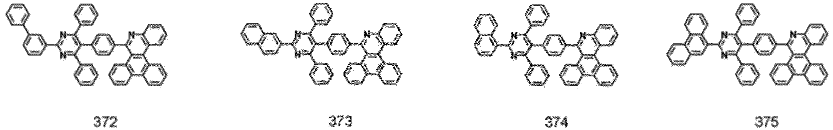

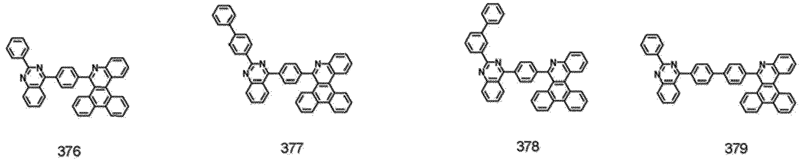

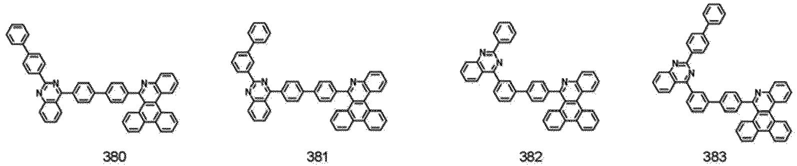

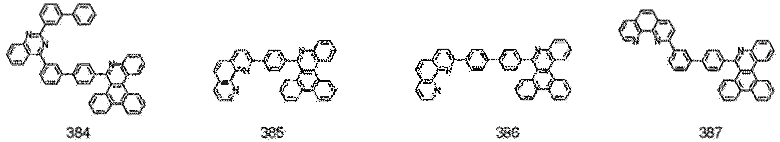

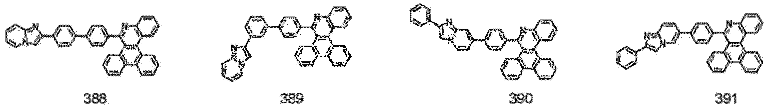

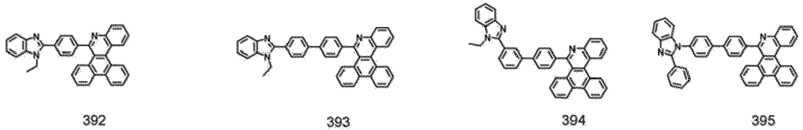

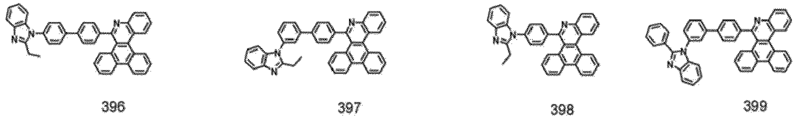

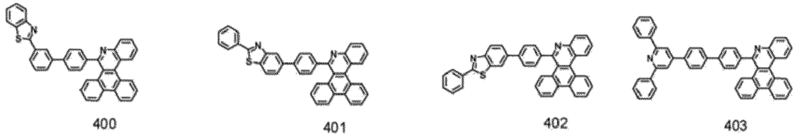

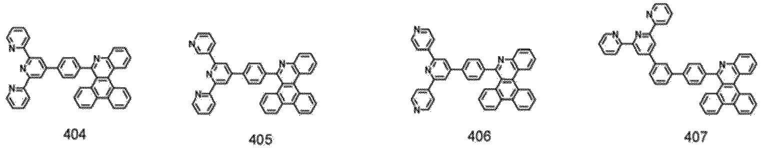

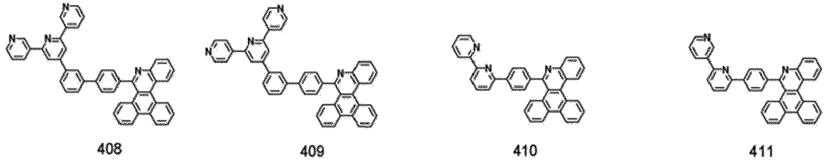

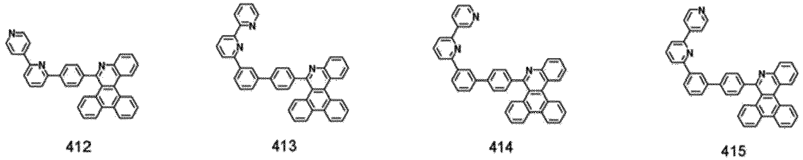

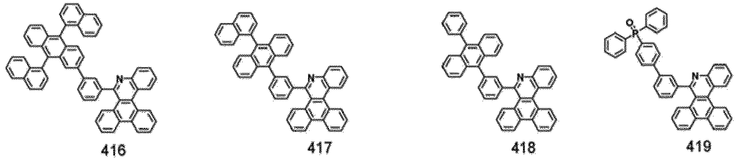

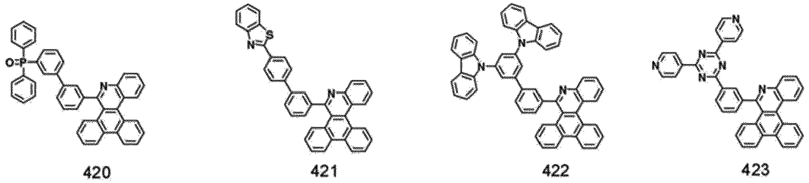

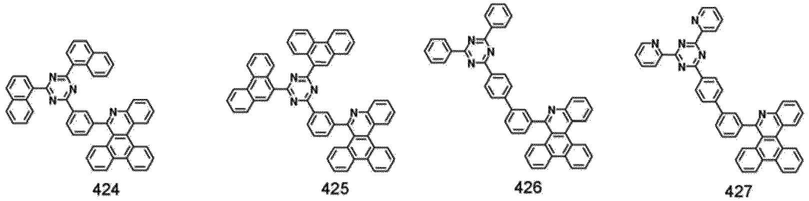

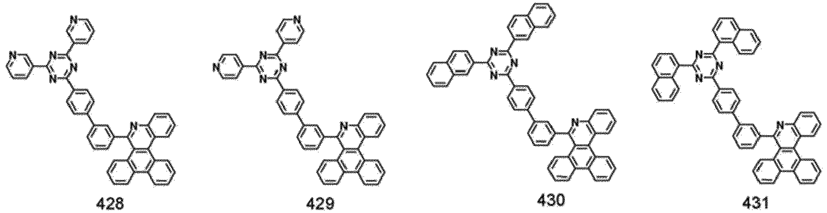

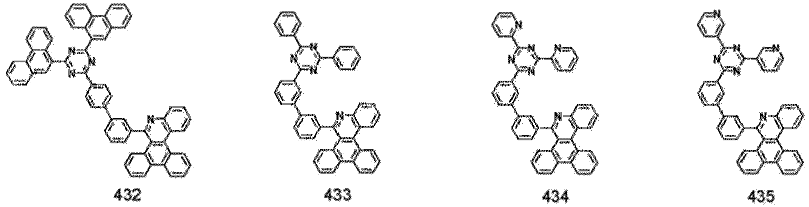

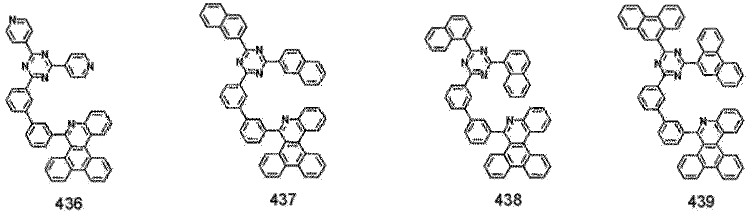

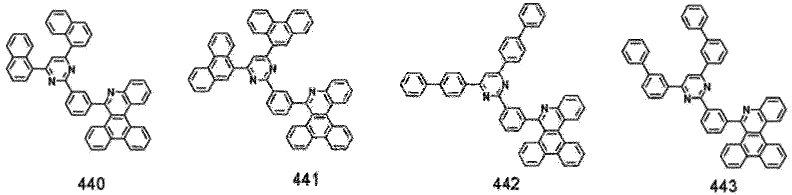

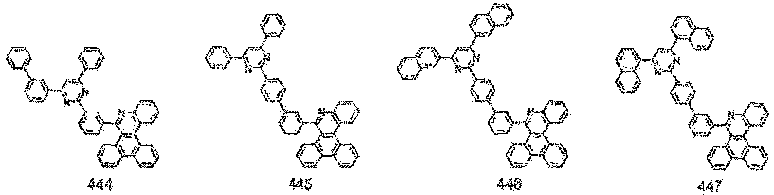

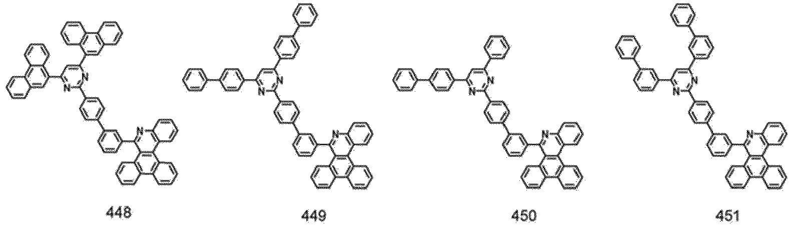

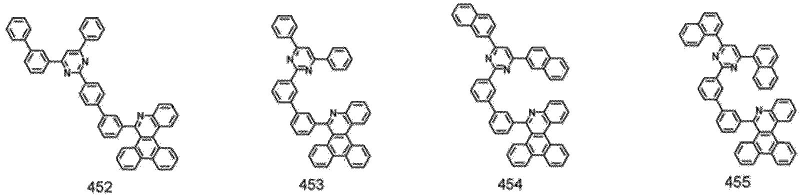

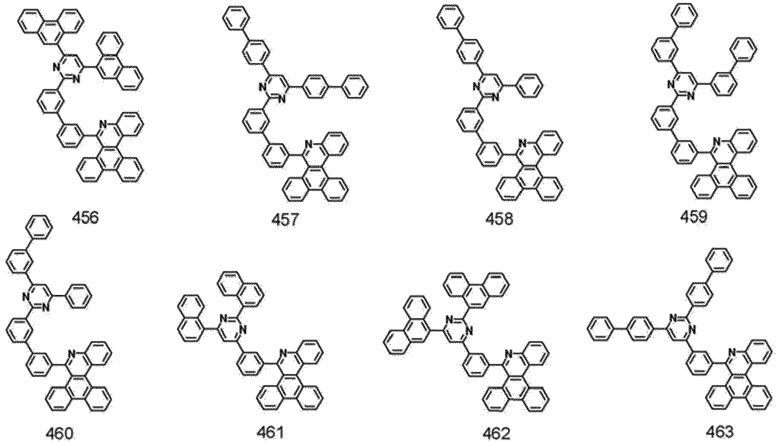

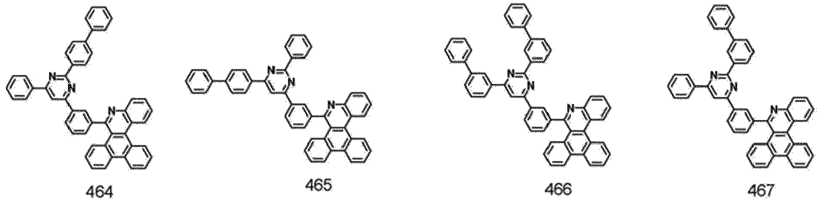

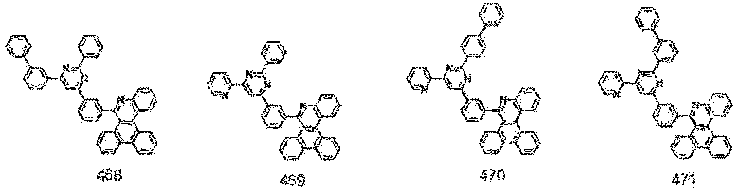

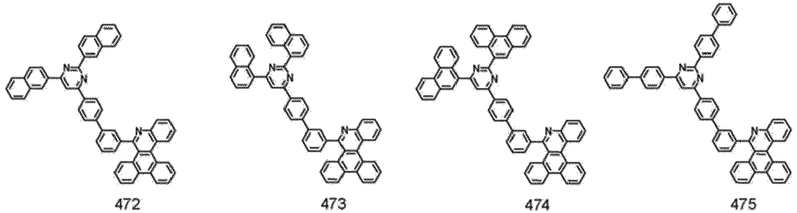

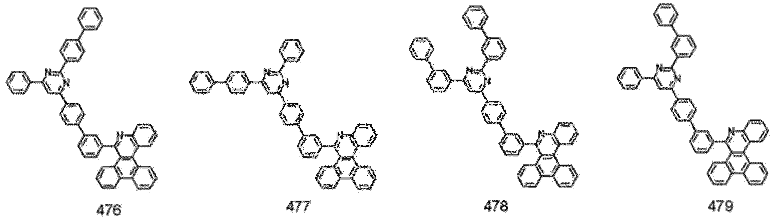

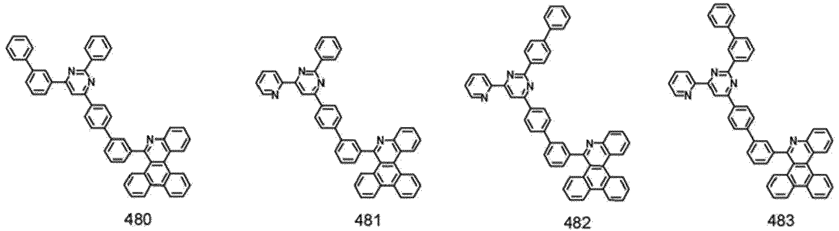

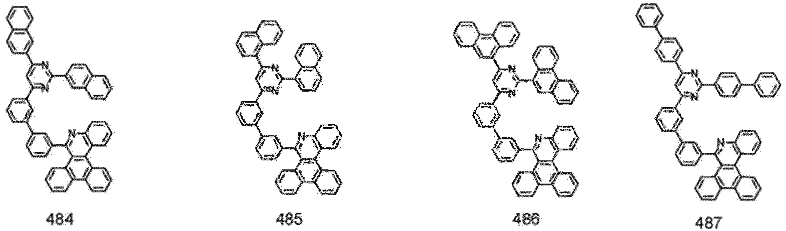

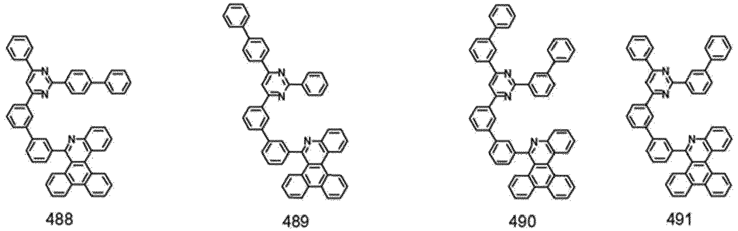

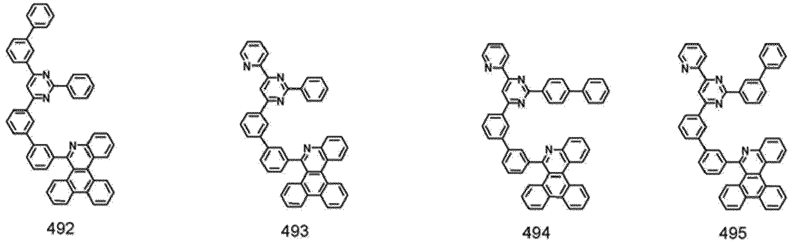

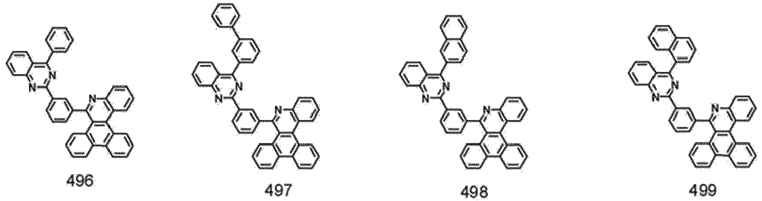

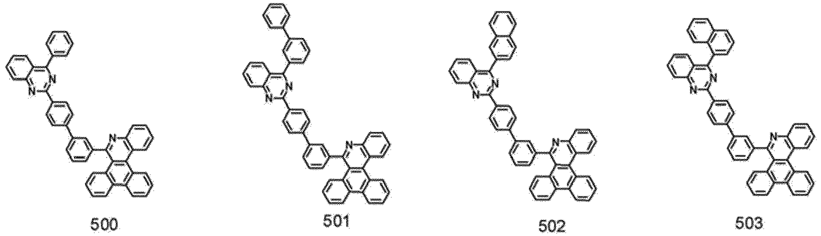

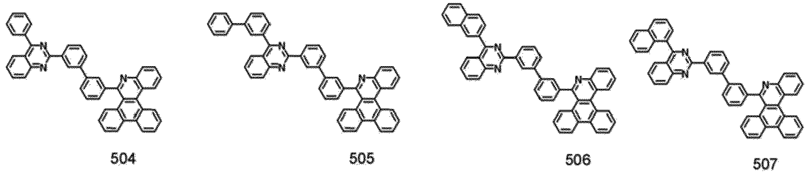

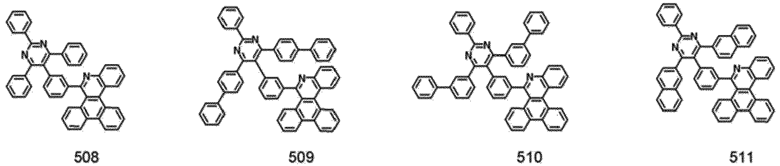

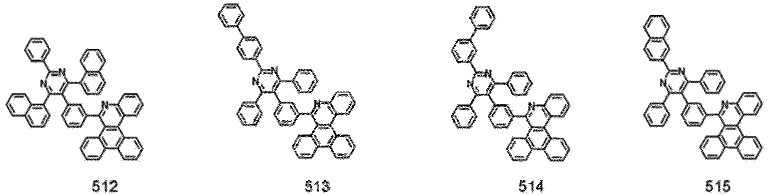

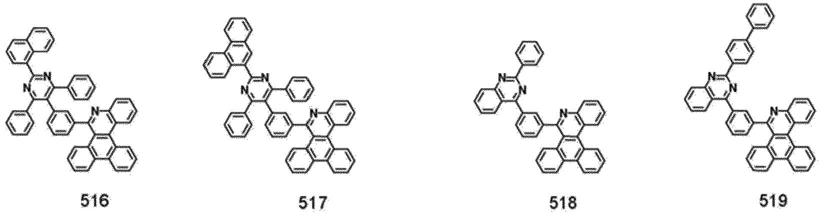

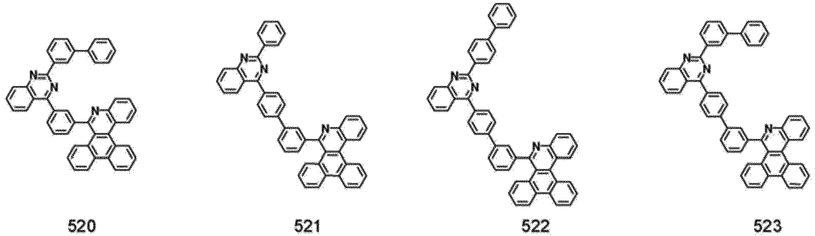

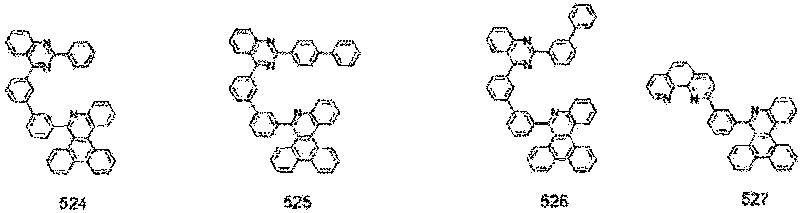

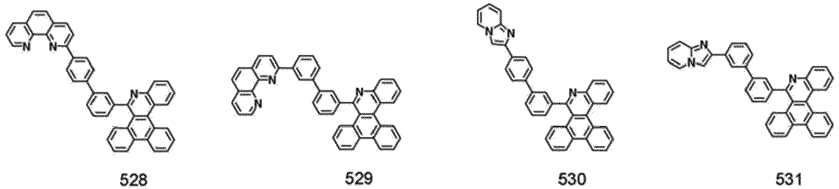

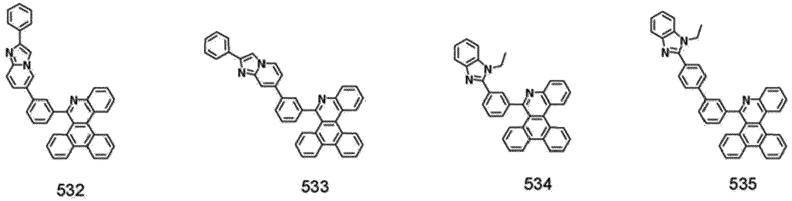

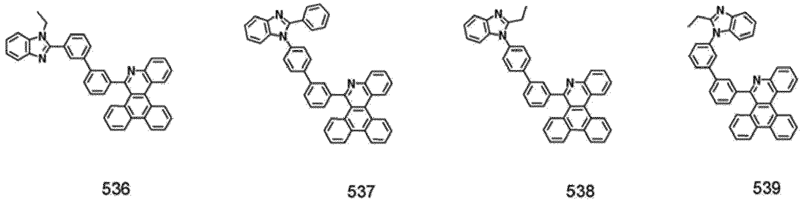

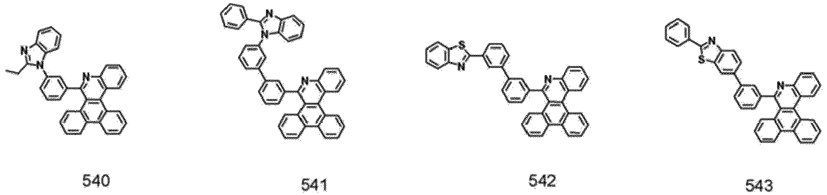

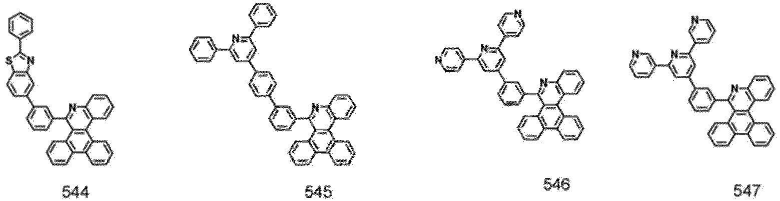

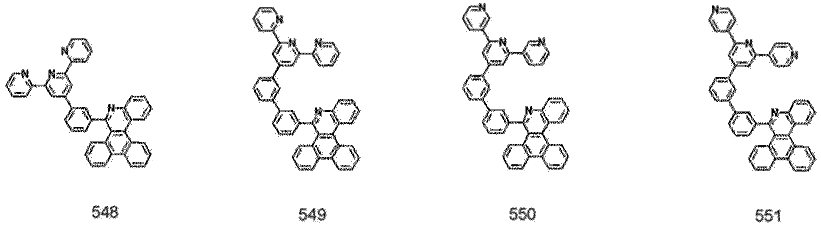

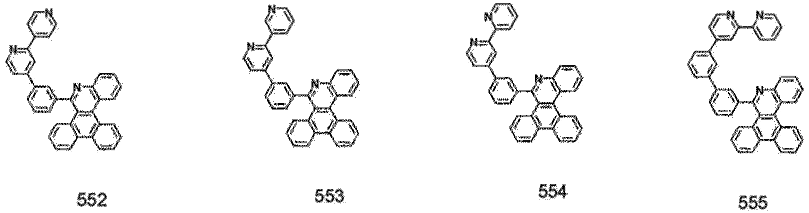

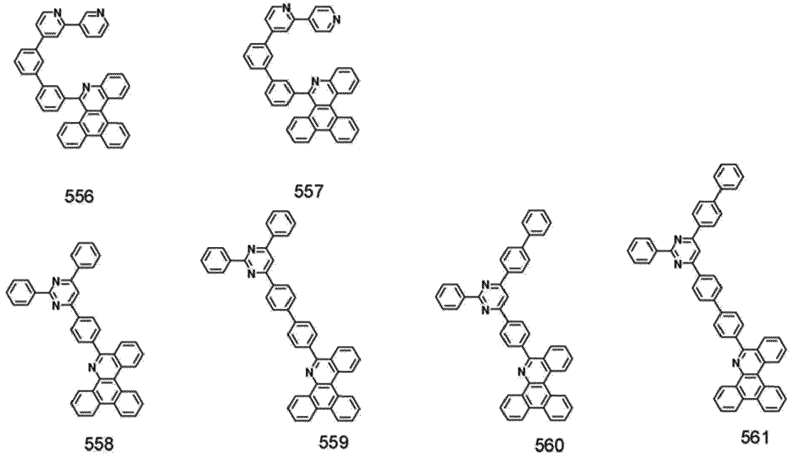

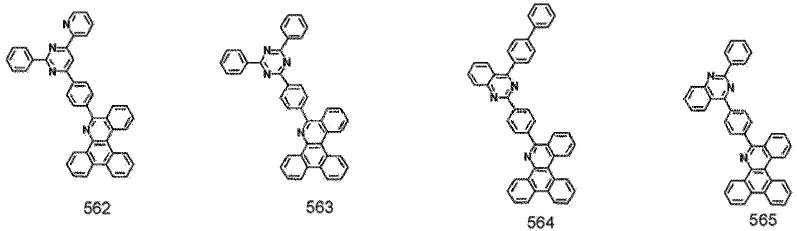

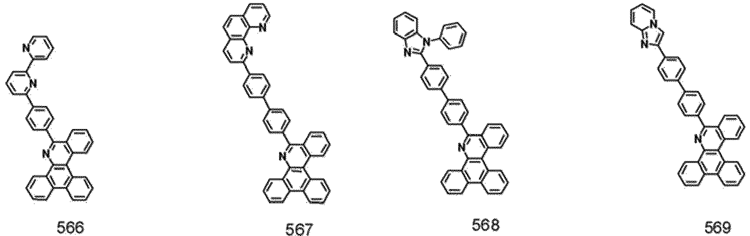

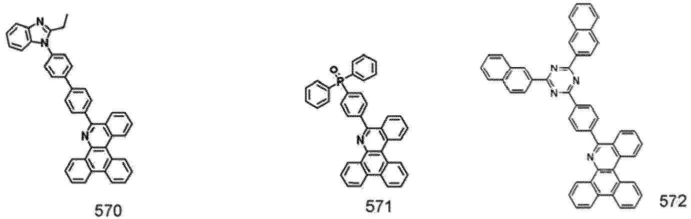

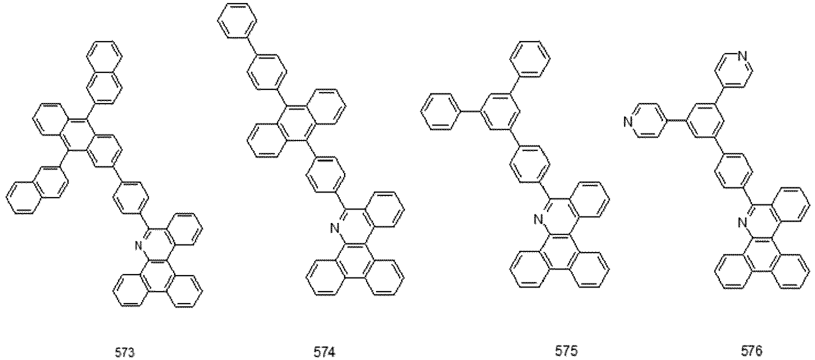

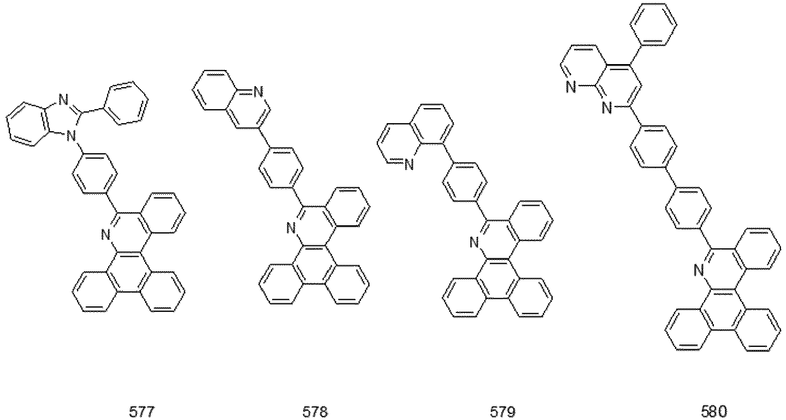

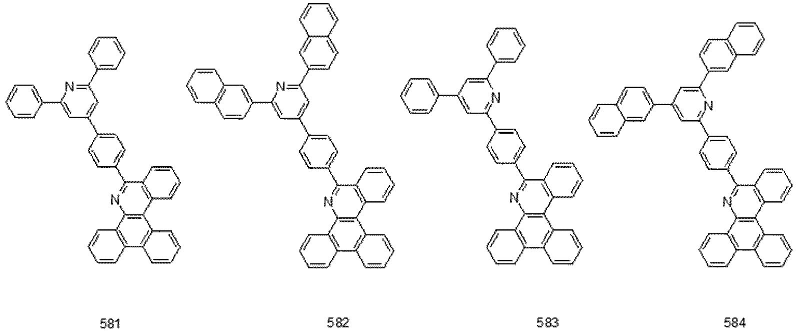

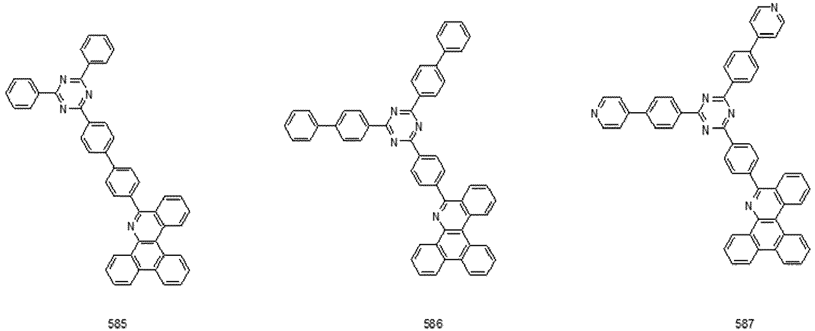

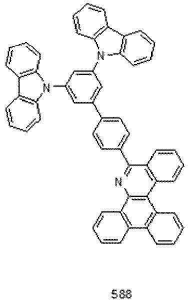

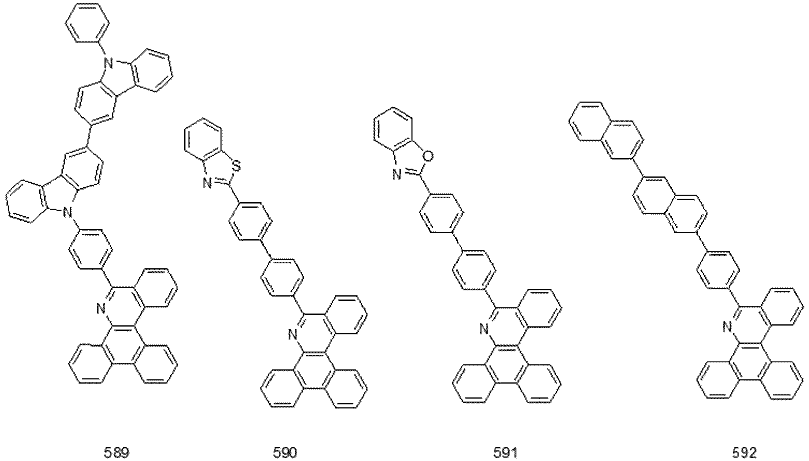

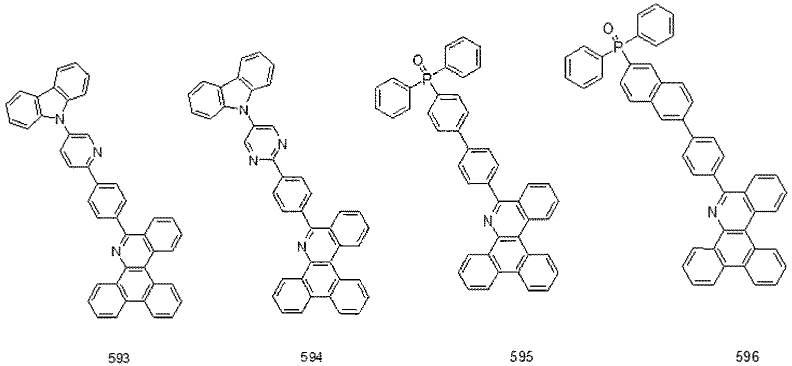

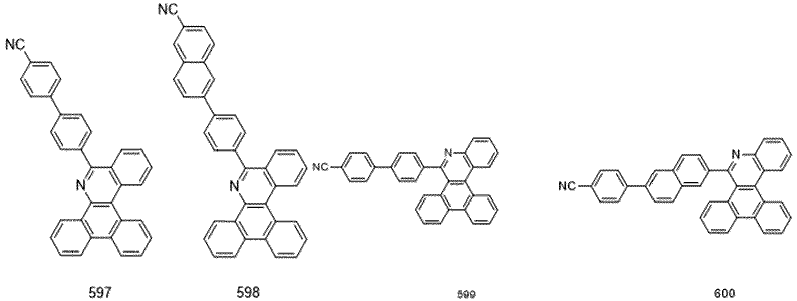

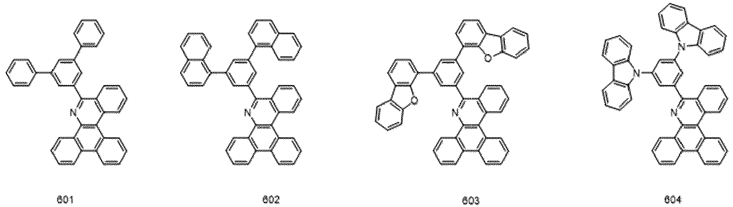

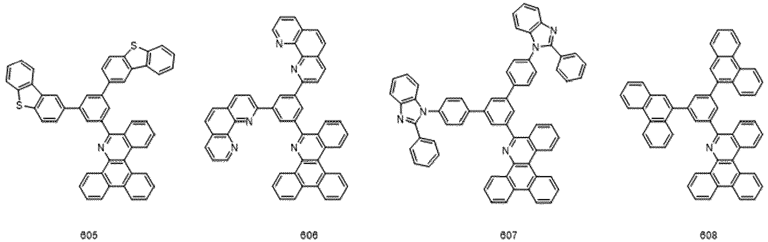

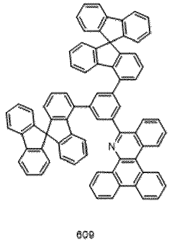

13. Organische lichtemittierende Vorrichtung, umfassend:
eine Anode;
eine Kathode; und
eine oder mehrere organische Materialschichten, die zwischen der Anode und der Kathode bereitgestellt sind,
wobei eine oder mehrere Schichten der organischen Materialschichten die Verbindung nach einem der Ansprüche 1 bis 12 einschließt bzw. einschließen.

14. Organische lichtemittierende Vorrichtung nach Anspruch 13, wobei eine organische Materialschicht, die die Verbindung nach einem der Ansprüche 1 bis 12 einschließt, eine oder mehrere Schichten ist, ausgewählt aus einer Lochinjektionsschicht, einer Lochtransportschicht, einer lichtemittierenden Schicht, einer Lochblockierschicht, einer Elektronentransportschicht und einer Elektroneninjektionsschicht.

15. Organische lichtemittierende Vorrichtung nach Anspruch 13, wobei eine organische Materialschicht, die die Verbindung nach einem der Ansprüche 1 bis 12 einschließt, eine Elektronentransportschicht ist.

16. Organische lichtemittierende Vorrichtung nach Anspruch 13, wobei eine organische Materialschicht, die die Verbindung nach einem der Ansprüche 1 bis 12 einschließt, eine lichtemittierende Schicht ist.

17. Organische lichtemittierende Vorrichtung nach Anspruch 13, wobei eine organische Materialschicht, die die Verbindung nach einem der Ansprüche 1 bis 12 einschließt, eine Lochblockierschicht ist.

## Revendications

1. Composé répondant à la formule chimique 2, 3 ou 9 suivante : 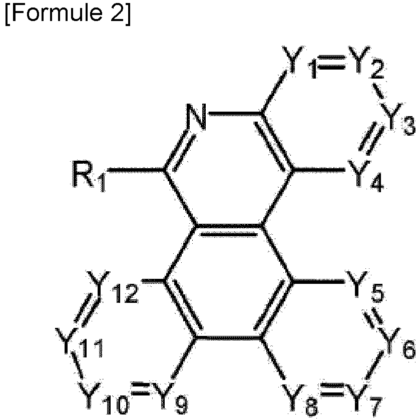 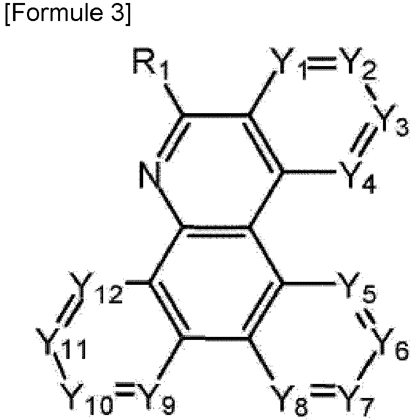 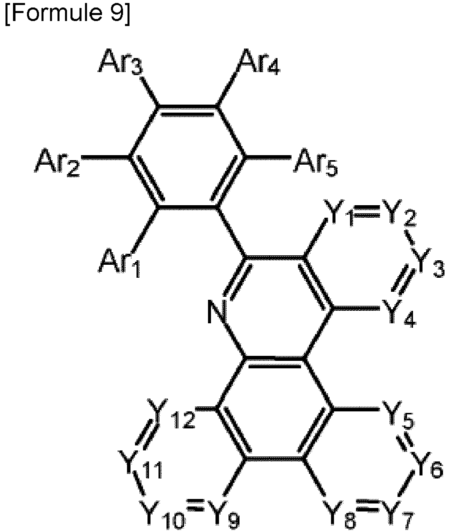 dans lesquelles
Y1 à Y12 sont identiques ou différents l'un de l'autre et représentent chacun indépendamment N ou CR,
R1 désigne -(L)m-(Z)n,
L représente un arylène en C6 à C60 monocyclique ou polycyclique substitué ou non substitué, ou un hétéroarylène en C2 à C60 monocyclique ou polycyclique substitué ou non substitué,
Z est sélectionné dans le groupe constitué d'un aryle en C6 à C60 en C2 à C60 monocyclique ou polycyclique substitué ou non substitué ; d'un hétéroaryle en C2 à C60 monocyclique ou polycyclique substitué ou non substitué ; de -SiR11R12R13 ; et de -P(=0)R14R15,
m représente un nombre entier valant de 1 à 5,
n représente un nombre entier valant de 1 à 3,
lorsque m et n représentent indépendamment des nombres entiers valant 2 ou plus, les L et Z multiples sont identiques ou différents les uns des autres,
R représente un élément sélectionné dans le groupe constitué d'hydrogène ; ou au moins deux R adjacents sont liés l'un à l'autre pour former un cycle hydrocarboné aliphatique ou aromatique monocyclique ou polycyclique substitué ou non substitué ;
où "substitué ou non substitué" se réfère à un groupe qui peut être substitué ou ne peut pas être substitué davantage par un ou plusieurs substituants sélectionnés dans le groupe constitué d'halogène ; de -CN ; d'alkyle en C1 à C60 linéaire ou ramifié; de cycloalkyle en C3 à C60 monocyclique ou polycyclique; d'aryle en C6 à C60 monocyclique ou polycyclique ; d'hétéroaryle en C2 à C60 monocyclique ou polycyclique; de -SiR11R12R13 ; et de P(=0)R14R15,
R11 à R15 sont identiques ou différents les uns des autres, et sont chacun indépendamment sélectionnés dans le groupe constitué d'un alkyle en C1 à C60 linéaire ou ramifié, substitué ou non substitué ; d'un cycloalkyle en C3 à C60 monocyclique ou polycyclique substitué ou non substitué ; d'un aryle en C6 à C60 monocyclique ou polycyclique substitué ou non substitué ; et d'un hétéroaryle en C2 à C60 monocyclique ou polycyclique substitué ou non substitué, et
au moins deux de Ar1 à Ar5 sont un aryle en C6 à C60 monocyclique ou polycyclique substitué ou non substitué ; ou un hétéroaryle en C2 à C60 monocyclique ou polycyclique substitué ou non substitué, et les autres représentent chacun indépendamment l'hydrogène ; le deutérium ; l'halogène ; un alkyle en C1 à C60 substitué ou non substitué à chaîne droite ou ramifiée ; un aryle en C6 à C60 monocyclique ou polycyclique substitué ou non substitué ; ou un hétéroaryle en C2 à C60 monocyclique ou polycyclique substitué ou non substitué.

2. Composé selon la revendication 1, dans lequel tous les Y1 à Y4 sont CR, ou l'un quelconque des Y1 à Y4 est N et les autres sont CR, et
le R est identique à celui défini dans la revendication 1.

3. Composé selon la revendication 1, dans lequel tous les Y5 à Y8 sont CR, ou l'un quelconque des Y5 à Y8 est N et les autres sont CR, et
le R est identique à celui défini dans la revendication 1.

4. Composé selon la revendication 1, dans lequel tous les Y9 à Y12 sont CR, ou l'un quelconque des Y9 à Y12 est N et les autres sont CR, et
le R est identique à celui défini dans la revendication 1.

5. Composé de la revendication 1, dans lequel les Y1 à Y12 sont CR, et le R est identique à celui défini dans la revendication 1.

6. Composé de la revendication 1, dans lequel
L est sélectionné dans le groupe constitué par un arylène en C6 à C60 monocyclique ou polycyclique, non substitué ou substitué par un halogène, un alkyle en C1 à C60 linéaire ou ramifié, un cycloalkyle en C3 à C60 monocyclique ou polycyclique, un aryle en C6 à C60 monocyclique ou polycyclique, et un hétéroaryle en C2 à C60 monocyclique ou polycyclique ; et un hétéroarylène en C2 à C60 monocyclique ou polycyclique non substitué ou substitué par un halogène, un alkyle en C1 à C60 linéaire ou ramifié, un cycloalkyle en C3 à C60 monocyclique ou polycyclique, un aryle en C6 à C60 monocyclique ou polycyclique et un hétéroaryle en C2 à C60 monocyclique ou polycyclique,
Z est sélectionné dans le groupe constitué par un aryle en C6 à C60 monocyclique ou polycyclique non substitué ou substitué par un halogène, un alkyle en C1 à C60 linéaire ou ramifié, un cycloalkyle en C3 à C60 monocyclique ou polycyclique, un aryle en C6 à C60 monocyclique ou polycyclique, et un hétéroaryle en C2 à C60 monocyclique ou polycyclique; un hétéroaryle en C2 à C60 monocyclique ou polycyclique non substitué ou substitué par un halogène, un alkyle en C1 à C60 linéaire ou ramifié, un cycloalkyle en C3 à C60 monocyclique ou polycyclique, un aryle en C6 à C60 monocyclique ou polycyclique, et un hétéroaryle en C2 à C60 monocyclique ou polycyclique ; -SiR11R12R13 ; et -P(=0)R14R15,
R représente l'hydrogène ; et les R11 à R15 sont identiques ou différents les uns des autres, et sont chacun indépendamment sélectionnés dans le groupe constitué d'hydrogène ; d'alkyle linéaire ou ramifié non substitué ou substitué par l'halogène, d'alkyle en C1 à C60 linéaire ou ramifié, de cycloalkyle en C3 à C60 monocyclique ou polycyclique, d'aryle en C6 à C60 monocyclique ou polycyclique, et d'hétéroaryle en C2 à C60 monocyclique ou polycyclique ; d'aryle en C6 à C60 monocyclique ou polycyclique non substitué ou substitué par l'halogène, d'alkyle en C1 à C60 linéaire ou ramifié, de cycloalkyle en C3 à C60 monocyclique ou polycyclique, d'aryle en C6 à C60 monocyclique ou polycyclique, et d'hétéroaryle en C2 à C60 monocyclique ou polycyclique; et d'hétéroaryle en C2 à C60 monocyclique ou polycyclique non substitué ou substitué par un l'halogène, d'alkyle en C1 à C60 linéaire ou ramifié, de cycloalkyle en C3 à C60 monocyclique ou polycyclique, d'aryle en C6 à C60 monocyclique ou polycyclique, et d'hétéroaryle en C2 à C60 monocyclique ou polycyclique, et
si deux ou plusieurs des Y1 à Y12 sont CR, les multiples R sont identiques ou différents les uns des autres.

7. Composé selon la revendication 1, dans lequel Z est un phényle substitué ou non substitué, un biphényle substitué ou non substitué, un naphtyle substitué ou non substitué, un chrysényle substitué ou non substitué, un pyrényle substitué ou non substitué, triphényle substitué ou non substitué, un anthracényle substitué ou non substitué , un phénanthrényle substitué ou non substitué , un fluorényle substitué ou non substitué , un benzo chrysényle substitué ou non substitué ou un spirobifluorényle substitué ou non substitué.

8. Composé selon la revendication 1, dans lequel Z est un hétéroaryle en C2 à C60 monocyclique ou polycyclique substitué ou non substitué, et l'hétéroaryle comprend au moins un élément sélectionné parmi N, O, S, Si et Se comme hétéroatome.

9. Composé selon la revendication 1, dans lequel Z représente 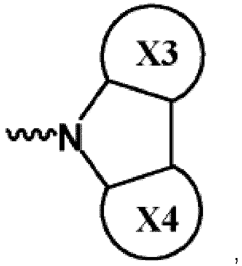 et X3 et X4 représentent des cycles hydrocarbonés aromatiques en C6 à C60, monocycliques ou polycycliques, substitués ou non substitués ou des cycles hétéro aromatiques en C2 à C60 monocycliques ou polycycliques, substitués ou non substitués.

10. Composé selon la revendication 9, dans lequel, 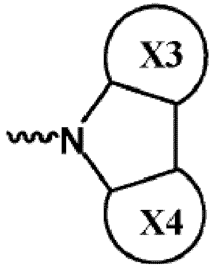 est représenté par l'une des formules structurales suivantes : 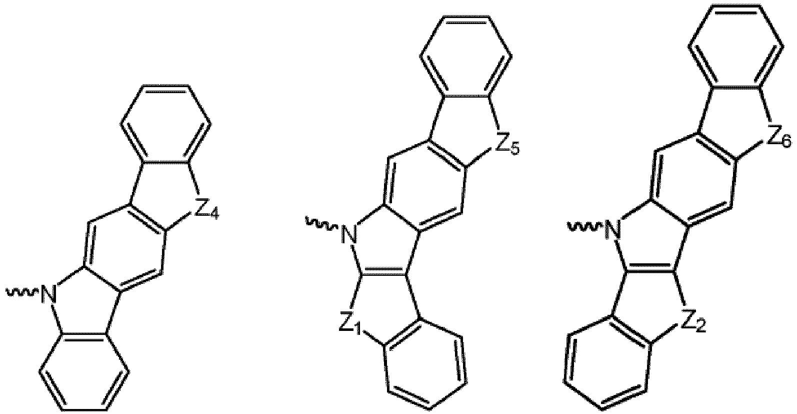 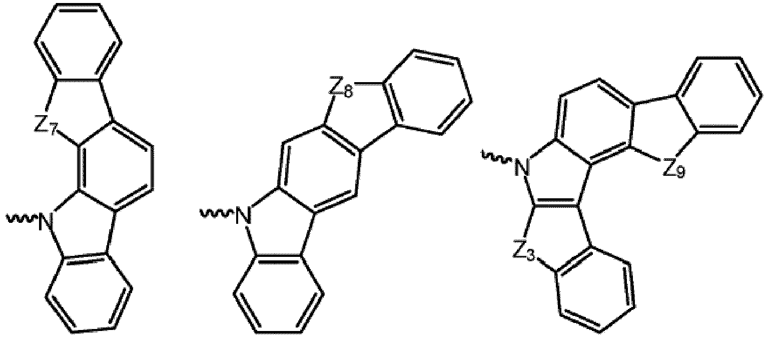
où, dans les formules chimiques structurales, Z1 à Z3 sont identiques ou différents l'un de l'autre et représentent indépendamment S ou O,
Z4 à Z9 sont identiques ou différents l'un de l'autre et représentent chacun indépendamment Cr' R", Nr', S ou O,
R' et R" sont identiques ou différents l'un de l'autre et représentent chacun indépendamment l'hydrogène, un alkyle en C1 à C60 linéaire ou ramifié, substitué ou non substitué ou un aryle en C6 à C60 monocyclique ou polycycliques substitué ou non substitué.

11. Composé selon la revendication 1, représenté par la formule chimique 4 ou 5 suivante : 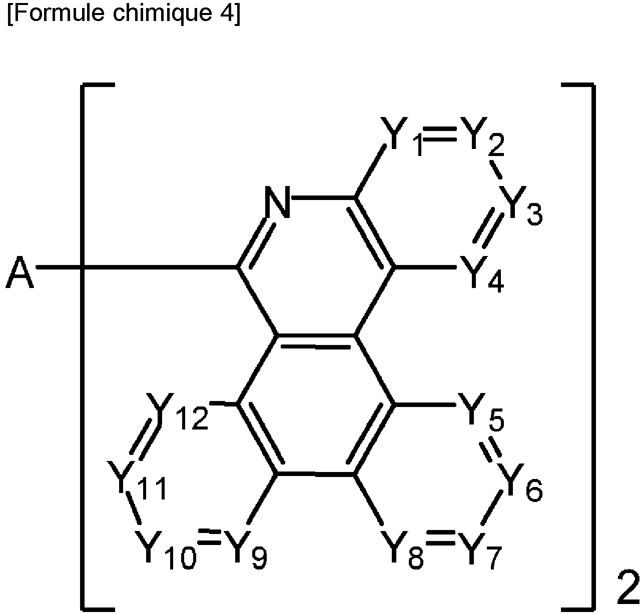 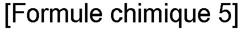 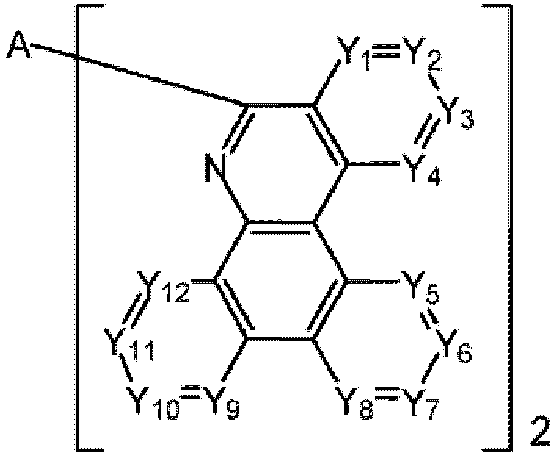 où dans les formules chimiques 4 et 5,
A est sélectionné dans le groupe constitué d'un arylène en C6 à C60 monocyclique ou polycyclique substitué ou non substitué, et d'un hétéroarylène en C2 à C60 monocyclique ou polycyclique substitué ou non substitué, et
Y1 à Y12 sont identiques à ceux définis dans la revendication 1.

12. Composé selon la revendication 1, le composé étant sélectionné parmi les composés suivants :

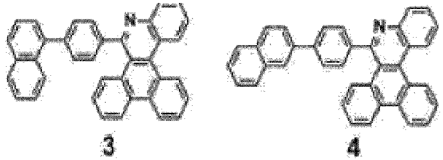

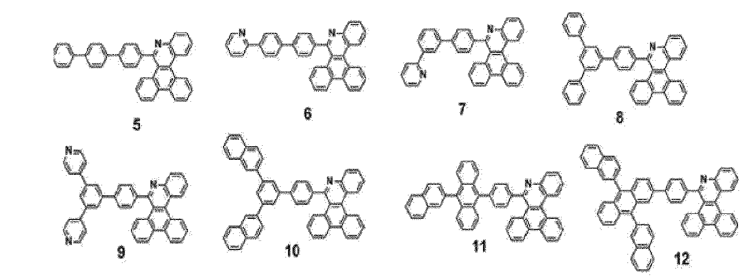

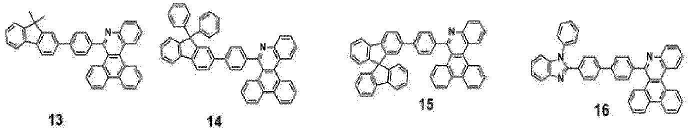

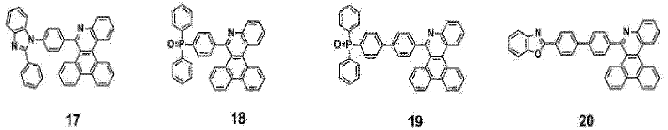

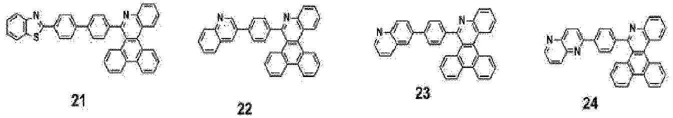

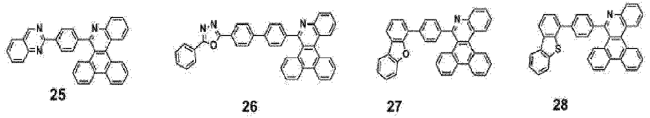

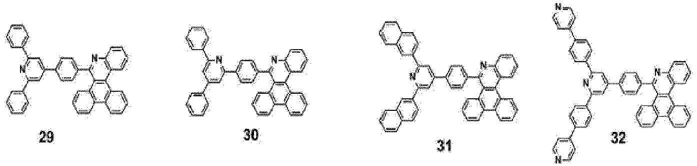

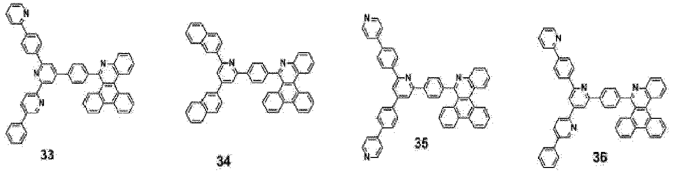

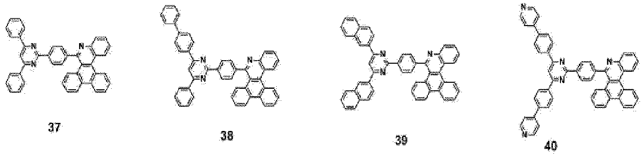

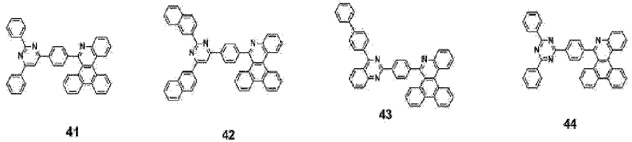

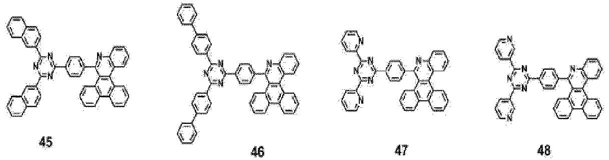

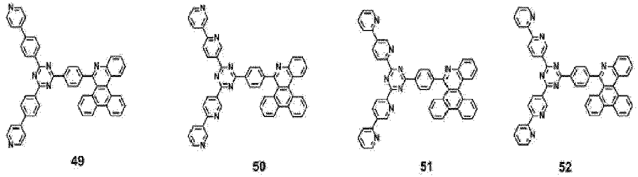

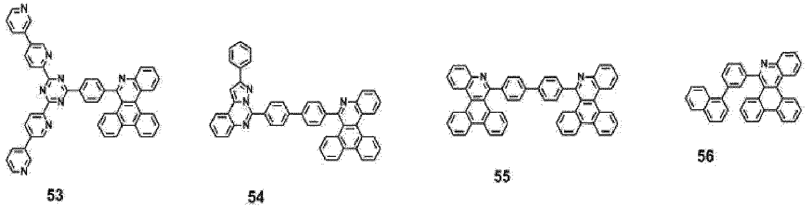

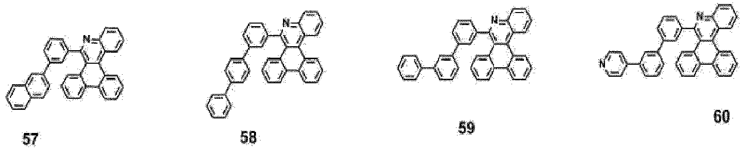

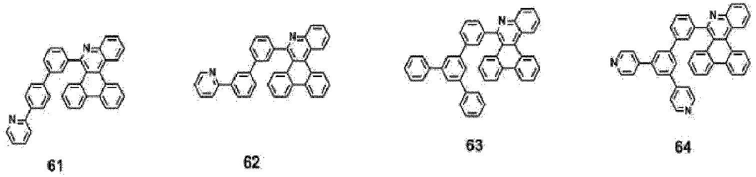

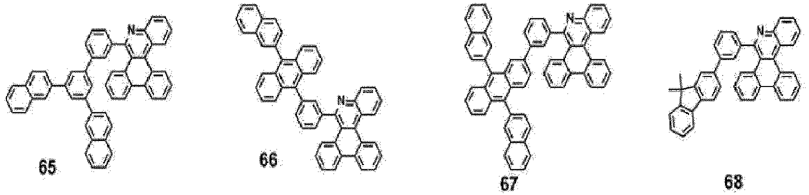

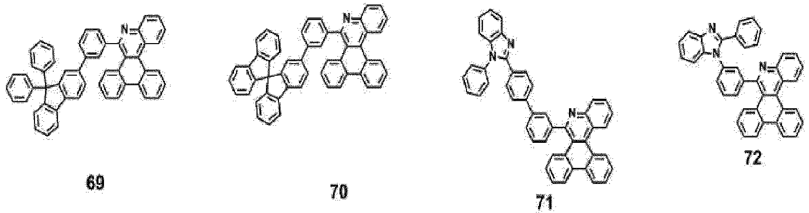

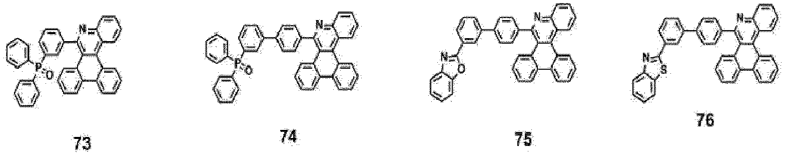

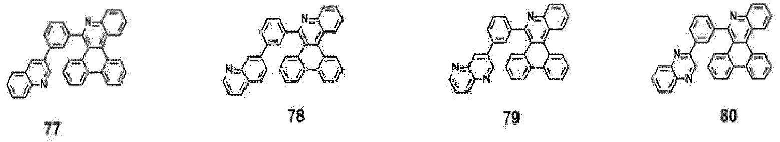

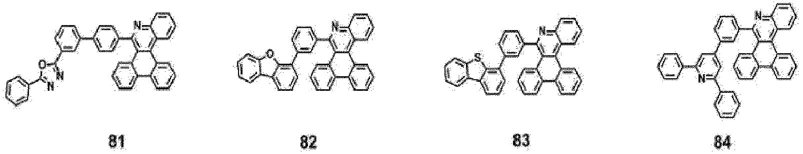

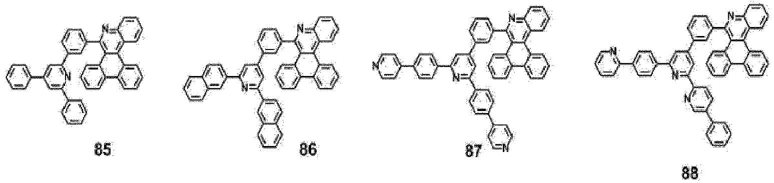

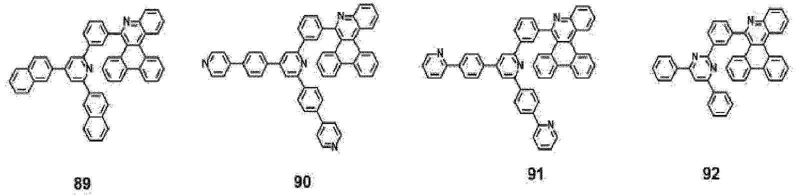

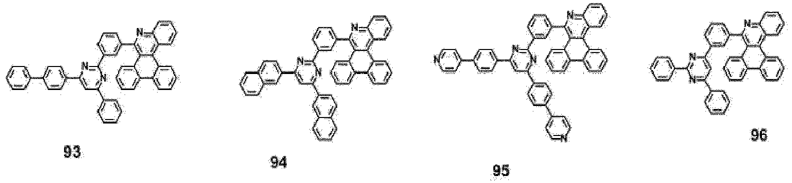

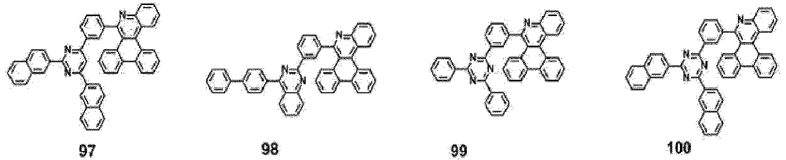

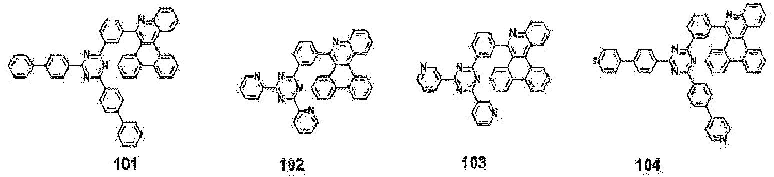

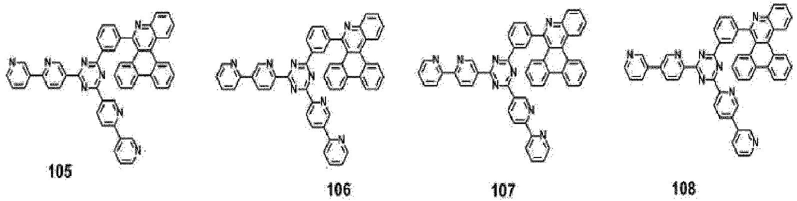

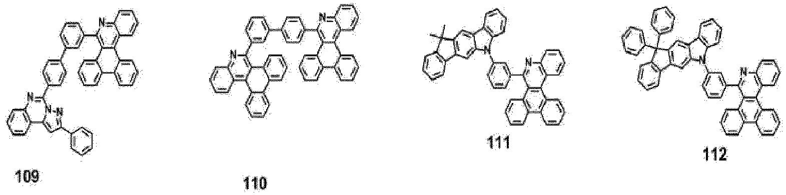

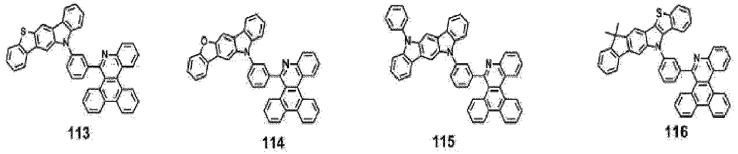

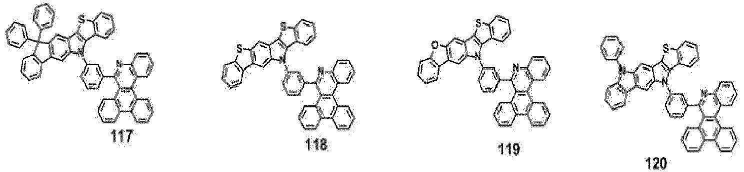

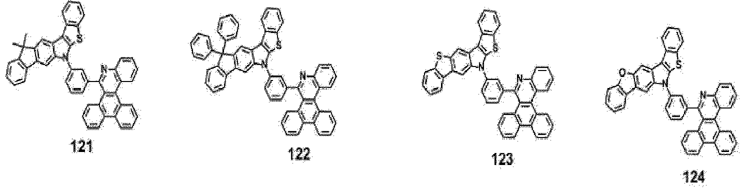

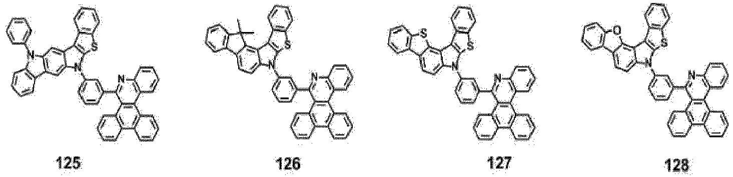

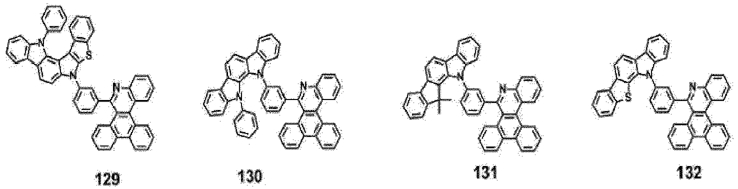

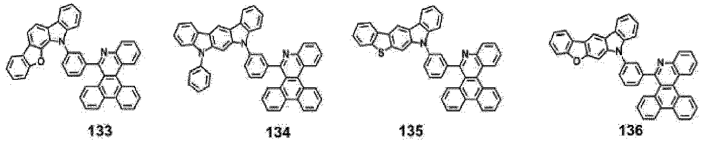

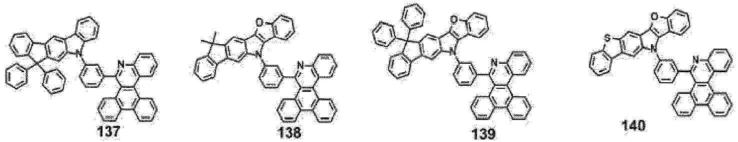

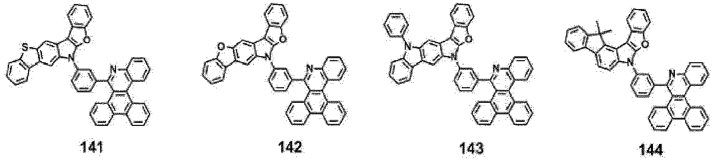

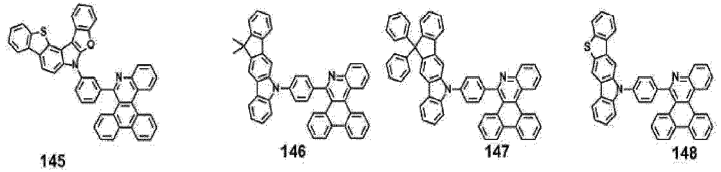

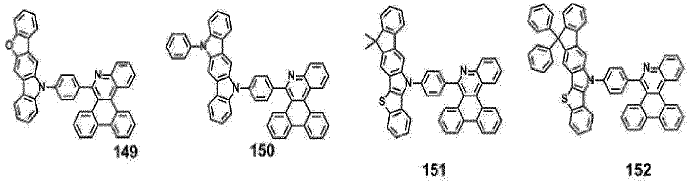

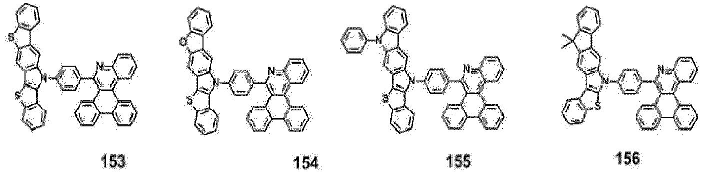

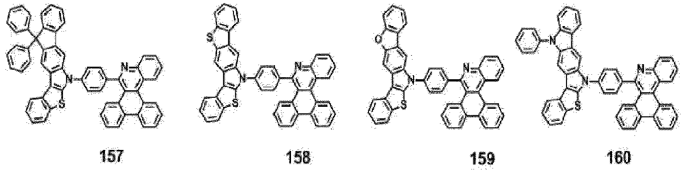

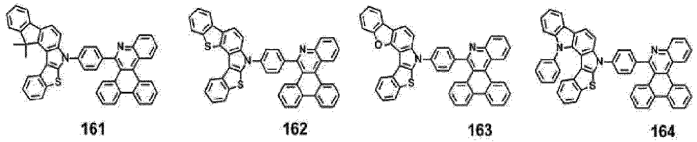

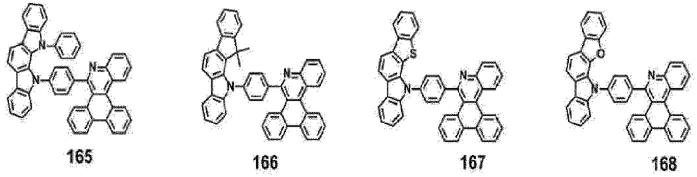

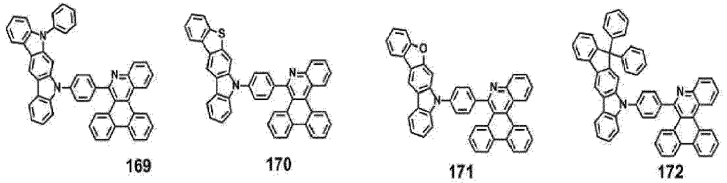

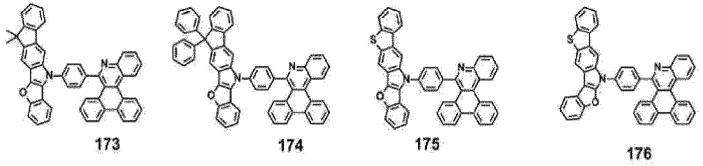

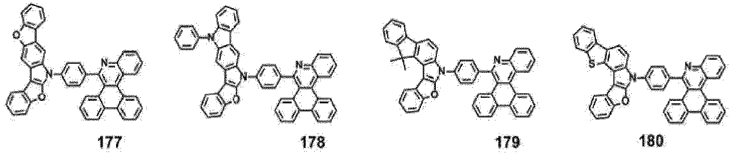

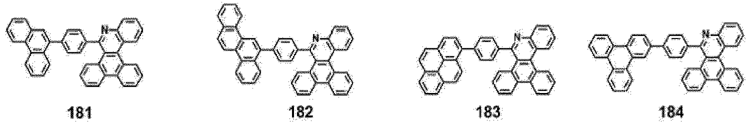

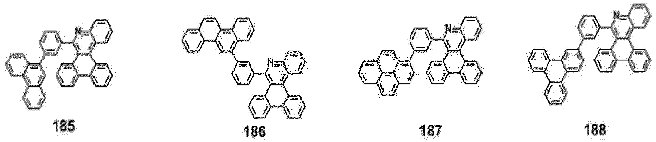

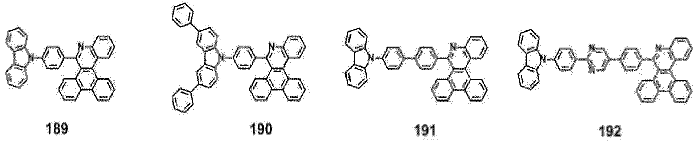

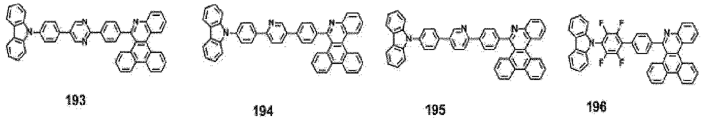

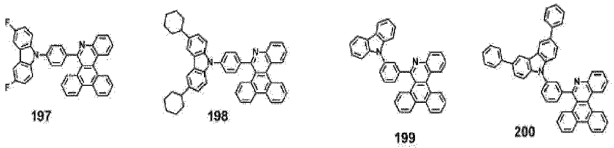

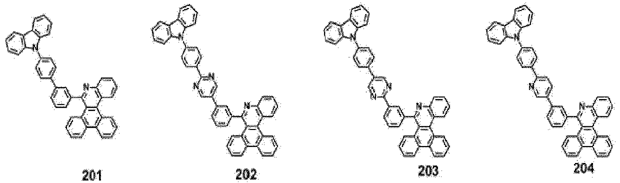

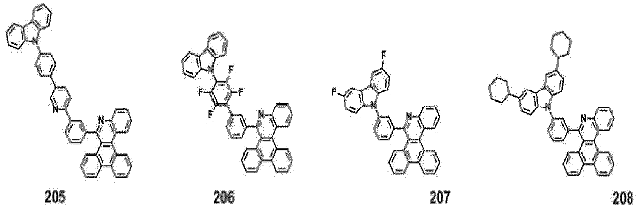

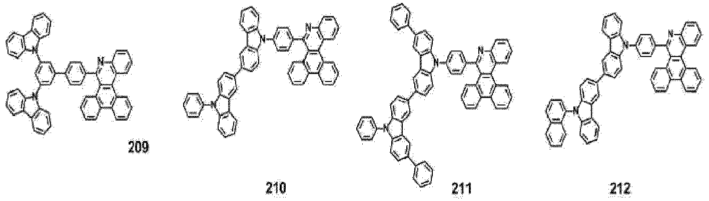

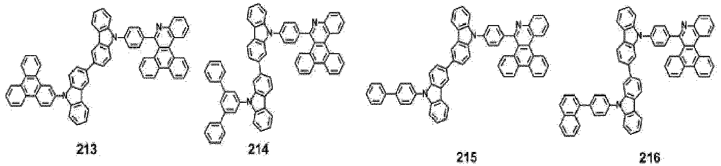

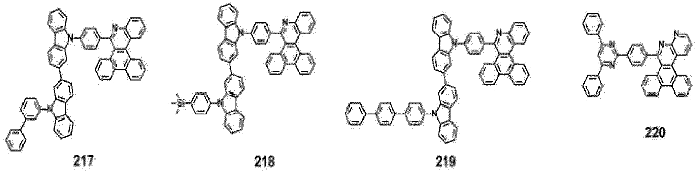

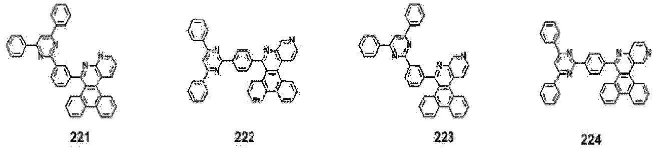

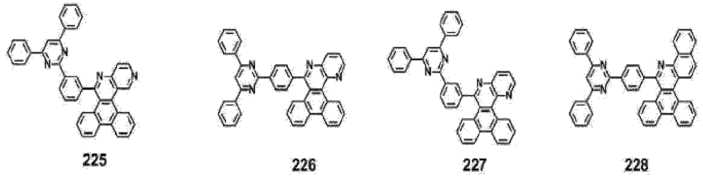

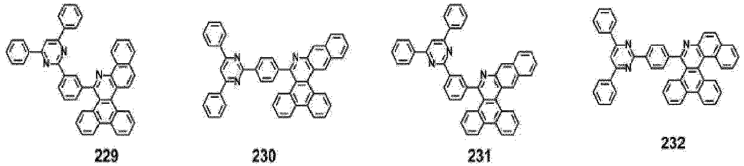

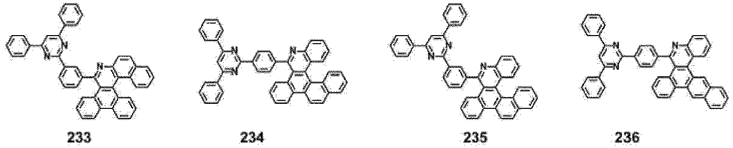

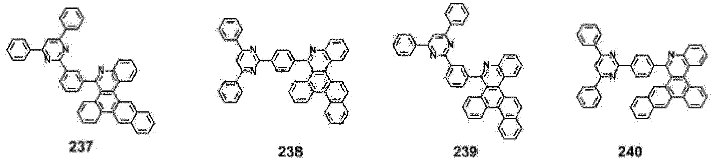

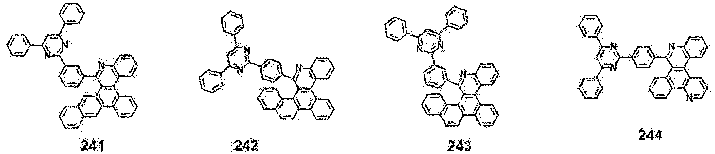

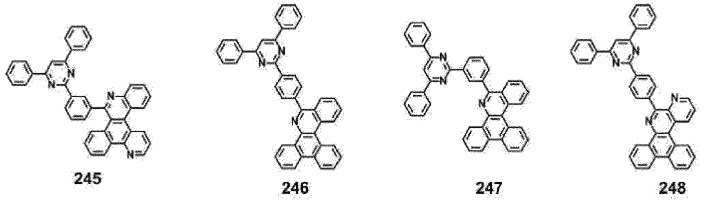

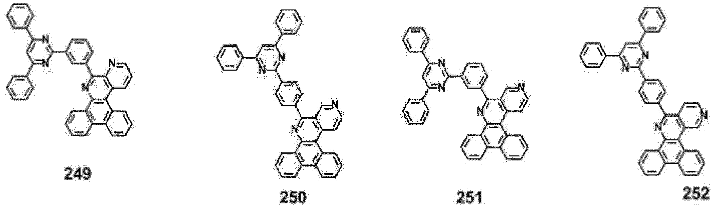

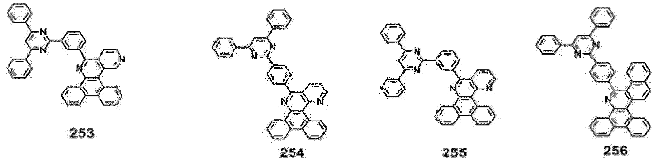

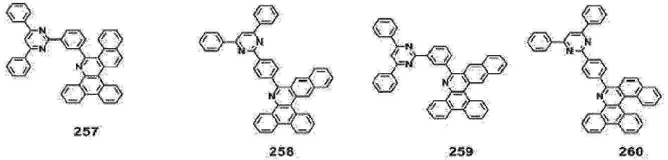

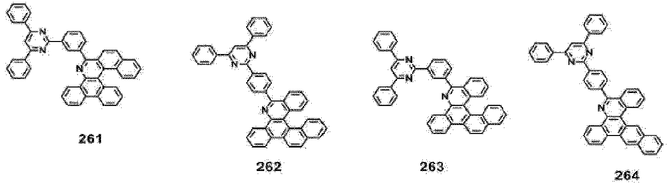

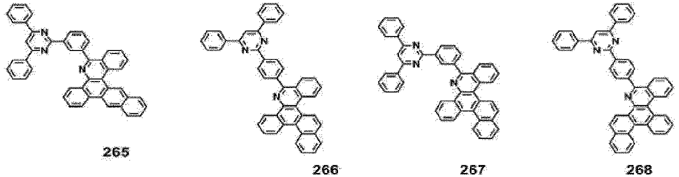

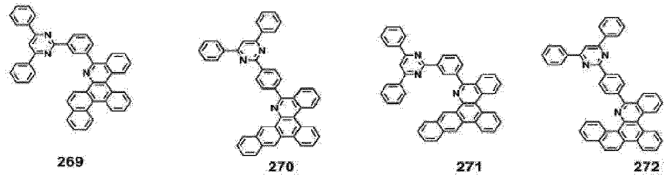

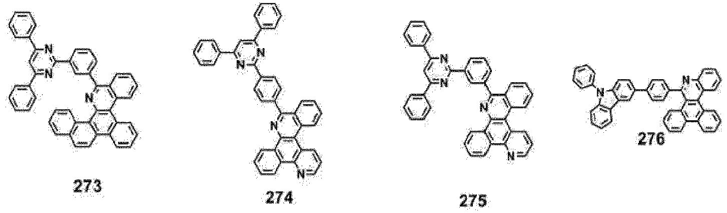

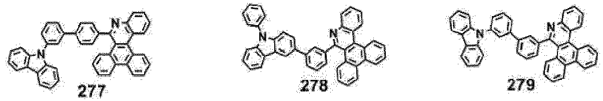

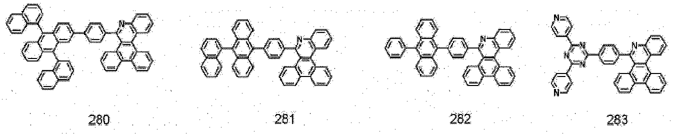

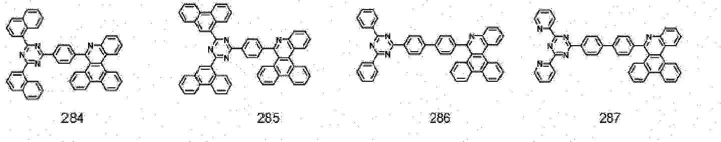

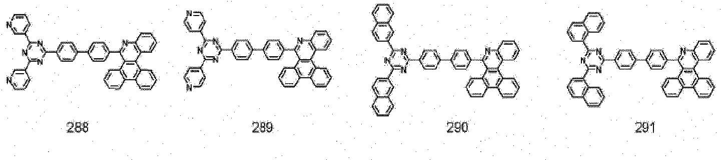

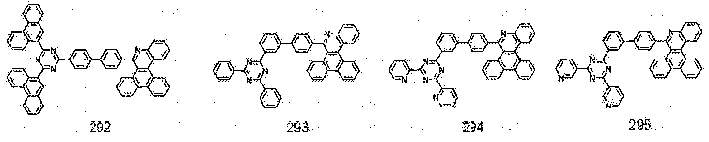

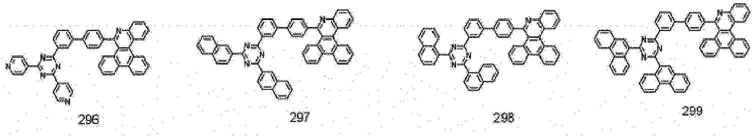

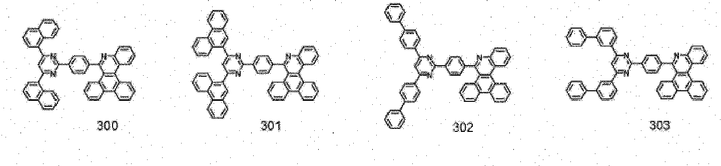

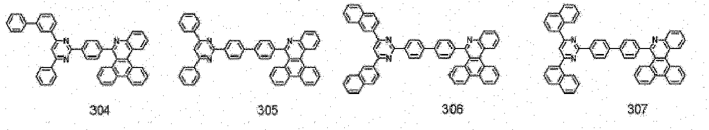

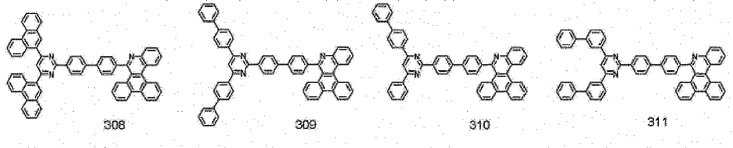

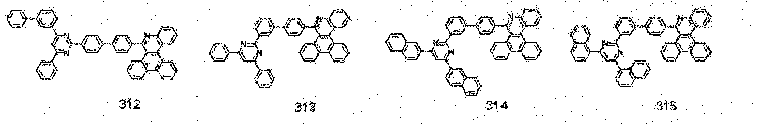

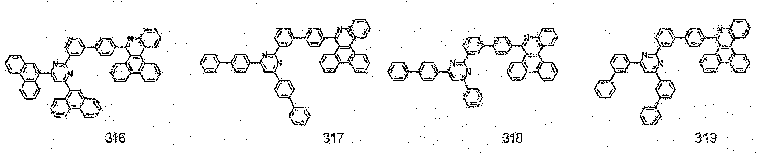

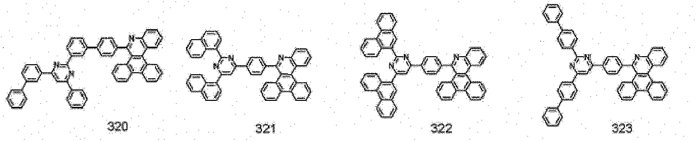

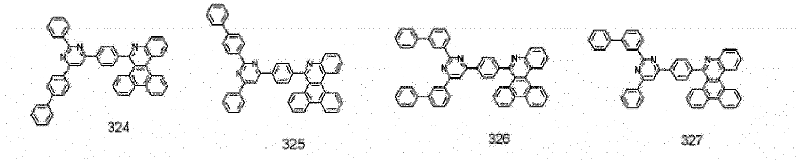

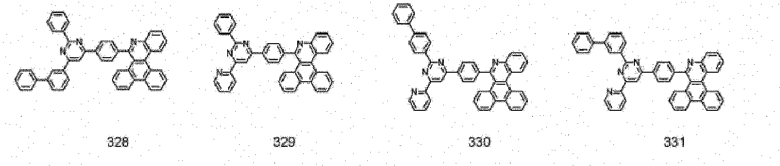

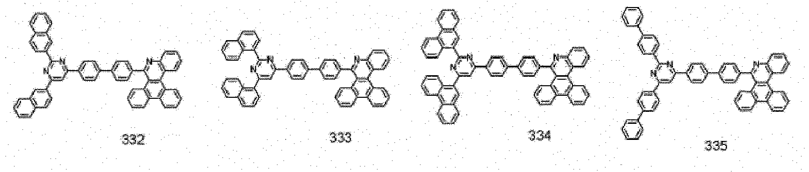

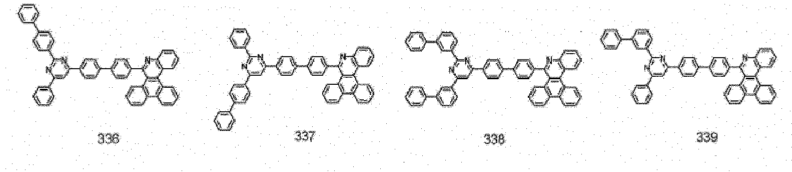

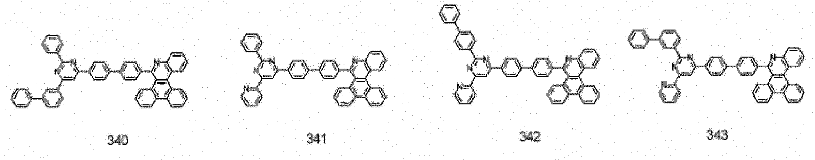

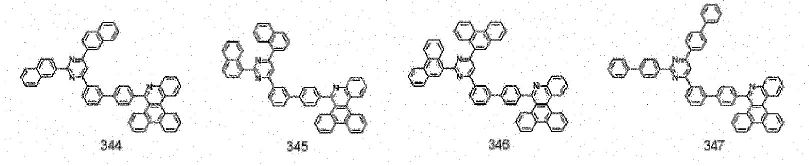

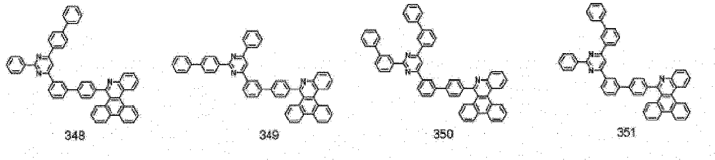

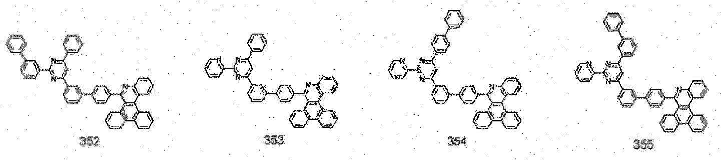

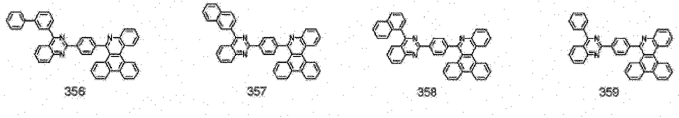

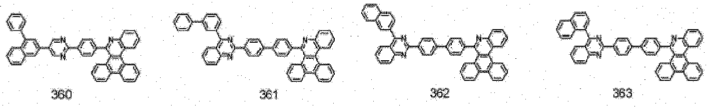

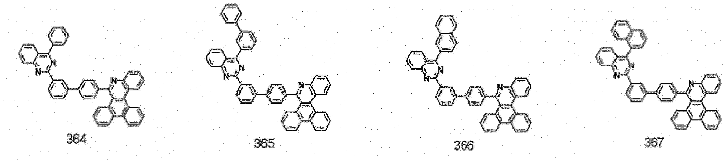

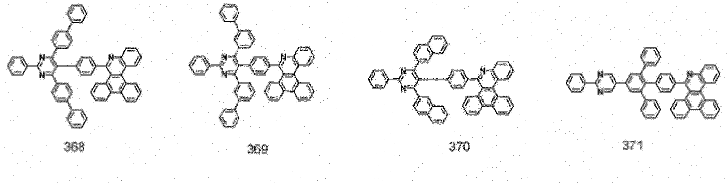

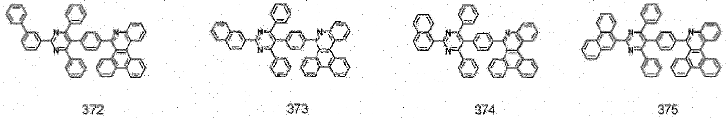

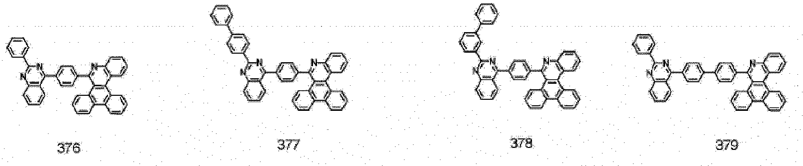

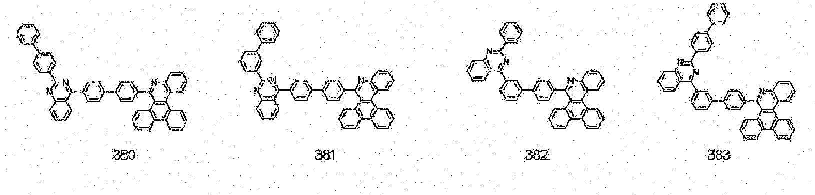

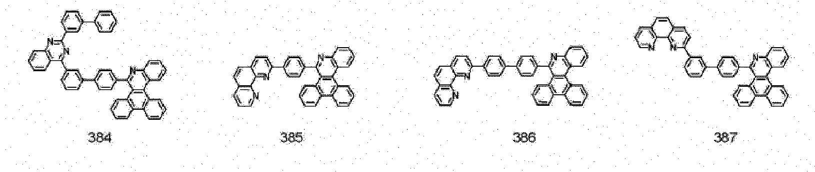

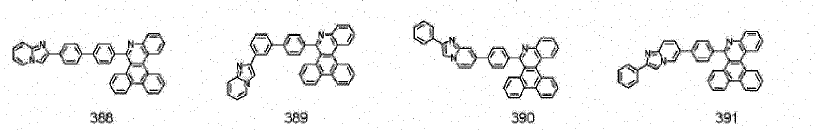

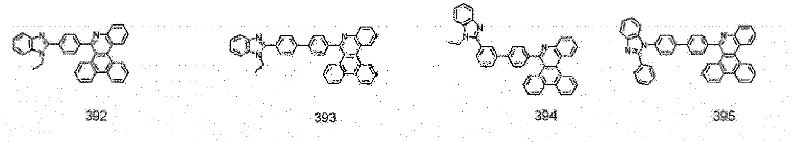

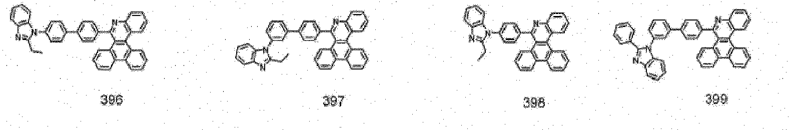

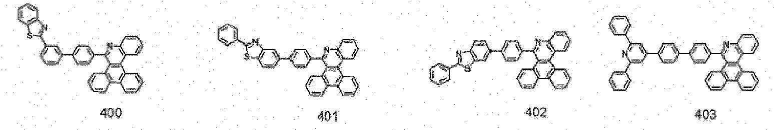

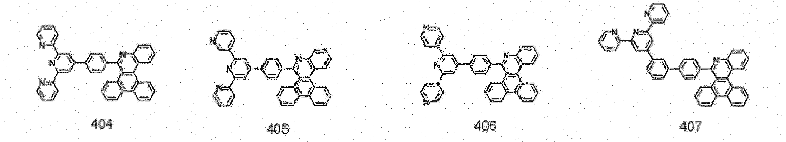

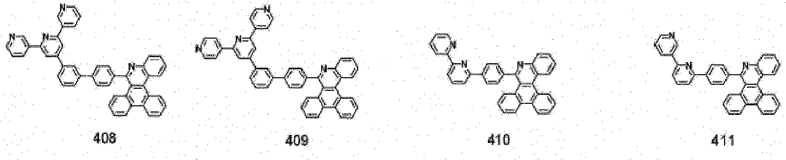

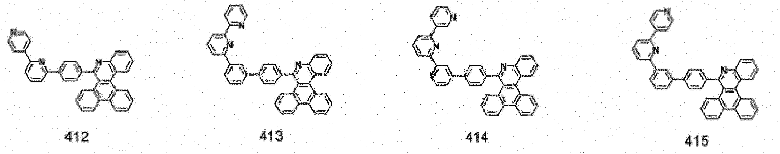

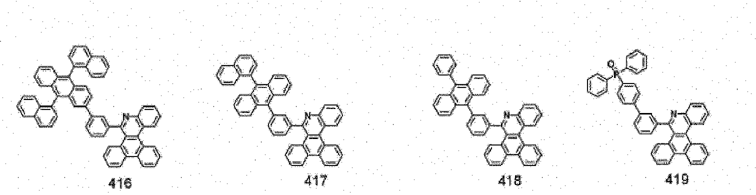

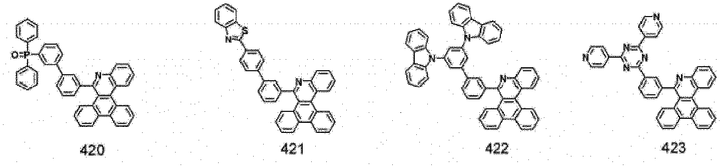

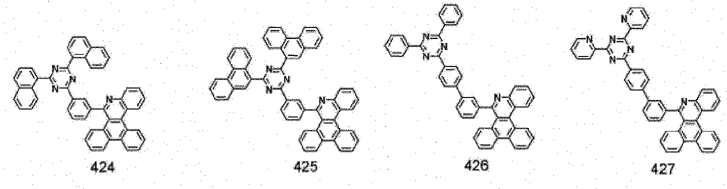

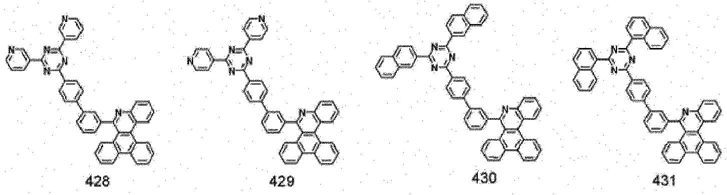

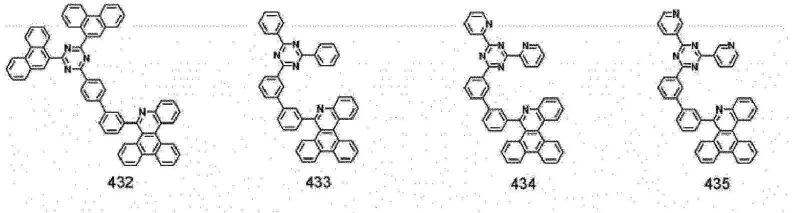

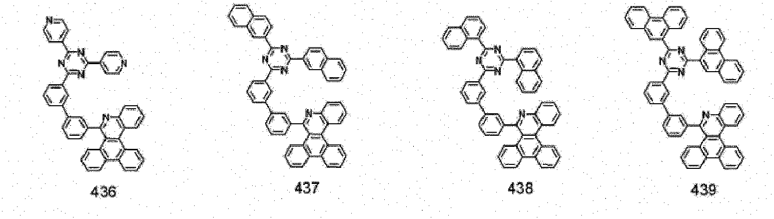

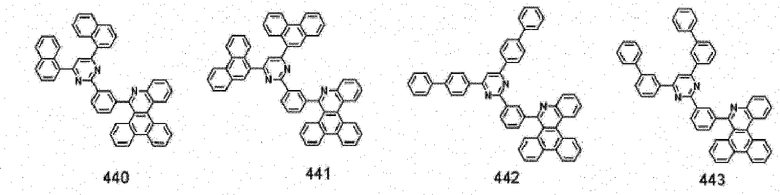

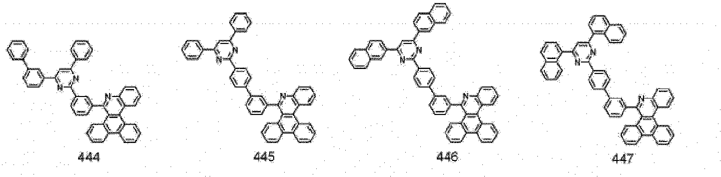

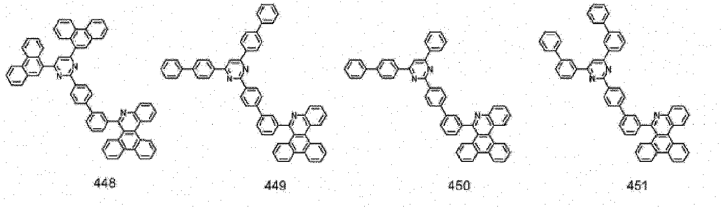

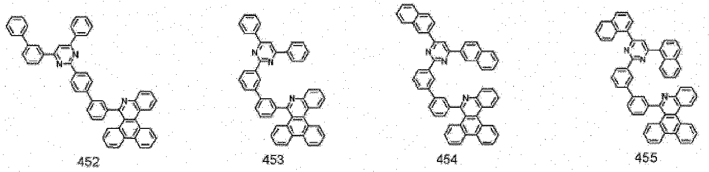

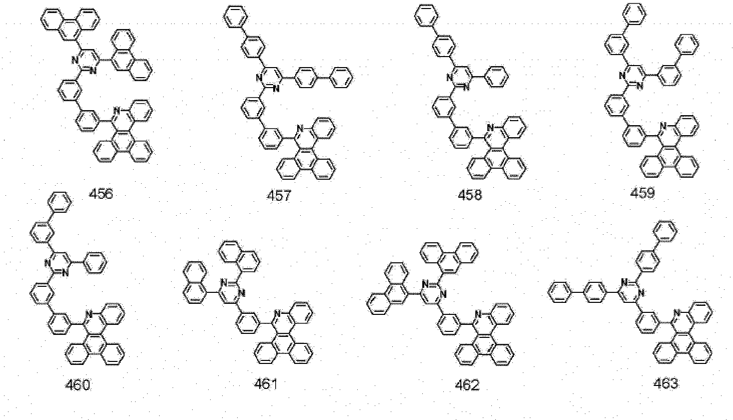

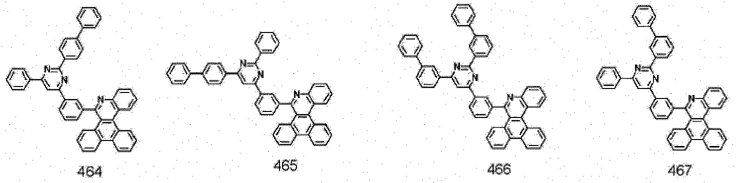

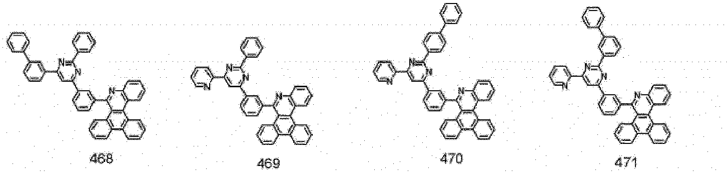

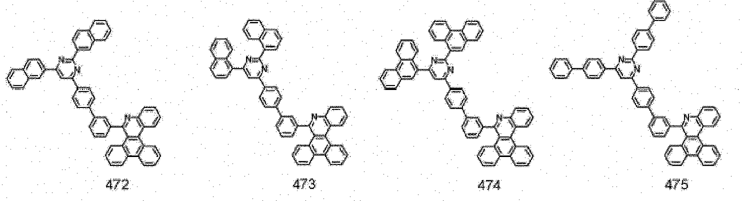

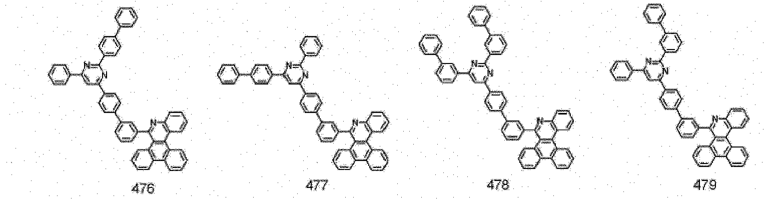

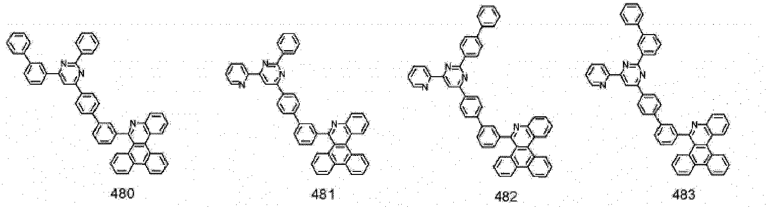

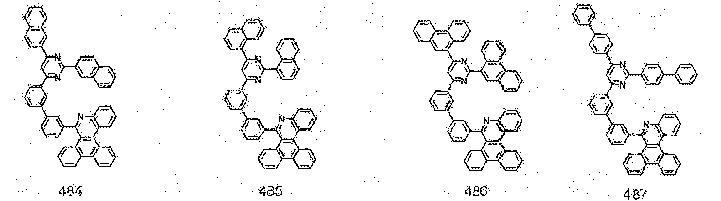

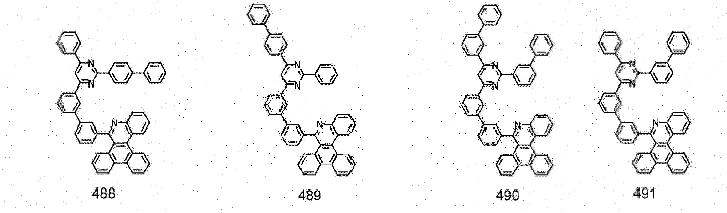

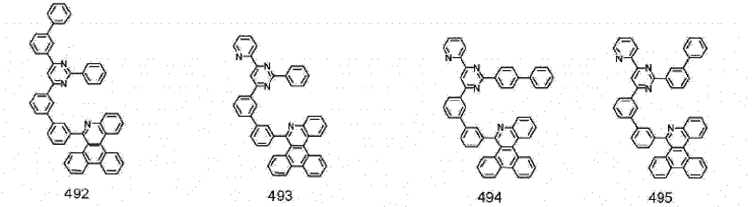

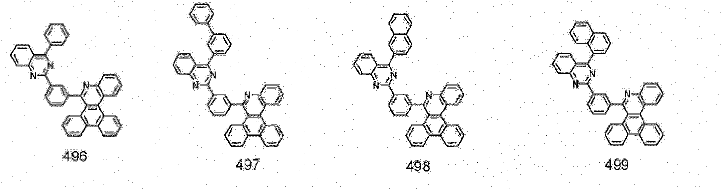

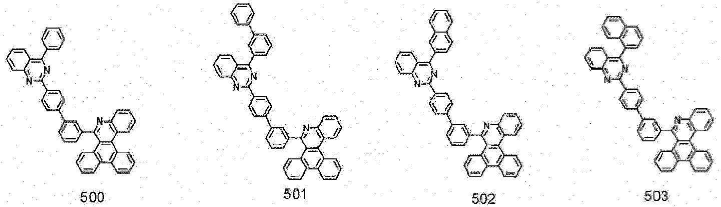

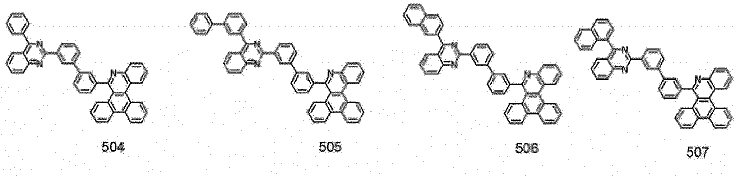

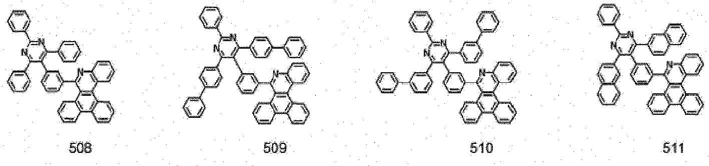

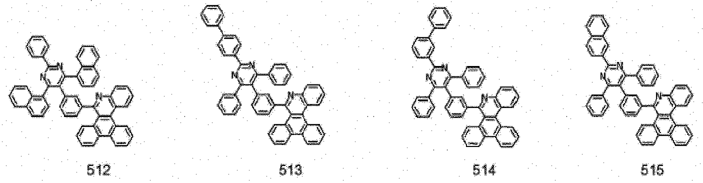

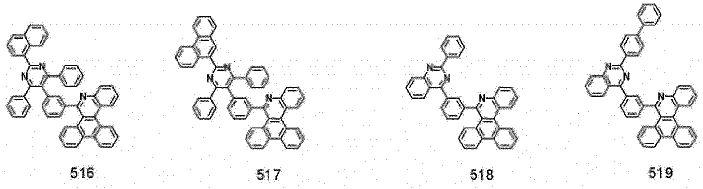

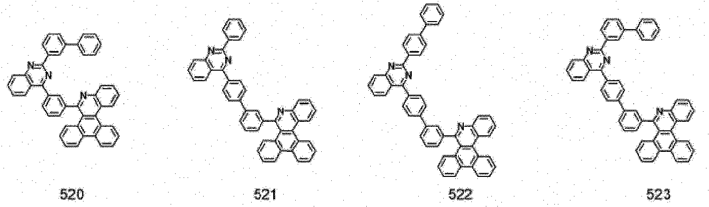

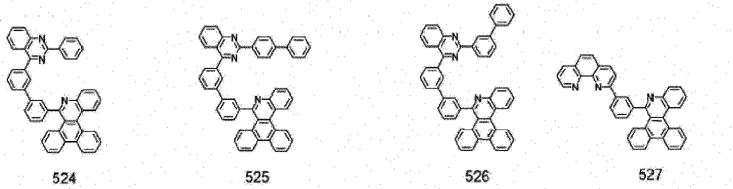

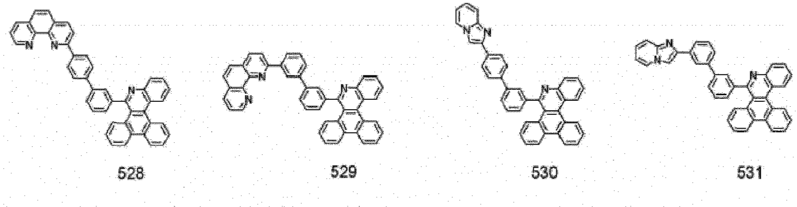

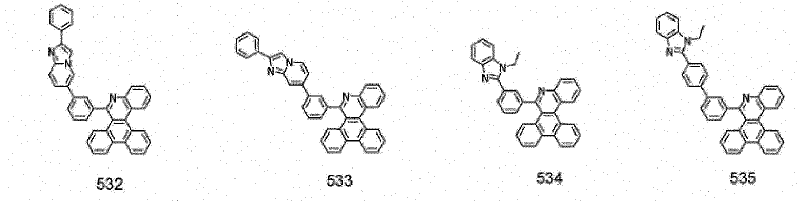

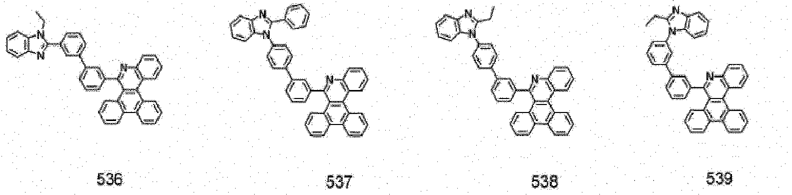

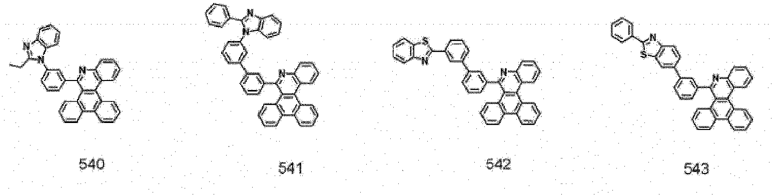

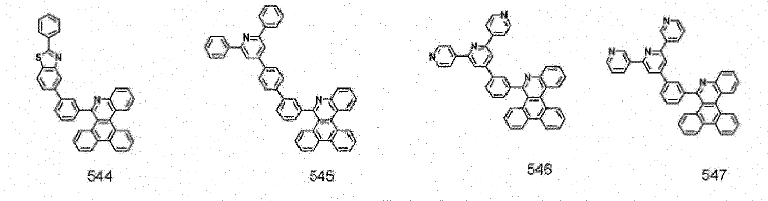

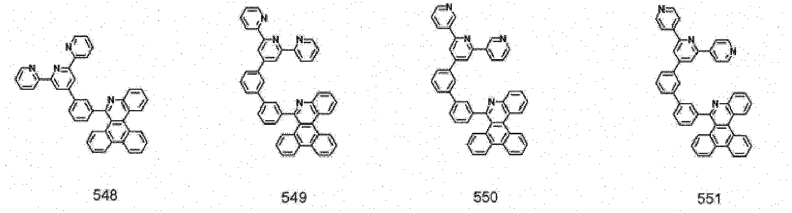

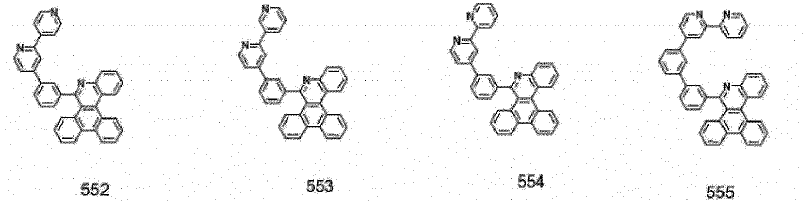

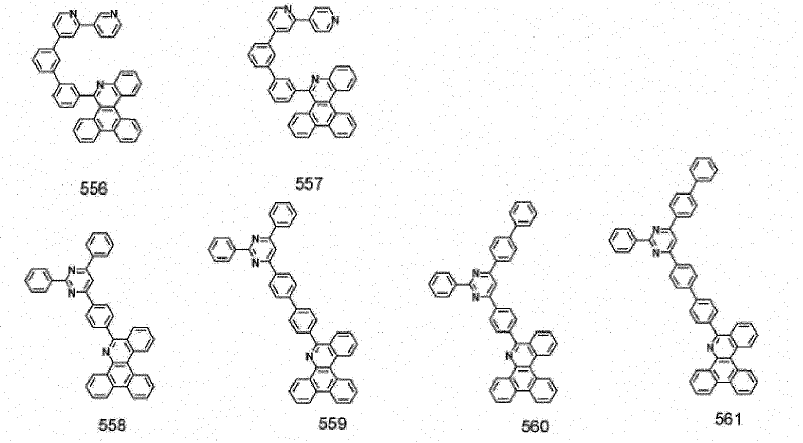

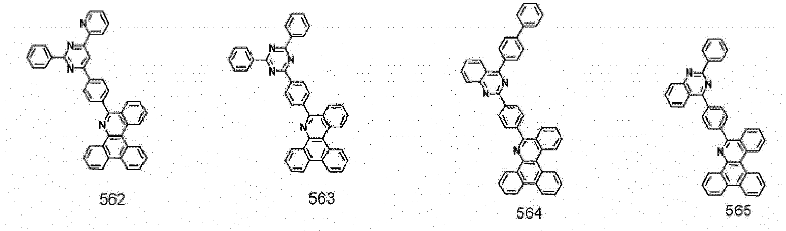

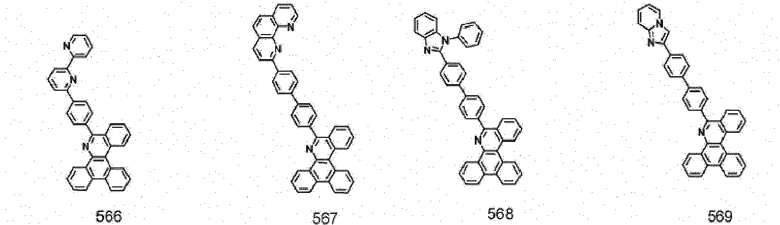

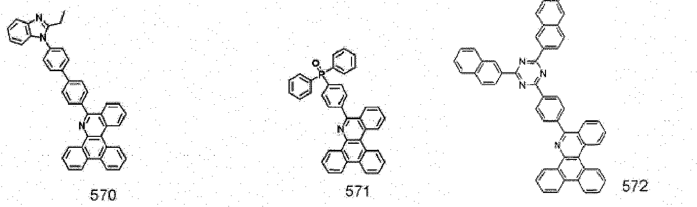

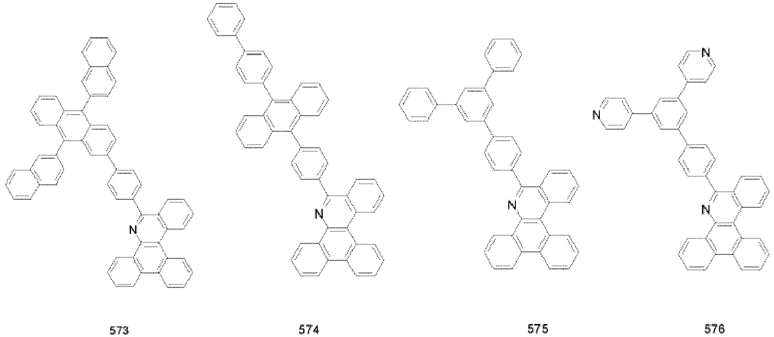

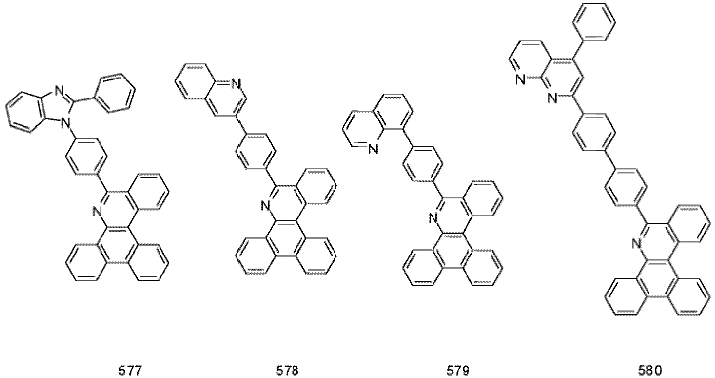

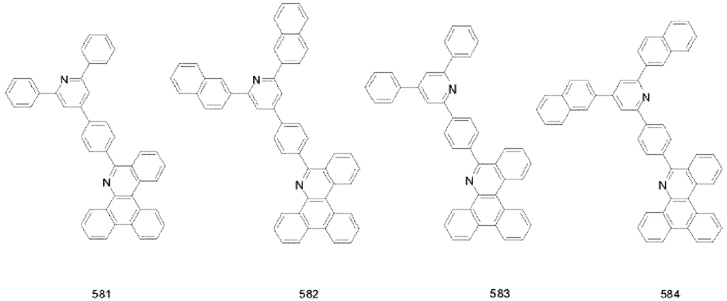

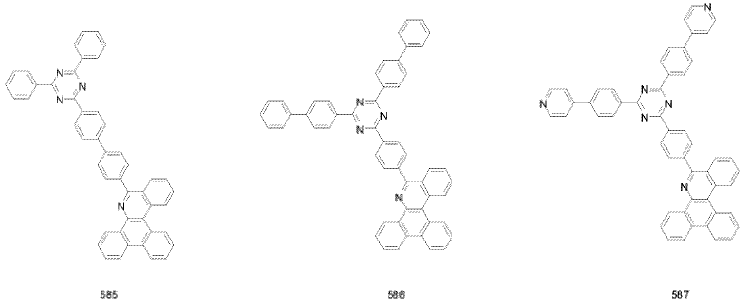

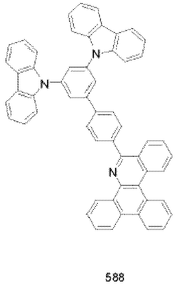

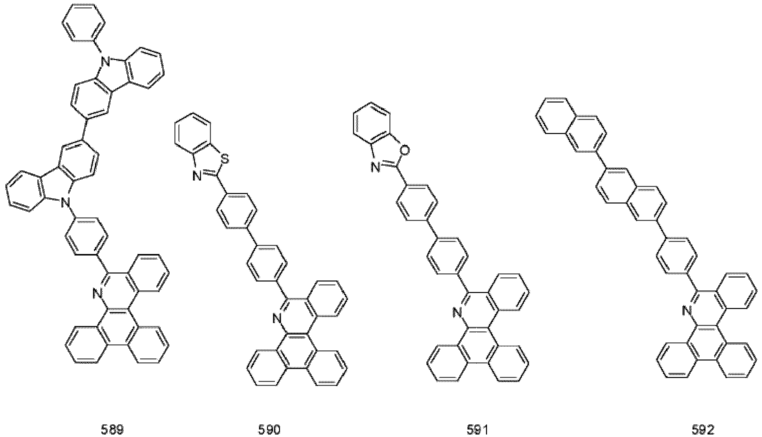

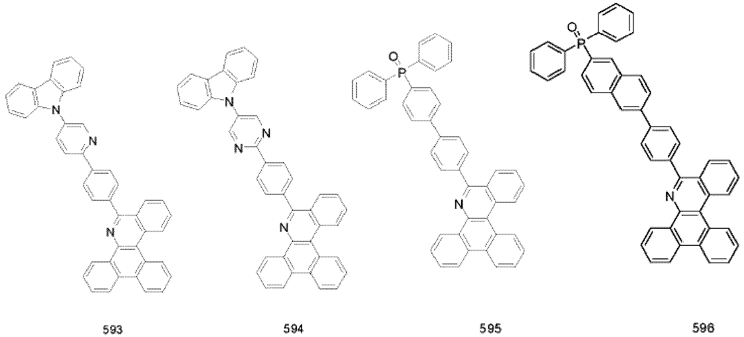

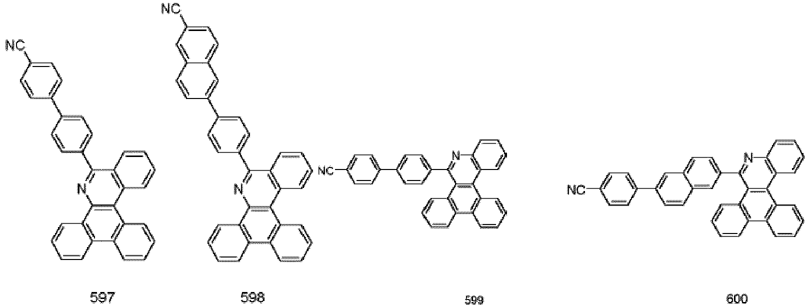

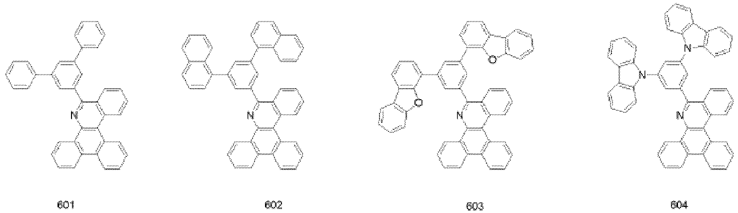

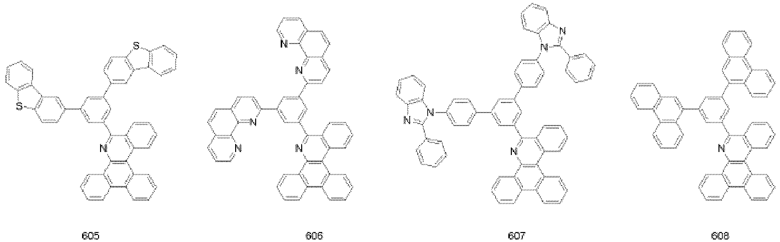

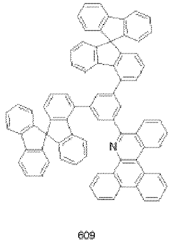

13. Dispositif électroluminescent organique, comprenant :
une anode
une cathode, et
une ou plusieurs couches de matériau organique prévues entre l'anode et la cathode, dans lequel une ou plusieurs couches des couches de matériau organique comprennent le composé selon l'une des revendications 1 à 12.

14. Dispositif électroluminescent organique selon la revendication 13, dans lequel une couche de matériau organique comprenant le composé selon l'une quelconque des revendications 1 à 12 est une ou plusieurs couches sélectionnées parmi une couche d'injection de trous, une couche de transport de trous, une couche électroluminescente, une couche de blocage de trous, une couche de transport d'électrons et une couche d'injection d'électrons.

15. Dispositif électroluminescent organique selon la revendication 13, dans lequel une couche de matériau organique comprenant le composé selon l'une quelconque des revendications 1 à 12 est une couche de transport d'électrons.

16. Dispositif électroluminescent organique selon la revendication 13, dans lequel une couche de matériau organique comprenant le composé selon l'une quelconque des revendications 1 à 12 est une couche électroluminescente.

17. Dispositif électroluminescent organique selon la revendication 13, dans lequel une couche de matériau organique comprenant le composé selon l'une quelconque des revendications 1 à 12 est une couche de blocage de trous.
